(19) Europäisches Patentamt
European Patent Office
Office européen des brevets

(11) EP 2 128 134 A1

(12) # EUROPEAN PATENT APPLICATION
published in accordance with Art. 153(4) EPC

(43) Date of publication:
**02.12.2009 Bulletin 2009/49**

(21) Application number: **07830926.7**

(22) Date of filing: **31.10.2007**

(51) Int Cl.:
*C07D 209/08* (2006.01) *A61K 31/4418* (2006.01)
*A61K 31/451* (2006.01) *A61K 31/495* (2006.01)
*A61K 31/496* (2006.01) *A61K 31/5377* (2006.01)
*A61K 31/551* (2006.01) *A61P 1/02* (2006.01)
*A61P 19/02* (2006.01) *A61P 27/02* (2006.01)
*A61P 29/00* (2006.01) *A61P 31/12* (2006.01)
*A61P 31/18* (2006.01) *A61P 35/00* (2006.01)
*A61P 35/04* (2006.01) *A61P 43/00* (2006.01)
*C07D 209/86* (2006.01) *C07D 211/96* (2006.01)
*C07D 213/81* (2006.01) *C07D 215/20* (2006.01)

(86) International application number:
**PCT/JP2007/071192**

(87) International publication number:
**WO 2008/053913 (08.05.2008 Gazette 2008/19)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **02.11.2006 JP 2006298795**
**20.03.2007 JP 2007072150**
**31.08.2007 JP 2007225075**

(71) Applicant: **Shionogi&Co., Ltd.**
**Osaka-shi, Osaka 5410045 (JP)**

(72) Inventors:
- **ENDOH, Takeshi**
  **Osaka-shi**
  **Osaka 5530002 (JP)**
- **FUJII, Yasuhiko**
  **Osaka-shi**
  **Osaka 5530002 (JP)**
- **KOJIMA, Eiichi**
  **Osaka-shi**
  **Osaka 5530002 (JP)**
- **TADANO, Genta**
  **Osaka-shi**
  **Osaka 5530002 (JP)**
- **YAMAGUCHI, Naoko**
  **Osaka-shi**
  **Osaka 5530002 (JP)**
- **ADACHI, Yo**
  **Osaka-shi**
  **Osaka 5530002 (JP)**
- **TAGASHIRA, Sachie**
  **Osaka-shi**
  **Osaka 5530002 (JP)**
- **TACHIBANA, Yuki**
  **Osaka-shi**
  **Osaka 5530002 (JP)**
- **ONODERA, Naohiro**
  **Osaka-shi**
  **Osaka 5530002 (JP)**

(74) Representative: **HOFFMANN EITLE**
**Patent- und Rechtsanwälte**
**Arabellastrasse 4**
**81925 München (DE)**

(54) **SULFONYLUREA DERIVATIVE CAPABLE OF SELECTIVELY INHIBITING MMP-13**

(57) A compound represented by the general formula (I):

[Chemical formula 1]

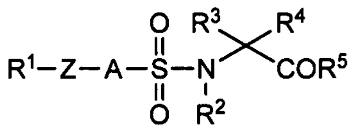

(I)

EP 2 128 134 A1

wherein $R^1$ is

[Chemical formula 2]

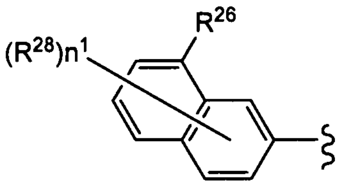

,

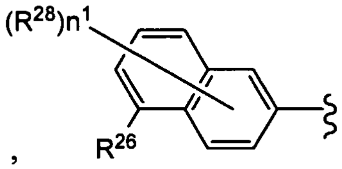

,

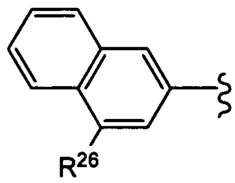

or

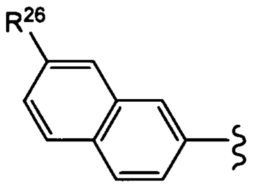

wherein $R^{26}$ and $R^{28}$ are each independently lower alkyloxy and the like; $n^1$ is an integer of 0 to 3; Z is a optionally substituted C1-C5 alkynylene which may be interrupted with a substituent selected from Substituent group a and the like; A is a group represented by the formula:

[Chemical formula 3]

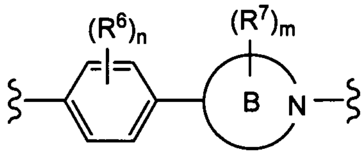

wherein $R^6$ and $R^7$ are each independently lower alkyl and the like; m and n are each independently 0, 1, or 2; $R^2$ is a hydrogen atom and the like; $R^3$ is optionally substituted lower alkyl and the like; $R^4$ is a hydrogen atom; or $R^3$ and $R^4$ may be taken together with an adjacent carbon atom to from a ring; $R^5$ is hydroxyl and the like, or an optically active isomer, a pharmaceutically acceptable salt or a solvate thereof.

## Description

[Technical field]

The present invention relates to sulfonylurea derivatives which selectively inhibit MMP-13.

[Background technique]

An extracellular matrix is composed of collagen, fibronectin, laminine, proteoglycan and the like, and has a role of tissue supporting function, proliferation, differentiation and adhesion of cells, and the like. Degradation of an extracellular matrix is associated with metalloprotease which is a protease containing a metal ion in an active center, particularly, matrix metalloprotease (MMP). As for the MMP family, many members, from MMP-1 (I type collagenase) to MMP-28, were reported and they act on growth, tissue metabolism and the like under the original physiological conditions. However, it was reported that the progress were correlated with the increase in expression (activity) of the above enzymes in various pathological states which were accompanied by the destruction of tissues and fibrosis such as osteoarthritis, articular rheumatism, corneal ulcer, periodontic disease, metastasis or infiltration of tumor, and virus infectious disease (HIV infectious disease).
The sulfonamide derivatives having MMP inhibiting activities were described in Patent Literature 1, Patent Literature 2, Patent Literature 3 and Non-Patent Literature 1.
The sulfonamide derivatives having MMP-12 inhibiting activity, and having a naphthyl group as a substituent were described in Patent Literature 4.
The thiazoles or oxazolesulfonamide derivatives having a naphtyl group as a substituent were described in Patent Literature 5.
The thiazoles or oxazolesulfonamide derivatives having a naphtyl group as a substituent were described in Patent Literature 6.
The sulfonamide derivatives having aglycanase inhibiting activity, and having a naphtyl group as a substituent were described in Patent Literature 7.
The sulfonamide derivatives having aggrecanase inhibiting activity and MMP-13 inhibiting activity were described in Patent Literature 8.
The sulfonamide derivatives having aggrecanase inhibiting activity and MMP inhibiting activities were described in Patent Literature 9.
The sulfonamide derivatives having TNF-$\alpha$ inhibiting activity and the MMP inhibiting activities were described in Patent Literature 10.

[Patent Literature 1] International Publication WO 97/27174
[Patent Litarature 2] International Publication WO 99/04780
[Patent Litarature 3] International Publication WO 00/15213
[Patent Litarature 4] International Publication WO 01/83431
[Patent Litarature 5 International Publication WO 02/28844
[Patent Litarature 6] International Publication WO 01/83461
[Patent Litarature 7] International Publication WO 03/080042
[Patent Litarature 8] Japanese Patent Application Laid-Open (JP-A) No.2000-319250
[Patent Litarature 9] International Publication WO 05/061459
[Patent Litarature 10] International Publication WO 98/32748
[Patent Litarature 11] International Publication WO 2007/102392
[Non-Patent Literature 1] Yoshinori Tamura et al., J. Med. Chem., 1998, vol.41, No.4, p.640-649

[Disclosure of the invention]

[Problems to be solved by the invention]

It seems that the inhibition of the activity of MMP-13 will contribute to improvement or prevention of progession of pathological states, particularly, osteoarthritis (OA), resulting from or associated with the MMP-13 activity. Therefore, the development of MMP-13 inhibitors is anticipated.

[Means to solve the problems]

In view of the aforementioned respects, the present inventors intensively continued to study. The inventors

found that some novel sulfonamide derivatives exhibit the MMP-13 selective inhibiting activity and completed the invention as follows.

The present invention relates to: 1') a compound represented by the general formula (I):

[Chemical formula 1]

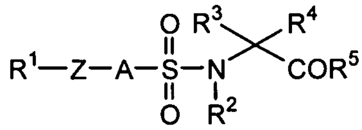

(I)

wherein $R^1$ is

a) a group represented by the formula:

[Chemical formula 2]

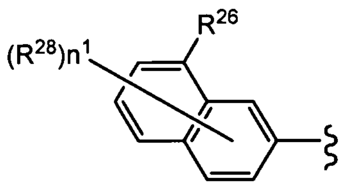

,

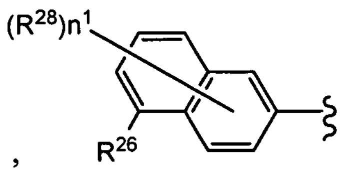

,

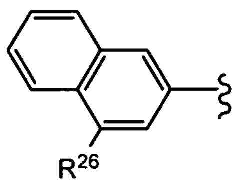

or

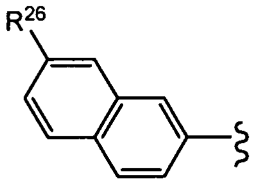

wherein $R^{26}$ and $R^{28}$ are each independently a halogen, optionally substituted lower alkyl, optionally substituted lower alkenyl, optionally substituted lower alkyloxy, lower alkylthio, aralkylthio, optionally substituted amino, carboxy, hydroxy, cyano, lower alkyloxycarbonyl, optionally substituted aminocarbonyl, optionally substituted aminoxalyl, acyl, lower alkylsulfonyl, lower alkylsulfinyl, aralkylsulfonyl, aralkylsulfinyl, optionally substituted aminosulfonyl, optionally substituted heteroaryl or an optionally substituted non-aromatic heterocyclic group;

$n^1$ is an integer of 0 to 3; and

$R^{28}$ may be substituted on all substitutable atoms on the ring;

b) a group represented by the formula:

[Chemical formula 3]

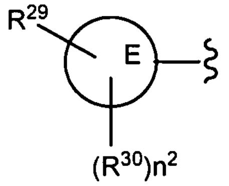

wherein E ring is benzene, pyridine, 2-pyridone, thiazole, tetrahydrothienopyrimidine or oxazole;

$R^{29}$ is a halogen, acyl, optionally substituted lower alkyl, optionally substituted lower alkyloxy, optionally substituted lower alkenyl, optionally substituted aryl, optionally substituted heteroaryl, an optionally substituted non-aromatic

carbocyclic group, an optionally substituted non-aromatic heterocyclic group, optionally substituted aminocarbonyl, optionally substituted amino or optionally substituted hydrazino;
$R^{30}$ is each independently halogen, hydroxy, nitro, optionally substituted lower alkyl, optionally substituted lower alkenyl, optionally substituted lower alkyloxy, optionally substituted aryloxy, optionally substituted aralkyl, optionally substituted heteroarylalkyl, lower alkylthio, aralkylthio, optionally substituted amino, carboxy, cyano, lower alkyloxycarbonyl, optionally substituted aminocarbonyl, optionally substituted aminoxalyl, acyl, lower alkylsulfonyl, lower alkylsulfinyl, aralkylsulfonyl, aralkylsulfinyl, optionally substituted aminosulfonyl, optionally substituted aryl, optionally substituted heteroaryl or an optionally substituted non-aromatic heterocyclic group;
$n^2$ is an integer of 0 to 2; and
$R^{29}$ and $R^{30}$ may be substituted on all substitutable atoms on the ring; or
c) a group represented by the formula:

[Chemical formula 4]

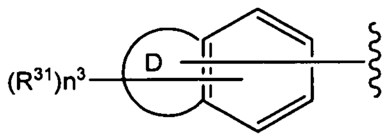

wherein D ring is a non-aromatic carbocyclic ring, a non-aromatic heterocyclic ring or an aromatic heterocyclic ring;
$R^{31}$ is each independently halogen, optionally substituted lower alkyl, optionally substituted lower alkenyl, optionally substituted lower alkyloxy, lower alkylthio, aralkylthio, optionally substituted amino, carboxy, cyano, lower alkyloxycarbonyl, lower alkyloxy lower alkylcarbonyl, optionally substituted aminocarbonyl, optionally substituted aminoxalyl, acyl, lower alkylsulfonyl, lower alkylsulfinyl, aralkylsulfonyl, aralkylsulfinyl, optionally substituted aminosulfonyl, oxo, thioxo, imino, hydroxy, hydroxyimino, optionally substituted lower alkyloxyimino, optionally substituted heteroaryl, optionally substituted aralkyl, optionally substituted heteroarylalkyl, optionally substituted aralkyloxy, optionally substituted aryl or an optionally substituted non-aromatic heterocyclic group;
all substitutable atoms of the benzene ring and all substitutable atoms of D ring may be bonded to "Z";
$n^3$ is an integer of 0 to 4;
$R^{31}$ may be substituted on all substitutable atoms of the benzene ring and all substitutable atoms of D ring;
provided that when $n^3$ is an integer of 0, D ring is not unsubstituted indole;
Z is -O-, -S-, -SO-, $-SO_2-$, $-N(R^{10})-$, $N(R^{10})-C(=O)-$, $-C(=O)-N(R^{10})-$, - $N(R^{10})-SO_2-$, $-SO_2-N(R^{10})-$, -C(=O)-, -O-C(=O)-, -C(=O)-O-, $N(R^{10})-C(=O)-N(R^{10})-$, $-N(R^{10})-C(=S)-N(R^{10})-$, $-O-N=C(R^8)-$, $-C(R^8)=N-O-$, $-O-C(=O)-N(R^{10})-$, $-N(R^{10})-C(=O)-O-$, -C(=O)-C(=O)-, an optionally substituted non-aromatic carbocyclic group, an optionally substituted non-aromatic heterocyclic group, optionally substituted C1-C5 alkylene which may be intervened with a substituent selected from Substituent_group a, or C2-C5 optionally substituted alkenylene which may be intervened with a substituent selected from Substituent group a, or optionally substituted C2-C5 alkynylene which may be intervened with a substituent selected from Substituent group a, wherein the Substituent group a consists of -O-, -S-, -SO-, $-SO_2-$, $-N(R^{10})-$, $N(R^{10})-C(=O)-$, $-C(=O)-N(R^{10})-$, $-N(R^{10})-SO_2-$, $-SO_2-N(R^{10})-$, -C(=O)-, -O-C(=O)-, -C(=O)-O-, $-N(R^{10})-C(=O)-N(R^{10})-$, $-N(R^{10})-C(=S)-N(R^{10})-$, $-ON=C(R^8)-$, $-C(R^8)=N-O-$, $-O-C(=O)-N(R^{10})-$, $-N(R^{10})-C(=O)-O-$, and - C(=O)-C(=O)-;
$R^8$ and $R^{10}$ are each independently a hydrogen atom or lower alkyl;
A is a group represented by the formula;

[Chemical formula 5]

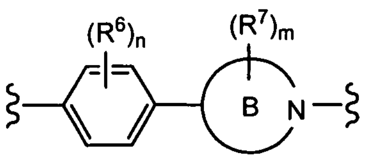

or

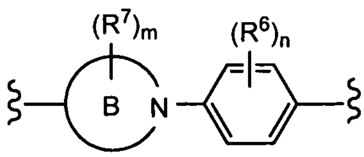

wherein $R^6$ and $R^7$ are each independently halogen, lower alkyl, cycloalkyl, lower alkenyl, lower alkynyl, lower alkyloxy, lower alkenyloxy, lower alkylthio, halo lower alkyl, halo lower alkyloxy, halo lower alkylthio, hydroxy, hydroxy lower alkyl, mercapto, carboxy, lower alkyloxycarbonyl, lower alkylsulfonyl, acyl, acyloxy, nitro, cyano, optionally substituted amino or optionally substituted aminocarbonyl; B ring is a nitrogen-containing heterocyclic ring which is optionally contained a further nitrogen atom, an oxygen atom, and/or a sulfur atom in the ring;
m and n are each independently an integer of 0 to 3; $R^2$ is a hydrogen atom, optionally substituted lower alkyl, optionally substituted aralkyl, optionally substituted heteroarylalkyl, optionally substituted aryl, or optionally substituted heteroaryl; $R^3$ is a hydrogen atom, optionally substituted lower alkyl, optionally substituted aralkyl, optionally substituted heteroarylalkyl, optionally substituted aryl, or optionally substituted heteroaryl; $R^4$ is a hydrogen atom; or $R^3$ and $R^4$ may be taken together with an adjacent carbon atom to form a 5- to 6-membered non-aromatic carbocyclic ring or a 5- to 6-membered non-aromatic heterocyclic ring;
$R^5$ is hydroxy, lower alkyloxy, or -NHOH;
an optically active isomer, a pharmaceutically acceptable salt or a solvate thereof.

More particularly, the present invention relates to the following 1) to 26).

1) a compound represented by the general formula (I):

[Chemical formula 6]

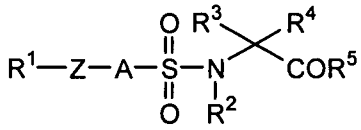

(I)

wherein $R^1$ is

a) a group represented by the formula:

[Chemical formula 7]

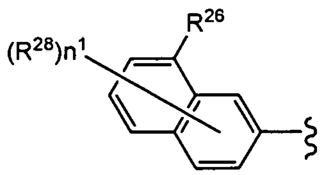

,

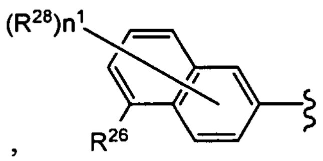

,

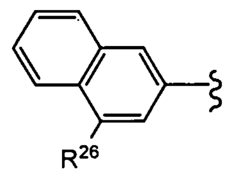

or

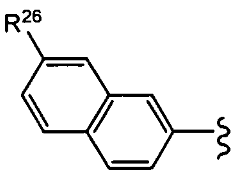

wherein $R^{26}$ and $R^{28}$ are each independently a halogen, optionally substituted lower alkyl, optionally substituted lower alkenyl, optionally substituted lower alkyloxy, lower alkylthio, aralkylthio, optionally substituted amino, carboxy, cyano, lower alkyloxycarbonyl, optionally substituted aminocarbonyl, optionally substituted aminoxalyl, acyl, lower alkylsulfonyl, lower alkylsulfinyl, aralkylsulfonyl, aralkylsulfinyl, optionally substituted aminosulfonyl, optionally substituted heteroaryl or an optionally substituted non-aromatic heterocyclic group;
$n^1$ is an integer of 0 to 3; and
$R^{28}$ may be substituted on all substitutable atoms on the ring;
b) a group represented by the formula:

[Chemical formula 8]

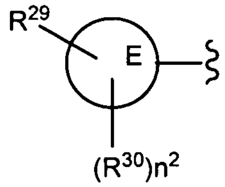

wherein E ring is benzene, pyridine or oxazole;
$R^{29}$ is an optionally substituted lower alkenyl, optionally substituted aryl, optionally substituted heteroaryl, an optionally substituted non-aromatic carbocyclic group, an optionally substituted non-aromatic heterocyclic group, optionally substituted aminocarbonyl or optionally substituted amino;
$R^{30}$ is halogen, optionally substituted lower alkyl, optionally substituted lower alkenyl, optionally substituted lower alkyloxy, lower alkylthio, aralkylthio, optionally substituted amino, carboxy, cyano, lower alkyloxycarbonyl, optionally substituted aminocarbonyl, optionally substituted aminoxalyl, acyl, lower alkylsulfonyl, lower alkylsulfinyl, aralkylsulfonyl, aralkylsulfinyl, optionally substituted aminosulfonyl, optionally substituted heteroaryl or an optionally substituted non-aromatic heterocyclic group;
$n^2$ is an integer of 0 to 2; and
$R^{29}$ and $R^{30}$ may be substituted on all substitutable atoms on the ring;
or
c) a group represented by the formula:

[Chemical formula 9]

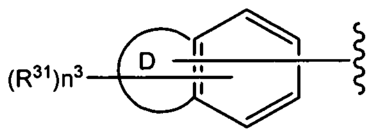

wherein D ring is a non-aromatic carbocyclic ring, a non-aromatic heterocyclic ring or an aromatic heterocyclic ring;
$R^{31}$ is halogen, optionally substituted lower alkyl, optionally substituted lower alkenyl, optionally substituted lower alkyloxy, lower alkylthio, aralkylthio, optionally substituted amino, carboxy, cyano, lower alkyloxycarbonyl, optionally substituted aminocarbonyl, optionally substituted aminoxalyl, acyl, lower alkylsulfonyl, lower alkylsulfinyl, aralkylsulfonyl, aralkylsulfinyl, optionally substituted aminosulfonyl, oxo, imino, hydroxyimino, optionally substituted lower alkyloxyimino, optionally substituted heteroaryl or an optionally substituted non-aromatic heterocyclic group;
$n^3$ is an integer of 0 to 2; and
all substitutable atoms of the benzene ring and all substitutable atoms of D ring may be bonded to "Z";
$R^{31}$ may be substituted on all substitutable atoms of the benzene ring; provided that when $n^3$ is an integer of 0, D ring is not unsubstituted indole;
Z is -O-, -S-, -SO-, $-SO_2-$, $-N(R^{10})-$, $-N(R^{10})-C(=O)-$, $-C(=O)-N(R^{10})-$, - $N(R^{10})-SO_2$ -, $-SO_2-N(R^{10})-$, -C(=O)-, -O-C(=O)-, -C(=O)-O-, $-N(R^{10})-C(=O)-N(R^{10})-$,- $N(R^{10})-C(=S)-N(R^{10})-$, $-O-N=C(R^8)-$, $-C(R^8)=N-O-$, $-O-C(=O)-N(R^{10})-$, $-N(R^{10})-C(=O)-O-$, -C(=O)-C(=O)-, an optionally substituted non-aromatic carbocyclic group, an optionally substituted non-aromatic heterocyclic group, optionally substituted C1-C5 alkylene which may be intervened a substituent selected from Substituent group a, or optionally substituted C2-C5 alkenylene which may be intervened with a substituent selected from Substituent group a, or optionally substituted C2-C5 alkynylene which may be intervened with a substituent selected from Substituent group a, wherein the Substituent group a consists of -O-, -S-, -SO-, $-SO_2-$, $-N(R^{10})-$, $-N(R^{10})-C(=O)-$, $-C(=O)-N(R^{10})-$, $-N(R^{10})-SO_2-$, $-SO_2-N(R^{10})-$, -C(=O)-, -O-C(=O)-, -C(=O)-O-, $-N(R^{10})-C(=O)-N(R^{10})-$, $-N(R^{10})-C(=S)-N(R^{10})-$, $-ON=C(R^8)-$, $-C(R^8)=N-O-$, $-O-C(=O)-N(R^{10})-$, $-N(R^{10})-C(=O)-O-$, and - C(=O)-C(=O)-;
$R^8$ and $R^{10}$ are each independently a hydrogen atom or lower alkyl;
A is a group represented by the formula;

[Chemical formula 10]

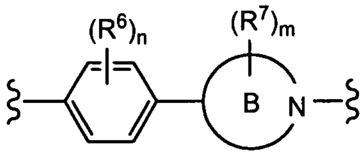

or

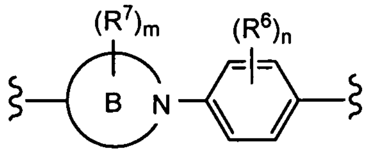

wherein $R^6$ and $R^7$ are each independently halogen, lower alkyl, cycloalkyl, lower alkenyl, lower alkynyl, lower alkyloxy, lower alkenyloxy, lower alkylthio, halo lower alkyl, halo lower alkyloxy, halo lower alkylthio, hydroxy, hydroxy lower alkyl, mercapto, carboxy, lower alkyloxycarbonyl, lower alkylsulfonyl, acyl, acyloxy, nitro, cyano, optionally substituted amino, or optionally substituted aminocarbonyl; B ring is a nitrogen-containing heterocyclic ring which is optionally contained a further nitrogen atom, an oxygen atom, and/or a sulfur atom in the ring;
m and n are each independently an integer of 0 to 3; $R^2$ is a hydrogen atom, optionally substituted lower alkyl, optionally substituted aralkyl, optionally substituted heteroarylalkyl, optionally substituted aryl, or optionally substituted heteroaryl;
$R^3$ is a hydrogen atom, optionally substituted lower alkyl, optionally substituted aralkyl, optionally substituted heteroarylalkyl, optionally substituted aryl, or optionally substituted heteroaryl;
$R^4$ is a hydrogen atom; or
$R^3$ and $R^4$ may be taken together with an adjacent carbon atom to form a 5- to 6-membered non-aromatic carbocyclic group or a 5- to 6-membered non-aromatic heterocyclic group;
$R^5$ is hydroxy, lower alkyloxy, or -NHOH;
an optically active isomer, a pharmaceutically acceptable salt or a solvate thereof.

2) The compound according to 1') or 1), wherein $R^1$ is a group represented by the formula:

[Chemical formula 11]

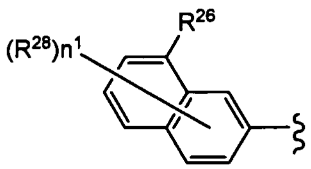

,

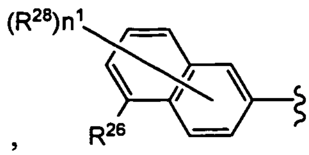

,

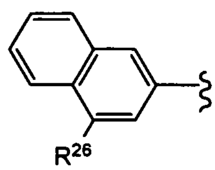

or

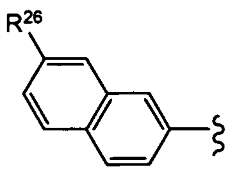

wherein $R^{26}$, $R^{28}$, and $n^1$ are as defined in above 1), an optically active isomer, a pharmaceutically acceptable salt or a solvate thereof.
3) The compound according to any one of 1'), 1) or 2), wherein $R^1$ is a group represented by the formula:

[Chemical formula 12]

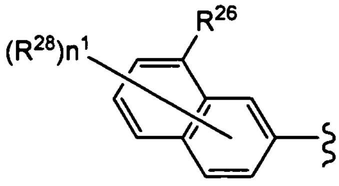

or

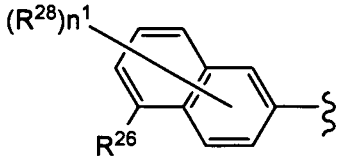

wherein $R^{26}$, $R^{28}$, and $n^1$ are as defined in above 1);, an optically active isomer, a pharmaceutically acceptable salt or a solvate thereof.

4) The compound according to any one of above 1'), or 1) to 3), wherein $R^1$ is a group represented by the formula:

[Chemical formula 13]

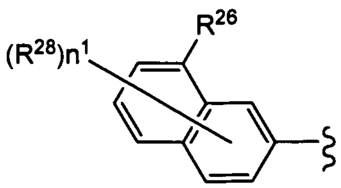

wherein $R^{26}$, $R^{28}$, and $n^1$ are as defined in above 1), an optically active isomer, a pharmaceutically acceptable salt or a solvate thereof.

5) The compound according to any one of 1') or 1) to 3), wherein $R^1$ is a group represented by the formula:

[Chemical formula 14]

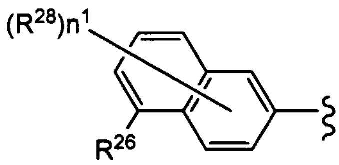

wherein $R^{26}$, $R^{28}$, and $n^1$ are as defined in above 1), an optically active isomer, a pharmaceutically acceptable salt or a solvate thereof.

6) The compound according to 1') or 1), wherein $R^1$ is a group represented by the formula:

[Chemical formula 15]

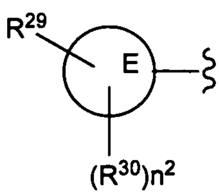

wherein E ring, $R^{29}$, $R^{30}$, and $n^2$ are as defined in above 1), an optically active isomer, a pharmaceutically acceptable salt or a solvate thereof.

7) The compound according to 1') or 1), wherein $R^1$ is a group represented by the formula:

[Chemical formula 16]

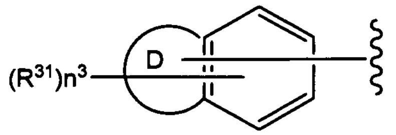

wherein D ring, $R^{31}$ and $n^2$ are as defined in above 1), an optically active isomer, a pharmaceutically acceptable salt or a solvate thereof.

8) The compound according to any one of 1') or 1) to 7), which is represented by the general formula (II):

[Chemical formula 17]

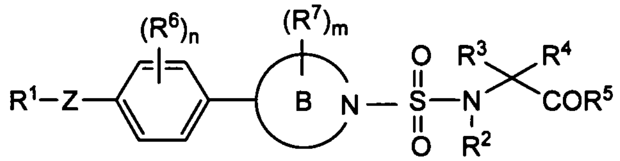

(II)

wherein $R^1$, $R^2$, $R^3$, $R^4$, $R^5$, $R^6$, $R^7$, Z, m, n and B ring are as defined in above 1),
an optically active isomer, a pharmaceutically acceptable salt or a solvate thereof.

9) The compound according to any one of 1') or 1) to 7), which is represented by the general formula (III):

[Chemical formula 17]

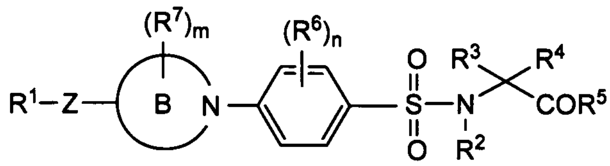

(III)

wherein $R^1$, $R^2$, $R^3$, $R^4$, $R^5$, $R^6$, $R^7$, Z, m, n and B ring are as defined in above 1),
an optically active isomer, a pharmaceutically acceptable salt or a solvate thereof.

10) The compound according to any one of 1') or 1) to 9), wherein Z is - $C(R^8)(R^9)$-, -O-, -S-, -SO-, $-SO_2-$, $-N(R^{10})-$, $-C(R^8)(R^9)-C(R^8)(R^9)-$, - $C(R^8)=C(R^9)$-, -C≡C-, $-O-C(R^8)(R^9)-$, $-C(R^8)(R^9)-O-$, $-S-C(R^8)(R^9)-$, - $C(R^8)(R^9)-S-$, $-SO-C(R^8)(R^9)-$, $-C(R^8)(R^9)-SO-$, $-SO_2-C(R^8)(R^9)-$, - $C(R^8)(R^9)-SO_2-$, $-N(R^{10})-C(R^8)(R^9)-$, $-C(R^8)(R^9)-N(R^{10})-$, $-N(R^{10})-C(=O)-$, $-C(=O)-N(R^{10})-$, - $-N(R^{10})-SO_2-$, $-SO_2-N(R^{10})-$, $-C(R^8)(R^9)-C(=O)-$, $-C(=O)-C(R^8)(R^9)-$, -C(=O)C(=O)-, $-C(R^8)(R^9)-C(R^8)(R^9)-C(R^8)(R^9)-$, $-C(R^8)(R^9)-C(R^8)=C(R^9)-$, $-C(R^8)=C(R^9)-C(R^8)(R^9)-$, - $C(R^8)(R^9)$-C≡C-, -C≡C-$C(R^8)(R^9)$-, $-O-C(R^8)(R^9)-C(R^8)(R^9)-$, - $C(R^8)(R^9)-O-C(R^8)(R^9)-$, $-C(R^8)(R^9)-C(R^8)(R^9)-O-$, $-S-C(R^8)(R^9)-C(R^8)(R^9)-$, $-C(R^8)(R^9)-S-C(R^8)(R^9)-$, $-C(R^8)(R^9)-C(R^8)(R^9)-S-$, $-SO-C(R^8)(R^9)-C(R^8)(R^9)-$, $-C(R^8)(R^9)-SO-C(R^8)(R^9)-$, $-C(R^8)(R^9)-C(R^8)(R^9)-SO-$, $-SO_2-C(R^8)(R^9)-C(R^8)(R^9)-$, $-C(R^8)(R^9)-SO_2-C(R^8)(R^9)-$, $-C(R^8)(R^9)-C(R^8)(R^9)-SO_2-$, $-N(R^{10})-C(R^8)(R^9)-C(R^8)(R^9)-$, - $C(R^8)(R^9)-N(R^{10})-C(R^8)(R^9)-$, $-C(R^8)(R^9)-C(R^8)(R^9)-N(R^{10})-$, $-C(=O)-C(R^8)(R^9)-C(R^8)(R^9)-$, $-C(R^8)(R^9)-C(=O)-C(R^8)(R^9)-$, $-C(R^8)(R^9)-C(R^8)(R^9)-C(=O)-$, $-C(R^8)(R^9)-N(R^{10})-C(=O)-$, $N(R^{10})-C(=O)-C(R^8)(R^9)-$, $-C(R^8)(R^9)-C(=O)-N(R^{10})-$, $-C(=O)-N(R^{10})-C(R^8)(R^9)-$, - $C(R^8)(R^9)-N(R^{10})-SO_2-$, $-N(R^{10})-SO_2-C(R^8)(R^9)-$, $-C(R^8)(R^9)-SO_2-N(R^{10})-$, $-SO_2-N(R^{10})-C(R^8)(R^9)-$, $N(R^{10})-C(=O)-N(R^{10})-$, - $N(R^{10})-C(=S)-N(R^{10})-$, $-O-N=C(R^8)-$, $-C(R^8)=N-O-$, $-O-C(=O)-C(R^8)(R^9)-$, - $C(R^8)(R^9)-C(=O)-O-$, $-O-C(=O)-N(R^{10})-$, or $N(R^{10})-C(=O)-O-$, wherein $R^8$, $R^9$ and $R^{10}$ are each independently a hydrogen atom or lower alkyl, an optically active isomer, a pharmaceutically acceptable salt or a solvate thereof.

11) The compound according to any one of 1') or 1) to 10), wherein Z is - $C(R^8)(R^9)-C(R^8)(R^9)-$, $-C(R^8)=C(R^9)-$,

-C≡C-, -O-C($R^8$)($R^9$)-, - C($R^8$)($R^9$)-O-, -S-C($R^8$)($R^9$)-, -C($R^8$)($R^9$)-S-, -SO-C($R^8$)($R^9$ )-, - C($R^8$)($R^9$)-SO-, -$SO_2$-C($R^8$)($R^9$)-, -C($R^8$)($R^9$)-$SO_2$-, -N($R^{10}$)-C($R^8$)($R^9$)-, -C($R^8$)($R^9$)-N($R^{10}$)-, N($R^{10}$)-C(=O)-, -C(=O)-N($R^{10}$)-, -N($R^{10}$)-$SO_2$-, -$SO_2$-N($R^{10}$)-, -C($R^8$)($R^9$)-C(=O)-, -C(=O)-C($R^8$)($R^9$)-, or -C(=O)C(=O)-, wherein $R^8$, $R^9$ and $R^{10}$ are each independently a hydrogen atom or lower alkyl,
an optically active isomer, a pharmaceutically acceptable salt or a solvate thereof.
12) The compound according to any one of 1') or 1) to 11), wherein Z is - $CH_2$-$CH_2$-, -CH=CH-, -C≡C-, -O-$CH_2$-, or -$CH_2$-O-,
an optically active isomer, a pharmaceutically acceptable salt or a solvate thereof.
13) The compound according to any one of 1') or 1) to 12), wherein Z is - C≡C- or -CH=CH-,
an optically active isomer, a pharmaceutically acceptable salt or a solvate thereof.
14) The compound according to any one of 1') or 1) to 13), wherein a group represented by the formula:

[Chemical formula 19]

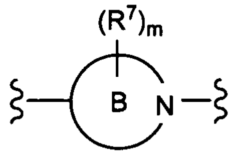

is a group represented by the formula:

[Chemical formula 20]

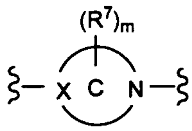

wherein X is a carbon atom or a nitrogen atom, a C ring is a 5- to 8-membered nitrogten-containing heterocyclic ring, and $R^7$ and m are as defined in above 1),
an optically active isomer, a pharmaceutically acceptable salt or a solvate thereof.
15) The compound according to any one of 1') or 1) to 14), wherein a group represented by the formula:

[Chemical formula 21]

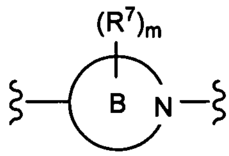

is a group represented by the formula:

[Chemical formula 22]

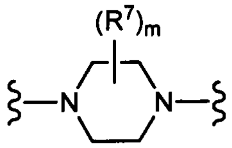, 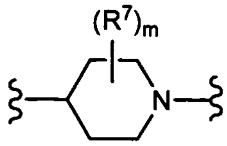, 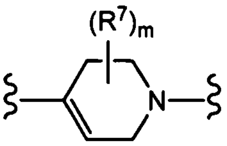,

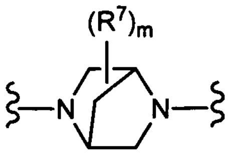 or 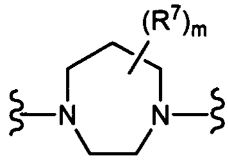

wherein $R^7$ and m are as defined in above 1),
an optically active isomer, a pharmaceutically acceptable salt or a solvate thereof.
16) The compound according to any one of 1') or 1) to 15), wherein $R^2$ is a hydrogen atom,
an optically active isomer, a pharmaceutically acceptable salt or a solvate thereof.
17) The compound according to any one of 1') or 1) to 16), wherein $R^3$ is a hydrogen atom, methyl, ethyl, n-propyl, isopropyl, n-butyl, s-butyl, isobutyl, tert-butyl, hydroxymethyl, 1-hydroxyethyl, 2-hydroxyethyl, carboxymethyl, carboxyethyl, methyloxymethyl, methylthiomethyl, 2-methylthioethyl, phenyl, 4-hydroxyphenyl, 4-fluorophenyl, 4-methyloxyphenyl, benzyl, 4-hydroxybenzyl, 4-fluorobenzyl, 4-chlorobenzyl, 4-methoxybenzyl, indol-3-ylmethyl, 1-carboxymethylindol-3-ylmethyl, thiazol-4-ylmethyl, carboxymethyloxybenzyl, or cyclopropylmethyl;
$R^4$ is a hydrogen atom; or
$R^3$ and $R^4$ may be taken together with an adjacent carbon to form a ring represented by the formula:

[Chemical formula 23]

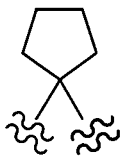、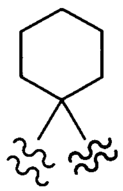 or 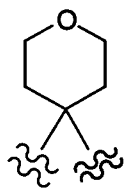

an optically active isomer, a pharmaceutically acceptable salt or a solvate thereof.
18) The compound according to any one of 1') or 1) to 17), wherein $R^3$ is a hydrogen atom, methyl, ethyl, n-propyl, isopropyl, n-butyl, s-butyl, isobutyl, tert-butyl, hydroxymethyl, 1-hydroxyethyl, 2-hydroxyethyl, carboxymethyl, carboxyethyl, methyloxymethyl, methylthiomethyl, 2-methylthioethyl, phenyl, 4-hydroxyphenyl, 4-fluorophenyl, 4-methyloxyphenyl, benzyl, 4-hydroxybenzyl, 4-fluorobenzyl, 4-chlorobenzyl, 4-methoxybenzyl, indol-3-ylmethyl, 1-carboxymethylindol-3-ylmethyl, thiazol-4-ylmethyl, carboxymethyloxybenzyl, or cyclopropylmethyl, and $R^4$ is a hydrogen atom, an optically active isomer, a pharmaceutically acceptable salt or a solvate thereof.
19) The compound according to any one of 1') or 1) to 18), wherein $R^5$ is hydroxy,
an optically active isomer, a pharmaceutically acceptable salt or a solvate thereof.
20) A pharmaceutical composition containing the compound as defined in any one of 1) to 19) as an active ingredient.
21) A pharmaceutical composition for inhibiting MMP-13 which contains the compound as defined in any one of 1) to 19) as an active ingredient.
22) A pharmaceutical composition for treating or preventing osteoarthritis containing the compound as defined in any one of 1) to 19) as an active ingredient.
23) A method of treating a disease resulting from, or associated with MMP-13 of a mammal, comprising administering to a mammal including a human therapeutically effective amount of the compound as defined in any one of 1) to 19).
24) A method of treating osteoarthritis of a mammal, comprising administering to a mammal including a human therapeutically effective amount of the compound as defined in any one of 1) to 19).
25) Use of the compound as defined in any one of 1) to 19), for preparing a medicament for treating a disease resulting from associated with MMP-13.

26) Use of the compound as defined in any one of 1) to 19), for preparing a medicament for treating osteoarthritis.

In the present specification, the term "lower alkyl" which is used alone or in combination with other terms includes straight chained or branched monovalent hydrocarbon groups comprised of 1 to 8 carbon atoms. For example, methyl, ethyl, n-propyl, isopropyl, n-butyl, isobutyl, s-butyl, tert-butyl, n-pentyl, isopentyl, neo-pentyl, n-hexyl, isohexyl, n-heptyl, n-octyl and the like are exeplified. Preferable is C1-C6 alkyl. Further, C1-C3 alkyl is preferable.
In the present specification, the term "cycloalkyl" includes C3-C8 cycloalkyl. For example they are cyclopropyl, cylobutyl, cyclopentyl, cyclohexyl, cycloheptyl and cyclooctyl. Preferable is C3-C6 cycloalkyl.
In the present specification, the "lower alkenyl" which is used alone or in combination with other terms includes straight chained or branched monovalent hydrocarbon group comprised of 2 to 8 carbon atoms, having one or more double bonds. For example, vinyl, allyl, propenyl, crotonyl, isopentenyl, various butenyl isomers and the like are exemplified. Preferable is C2-C6 alkenyl. Further, C2-C4 alkenyl is preferable.
In the present specification, the "cycloalkenyl" includes cycloalkenyls comprised of 3 to 8 carbon atoms, having one or more double bonds in the ring. For example, cyclopropenyl, cyclobutenyl, cyclopentenyl, cyclohexenyl, cycloheptenyl, and cyclooctenyl are exemplifired. Preferable is C3-C6 cycloalkenyl.
In the present specification, the "lower alkynyl" includes straight chained or branched monovalent hydrocarbon groups comprised of 2 to 8 carbon atoms, having one or more triple bonds, which may have a double bond. For example, ethynyl, 2-propynyl, 3-butynyl, 4-pentynyl, 5-hexynyl, 6-heptynyl, 7-octynyl and the like are exemplified. Preferable is C2-C6 alkynyl. Further, C2-C4 alkynyl is preferable.
In the present specification, the "cycloalkynyl" includes cycloalkynyl comprised of 4 to 8 carbon atoms, having one or more triple bonds in the ring, which may have a double bond. For example, cyclobutynyl, cyclopentynyl, cyclohexynyl, cycloheptynyl, and cyclooctynyl are exemplified. Preferable is C4-C6 cycloalkynyl.
In the present specification, the "alkylene" includes divalent hydrocarbon group corresponding to the above "lower alkyl".
In the present specification, the "alkenylene" includes divalent hydrocarbon group corresponding to the above "lower alkenyl".
In the present specification, the "alkynylene" includes divalent hydrocarbon group corresponding to the above "lower alkynyl".
In the present specification, the " C1-C5 alkylene which may be intervened with a substituent selected from Substituent group a " includes (substituent selected from Substituent group a)-(C1-C5 alkylene), (C1-C5 alkylene)-(substituent selected from Substituent group a), and (alkylene)-(substituent selected from Substituent group a)-(alkylene), provided that the sum of lengths of both alkylene chains is an integer of 1 to 5.
In the present specification, the term "aryl" which is used alone or in combination with other terms includes monocyclic aromatic hydrocarbon group or fused aromatic hydrocarbon group in which 2 or 3 aromatic rings are fused. For example, phenyl, 1-naphthyl, 2-naphthyl, anthryl and the like are exemplified.
As the "aryl" in $R^2$, phenyl is preferable.
As the "aryl" in $R^3$, phenyl is preferable.
In the present specification, the term "naphthyl" includes 1-naphthyl and 2-naphthyl.
In the present specification, the term "aralkyl" which is used alone or in combination with other terms is the abovementioned "lower alkyl" which is substituted with one or more of the abovementioned "aryl", which may be substituted at all possible positions. For example, benzyl, phenylethyl (e.g. 2-phenylethyl, etc.), phenylpropyl (e.g. 3-phenylpropyl, etc.), naphthylmethyl (e.g. 1-naphthylmethyl, 2-naphthylmethyl, etc.), anthrylmethyl (e.g. 9-anthrylmethyl, etc.), biphenylmethyl (e.g. 4-biphenylmethyl, etc.) and the like are exemplified. Preferably, benzyl,and phenylethyl are exemplified.
As the "aralkyl" in $R^2$, phenyl C1-C3 alkyl is preferable, and benzyl is particularly preferable.
As the "aralkyl" in $R^3$, phenyl C1-C6 alkyl and naphthyl C1-C6 alkyl are preferable. Further preferable are phenyl C1-C3 alkyl and naphthyl C1-C3 alkyl, for example, benzyl, phenylethyl, 1-naphthylmethyl, and 2-naphthylmethyl.
In the present specification, the term "heteroaryl" which is used alone or in combination with other terms includes 5- to 6-membered aromatic ring containing, in the ring, one or more atoms arbitrarily selected from oxygen atoms, sulfur atoms and nitrogen atoms, and these may be fused with cycloalkyl, aryl, non-aromatic heterocyclic group, or other heteroaryl, and may be fused at all possible positions. For example, pyrrolyl (e.g. 1-pyrrolyl, 2-pyrrolyl, 3-pyrrolyl), furyl (e.g. 2-furyl, 3-furyl), thienyl (e.g. 2-thienyl, 3-thienyl), imidazolyl (e.g. 2-imidazolyl, 4-imidazolyl), pyrazolyl (e.g. 1-pyrazolyl, 3-pyrazolyl), isothiazolyl (e.g. 3-isothiazolyl), isoxazolyl (e.g. 3-isoxazolyl), oxazolyl (e.g. 2-oxazolyl), thiazolyl (e.g. 2-thiazolyl), pyridyl (e.g. 2-pyridyl, 3-pyridyl, 4-pyridyl), pyrazinyl (e.g. 2-pyrazinyl), pyrimidinyl (e.g. 2-pyrimidinyl, 4-pyrimidinyl), pyridazinyl (e.g. 3-pyridazinyl), tetrazolyl (e.g. 1H-tetrazolyl), oxadiazolyl (e.g. 1,3,4-oxadiazolyl), thiadiazolyl (e.g. 1,3,4-thiadiazolyl), indolizinyl (e.g. 2-indolizinyl, 6-indolizinyl), isoindolyl (e.g. 2-isoindolyl), indolyl (e.g. 1-indolyl, 2-indolyl, 3-indolyl, 5-indolyl, 6-indolyl), indazolyl (e.g. 3-indazolyl), purinyl (e.g. 8-purinyl), quinolizinyl (e.g. 2-quinolizinyl), isoquinolyl (e.g. 3-isoquinolyl), quinolyl (e.g. 2-quinolyl, 5-quinolyl), phthalazinyl (e.g. 1-phthalazinyl), naphthyridinyl (e.g. 2-naphthyridinyl), quinoxanyl (e.g. 2-quinoxanyl), quinazolinyl (e.g. 2-quinazolinyl), cinnolinyl (e.g. 3-cinnolinyl), pteridinyl (e.g. 2-pteridinyl), carbazolyl (e.g. 2-carbazolyl, 3-carbazolyl), phenanthridinyl (e.g. 2-phenanthridinyl, 3-phenanthridinyl),

acridinyl (e.g. 1-acridinyl, 2-acridinyl), dibenzofuranyl (e.g. 1-dibenzofuranyl, 2-dibenzofuranyl, 3-dibenzofuranyl), benzimidazolyl (e.g. 2-benzimidazolyl), benzisoxazolyl (e.g. 3-benzisoxazolyl), benzoxazolyl (e.g. 2-benzoxazolyl), benzoxadiazolyl (e.g. 4-benzoxadiazolyl), benzisothiazolyl (e.g. 3-benzisothiazolyl), benzothiazolyl (e.g. 2-benzothiazolyl, 5-benzothiazolyl), benzofuryl (e.g. 2-benzofuryl, 3-benzofuryl, 5-benzofuryl), benzothienyl (e.g. 2-benzothienyl), thienopyrimidinyl and the like are exemplified.
As the "heteroaryl" in $R^{29}$, thiazolyl, oxazoly, benzoxazolyl benzothienyl, benzothiazolyl and the like are preferable.
As the "heteroaryl" in $R^{26}$, $R^{28}$, $R^{30}$ and $R^{31}$, pyrrolyl, furyl, thienyl, oxazolyl, thiazolyl and the like are preferable.
As the "heteroaryl" in $R^2$, pyridyl, thienyl, furyl, imidazolyl and the like are preferable.
As the "heteroaryl" in $R^3$, indolyl, imidazolyl, furyl, thienyl and the like are preferable.

In the present specification, the term "heteroarylalkyl" is the abovementioned "lower alkyl" substituted with one or more of the abovementioned "heteroaryl" at the arbitrary positions, and these can be substituted at all possible positions. For example, oxazolylmethyl (e.g. 2-oxazolylmethyl, 4-oxazolylmethyl), thiazolylmethyl (e.g. 2-thiazolylmethtyl, 4-thiazolylmethyl), oxazolylethyl (e.g. 5-oxazolyl-2-ethyl), thiazolylethyl (e.g. 5-thiazolyl-2-ethyl), benzoxazolylmethyl (e.g. benzoxazol-2-ylmethyl), benzothiazolylmethyl (e.g. benzothiazol-2-ylmethyl), indolylmethyl (e.g. indol-3-ylmethyl), imidazolylmethyl (e.g. 4-imidazolylmethyl), indazolylmethyl (e.g. 1-indazolylmethyl), benzotriazolylmethyl (e.g. 1-benzotriazolylmethyl), benzoquinolylmethyl (e.g. 2-benzoquinolylmethyl), benzimidazolylmethyl (e.g. benzimidazol-2-methyl), pyridylmethyl (e.g. 4-pyridylmethyl), furylmethyl (e.g. furan-2-ylmethyl), thienylmethyl (e.g. thiophen-2-ylmethyl) and the like are exemplified.
Examples of the "heteroarylalkyl" in $R^2$ are indolylmethyl (e.g. indol-3-ylmethyl), imidazolylmethyl (e.g. imidazol-5-ylmethyl), thienylmethyl (e.g. thiophen-2-ylmethyl), thiazolylmethyl (e.g. 2-thiazolylmethyl, 4-thiazolylmethyl), pyridylmethyl and the like.
Examples of the "heteroarylalkyl" in $R^3$, $R^{30}$ and $R^{31}$ are indolylmethyl (e.g. indol-3-ylmethyl), imidazolylmethyl (e.g. imidazol-5-ylmethyl), benzoxazolylmethyl (e.g. benzoxazol-2-ylmethyl), benzothiazolylmethyl (e.g. benzothiazol-2-ylmethyl), benzimidazolylmethyl (e.g. benzimidazol-2-ylmethyl), thienylmethyl (e.g. thiophen-2-ylmethyl), thiazolylmethyl (e.g. 2-thiazolylmethyl, 4-thiazolylmethyl) and the like.

In the present specification, the term "aromatic heterocyclic ring" includes 5- to 6-membered aromatic ring containing, in the ring, one or more atoms arbitrarily selected from oxygen atoms, sulfur atoms and nitrogen atoms, and these may be fused with cycloalkyl, aryl, a non-aromatic heterocyclic group, or other heteroaryl, and may be fused at all possible positions.
Examples are pyrrole, furan, thiophene, imidazole, pyrazole, isothiazole, isoxazole, oxazole, thiazole, pyridine, pyrazine, pyrimidine, pyridazine, tetrazole, oxadiazole, thiadiazole, indolizine, isoindole, indole, indazole, purine, quinolizine, isoquinoline, quinoline, phthalazine, naphthylidine, quinoxaline, quinazoline, cinnoline, pteridine, carbazol, phenanthridine, acridine, dibenzofuran, benzimidazole, benzisoxazole, benzoxazole, benzoxadiazole, benzisothiazole, benzothiazole, benzofuran, benzothiophene and thienopyrimidine and the like.
As the "aromatic heterocycric ring " in D ring, pyrrole, furan, thiophene, benzofuran, pyridine, pyrimidine, indole, and the like are preferable.
In the present specification, the term "non-aromatic carbocyclic group" which is used alone or in combination with other terms includes "cycloalkyl", "cycloalkenyl", "cycloalkynyl", and the fused ring which "cycloalkyl", "cycloalkenyl", or "cycloalkynyl" may be fused with the above "aryl". For example, cyclobutyl, cyclopentyl, cyclohexyl, indanyl, tetrahydronaphthyl, and the like are exemplified.
As the "non-aromatic carbocyclic group" in Z, cyclobutyl is preferable.
As the "non-aromatic carbocyclic ring" in D ring, cyclopentane, cyclohexane, cycloheptane and cyclohexene and the like are preferable.
Examples of the "non-aromatic carbocyclic group" in which $R^3$ and $R^4$ are taken together with an adjacent carbon to form a 5- to 6-membered non-aromatic carbocyclic group, are cycloalkane, cycloalkene and cycloalkyne. Cyclopentane, cyclohexane and the like are preferable.
In the present specification, a term "non-aromatic heterocyclic group" which is used alone or in combination with other terms includes a non-aromatic 5- to 7- membered ring containing in the ring, one or more atoms, arbitrarily selected from oxygen atoms, sulfer atoms and nitrogen atoms, and the ring group in which two or more of the above rings are fused, and the ring group in which the abovementioned "ring" is fused with the abovementioned "aryl". For example, pyrrolidinyl (e.g. 1-pyrrolidinyl, 2-pyrrolidinyl), pyrrolinyl (e.g. 3-pyrrolinyl), imidazolidinyl (e.g. 2-imidazolidinyl), imidazolinyl (e.g. imidazolinyl), pyrazolidinyl (e.g. 1-pyrazolidinyl, 2-pyrazolidinyl), pyrazolinyl (e.g. pyrazolinyl), piperidyl (e.g. piperidino, 2-piperidyl), piperazinyl (e.g. 1-piperazinyl), indolinyl (e.g. 1-indolinyl), isoindolinyl (e.g. isoindolinyl), morpholinyl (e.g. morpholino, 3-morpholinyl), dihydropyridyl, tetrahydropyridyl, tetrahydroquinolyl, tetrahydrofuryl, tetrahydropyranyl, 1,3-benzodioxolanyl, 1,4-benzodioxanyl, 1-benzoxolanyl, oxetanyl, azetidinyl, diazetidinyl, chromanyl and the like are exemplified.
As the "non-aromatic heterocyclic group" in $R^{29}$, morpholinyl, tetrahydrofuranyl, tetrahydropyranyl, piperidyl, piperazinyl, and the like are preferable.

As the "non-aromatic heterocyclic group" in $R^{26}$, $R^{28}$, $R^{30}$ and $R^{31}$ morpholinyl and the like are preferable.
As the "non-aromatic heterocyclic group" in Z, oxetanyl, azetidinyl, and diazetidinyl are preferable.
As the "non-aromatic heterocyclic ring" in D ring, pyrrolidine, 2H-pyran, dihydro-2H-oxazine, pyrroline, tetrahydropyran, tetrahydroxazepine, dihydropyran, dihydropyridine, dihydropyrimidine, tetrahydropyridine, tetrahydropyrimidine, tetrahydroxazepine, tetrahydrodiazepine and the like are preferable.
As the "non-aromatic heterocyclic ring" in which $R^3$ and $R^4$ are taken together with an adjacent carbon to form a 5- to 6- membered non-aromatic heterocyclic ring, tetrahydropyran and the like are preferable.

The "nitrogen-containing heterocyclic ring which is optionally contained a further nitrogen atom, an oxygen atom, and/or a sulfur atom in a ring" in the B ring means a nitrogen-containing heterocyclic ring optionally further containing a nitrogen atom in addition to a nitrogen atom on the B ring, an oxygen atom, and/or a sulfur atom in the ring,. The "nitrogen-containing heterocyclic ring" includes monocyclic 5- to 8- membered nitrogen-containing heterocyclic ring, and bridged nitrogen-containing heterocyclic ring. Preferable are nitrogen-containing heterocyclic ring represented by the formula:

[Chemical formula 24]

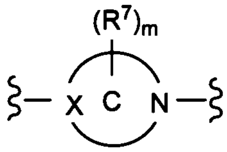

wherein X is a carbon atom or a nitrogen atom, C ring is a 5- to 8-membered nitrogen-containg heterocyclic ring or a bridged ring. Further preferable are nitrogen-containing heterocyclic ring represented by the formula:

[Chemical formula 25]

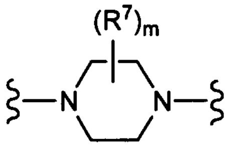
,
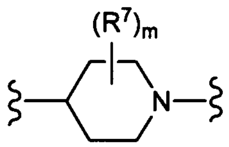
,
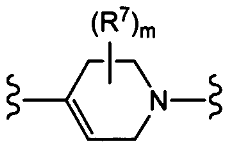
,

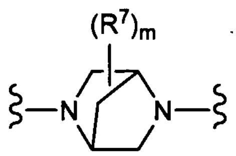
or
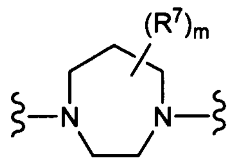

Particularly preferable are nitrogen-containing heterocyclic ring represented the formula:

[Chemical formula 26]

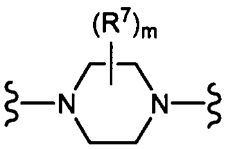

In the present specification, "halogen" means fluorine, chlorine, bromine and iodine. Preferable are fluorine,

chlorine, and bromine.
In the present specification, examples of the "lower alkyloxy" are methyloxy, ethyloxy, n-propyloxy, isopropyloxy, n-butyloxy, isobutyloxy, s-butyloxy, tert-butyloxy, n-pentyloxy, n-hexyloxy and the like. Preferable is C1-C6 alkyloxy. Further preferable is C1-C3 alkyloxy.
In the present specification, "lower alkyloxy lower alkylcarbonyl" means the abovementioned "lower alkylcarbonyl" is substituted with the abovementioned "lower alkyl". Examples of the "lower alkyloxy lower alkylcarbonyl" are methyloxymethylcarbonyl, ethyloxymethylcarbonyl, methyloxyethylcarbonyl, isopropyloxymethylcarbonyl, isopropyloxyethylcarbonyl and the like.
In the present specification, examples of the "lower alkenyloxy" are vinyloxy, allyloxy, propenyloxy, 3-butenyloxy, 2-butenyloxy (crotonyloxy, isocrotonyloxy), 1-butenyloxy, isopentenyloxy and the like. C2-C3 alkenyloxy is preferable.
In the present specification, examples of the "lower alkylthio" are methylthio, ethylthio, propylthio, isopropylthio, butylthio, s-butylthio, isobutylthio, t-butylthio and the like. C1-C3 alkylthio is preferable. Particularly preferable is methylthio.

In the present specification, the term "halo lower alkyl" which is used alone or in combination with other terms includes the abovementioned "lower alkyl", 1 to 8 atoms, preferably 1 to 5 atoms of which are substituted with the abovementioned "halogen". For example, trifluoromethyl, trichloromethyl, difluoroethyl, trifluoroethyl, dichloroethyl, trichloroethyl and the like are exemplified. Preferable examples are C1-C3 lower alkyl, 1 to 5 atoms of which are substituted with the abovementioned "halogen". Particularly preferable example is trifluoromethyl.
In the present specification, as the "halo lower alkyloxy", C1-C3 lower alkyloxy, 1 to 5 atoms of which are substituted with the above "halogen", is preferable. Particularly preferable example is trifluoromethyloxy.
In the present specification, as the "halo lower alkylthio", C1-C3 lower alkylthio, 1 to 5 atoms of which are substituted with the above "halogen", is preferable. Particularly preferable example is trifluoromethylthio.
In the present specification, the term "hydroxy lower alkyl" includes the abovementioned "lower alkyl" substituted with a hydroxy group. For example, hydroxymethyl, 1-hydroxyethyl, 2-hydroxyethyl, 1-hydroxypropyl, 2-hydroxypropyl, 3-hydroxypropyl and the like are exemplified. Preferable examples are hydroxymethyl, 1-hydroxyethyl, and 2-hydroxyethyl.
In the present specification, examples of the "lower alkyloxycarbonyl" are methyloxycarbonyl, ethyloxycarbonyl, n-propyloxycarbonyl, isopropyloxycarbonyl, tert-butyloxycarbonyl and the like.
In the present specification, examples of the "lower alkylsulfonyl" are methylsulfonyl, ethylsulfonyl and the like. Preferable example is methylsulfonyl.
In the present specification, examples of the "lower alkylsulfinyl" are methylsulfinyl, ethylsulfinyl and the like. Preferable example is methylsulfinyl.
In the present specification, examples of the "lower alkyloxyimino" are methyloxyimino, ethyloxyimino, propyloxyimino, isopropyloxyimino butyloxyimino isobutyloxyimino and the like.
In the present specification, examples of the "aralkyloxy" are phenyl C1-C3 alkyloxy, naphthyl C1-C3 alkyloxy and the like. Preferable example is benzyloxy.
In the present specification, examples of the "aralkylthio" are phenyl C1-C3 alkylthio, naphthyl C1-C3 alkylthio and the like. Preferable example is benzylthio.
In the present specification, examples of the "aralkylsulfonyl" are phenyl C1-C3 alkylsulfonyl, naphthyl C1-C3 alkylsulfonyl and the like. Preferable example is benzylsulfonyl.
In the present specification, examples of the "aralkylsulfinyl" are phenyl C1-C3 alkylsulfinyl, naphthyl C1-C3 alkylsulfinyl and the like. Preferable example is benzylsulfinyl.
In the present specification, a term "acyl" which is used alone or in combination with other terms includes alkylcarbonyl in which an alkyl moiety is the abovementioned "lower alkyl" or "haro lower alkyl", arylcarbonyl in which an aryl moiety is the abovementioned "aryl", heteroarylcarbonyl in which a heteroaryl moiety is the abovementioned "heteroaryl", aralkylcarbonyl in which an aralkyl moiety is the abovementioned " aralkyl ", heteroarylalkylcarbonyl in which a heteroarylalkyl moiety is the abovementioned "heteroarylalkyl" and non-aromatic heterocyclic carbonyl in which a non-aromatic heterocyclic moiety is the abovementioned " non-aromatic heterocyclic group ". For example, acetyl, propionyl, benzoyl, pyridylcarbonyl (e.g. nicotinoyl, isonicotinoyl and the like), morpholinocarbonyl, benzyl carbonyl and the like are exemplified. The "lower alkyl", the "aryl", the "heteroaryl" and "non-aromatic heterocyclic group" may be substituted with a substituent described later.
In the present specification, a term "acyloxy" includes acyloxy in which an acyl moiety is the abovementioned "acyl". For example, they are acetyloxy, propionyloxy, benzoyloxy and the like.

In the present specification, the "optionally substituted amino" which is used alone or in combination with other terms includes amino and amino, 1 or 2 atoms of which are substituted with the abovementioned "lower alkyl", the abovementioned "aralkyl", the abovementioned "heteroarylalkyl",the abovementioned " lower alkyloxycarbonyl", the abovementioned "lower alkylsulfonyl", or the abovementioned "acyl". Preferable examples are unsubstituted amino, and amino substituted with 1 or 2 substituents selected from the group consisting of C1-C6 alkyl, phenyl C1-C3 alkyl, pyridyl C1-C3 alkyl, C1-C6 alkylsulfonyl, C1-C6 alkylcarbonyl and arylcarbonyl. For example, amino, methylamino, dimethylamino; ethylmethylamino, diethylamino, benzylamino, pyridylmethylamino, tert-butoxycarbonylamino, methylsulfonylamino, acetylamino, benzoylamino and the like are exemplified. Preferable examples are amino, methylamino, dimethylamino, ethylmethylamino, diethylamino, methylsulfonylamino and acetylamino.

In the present specification, as the "optionally substituted aminocarbonyl", unsubstitued aminocarbonyl, arylaminocarbonyl optionally substituted with a substituent selected from Substituent group c, aralkylaminocarbonyl optionally substituted with a substituent selected from Substituent group c, heteroarylalkylaminocarbonyl optionally substituted with a substituent selected from Substituent group c, aryloxyaminocarbonyl optionally substituted with a substituent selected from Substituent group c, lower alkyloxyaminocarbonyl optionally substituted with a substituent selected from Substituent group c, lower alkyaminocarbonyl optionally substituted with a substituent selected from Substituent group c, lower alkenylaminocarbonyl optionally substituted with a substituent selected from Substituent group c, lower alkynylaminocarbonyl optionally substituted with a substituent selected from Substituent group c, lower alkylaminocarbonyl substituted with a cycloalkyl optionally substituted with a substituent selected from Substituent group c, lower alkylaminocarbonyl substituted with a non-aromatic heterocyclic group optionally substituted with a substituent selected from Substituent group c, aminocarbonyl wherein nitrogen atom forms a non-aromatic heterocyclic group, aminocarbonyl optionally substituted with 1 or 2 non-aromatic heterocyclic group(s) substituted with a substituent selected from Substituent group c, or aminocarbonyl optionally substituted with 1 or 2 cycloalkyl substituted with a substituent selected from Substituent group c are preferable. For example, aminocarbonyl, methylaminocarbonyl, dimethylaminocarbonyl, ethylmethylaminocarbonyl, diethylaminocarbonyl, isobutylaminocarbonyl, isopropylaminocarbonyl, aminoethylaminocarbonyl, 2-(tert-butoxycarbonylamino)-ethylaminocarbonyl, 2-(tert-butoxycarbonyl(methyl) amino) ethylaminocarbonyl, 2-methylaminoethylcarbonyl, 3-prop-2-ylaminocarboyl, cyanomethylaminocarbonyl, cyanoethylaminocarbonyl, phenyloxyamino, benzylaminocarbonyl, 4-chlorobenzylaminocarbonyl, 3-chlorobenzylaminocarbonyl, 2-chlorobenzylaminocarbonyl, 4-fluorobenzylaminocarbonyl, 3-fluorobenzylaminocarbonyl, 4-bromobenzylaminocarbonyl, 4-methoxybenzylaminocarbonyl, 3-methoxybenzylaminocarbonyl, 4-tert-butylbenzylaminocarbonyl, phenylaminocarbonyl, pyridylmethylaminocarbonyl, 4-chlorophenylaminocarbonyl, 4-trifluoromethylbenzylaminocarbonyl, 3-trifluoromethylbenzylaminocarbonyl, 3-methylbenzylaminocarbonyl, 4-trifluoromethoylbenzylaminocarbonyl, 3-trifluoromethoylbenzylaminocarbonyl, 3, 4-dimethoylbenzylaminocarbonyl, 3, 4-dichlorobenzylaminocarbonyl, 3, 4-difluorobenzylaminocarbonyl, 3, 5-difluorobenzylaminocarbonyl, 2, 5-dichlorobenzylaminocarbonyl, 4-dimethylaminobenzylaminocarbonyl, 3-dimethylaminomethyl-4- fluorobenzylaminocarbonyl, chloropyridylmethylaminocarbonyl, 2-methanesulfonylaminoethylaminocarbonyl, 3-methanesulfonylaminopropylaminocarbonyl, 4-methanesulfonylaminobutylaminocarbonyl, 1-(methanesulfonylpiperidin-4-ylmethyl)aminocarbonyl, 4-methanesulfonylbenzylaminocarbonyl, 4-piperidin-1-ylbenzylaminocarbonyl, 4-(2-oxopyrrolidin-1-yl)-benzylaminocarbonyl, 4-methylaminocarbonylbenzylaminocarbonyl, 4-isopropylcarbamoylbenzylaminocarbonyl, 3-isopropylcarbamoylbenzylaminocarbonyl, 4-acetylaminobenzylaminocarbonyl, (thiophen-2-ylmethyl)-aminocarbonyl, isopropylaminocarbonylaminopropylaminocarbonyl, ethoxycarbonylaminopropylaminocarbonyl, isopropylsulfonylaminopropylaminosulfonyl, 4-sulfamoylbenzylaminocarbonyl, 3-chloro-4-fluorobenzylaminocarbonyl, 4-dimethylaminocarbonylbenzyl, 4-fluoro-3-methylbenzylaminocarbonyl, (furan-2-ylmethyl)- aminocarbonyl, (1-methyl-1H-pyrazol-3-ylmethyl)-aminocarbonyl, 4-fluoro-3-methoxymethylbenzylaminocarbonyl, 4-methylbenzylaminocarbonyl, 4-isopropylbenzylaminocarbonyl, (benzo[1,3]dioxol-5-ylmethyl)-aminocarbonyl, (2,3-dihydrobenzofuran-5-ylmethyl)-aminocarbonyl, 4-sulfamoylbenzylaminocarbonyl, (5-methylisoxazol-3-ylmethyl)-aminocarbonyl, 4-methylfurazan-3-ylmethyl)-aminocarbonyl, 4-fluoro-3-trifluoromethylbenzylaminocarbonyl, (2, 3-dihydrobenzo[1, 4] dioxin-6-ylmethyl) aminocarbonyl, 4-morpholin-4-ylbenzylaminocarbonyl, (5-methyl-[1, 2, 4]oxadiazol-3-ylmethyl)aminocarbonyl, 3-(3-methoxyphenoxy)-benzylaminocarbonyl, 3-bromo-4-fluorobenzylaminocarbonyl, 4-fluoro-3-methoxybenzylaminocarbonyl, (1, 5-dimethyl-1H-pyrazol-3-ylmethyl)-aminocarbonyl, 4-pyrazol-1-ylbenzylaminocarbonyl, (2, 5-dimethyloxazol-4-ylmethyl)-aminocarbonyl, 4-pyrrol-1-ylbenzylaminocarbonyl, (biphenyl-4-ylmethyl)-aminocarbonyl, 3-hydroxy-4-methoxybenzylaminocarbonyl, 4-phenoxybenzylaminocarbonyl, 4-trifluoromethylsulfanylbenzylaminocarbonyl, 4-(tert-butoxycarbonylaminomethyl)-benzylaminocarbonyl, 3-(tert-butoxycarbonylaminomethyl)-benzylaminocarbonyl, 4-fluoro-2-methoxymethylbenzylaminocarbonyl, 3-iodobenzylaminocarbonyl, 4-fluoro-2-methoxybenzylaminocarbonyl, 4-aminocarbonylbenzylaminocarbonyl, 4-fluoro-2-methanesulfonylaminobenzylaminocarbonyl, 3-fluoro-4-methxybenzylaminocarbonyl, [5-(4-chlorophenyl)-thiophen-2-ylmethyl]-aminocarbonyl, 2-dimethylcarbamoyl-4-fluorobenzylaminocarbonyl, 2-fluoro-ethylaminocarbonyl, 2-oxo-propylaminocarbonyl, cyclopropylaminocarbonyl, cyclohexylmethylaminocarbonyl, 3, 4-dichlorobenzylaminocarbonyl, 3, 5-dichlorobenzylaminocarbonyl and the like are exemplified.

Preferable examples are aminocarbonyl, dimethylaminocarbonyl, dichlorobenzylaminocarbonyl, 4-chlorobenzylaminocarbonyl, 3-chlorobenzylaminocarbonyl, 4-methoxybenzylaminocarbonyl, 4-fluorobenzylaminocarbonyl, 4-tert-butylbenzylaminocarbonyl, (3-methyl-2-oxo-2, 3-dihydrobenzoxazol-6-ylmethyl)-aminocarbonyl, pyridin-4-ylmethylaminocarbonyl, 4-cyanobenzylaminocarbonyl, and piperidinecarbonyl.

Substituent group c : halogen; hydroxy; cyano; acyl; lower alkyloxy; lower alkyloxycarbonyl; lower alkylthio; lower alkyl substituted with lower alkyloxy; lower alkylsulfonyl; aminosulfonyl; lower alkyl; halo lower alkyl; halo lower alkyloxy; halo lower alkylthio; amino optionally substituted with 1 or 2 lower alkyl and/or lower alkyloxycarbonyl; amino substituted with lower alkylcarbonyl; lower alkylaminocarbonyl optionally substituted with cyano; amino substituted with aminocarbonyl; amino substituted with lower alkylaminocarbonyl; sulfonylamino substituted with lower alkyl; aminoalkyl optionally substituted with 1 or 2 lower alkyl and/or lower alkyloxycarbonyl; optionally substituted aryloxy wherein the substituent is selected from the group consisting of halogen, lower alkyl, lower alkykoxy, hydroxy, lower alkyl substituted with hydroxy, halo lower alkyl, lower alkylsulfonyl, lower alkyloxycarbonyl, carboxy, aminocarbonyl, lower alkylaminocarbonyl, and the like; cycloalkyl; a non-aromatic heterocyclic group; cycloalkyl in which hydrogen atom on the ring is substituted with oxo; a non-aromatic heterocyclic group in which hydrogen atom on the ring is substituted with oxo; lower alkylsulfoneamide; optionally substituted heteroaryl; oxo; amino optionally substituted with 1 or 2 optionally substituted aryl and/or lower alkyloxycarbonyl;

a group represented by the following formula:

[Chemical formula 27]

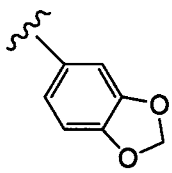
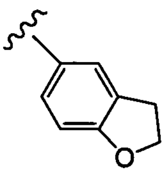
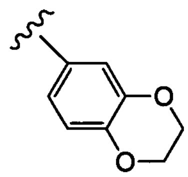
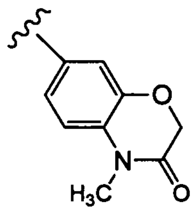
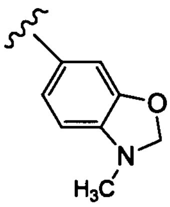
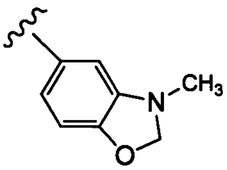
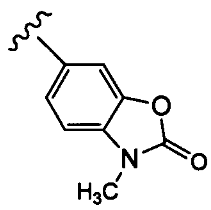
or
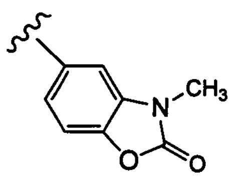

and aryl optionally substituted with the group represented by the formula:

[Chemical formula 28]

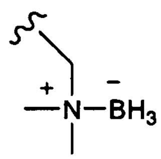

In the present specification, as the "optionally substituted hydrazino", unsunstituted hydrazino, lower alkyl hydrazino wherein the lower alkyl is abovementioned "lower alkyl", aryl hydrazino optionally substituted with a substituent selected from Substituent group c and heteroaryl hydrazino optionally substituted with a substituent selected from Substituent group c are preferable. Examples are hydrazino, methyhydrazino, ethyhydrazino, phenylhydrazino, pyridylhydrazino and the like.

In the present specification, as the "optionally substituted aminoxalyl", unsubstituted aminoxalyl or aminoxalyl substituted with 1 or 2 C1-C6 alkyl are preferable. For example, aminoxalyl, methylaminoxalyl, dimethylaminooxalyl, ethylmethylaminoxalyl, diethylaminoxalyl, isopropylaminoxalyl and the like are exemplified. Preferable examples are aminocarbonyl and dimethylaminoxalyl.

In the present specification, as the "optionally substituted aminosulfonyl", unsubstituted aminosulfonyl or aminosulfonyl substituted with 1 or 2 C1-C6 alkyl are preferable. For example, aminosulfonyl, methylaminosulfonyl, dimethylaminosulfonyl, ethylmethylaminosulfonyl, diethylaminosulfonyl, isopropylaminosulfonyl and the like are exemplified. Preferable examples are aminosulfonyl, dimethylaminosulfonyl and the like.

In the present specification, a term "lower alkylsulfonamide" includes the sulfonamide substituted with the abovemenrioned "lower alkyl". For example, methylsulfonamide, ethylsulfonamide and isopropylsulfonamide and the like are exemplified.

In the present specification, examples of a substituent of the "optionally substituted lower alkyl", "optionally substituted lower alkenyl"and "optionally substituted lower alkyloxy" are lower alkylthio, cycloalkyl, carboxy, lower alkyloxy, hydroxy, mercapto, halogen, nitro, cyano, lower alkyloxycarbonyl, halo lower alkyl, halo lower alkyloxy, optionally substituted amino, optionally substituted aminocarbonyl, acyl, acyloxy, aryl optionally substituted with a substituent selected from Substituent group b, hetetoaryl optionally substituted with a substituent selected from Substituent group b, a non-aromatic heterocyclic group optionally substituted with a substituent selected from Substituent group b, lower alkyloxyimino, aryloxy, aralkyloxy, lower alkylsulfonyl, guanidino, an azo group, ureido optionally substituted with a substituent selected from Substituent group b, carbamoyl and the like. These may substitute at one or more of all possible positions.

Substituent group b: lower alkyl, cycloalkyl, lower alkenyl, lower alkynyl, hydroxy, lower alkyloxy, mercapto, lower alkylthio, lower alkenyloxy, halogen, nitro, cyano, carboxy, lower alkyloxycarbonyl, halo lower alkyl, halo lower alkyloxy, halo lower alkylthio, optionally substituted amino, optionally substituted aminocarbonyl, acyl, acyloxy, aryl, heteroaryl, a non-aromatic heterocyclic group, aralkyl, lower alkylsulfonyl, guanidino, an azo group, and ureido.

As a substituent of the "optionally substituted lower alkyl", "optionally substituted lower alkenyl" and "optionally substituted lower alkyloxy" in $R^{26}$, $R^{28}$ ,$R^{30}$ and $R^{31}$, cycloalkyl, carboxy, lower alkyloxy, lower alkyloxycarbonyl, hydroxy, halogen, cyano, amino optionally substituted with 1 or 2 lower alkyl, aminocarbonyl optionally substituted with 1 or 2 lower alkyl, tetrazolyl, aminocarbonyl optionally substituted with a substituent selected from the abovementioned Substituent group b, aryl optionally substituted with a substituent selected from the abovementioned Substituent group b, heteroaryl optionally substituted with a substituent selected from the abovementioned Substituent group b, a non-aromatic hetorocyclic group optionally substituted with a substituent selected from the abovementioned Substituent group b, or lower alkyloxyimino are preferable.

As a substituent of the "optionally substituted lower alkenyl" in $R^{29}$, halogen, cyano, carboxy, lower alkyloxycarbonyl, optionally substituted aminocarbonyl, tetrazolyl and the like are preferable.

As a substituent of the "optionally substituted lower alkyl" and "optionally substituted alkyl" in $R^2$, hydroxy, lower alkyloxy, a non-aromatic heterocyclic group optionally substituted with a substituent selected from the abovementioned Substituent group b and the like are preferable.

As a substituent of the "optionally substituted lower alkyl" in $R^3$, lower alkyloxy, lower alkylthio, cycloalkyl, carboxy, halogen, hydroxy, carbamoyl and mercapto are preferable.

As a substituent of the "optionally substituted lower alkyloxyimino" in $R^{31}$, halogen, hydroxy, aryl optionally substituted with lower alkyloxy, cyano, aminocarbonyl, amino optionally substituted with 1 or 2 lower alkyl or lower alkyloxycarbonyl and the like are preferable.

In the present specification, examples of the substituents in the "optionally substituted aryl", the "optionally substituted heteroaryl", the "optionally substituted aralkyl", and the "optionally substituted heteroarylalkyl" in $R^2$ and $R^3$ are lower alkyl optionally substituted with a substituent selected from abovementioned Substituent group b, cycloalkyl, lower alkenyl, lower alkynyl, hydroxy, lower alkyloxy, mercapto, lower alkylthio, lower alkenyloxy, halogen, nitro, cyano, carboxy, lower alkyloxycarbonyl, halo lower alkyl, halo lower alkyloxy, halo lower alkylthio, optionally substituted amino, optionally substituted aminocarbonyl, acyl, acyloxy, aryl optionally substituted with a substituent setected from abovementioned Substituent group b, hetroaryl optionally substituted with a substituent setected from abovementioned Substituent group b, a non-aromatic heterocyclic group optionally substituted with a substituent setected from abovementioned Substituent group b, aralkyl optionally substituted with a substituent setected from abovementioned Substituent group b, lower alkylsulfonyl, guanidino, an azo group, ureido optionally substituted with a substituent setected from abovementioned Substituent group b and the like. These can substitute at one or more of all possible positions.

As a substituent of the "optionally substituted aryl" in $R^2$, hydroxy, lower alkyloxy, halogen, and halo lower alkyl are preferable.

As a substitutent of the "optionally substituted aryl" in $R^3$, hydroxy is preferable.

As a substituent of the "optionally substituted heteroaryl" in $R^2$, hydroxy, lower alkyloxy, halogen, and halo lower alkyl are preferable.

As a substitutent of the "optionally substituted heteroaryl" in $R^3$, hydroxy and halogen are preferable.

As a substituent of the "optionally substituted aralkyl" in $R^2$, hydroxy, lower alkyloxy, halogen, halo lower alkyl, and nitro are preferable.

As a substituent of the "optionally substituted aralkyl" in $R^3$, hydroxy, halogen, nitro, lower alkyloxy, halo lower alkyl, and carboxy lower alkyloxy are preferable.

As a substituent of "optinally substituted heteroarylalkyl" in $R^2$, hydroxy, lower alkyloxy, halogen, and halo lower alkyl are preferable.

As a substituent of the "optionally substituted heteroarylalkyl" in $R^3$, a halogen, hydroxy, and carboxy are preferable.

In the present specification, as a substituent of "optinally substituted heteroaryl" and "optinally substituted non-aromatic heterocyclic group" in $R^{26}$ and $R^{28}$, halogen, lower alkyl, lower alkyloxy, hydroxy, lower alkyl substituted with hydroxy, halo lower alkyl, lower alkylsulfonyl, lower alkyloxycarbonyl and the like.

In the present specification, examples of the substituent of the "optionally substituted aryl", the "optionally substituted heteroaryl", the "optionally substituted non-aromatic heterocyclic group", the "optionally substituted aralkyl", and the

"optionally substituted heteroarylalkyl" in $R^{30}$ and $R^{31}$ are halogen, lower alkyl, lower alkyloxy, hydroxy, lower alkyl substituted with hydroxy, halo lower alkyl, lower alkylsulfonyl, lower alkyloxycarbonyl and the like.
In the present specification, examples of the substituent of the "optionally substituted aryl", the "optionally substituted heteroaryl", the "optionally substituted non-aromatic carbocyclic group" and the "optionally substituted non-aromatic heterocyclic group" in $R^{29}$ are halogen; optionally substituted lower alkyl; hydroxy; lower alkyl substituted with hydroxy; halo lower alkyl; lower alkyloxy; halo lower alkyloxy; optionally substituted amino; optionally substituted aminocarbonyl; optionally substituted aryl wherein the substituent is selected from the group consisting of halogen, lower alkyl, lower alkyloxy, hydroxy, lower alkyl substituted with hydroxy, halo lower alkyl, lower alkylsulfonyl, lower alkyloxycarbonyl, carboxy, aminocarbonyl, lower alkylaminocarbonyl and the like; optionally substituted aryloxy wherein the substituent is selected from the group consisting of halogen, lower alkyl, lower alkyloxy, hydroxy, lower alkyl substituted with hydroxy, halo lower alkyl, lower alkylsulfonyl, lower alkyloxycarbonyl, carboxy, aminocarbonyl, lower alkylaminocarbonyl and the like; optionally substituted aralkyl wherein the substituent is selected from the group consiting of halogen, lower alkyl, lower alkyloxy, hydroxy, lower alkyl substituted with hydroxy, halo lower alkyl, lower alkylsulfonyl, lower alkyloxycarbonyl, carboxy, aminocarbonyl, lower alkylaminocarbonyl and the like; optionally substituted aralkyloxy whrein the substituent is selected from the group consisting of halogen, lower alkyl, lower alkyloxy, hydroxy, lower alkyl substituted with hydroxy, halo lower alkyl, lower alkylsulfonyl, lower alkyloxycarbonyl, carboxy, aminocarbonyl, lower alkylaminocarbonyl and the like.
In the present specification, as a substituent of the "optinally substituted aryloxy" in $R^{30}$, halogen, lower alkyl, lower alkyloxy, hydroxy, lower alkyl substituted with hydroxy, halo lower alkyl, lower alkylsulfonyl, lower alkyloxycarbonyl and the like.
In the present specification, examples of a substituent of the "optionally substituted aralkyloxy" in $R^{31}$, halogen, lower alkyl, lower alkyloxy, hydroxy, lower alkyl substituted with hydroxy, halo lower alkyl, lower alkylsulfonyl, lower alkyloxycarbonyl and the like.
As the present compound, particularly, a compound represented by the following A) to AB) is preferable.

A) A compound represented by the general formula (IV):

[Chemical formula 29]

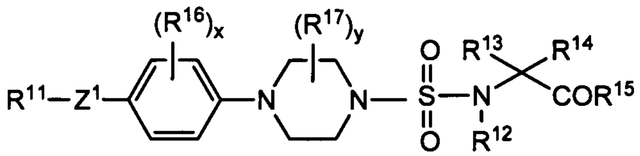

(IV)

wherein $R^{11}$ is

a) a group of the formula:

[Chemical formula 30]

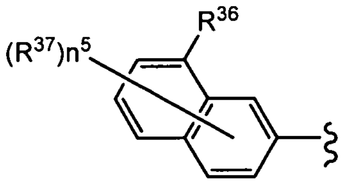
,
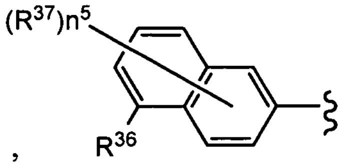
,
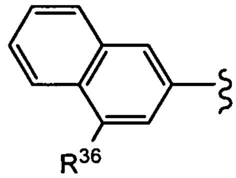

or
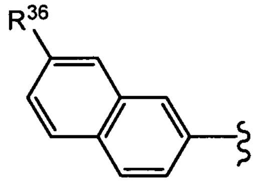

wherein $R^{36}$ and $R^{37}$ are each independently halogen, optionally substituted lower alkyl, optionally substituted lower alkenyl, optionally substituted lower alkyloxy, lower alkylthio, aralkylthio, optionally substituted amino, carboxy, hydroxy, cyano, lower alkyloxycarbonyl, optionally substituted aminocarbonyl, optionally substituted aminoxalyl, acyl, lower alkylsulfonyl, lower alkylsulfinyl, aralkylsulfonyl, aralkylsulfinyl, optionally substituted aminosulfonyl, optionally substituted heteroaryl or an optionally substituted non-aromatic heterocyclic group;
$n^5$ is an integer of 0 to 3, and
$R^{37}$ may be substituted on all substitutable atoms on the ring;
b) a group of the formula:

[Chemical formula 31]

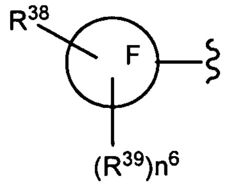

wherein F ring is benzene, pyridine, 2-pyridone, thiazole, tetrahydrothienopyrimidine or oxazole;
$R^{38}$ is halogen, acyl, optionally substituted lower alkyl, optionally substituted lower alkyloxy, optionally substituted lower alkenyl, optionally substituted aryl, optionally substituted heteroaryl, an optionally substituted non-aromatic carbocyclic group, an optionally substituted non-aromatic heterocyclic group, optionally substituted aminocarbonyl or optionally substituted amino;
$R^{39}$ is each independently halogen, hydroxy, nitro, optionally substituted lower alkyl, optionally substituted lower alkenyl, optionally substituted lower alkyloxy, optionally substituted aryloxy, optionally substituted aralkyl, optionally substituted heteroarylalkyl, lower alkylthio, aralkylthio, optionally substituted amino, carboxy, cyano, lower alkyloxycarbonyl, optionally substituted aminocarbonyl, optionally substituted aminoxalyl, acyl, lower alkylsulfonyl, lower alkylsulfinyl, aralkylsulfonyl, aralkylsulfinyl, optionally substituted aminosulfonyl, optionally substituted aryl, optionally substituted heteroaryl or an optionally substituted non-aromatic heterocyclic group;
$n^6$ is an integer of 0 to 2, and
$R^{38}$ and $R^{39}$ may be substituted on all substitutable atoms on the ring, or
c) a group of the formula:

[Chemical formula 32]

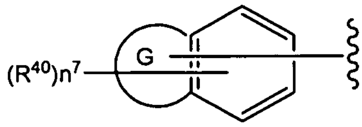

wherein G ring is a non-aromatic carbocyclic ring, a non-aromatic heterocyclic ring or an aromatic heterocyclic ring;
$R^{40}$ is each independently halogen, optionally substituted lower alkyl, optionally substituted lower alkenyl, optionally substituted lower alkyloxy, lower alkylthio, aralkylthio, optionally substituted amino, carboxy, cyano, oxo, lower alkyloxycarbonyl, lower alkyloxy lower alkylcarbonyl, optionally substituted aminocarbonyl, optionally substituted aminoxalyl, acyl, lower alkylsulfonyl, lower alkylsulfinyl, aralkylsulfonyl, aralkylsulfinyl, optionally substituted aminosulfonyl, imino, hydroxyimino, lower alkyloxyimino, optionally substituted heteroaryl, optionally substituted aralkyl, optionally substituted heteroarylalkyl, optionally substituted aralkyloxy, optionally substituted aryl or an optionally substituted non-aromatic heterocyclic group;
$n^7$ is an integer of 0 to 4, and
all substitutable atoms of the benzene ring and all substitutable atoms of G ring may be bonded to $Z^1$,
$R^{40}$ may be substituted on all substitutable atoms of the benzene ring and and all substitutable atoms of the G ring provided that when $n^7$ is an integer of 0, G ring is not unsubstituted indole,
$Z^1$ is $-C(R^8)(R^9)-C(R^8)(R^9)-$, $-C(R^8)=C(R^9)-$, $-C\equiv C-$, $-O-C(R^8)(R^9)-$, $C(R^8)(R^9)-O-$, $-S-C(R^8)(R^9)-$, $-C(R^8)(R^9)-S-$, $-SO-C(R^8)(R^9)-$, $-C(R^8)(R^9)-SO-$, $-SO_2-C(R^8)(R^9)-$, $-C(R^8)(R^9)-SO_2-$, $-N(R^{10})-C(R^8)(R^9)-$, $-C(R^8)(R^9)-N(R^{10})-$, $N(R^{10})-C(=O)-$, $-C(=O)-N(R^{10})-$, $-N(R^{10})-SO_2-$, $-SO_2-N(R^{10})-$, $-C(R^8)(R^9)-C(=O)-$, $-C(=O)-C(R^8)(R^9)-$, or $-C(=O)C(=O)-$, wherein $R^8$, $R^9$ and $R^{10}$ are each independently a hydrogen atom or lower alkyl;
$R^{12}$ is a hydrogen atom or lower alkyl;
$R^{13}$ is a hydrogen atom, methyl, ethyl, n-propyl, isopropyl, n-butyl, s-butyl, isobutyl, tert-butyl, hydroxymethyl, 1-hydroxyethyl, 2-hydroxyethyl, carboxymethyl, carboxyethyl, methyloxymethyl, methylthiomethyl, 2-methylthioethyl, phenyl, 4-hydroxyphenyl, 4-fluorophenyl, 4-methyloxyphenyl, benzyl, 4-hydroxybenzyl, 4-fluorobenzyl, 4-chlorobenzyl, 4-methoxybenzyl, indol-3-ylmethyl, 1-carboxymethylindol-3-ylmethyl, thiazol-4-ylmethyl, carboxymethyloxybenzyl, or cyclopropylmethyl,
$R^{14}$ is a hydrogen atom; or
$R^{13}$ and $R^{14}$ may be taken together with an adjacent carbon to form the ring represented by the formula:

[Chemical formula 33]

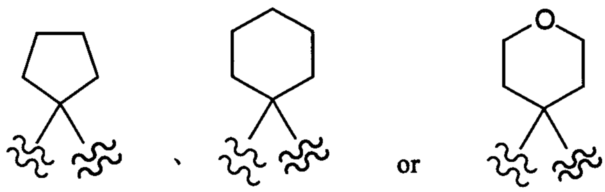

$R^{15}$ is hydroxy or lower alkyloxy;
$R^{16}$ and $R^{17}$ are each independently halogen, lower alkyl, lower alkyloxy, or hydroxy;
x and y are each independently an integer of 0 to 3,
an optically active isomer, a pharmaceutically acceptable salt, or a solvate thereof.

B) A compound represented by the general formula (V):

[Chemical formula 34]

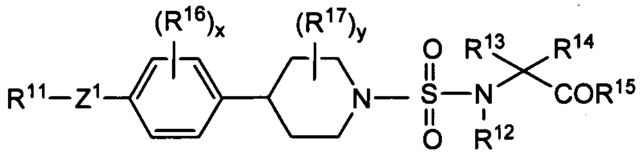 (V)

wherein, $R^{11}$, $Z^1$, $R^{12}$, $R^{13}$, $R^{14}$, $R^{15}$, $R^{16}$, $R^{17}$, x, and y are as defined in A), an optically active isomer, a pharmaceutically acceptable salt or a solvate thereof.

C) A compound represented by the general formula (VI):

[Chemical formula 35]

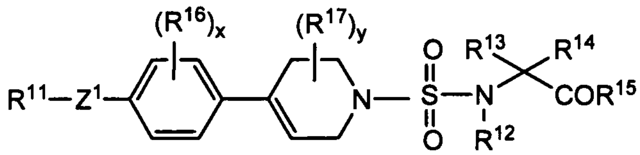 (VI)

wherein $R^{11}$, $Z^1$, $R^{12}$, $R^{13}$, $R^{14}$, $R^{15}$, $R^{16}$, $R^{17}$, x, and y are as defined in A), an optically active isomer, a pharmaceutically acceptable salt, or a solvate thereof.

D) A compound represented by the general formula (VII):

[Chemical formula 36]

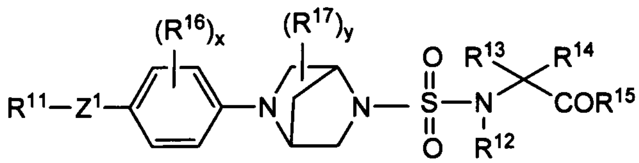 (VII)

wherein $R^{11}$, $Z^1$, $R^{12}$, $R^{13}$, $R^{14}$, $R^{15}$, $R^{16}$, $R^{17}$, x, and y are as defined in A), an optically active isomer, a pharmaceutically acceptable salt, or a solvate thereof.

E) A compound represented by the general formula (VIII):

[Chemical formula 37]

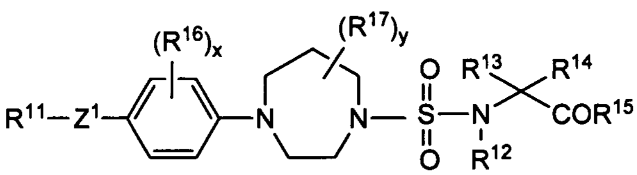 (VIII)

wherein $R^{11}$, $Z^1$, $R^{12}$, $R^{13}$, $R^{14}$, $R^{15}$, $R^{16}$, $R^{17}$, x, and y are as defined in A), an optically active isomer, a pharmaceutically acceptable salt, or a solvate thereof.

F) A compound represented by the general formula (IX):

[Chemical formula 38]

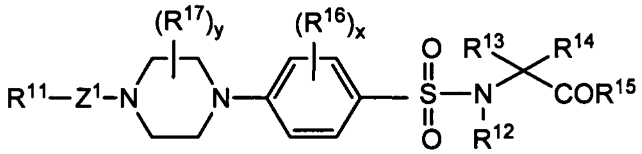

(IX)

wherein $R^{11}$, $Z^1$, $R^{12}$, $R^{13}$, $R^{14}$, $R^{15}$, $R^{16}$, $R^{17}$, x, and y are as defined in A), an optically active isomer, a pharmaceutically acceptable salt, or a solvate thereof.

G) A comound represented by the general formula (X):

[Chemical formula 39]

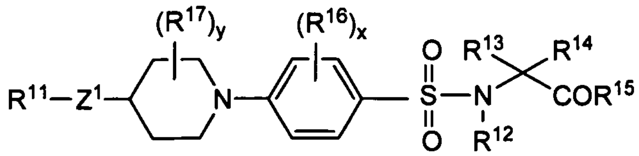

(X)

wherein $R^{11}$, $Z^1$, $R^{12}$, $R^{13}$, $R^{14}$, $R^{15}$, $R^{16}$, $R^{17}$, x, and y are as defined in A), an optically active isomer, a pharmaceutically acceptable salt, or a solvate thereof.

H) A compound represented by the general formula (XI):

[Chemical formula 40]

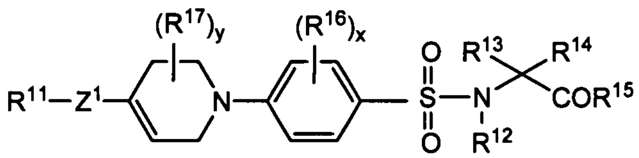

(XI)

wherein $R^{11}$, $Z^1$, $R^{12}$, $R^{13}$, $R^{14}$, $R^{15}$, $R^{16}$, $R^{17}$, x and y are as defined in A), an optically active isomer, a pharmaceutically acceptable salt, or a solvate thereof.

I) A compound represented by the general formula (XII):

[Chemical formula 41]

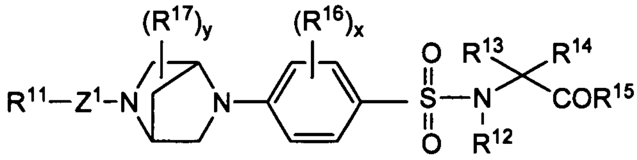

(XII)

wherein $R^{11}$, $Z^1$, $R^{12}$, $R^{13}$, $R^{14}$, $R^{15}$, $R^{16}$, $R^{17}$, x and y are as defined in A), an optically active isomer, a pharmaceutically acceptable salt, or a solvate thereof.

J) A compound represented by the general formula (XIII):

[Chemical formula 42]

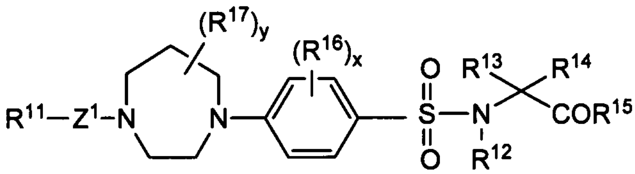

(XIII)

wherein $R^{11}$, $Z^1$, $R^{12}$, $R^{13}$, $R^{14}$, $R^{15}$, $R^{16}$, $R^{17}$, x and y are as defined in A), an optically active isomer, a pharmaceutically acceptable salt, or a solvate thereof.

K) The compound according to any one of A) to J), wherein $R^{11}$ is a group represented by the formula:

[Chemical formula 43]

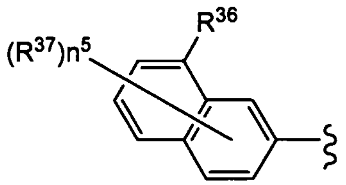

,

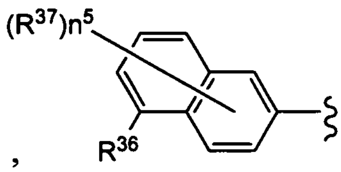

,

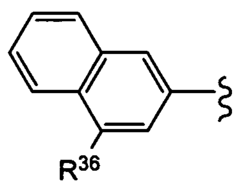

or

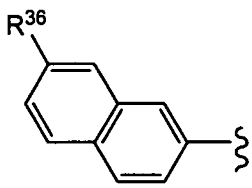

wherein $R^{36}$, $R^{37}$ and $n^5$ are as defined in A),
an optically active isomer, a pharmaceutically acceptable salt, or a solvate thereof.

L) The compound accoring to any one of A) to K), wherein $R^{11}$ is a group represented by the formula:

[Chemical formula 44]

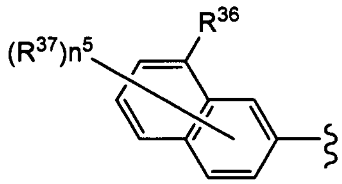

or

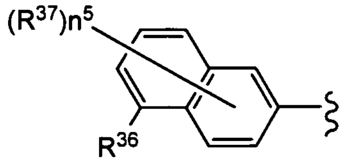

wherein $R^{36}$, $R^{37}$ and $n^5$ are as defined in A), an optically active isomer, a pharmaceutically acceptable salt, or a solvate thereof.

M) The compound according to any one of A) to L), wherein $R^{11}$ is a group represented by the formula:

[Chemical formula 45]

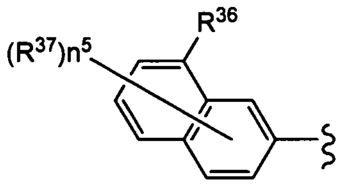

wherein $R^{36}$, $R^{37}$ and $n^5$ are as defined in A), an optically active isomer, a pharmaceutically acceptable salt, or a solvate thereof.

N) The compound accoring to any one of A) to L), wherein $R^{11}$ is a group represented by the formula:

[Chemical formula 46]

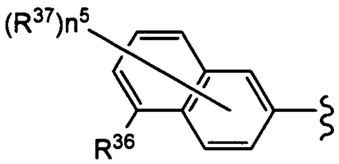

wherein $R^{36}$, $R^{37}$ and $n^5$ are as defined in A), an optically active isomer, a pharmaceutically acceptable salt, or a solvate thereof.

O) The compound according to any one of A) to J), wherein $R^{11}$ is a group represented by the formula:

[Chemical formula 47]

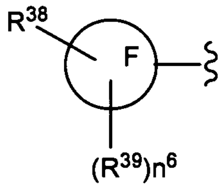

wherein $R^{38}$, $R^{39}$, $n^6$ and F ring are as defined in A), an optically active isomer, a pharmaceutically acceptable salt, or a solvate thereof.

O') The compound according to any one of A) to J), wherein $R^{11}$ is a group represented by the formula:

[Chemical formula 48]

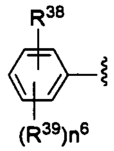 , 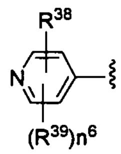 , 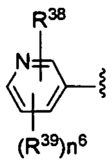 , 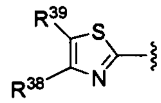 , 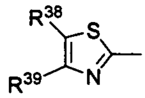 ,

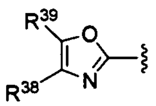 , 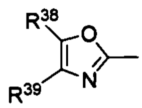 , 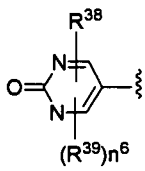 or 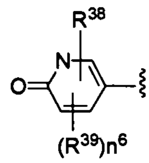

wherein $R^{38}$, $R^{39}$, and $n^6$ are as defined in A), an optically active isomer, a pharmaceutically acceptable salt, or a solvate thereof.

P) The compound according to any one of A) to J), wherein $R^{11}$ is a group represented by the formula:

[Chemical formula 49]

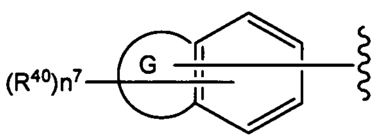

wherein $R^{40}$, $n^7$ and G ring are as defined in A), an optically active isomer, a pharmaceutically acceptable salt, or a solvate thereof.

P') The compound accoring to any one of A) to J), wherein $R^{11}$ is a group represented by the formula:

[Chemical formula 50]

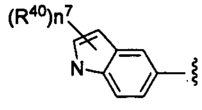,
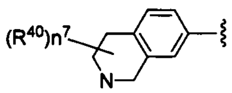,
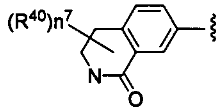,
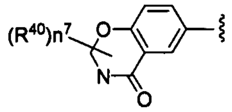

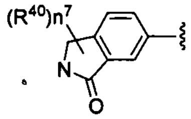,
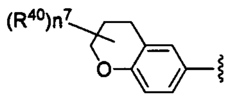,
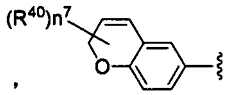,
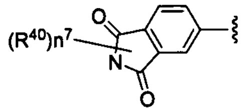

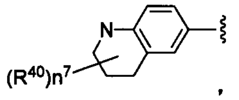,
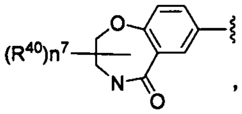,
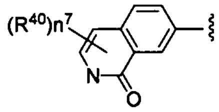,
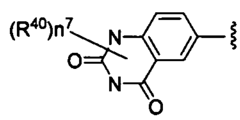

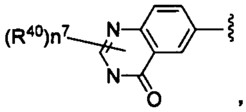,
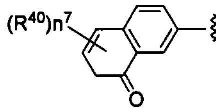,
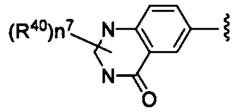,
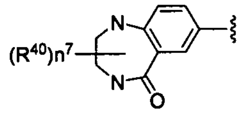

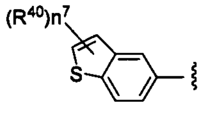 or 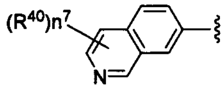

wherein $R^{40}$ and $n^7$ are as defined in A), provided that $R^{40}$ is not oxo, an optically active isomer, a pharmaceutically acceptable salt, or a solvate thereof.

Q) The compound according to any one of A) to P, O') or P'), wherein $R^{36}$, $R^{37}$, $R^{38}$, $R^{39}$ and/or $R^{40}$ are each independently a substituent selected from Substituent group d, an optically active isomer, a pharmaceutically acceptable salt or a solvate thereof.
Substituent group d : halogen, hydroxy, nitro, lower alkyl optionally substituted with a substituent selected from Substituent group e, lower alkenyl optionally substituted with a substituent selected from Substituent group e, lower alkyloxy optionally substituted with a substituent selected from Substituent group e, lower alkyloxy lower alkylcarbonyl optionally substituted with a substituent selected from Substituent group e, lower alkylthio, aralkylthio, amino optionally substituted with 1 or 2 substituent(s) selected from Substituent group f, carboxy, cyano, lower alkyloxycarbonyl, aminocarbonyl optionally substituted with 1 or 2 substituent(s) selected from Substituent group f, aminoxalyl optionally substituted with 1 or 2 substituent(s) selected from Substituent group f, acyl, oxo, thioxo, imino, hydroxyimino, lower alkyloxyimino optionally substituted with a substituent selected from Substituent group e, lower alkylsulfonyl, lower alkylsulfinyl, aralkylsulfonyl, aralkylsulfinyl, hydrazino optionally substituted with a substituent selected from Substituent group k, aminosulfonyl optionally substituted with 1 or 2 substituent(s) selected from Substituent group f, aralkyl optionally substituted with a substituent selected from Substituent group g, heteroarylalkyl optionally substituted with a substituent selected from Substituent group g, aralkyloxy optionally substituted with a substituent selected from Substituent group g, aryl optionally substituted with a substituent selected from Substituent group g, aryloxy optionally substituted with a substituent selected from Substituent group g, heteroaryl optionally substituted with a substituent selected from Substituent group g, a non-aromatic carbocyclic group optionally substituted with a substituent selected from Substituent group g and a non-aromatic heterocyclic group optionally substituted with a substituent selected from Substituent group g;
Substituent group e : cycloalkyl, carboxy, lower alkyloxy, hydroxy, halogen, cyano, aminocarbonyl optionally substituted with 1 or 2 lower alkyl, aryl optionally substituted with a substituent selected from Substituent group g, heteroaryl optionally substituted with a substituent selected from Substituent group g, a non-aromatic heterocyclic group optionally substituted with a substituent selected from Substituent group g and lower alkyloxyimino;
Substituent group f : aryl optionally substituted with a substituent selected from Substituent group h, aralkyl optionally substituted with a substituent selected from Substituent group h, heteroarylalkyl optionally substituted with a substituent selected from Substituent group h, aryloxy optionally substituted with a substituent selected from Substituent group h, arylamino optionally substituted with a substituent selected from Substituent group h, lower alkyloxy optionally substituted with a substituent selected from Substituent group h, lower alkyl optionally substituted with a substituent selected from Substituent group h, lower alkenyl optionally substituted with a substituent selected from Substituent group h, lowe alkynyl optionally substituted with a substituent selected from Substituent group h, lower alkyl substituted with cycloalkyl optionally substituted with a substituent selected from Substituent group h, lower alkyl substituted with a non-aromatic heterocyclic group optionally substituted with a substituent selected from Substituent group h, cycloalkyl optionally substituted with a substituent selected from Substituent group h and a non-aromatic heterocyclic group optionally substituted with a substituent selected from Substituent group h;

Substituent group g : lower alkyl; cycloalkyl; lower alkenyl; lower alkynyl; hydroxy; lower alkyloxy; mercapto; lower alkylthio; lower alkenyloxy; halogen; nitro; cyano; carboxy; lower alkyloxycarbonyl; halo loweralkyl; halo lower alkyloxy; halo lower alkylthio; amino optionally substituted with 1 or 2 lower alkyl; unsubstituted aminocarbonyl; arylaminocarbonyl optionally substituted with a substituent selected from Substituent group h; aralkylaminocarbonyl optionally substituted with a substituent selected from Substituent group h; heteroarylalkylaminocarbonyl optionally substituted with a substituent selected from Substituent group h; aryloxylaminocarbonyl optionally substituted with a substituent selected from Substituent group h; lower alkyloxyaminocarbonyl optionally substituted with a substituent selected from Substituent group h; lower alkylaminocarbonyl optionally substituted with a substituent selected from Substituent group h; lower alkenylaminocarbonyl optionally substituted with a substituent selected from Substituent group h; lower alkynylaminocarbonyl optionally substituted with a substituent selected from Substituent group h; lower alkylaminocarbonyl substituted with cycloalkyl optionally substituted with a substituent selected from Substituent group h; lower alkylaminocarbonyl substituted with a non-aromatic heterocyclic group optionally substituted with a substituent selected from Substituent group h; aminocarbonyl wherein nitrogen atom of the aminocarbonyl forms a non-aromatic heterocyclic ring; aminocarbonyl optionally substituted with 1 or 2 non-aromatic heterocyclic group substituted with a substituent selected from Substituent group h or aminocarbonyl optionally substituted with 1 or 2 cycloalkyl substituted with a substituent selected from Substituent group h; acyl; acylamino; acyloxy; aryloxy optionally substituted with a substituent selected from Substituent group h; aryl optionally substituted with a substituent selected from Substituent group h; heteroaryl optionally substituted with a substituent selected from Substituent group h; heteroaryloxy optionally substituted with a substituent selected from Substituent group h; a non-aromatic heterocyclic group optionally substituted with a substituent selected from Substituent group h; aralkyl optionally substituted with a substituent selected from Substituent group h; aralkyloxy optionally substituted with a substituent selected from Substituent group h; lower alkyksulfonyl; guanidino; an azo group; and ureido;

Substituent group k : optionally substituted lower alkyl (Examples of the substituent are halogen, lower alkyloxy, hydroxy, lower alkylsulfonyl, lower alkyloxycarbonyl, carboxy, aminocarbonyl, lower alkylaminocarbonyl and the like), optionally substituted arylhydrazino (Examples of the substituent are halogen, lower alkyloxy, hydroxy, lower alkylsulfonyl, lower alkyloxycarbonyl, carboxy, aminocarbonyl, lower alkylaminocarbonyl and the like), optionally substituted heteroarylhydrazino (Examples of the substituent are halogen, lower alkyloxy, hydroxy, lower alkylsulfonyl, lower alkyloxycarbonyl, carboxy, aminocarbonyl, lower alkylaminocarbonyl and the like);

Substituent group h : halogen, hydroxy, cyano, acyl, lower alkyloxy, lower alkyloxycarbonyl, lower alkylthio, lower alkyl substituted with lower alkyloxy, lower alkylsulfonyl, aminosulfonyl, lower alkyl, halo lower alkyl, halo lower alkyloxy, halo lower alkylthio, amino optionally substituted with 1 or 2 lower alkyl and/or lower alkylowycarbonyl, amino substituted with lower alkylcarbonyl, lower alkylaminocarbonyl optionally substituted with cyano, amino substituted with lower alkylaminocarbonyl, sulfonylamino substituted with lower alkyl, aminoalkyl optionally substituted with 1 or 2 lower lower alkyl and/or lower alkyloxycarbonyl, optionally substituted aryloxy (Examples of the substituent are halogen, lower alkyl, lower alkyloxy, hydroxy, lower alkyl substituted with hydroxy, halo lower alkyl, lower alkylsulfonyl, lower alkyloxycarbonyl, carboxy, aminocarbonyl, lower alkylaminocarbonyl and the like), cycloalkyl, a non-aromatic heterocyclic group, cycloalkyl in which hydrogen atoms on the ring are substituted with oxo, a non-aromatic heterocyclic group in which hydrogen atoms on the ring are substituted with oxo, lower alkylsulfonamide, optionally substituted heteroaryl (Examples of the substituent are halogen, lower alkyl, lower alkyloxy, hydroxy, lower alkyl substituted with hydroxy, halo lower alkyl, lower alkylsulfonyl, lower alkyloxycarbonyl, carboxy, aminocarbonyl, lower alkylaminocarbonyl and the like), oxo, amino optionally substituted with 1 or 2 optionally substituted aryl and/or lower alkyloxycarbonyl (Examples of the substituent are halogen, lower alkyl, lower alkyloxy, hydroxy, lower alkyl substituted with hydroxy, halo lower alkyl, lower alkylsulfonyl, lower alkyloxycarbonyl, carboxy, aminocarbonyl, lower alkylaminocarbonyl and the like); anda group represented by the formula:

[Chemical formula 51]

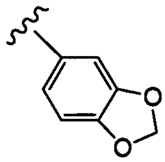 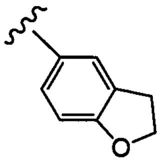 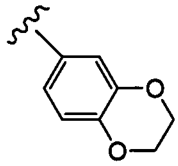 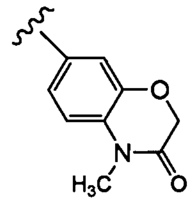

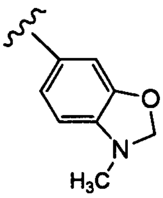 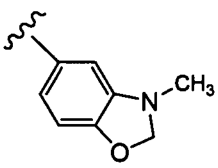 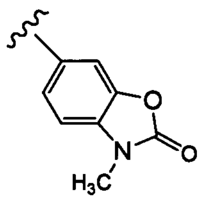 or 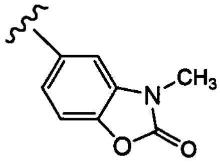

Q') The compound accoring to any one of A) to P, O') and P'), wherein $R^{36}$, $R^{37}$, $R^{38}$, $R^{39}$ and/or $R^{40}$ are each independently aminocarbonyl optionally substituted with a substituent selected from Substituent group m, an optically active isomer, a pharmaceutically acceptable salt or a solvate thereof.

Substituent group m : optionally substituted aminocarbonyl (Examples of the substituent are aryl optionally substituted with a substituent selected from Substituent group h, aralkyl optionally substituted with a substituent selected from Substituent group h, heteroarylalkyl optionally substituted with a substituent selected from Substituent group h, aryloxy optionally substituted with a substituent selected from Substituent group h, lower alkyloxy optionally substituted with a substituent selected from Substituent group h, lower alkyl optionally substituted with a substituent selected from Substituent group h, lower alkenyl optionally substituted with a substituent selected from Substituent group h, lower alkynyl optionally substituted with a substituent selected from Substituent group h, lower alkyl substituted with cycloalkyl substituted with a substituent selected from Substituent group h, lower alkyl substituted with a non-aromatic heterocyclic group substituted with a substituent selected from Substituent group h, a non-aromatic heterocyclic group substituted with a substituent selected from Substituent group h, cycloalkyl substituted with a substituent selected from Substituent group h and the like), oxo, optionally substituted lower alkyloxyimino (Examples of the substituent are cycloalkyl, carboxy, lower alkyloxy, hydroxy, halogen, cyano, aminocarbonyl optionally substituted with 1 or 2 lower alkyl, aryl optionally substituted with a substituent selected from Substituent group g, heteroaryl optionally substituted with a substituent selected from Substituent group g, a non-aromatic heterocyclic group optionally substituted with a substituent selected from Substituent group g, lower alkyloxyimino and the like).

R) The compound according to any one of A) to Q), O'), P') and Q'), wherein $Z^1$ is $-CH_2$ $-CH_2-$, -CH=CH-, -C≡C-, $-O-CH_2-$, $-CH_2-O-$, $-S-CH_2-$, $-CH_2-S-$, $-SO-CH_2-$, $-CH_2-SO-$, $-SO_2-CH_2-$, $-CH_2-SO_2-$, $-N(R^{10})-CH_2-$, $-CH_2-N(R^{10})-$, -NH-C(=O)-, -C(=O)-NH-, $-CH_2-C(=O)-$, $-C(=O)-CH_2-$, or -C(=O)C(=O)-, an optically active isomer, a pharmaceutically acceptable salt, or a solvate thereof.

T) The compound accoring to any one of A) to S), O'), P') and Q'), wherein $Z^1$ is $-CH_2-CH_2-$, -CH=CH- or -C=C-, an optically active isomer, a pharmaceutically acceptable salt, or a solvate thereof.

U) The compound according to any one of A) to T), O'), P') and Q'), wherein $Z^1$ is -C≡C-, an optically active isomer, a pharmaceutically acceptable salt, or a solvate thereof.

V) The compound according to any one of A) to U), O'), P') and Q'), wherein $R^{12}$ is a hydrogen atom, an optically active isomer, a pharmaceutically acceptable salt, or a solvate thereof.

W) The compound according to any one of A) to V), O'), P') and Q'), wherein $R^{13}$ is a hydrogen atom, methyl, ethyl, n-propyl, isopropyl, n-butyl, s-butyl, isobutyl, tert-butyl, hydroxymethyl, 1-hydroxyethyl, 2-hydroxyethyl, carboxymethyl, carboxyethyl, methyloxymethyl, methylthiomethyl, 2-methylthioethyl, phenyl, 4-hydroxyphenyl, 4-fluorophenyl, 4-methyloxyphenyl, benzyl, 4-hydroxybenzyl, 4-fluorobenzyl, 4-chlorobenzyl, 4-methoxybenzyl, indol-3-ylmethyl, 1-carboxymethylindol-3-ylmethyl, thiazol-4-ylmethyl, carboxymethyloxybenzyl, or cyclopropylmethyl, and $R^{14}$ is a hydrogen atom,

an optically active isomer, a pharmaceutically acceptable salt, or a solvate thereof.

X) The compound according to any one of A) to W), O'), P') and Q'), wherein $R^{13}$ is methyl, ethyl, n-propyl, isopropyl, n-butyl, s-butyl, isobutyl, tert-butyl, hydroxymethyl, 1-hydroxyethyl, 2-hydroxyethyl, carboxymethyl, carboxyethyl, methyloxymethyl, methylthiomethyl, 2-methylthioethyl, phenyl, 4-hydroxyphenyl, 4-fluorophenyl, 4-methyloxyphenyl, benzyl, 4-hydroxybenzyl, 4-fluorobenzyl, 4-chlorobenzyl, 4-methoxybenzyl, indol-3-ylmethyl, 1-carboxymethylindol-3-ylmethyl, thiazol-4-ylmethyl, carboxymethyloxybenzyl, or cyclopropylmethyl, and $R^{14}$ is a hydrogen atom, an optically active isomer, a pharmaceutically acceptable salt, or a solvate thereof.

Y) The compound according to any one of A) to V), O'), P') and Q'), wherein $R^{13}$ and $R^{14}$ are taken together with an adjacent carbon to form a ring represented by the formula:

[Chemical formula 52]

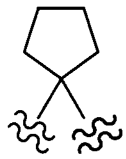 , 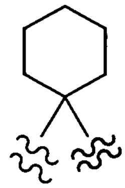 or 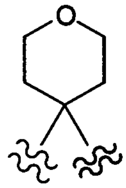

an optically active isomer, a pharmaceutically acceptable salt, or a solvate thereof.

Z) The compound according to any one of A) to Y), O'), P') and Q'), wherein $R^{15}$ is hydroxy, an optically active isomer, a pharmaceutically acceptable salt, or a solvate thereof.

AA) The compound according to any one of A), F), K) to Z), O'), P') and Q'), wherein the group represented by the formula:

[Chemical formula 53]

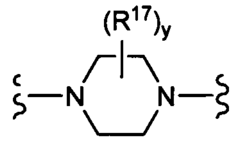

is the group represented by the formula:

[Chemical formula 54]

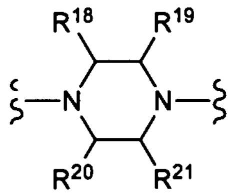

wherein in $R^{18}$, $R^{19}$, $R^{20}$, and $R^{21}$ are selected from the following i) to vi);

i) all of them are hydrogen atom,
ii) only $R^{18}$ is lower alkyl, and all of the rest are hydrogen atom,

iii) only $R^{19}$ is lower alkyl, and all of the rest are hydrogen atom,
iv) $R^{18}$ and $R^{20}$ are each independently lower alkyl, and all of the rest are hydrogen atom,
v) $R^{18}$ and $R^{21}$ are each independently lower alkyl, and all of the rest are hydrogen atoms,
vi) $R^{19}$ and $R^{21}$ are each independently lower alkyl, and all of the rest are hydrogen atom,

an optically active isomer, a pharmaceutically acceptable salt, or a solvate thereof.

AB) The compound described in any of B), G), K) to Z), O'), P') and Q'), wherein the group represented by the formula:

[Chemical formula 55]

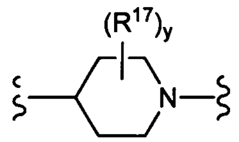

is the group represented by the formula:

[Chemical formula 56]

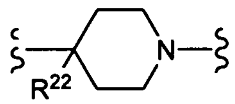

wherein $R^{22}$ is a hydrogen atom, halogen, hydroxy, or lower alkyloxy, an optically active isomer, a pharmaceutically acceptable salt, or a solvate thereof.

[Effect of the invention]

It was found that a compound of the present invention exhibits a specifically inhibiting activity for MMP-13 and is useful for treating and/or preventing disorders associated with MMP-13.
These derivatives can be utilized as an agent for treating or preventing a disease resulting from, or associated with MMP-13, particularly, osteoarthritis (OA). Also, the invented compounds have excellent profile as a medicament, such as relatively low protein binding rate, excellent solubility, good oral absorbability, and/or low toxicity.

[Best mode for carrying out the invention]

The present compound can be synthesized according to the method described in WO97/27174, WO00/46189 and the like, and the known method. As the methods for synthesizing the invented compounds, Method A and Method B are exemplified below.

(Method A)

[Chemical formula 57]

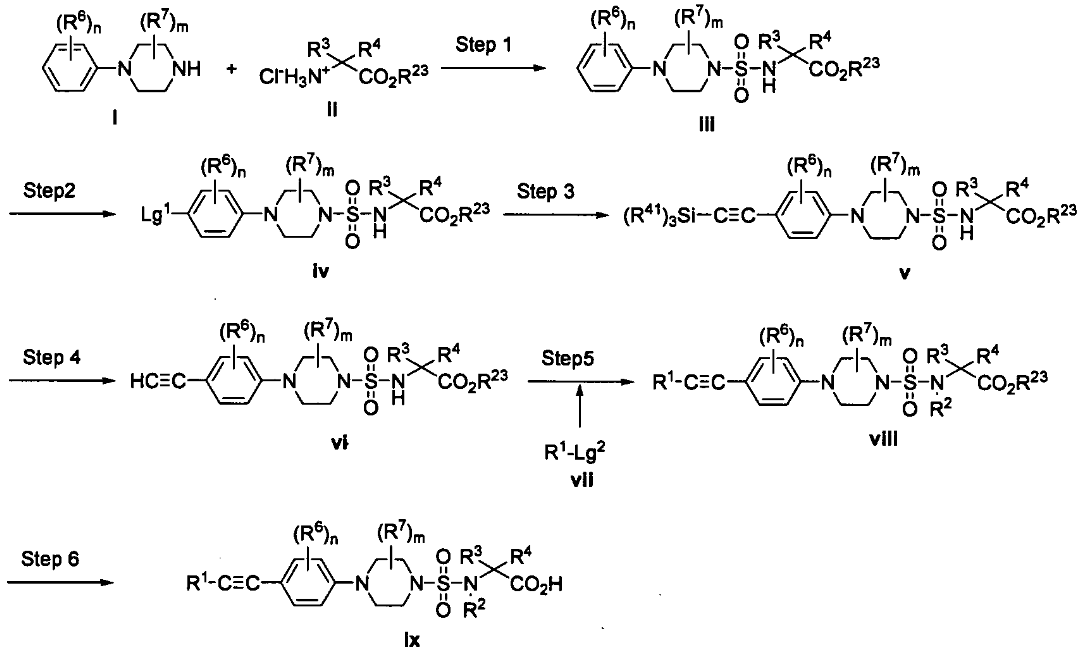


wherein $R^1$, $R^2$, $R^3$, $R^4$, $R^6$, $R^7$, m and n are as defined above; $R^{23}$ is a protecting group of carboxyl group; $R^{41}$ is lower alkyl, aryl or aralkyl and the like.; $Lg^1$ and $Lg^2$ are leaving groups in substitution reactions such as halogen, sulfonate and the like

(Step 1)

Some starting materials represented by the formula (i) are commercially available. The other compounds can be synthesized according to the method described in Tetrahedron Lett. 39 (1998) 617-620.

Some starting amino acids and their acid addition salts (e.g. hydrochloride, *p*-toluenesulfonate, trifluoroacetate) represented by the formula (II) are commertially available. The other amino acids and their acid addition salts can be synthesized according to the method for amino acid synthesis, described in Experimental Chemistry Course, vol.22, 4 edition (edited by The Chemical Society of Japan), or the method described in J. Med. Chem. 38, 1689-1700 (1995) Gary M. Ksander et. al. and the like.

A sulfamoyl halide can be obtained by reacting a halo alcohol derivative such as 2-chloroethanol, 2-bromoethanol, 2-chloropropanol, 2-chloro-1-methylethanol and the like, with a halo sulfonyl isocyanate derivative such as chlorosulfonyl isocyanate and the like at -20 °C to 50 °C, preferably at 0 °C to room temperature, for 0.1 to 10 hours, preferably for 1 to 5 hours, in a solvent such as acetonitrile, tetrahydrofuran, diethyl ether, toluene an the like.

A sulfamoyl oxazolinone derivative can be obtained by reacting the above sulfamoyl halide with the amino acid or its acid addition salt represented by the formula (II) in the presence of a base such as N-methylmorpholine, pyridine, triethylamine, potassium carbonate and sodium bicarbonate and the like at -20 °C to 50 °C, preferably at 0 °C to room temperature, for 1 to 48 hours, preferably for 3 to 24 hours.

The desired compound represented by the formula (III) can be obtained by reacting the above sulfamoyl oxazolinone derivative with the compound represented by the formula (i) in the presence of a silylation agent such as chlorotrimethylsilane etc. at 0 °C to 50 °C, preferably at room temparature to 100 °C, for 1 to 24 hours, preferably for 2 to 10 hours.

(Step 2)

The compound represented by the formula (iv), where $Lg^1$ is iodine, can be obtained by reacting the compound represented by the formula (III) with iodine in the presence of an inorganic base such as sodium bicarbonate and the like, at -20 °C to 50 °C, preferably at 0 °C to room temperature, for 1 to 10 hours, preferably for 2 to 5 hours, in a solvent such as methylene chloride, diethyl ether, n-hexane and the like.

Alternatively the compound can be synthesized according to the known methods for halogenation of aryls,

described in New Experimental Chemistry Course, vol.14, 2 edition (edited by The Chemical Society of Japan) and the like.

(Step 3)

By reacting with an acetylene derivative protected by an silyl group such as trimethylsilylacetylene and the like, in the presence of a palladium catalyst (e.g. $Pd(Ph_3P)_2Cl_2$ etc.), a divalent copper reagent (e.g. CuI etc.), and an organic base (e.g. triethylamine, diisopropylethylamine etc.), in a solvent such as dimethylformamide, toluene, xylene, benzene, tetrahydrofuran and the like, the compound (iv) can be converted into the desired compound represented by the formula (v) (Sonogashira reaction).

The reaction temperature is from room temperature to 100°C, preferably from room temperature to 80°C, and the reaction time is from 3 to 30 hours, preferably from 10 to 20 hours.

(Step 4)

The desired compound represented by the formula (vi) can be synthesized by removing the silyl group, which is a protecting group for acetylene groups of the compound (v), according to a conventional method.

(Step 5)

By reacting with the compound represented by the formula (vII), by the same method as the Step 3 (Sonogashira reaction), the compound (vi) can be converted into the desired compound (vIII).

When an optionally substituted aryl halide derivatives or an optionally substituted heteroaryl halide derivative has a substituent which is harmful to the present reaction, the substituent can be protected in advance by the method described in Protective Groups in Organic Synthesis, Theodora W Green (John Wiley & Sons), and the protecting group can be removed at a preferable stage.

When $R^2$ is not a hydrogen atom, the desired N-$R^2$ analogs can be obtained by adding an alkyl halide (e.g. methyl iodide, ethyl iodide etc.) or an aralkyl halide (e.g. benzyl chloride, benzyl bromide etc.) to the compound obtained by abovementioned reaction, at ice-bath temperature to 80°C, preferably at ice-bath temperature to room temperature, in a solvent such as dimethylformamide, tetrahydrofuran, dioxane and the like, and by stirring the mixture for 3 to 30 hours, preferably 10 to 20 hours.

Some compounds represented by the formula (vII) are commertially available. The other compounds can be prepared from a commercially available starting material according to the same method as step 2.

(Step 6)

The compound represented by the general formula (I) can be synthesized by deprotecting the compound (vIII) according to a conventional method.

(B method)

[Chemical formula 58]

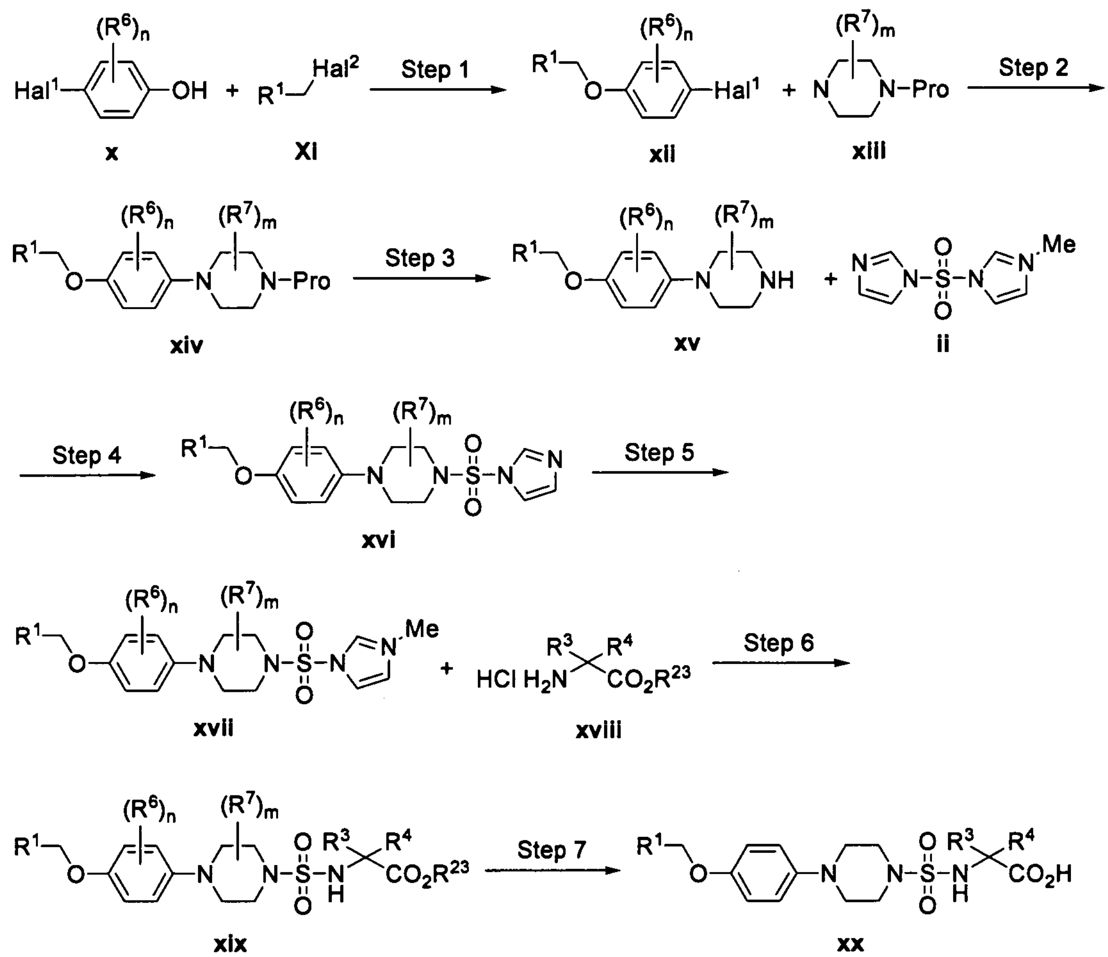


x XI xii xiii xiv xv ii xvi xvii xviii xix xx

(wherein $R^1$, $R^2$, $R^3$, $R^4$, $R^6$, $R^7$, $R^{23}$, m and n are as defined above; $Lg^3$ is a leaving group in substitution reaction, such as a halogen, sulfonate etc.)

(Step 1)

Some starting materials represented by the formula (x) are commercially available. The other compounds can be synthesized according to the method described in Tetrahedron Lett. 39 (1998) 617-620.

This step can be conducted by the similar manner as Step 1 of the Method A.

(Step 2)

The desired compound represented by the formula (xIII) can be obtained by reacting the compound represented by the formula (xi) with the compound represented by the formula (xII), under the anhydrous atmosphere such as nitrogen gas, argon gas and the like, in the presence of a palladium catalyst such as tris (benzylideneacetone) dipalladium (0) and the like, a phosphine reagent such as triphenylphosphine and tri-tert-butylphosphine and the like, and a base such as triethylamine and N, N-dicyclohexylmethylamine and the like, in a solvent such as 1, 4-dioxane, tetrahydrofuran, diethyl ether, N, N-dimethylformamide, dimethyl sulfoxide and toluene and the like.

The reaction temperature is from 0°C to 100°C, preferably from room temperature to 80°C, and the reaction time is from 0.5 to 10 hours, preferably from 1 to 5 hours.

Some compounds represented by the formula (xII) are commercially available. The other compounds can be synthesized from the commercially available starting materials according to the known method

(Step 3)

This step can be conducted by the similar manner as Step 6 of the Method A.

The therapeutic effect of the present compound regarding osteoarthritis and adjuvant arthritis can be confirmed by the following method.

A) Osteoarthritis

Using a 12 week-old female Hartley guinea pig (Charles River Laboratories Japan, Inc.), a right knee joint meniscus and an inside collateral ligament are excised according to the method of Meacock et al. (J. Exp. Path. 71: 279-293, 1990), by which a guinea pig knee osteoarthritis model is prepared. From the following date of operation, a 0.5% methylcellulose solution as a solvent, or a 30mg/kg test compound solution is orally administered once a day every day for 10 days.

On the following day of the final administration date, a right os femoris distal portion and a cnemis proximal part are taken. After a joint cartilage surface is stained with India ink (manufactured by Kuretake Co, Ltd.), a cartilage surface is photographed with a digital camera (manufactured by Nikon Corporation). Assessment of compounds is performed by scoring a degree of an OA lesion on a joint cartilage surface using a photographed image by a blind method.

In addition, similarly, the effect can be also confirmed by oral administration every day for 6 weeks using a 12 week-old female NZW rabbit (KITAYAMA LABES Co., Ltd.) or a 12 week-old female SD rat (CLEA Japan, Inc.).

B) Adjuvant arthritis

Using a 7-week old female Lewis rat (manufactured by Charles River Laboratories Japan, Inc.), and according to the method of Fletcher et al. (J. Pharmacol. Exp. Ther. 284(2): 714-721), dead mycobacterium butyricum is administered to a right posterior limb footpad to prepare a rat adjuvant arthritis model. From the following day of administration, a 0.5% methylcellulose solution which is a solvent, or a 30mg/kg test compound solution is orally administered once a day. After 21 days from administration, a degree of posterior limb bloating is measured using a volume recording meter (manufactured by Shionogi & Co., Ltd.), a spleen and a thymus are taken, and their weight are measured. In addition, both posterior limbs are subjected to X-ray photographing (manufactured by OHMIC Co., Ltd.). Determination of X-ray image is performed by a blind method using a score from 0 (normal) to 3 (bone/cartilage complete destruction), depending on a degree of joint destruction.

Herein, the "solvate" includes, for example, a solvate with an organic solvent, a hydrate and the like. When a hydrate is formed, an arbitrary number of water molecules may be coordinated.

The "present compound" also includes a pharmaceutically acceptable salt, and a solvate thereof. For example they are salts with alkali metals (lithium, sodium, potassium etc.), alkaline earth metals (magnesium, calcium etc.), ammonium, organic bases and amino acids, and salts with inorganic acids (hydrochloric acid, hydrobromic acid, phosphoric acid, sulfuric acid etc.), and organic acids (acetic acid, citric acid, maleic acid, fumaric acid, benzenesulfonic acid, p-toluenesulfonic acid etc.). These salts can be formed by the method which is conventionally performed.

And, the present compound is not limited to a specified isomer, and includes all possible isomers (e.g. optically active body) or racemates.

The present compound does not exhibit the inhibiting activity on MMP-2 and MMP-9 compared with MMP-13, but exhibits the selective and excellent MMP-13 inhibiting activity as described in Test Example described later.

The present compound can be used as a remedy for osteoarthritis, arthritis rheumatism, adjuvant arthritis, periodontitis, cirrhosis, osteoporosis, bone resorption, gingivitis, large intestine-rectum cancer, squamous epithelial cancer, epithelial cell cancer, prostate cancer, ovary cancer, tongue cancer, endometrium cancer, or stomach cancer.

When the present compound is administered to a human for the purpose of treating the aforementioned diseases, it can be orally administered as powders, granules, tablets, capsules, pills, solutions or the like, or can be parenterally administered as injectables, suppositories, transdermally absorbing agents, inhalants or the like. And, pharmaceutical additives suitable for a dosage form, such as excipients, binders, wetting agents, disintegrating agents, lubricants and the like, can be, if necessary, mixed into an effective amount of the present compound to obtain pharmaceutical preparations. In the case of injectables, they are sterilization-treated together with suitable carriers to obtain preparations.

A dose is different depending on condition of a patient, an administration route, an age and a weight of a patient and, when orally administered to an adult, the dose is usually 0.1 to 100 mg/kg/day, preferably 1 to 20 mg/kg/day.

The present invention will be explained in more detail below by way of Examples and Test Examples, but the present invention is not limited to them.

Examples

In Examples, following abbreviations are used.

Me: methyl

Tf: trifluoromethanesulfonyl
Boc: tert-butoxycarbonyl
TMS: trimethylsilyl
MOM: methoxymethyl

[Example 1]

Synthesis of (R)-2-{4-[4-(3-benzylcarbamoylphenylethynyl)-phenyl]-piperazine-1-sulfonylamino}-proponic acid (I-1)

## [Chemical formula 59]

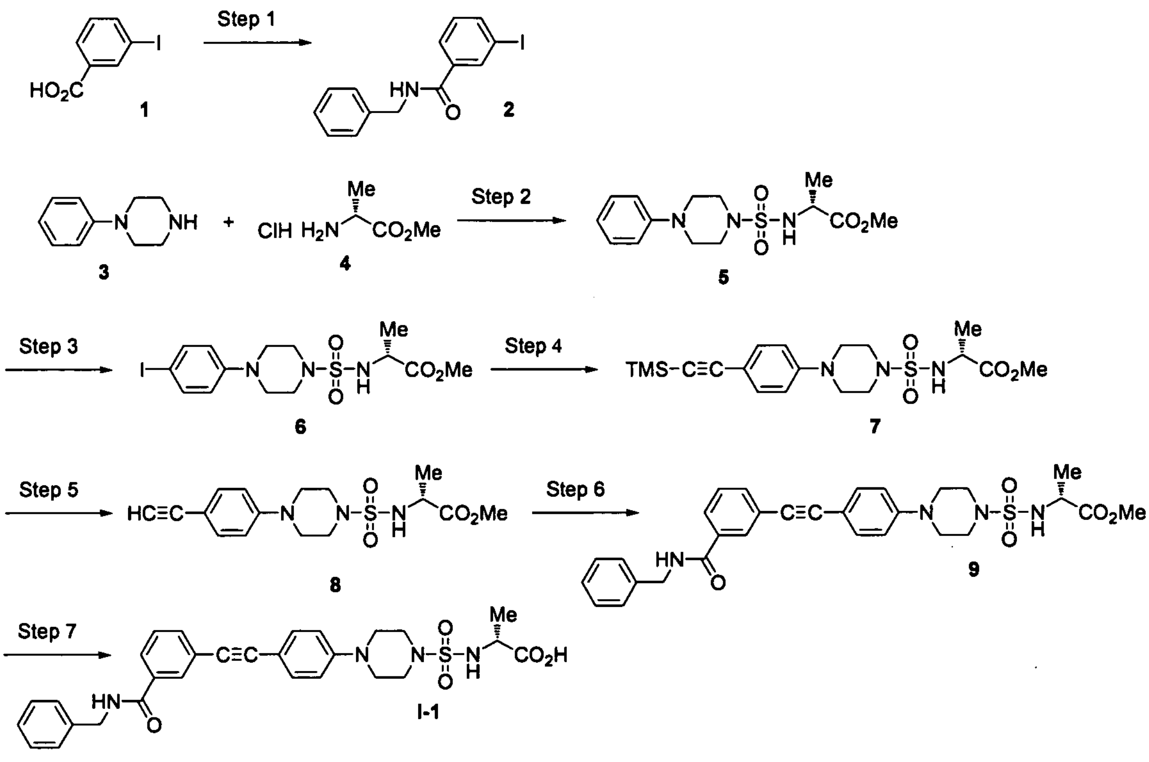


1, 2, 3, 4, 5, 6, 7, 8, 9, I-1

(Step 1)

To a solution of 3-iodobenzoic acid (1) (0.500 g, 2.02 mmol) in N, N-dimethylformamide (2 mL) were added 1-hydroxybenzotriazole (0.300 g, 2.22 mmol) and 1-ethyl-3-(3-dimethylaminopropyl) carbodiimide hydrochloride (0.580 g, 3.02 mmol) at room temperature, and the mixture was stirred for 15 minites. Next, benzylamine (0.440 mL, 4.03 mmol) was added to the reaction mixture , which was stirred for 6.5 hours. To the reaction mixture was added 5% citric acid aq., and the mixture was extracted with ethyl acetate. The organic layer was washed with saturated sodium bicarbonate aq., and saturated sodium chloride aq., dried over anhydrous sodium sulfate, and concentrated under reduced pressure. The residue was recrystallized from n-hexane/ethyl acetate to afford a colorless compound (2) (0.573 g, yield 84.3%).

(Step 2)

To a solution of 2-chloroethanol (2.47 mL, 37.0 mmol) in acetonitrile (40 mL) was added chlorosulfonyl isocyanate (3.18 mL, 36.5 mmol) under ice cooling, and the mixture was stirred at room temperature for 2 hours.Separately, to a solution of D-alanine methyl ester hydrochloride (5.00 g, 35.8 mmol) in acetonitrile (40 mL) was added N-methylmorpholine (19.7 mL, 179 mmol) under ice cooling, and the mixture was stirred for 3 hours.The former mixture was

added to the latter mixture, which was stirred at room temperature for 20 hours. Next, 1-phenylpiperazine (3) (5.60 g, 34.5 mmol) and chlorotrimethylsilane (2.30 mL, 18.1 mmol) were added to the reaction mixture, which was stirred at 80°C for 5 hours. The reaction mixture was poured into ice-water, neutralized with 5% citric acid aq., and extracted with ethyl acetate. The organic layer was washed with saturated sodium bicarbonate aq., and saturated sodium chloride aq., dried over anhydrous sodium sulfate, and concentrated under reduced pressure. The residue was subjected to silica gel chromatography, the fractions eluted with n-hexane/ethyl acetate = 1/2 were collected, and evaporated to dryness to give a colorless compound (5) (6.26 g, yield 55.4 %).

(Step 3)

To an aqueous solution (43 mL) of sodium bicarbonate (2.21 g, 26.3 mmol) were added methylene chloride (43 mL), the compound (5) (4.30 g, .13.1 mmol) and iodine(3.50 g, 13.8 mmol), and the mixture was stirred at room temperature for 3.5 hours. To the reaction mixture was added 1mol/L sodium thiosulfate aq. (10mL), and the mixture was extracted with chloroform. The organic layer was washed with water, dried over anhydrous sodium sulfate, and concentrated under reduced pressure. The residue was washed with n-hexane to give a colorless compound (6) (6.24 g) quantitatively.

(Step 4)

To a solution of the compound (6) (6.24 g, 13.8 mmol) in tetrahydrofuran (125 mL) was added trimethylsilylacetylene (2.92 mL, 20.7 mmol), and the mixture was degassed under nitrogen gas atmosphere. Then, to the mixture were added bis (triphenylphosphine) palladium (II) chloride (0.48 g, 0.68 mmol), copper (I) iodide (0.26 g, 1.37 mmol), and triethylamine (3.84 ml, 27.6 mmol), the mixture was degassed again under nitrogen gas atmosphere, and stirred at room temperature for 15 hours. The reaction mixture was poured into ice water, and this was neutralized with 5% citric acid aq., and extracted with ethyl acetate. The organic layer was washed with saturated sodium bicarbonate aq., and saturated sodium chloride aq., dried over anhydrous sodium sulfate, and concentrated under reduced pressure. The residue was subjected to silica gel chromatography, the fractions eluted with n-hexane/ethyl acetate = 3/1 were collected, and evaporated to dryness to give a pale brown compound (7) (4.32 g, yield 74.1%).

(Step 5)

To a solution of the compound (7) (1.80 g, 4.25 mmol) in methanol (18 mL) was added potassium carbonate (0.76 g, 5.50 mmol), and the mixture was stirred at room temperature for 1.5 hours. The reaction mixture was poured into ice water-5% citric acid aq., and the mixture was extracted with ethyl acetate. The organic layer was washed with water, dried over anhydrous sodium sulfate, and concentrated under reduced pressure. The residue was recrystallized from n-hexane/ethyl acetate to afford a pale yellow compound (8) (1.12 g, yield 75.2 %).

(Step 6)

A solution of the compound (2) (0.120 g, 0.356 mmol) and the compound (8) (0.125 g, 0.356 mmol) in N,N-dimethylformamide (1.2 mL) was degassed well, and the atmosphere was replaced with a nitrogen gas. Then, to the mixture were added bis(triphenylphosphine) palladium (II) chloride (0.013 g, 0.018 mmol), copper (I) iodide (0.007 g, 0.036 mmol), and triethylamine (0.099 ml, 0.71 mmol), this was degassed again under nitrogen gas atmosphere, and stirred at room temperature for 1 hours.

The reaction mixture was neutralized with 5% citric acid aq., and the mixture was extracted with ethyl acetate. The organic layer was washed with saturated sodium bicarbonate aq., and saturated sodium chloride aq., dried over anhydrous sodium sulfate, and concentrated under reduced pressure. The residue was subjected to silica gel chromatography, and the fractions eluted with n-hexane/ethyl acetate = 2/3 were collected, and evaporated to dryness to give a pale brown compound (9) (0.153 g, yield 76.7%).

(Step 7)

To a solution of the compound (9) (0.151 g, 0.269 mmol) in dimethyl sulfoxide (1.2 mL) was added 2 mol/L sodium hydroxide aq. (0.41 mL) at room temperature, and the mixture was stirred for 16 hours. The reaction mixture was poured into ice water-5% citric acid aq., and the mixture was extracted with ethyl acetate. The organic layer was washed with water, dried over anhydrous sodium sulfate, and concentrated under reduced pressure. The residue was recrystallized from n-hexane/ethyl acetate to give (R)-2-{4-[4-(3-carbamoylphenylethynyl)-phenyl]-piperazine-1-sulfonylamino}-proponic acid (I-1) (0.125 g, yield 85.0%).

$^1H$ NMR (DMSO-d6) δ: 1.30 (d, J=7.2 Hz, 3H), 3.11-3.19 (m, 4H), 3.30 (br s, 4H), 3.79-3.84 (m, 1H), 4.49 (d, J=6.0 Hz, 2H), 7.00 (d, J=8.8 Hz, 2H), 7.23-7.56 (m, 8H), 7.66 (d, J=8.0 Hz, 1H), 7.87-7.94 (m, 2H), 8.03 (s, 1H), 9.16 (t, J=6.4 Hz, 1H), 12.80 (br s, 1H).

[Example 2]

Synthesis of (R)-2-(4-{4-[(E)-2-(3-benzylcarbamoylphenyl)-vinyl]-phenyl}-piperazine-1-sulfonylamino)-proponic acid(I-2)

[Chemical formula 60]

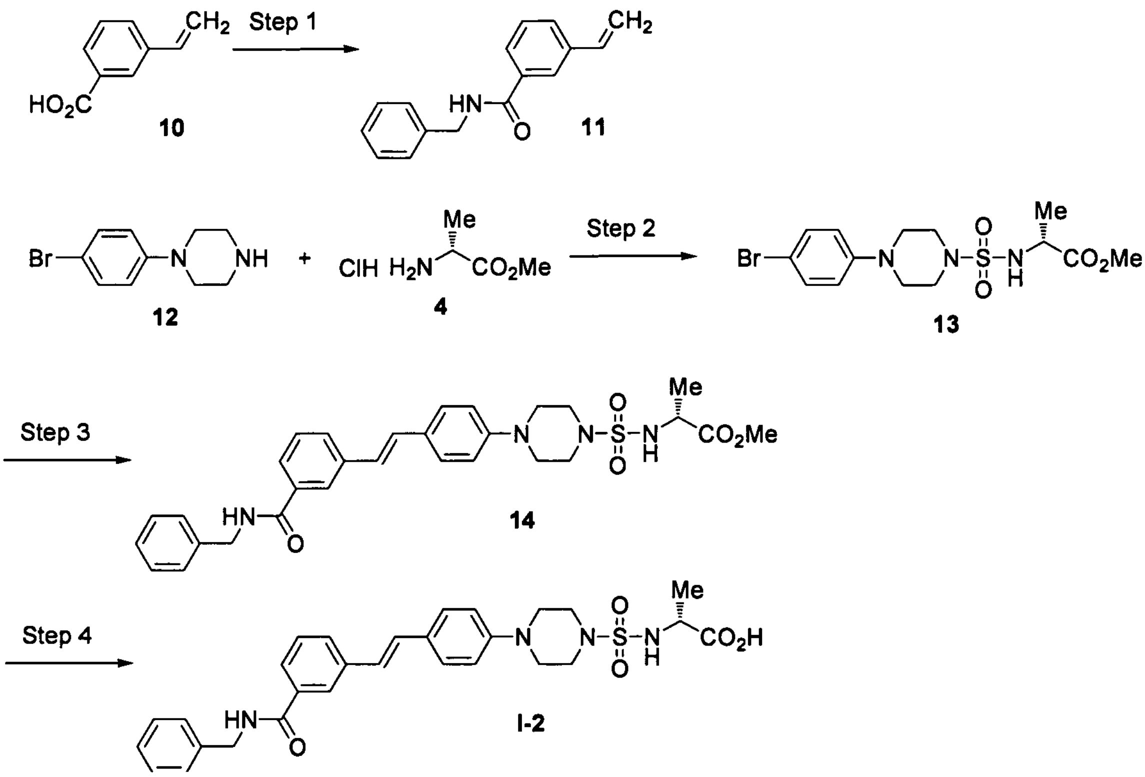


10 11 12 4 13 14 I-2

(Step 1)

To a solution of 3-vinyl benzoic acid (10) (0.200 g, 1.35 mmol) in N, N-dimethylformamide (2mL) were added benzylamine (0.177 mL, 1.62 mmol), 1-hydroxybenzotriazole (0.201 g, 1.49 mmol) and 1-ethyl-3-(3-dimethylaminopropyl) carbodiimide hydrochloride (0.388 g, 2.03 mmol) at room temperature, and the mixture was stirred for 4.5 hours. To the reaction mixture was added 5% citric acid aq., and the mixture was extracted with ethyl acetate. The organic layer was washed with saturated sodium bicarbonate aq., and saturated sodium chloride aq., dried over anhydrous magnesium sulfate, and concentrated under reduced pressure. The residue was subjected to silica gel chromatography, and the fractions eluted with -hexane/ethyl acetate = 3/2 were collected, and evaporated to dryness to afford a colorless compound (11) (0.300 g, yield 93.6%).

(Step 2)

To a solution of 2-chloroethanol (1.62 mL, 24.2 mmol) in acetonitrile (18 mL) was added chlorosulfonyl isocyanate (2.10 mL, 24.1 mmol) under ice cooling, and this was stirred at room temperature for 1 hours. Separately, to a solution of D-alanine methyl ester hydrochloride (4) (3.30 g, 23.6 mmol) in acetonitrile (7 mL) was added N-methylmorpholine (10.5 mL, 95.4 mmol) under ice cooling, and this was stirred for 2 hours. The former mixture was added to the latter mixture, which was stirred at room temperature for 20 hours. Next, to the reaction mixture were added 1-(4-bromophenyl) piperazine (12) (4.60 g, 19.1 mmol), chlorotrimethylsilane (1.52 mL, 12.0 mmol), and the mixture was stirred at 80°C for 7 hours. The reaction mixture was poured into ice-water, neutralized with 5% citric acid aq, and extracted with ethyl acetate. The organic layer was washed with saturated sodium bicarbonate aq., and saturated sodium chloride aq., dried over anhydrous sodium sulfate, and concentrated under reduced pressure. The residue was subjected to silica gel chromatography, and the fractions eluted with n-hexane/ethyl acetate = 1/1 were collected, and recrystallized with n-hexane/ethyl acetate to give a colorless compound (13) (6.85 g, yield 88.4%).

(Step 3)

A solution of the compound (11) (0.131 g, 0.553 mmol) and the compound (13) (0.150 g, 0.369 mmol) in 1,4-dioxane (3 mL) was degassed well, and the atmosphere was replaced with nitrogen gas. Then, to the mixture were added tris(dibenzylideneacetone) dipalladium (0) (0.017 g, 0.018 mmol), tri-butylphosphine(0.018 mL, 0.074 mmol) and N, N-dicyclohexylmethylamine (0.158 ml, 0.738 mmol), this was degassed again under nitrogen gas atmosphere, and stirred under reflux at 70°C for 1.5 hours. The reaction mixture was cooled to room temperature, water (6 mL) was added to the reaction mixture. The precipitated crystals were collected by filtration, and dissolved in ethyl acetate. The organic layer was dried over anhydrous magnesium sulfate, and concentrated under reduced pressure. The residue was recrystallized with ethyl acetate to afford a pale yellow compound (14) (0.128 g, yield 61.5 %).

(Step 4)

To a solution of the compound (14) (0.122 g, 0.217 mmol) in dimethyl sulfoxide (2.4 mL) was added 1 mol/L sodium hydroxide aq. (0.868 mL) at room temperature, and the mixture was stirred for 5.5 hours. The reaction mixture was poured into ice water-5% citric acid aq., the precipitated crystals were collected by filtration, washed with ethyl acetate. The solid was subjected to silica gel chromatography, the fractions eluted with chloroform/methanol/water = 4/1/0.1 were collected and concentrated under reduced pressure. The precipitated crystals were washed with diethyl ether to give (R)-2-(4-{4-[(E)-2-(3-benzylcarbamoylphenyl)-vinyl]-phenyl}-piperazine-1-sulfonylamino)-proponic acid (I-2) (0.0725 g, yield 60.9 %).
$^1$H NMR (DMSO-d6) δ: 1.29 (d, J=7.2 Hz, 3H), 3.14 (m, 4H), 3.24 (m, 4H), 3.79 (m, 1H), 4.50 (d, J=5.6 Hz, 2H), 6.98 (d, J=8.7 Hz, 2H), 7.11 (d, J=16.3 Hz, 1H), 7.23 (m, 1H), 7.24 (d, J=16.6 Hz, 1H), 7.33 (d, J=4.3 Hz, 4H), 7.44 (t, J=7.9 Hz, 1H), 7.49 (d, J=8.9 Hz, 2H), 7.68 (d, J=8.1 Hz, 1H), 7.72 (d, J=7.8 Hz, 1H), 7.90 (d, J=7.8 Hz, 1H), 8.07 (s, 1H), 9.09 (t, J=5.9 Hz, 1H).

[Example 3]

According to the same manner as Example 1 or 2, the following compounds were synthesized.

Synthesis of (R)-2-{4-[4-(3-methyl4- piperazine-1phenylethynyl)-phenyl]-piperazine-1-sulfonylamino}-proponic acid (I-3)

$^1$H NMR (DMSO-d6) δ: 1.30 (d, J=6.8 Hz, 3H), 2.64 (s, 3H), 3.16 (br s, 4H), 3.31 (br s, 4H), 3.79-3.84 (m, 1H), 7.00 (d, J=8.4 Hz, 2H), 7.42-7.52 (m, 3H), 7.89-7.96 (m, 2H), 8.23 (s, 1H), 12.68 (br s, 1H).

Synthesis of (R)-2-(4-{4-[3-methyl4-(4-methylthiazol-2-yl)-phenylethynyl]-phenyl}-piperazine-1-sulfonylamino)-proponic acid (I-4)

$^1$H NMR (DMSO-d6) δ: 1.30 (d, J=7.2 Hz, 3H), 2.45 (s, 3H), 2.57 (s, 3H), 3.05-3.40 (m, 8H), 3.80 (m, 1H), 7.00 (d, J=8.7 Hz, 2H), 7.38-7.46 (m, 4H), 7.51 (m, 1H), 7.80 (d, J=7.8 Hz, 1H), 7.90 (m, 1H), 12.65 (br s, 1H).

Synthesis of (R)-2-[4-(4-{3-methyl4-[(pyridine 4-carbonyl)-amino]-phenylethynyl}-phenyl)-piperazine-1-sulfonylamino]-proponic acid (I-5)

$^1$H NMR (DMSO-d6) δ: 1.30 (d, J=7.2 Hz, 3H), 3.26 (s, 3H), 3.05-3.40 (m, 8H), 3.81 (m, 1H), 7.00 (d, J=8.7 Hz, 2H), 7.34-7.48 (m, 5H), 7.84-7.96 (m, 3H), 8.80 (d, J=5.1 Hz, 1H), 10.19 (s, 1H), 12.80 (br s, 1H).

Synthesis of (R)-2-{4-[4-(3-cyano4-oxazol-2-ylphenylethynyl)-phenyl]-piperazine-1-sulfonylamino}-proponic acid (I-6)

$^1$H NMR (DMSO-d6) δ: 1.30 (d, J=6.8 Hz, 3H), 3.13-3.20 (m, 4H), 3.33 (br s, 4H), 3.78-3.86 (m, 1H), 7.02 (d,

J=8.4 Hz, 2H), 7.47 (d, J=8.8 Hz, 2H), 7.57 (s, 1H), 7.90-7.94 (m, 2H), 8.10-8.18 (m, 2H), 8.41 (s, 1H), 12.40 (br s, 1H).

Synthesis of (R)-2-{4-[4-(3-oxazol-2-ylphenylethynyl)-phenyl]-piperazine-1-sulfonylamino}-proponic acid (I-7)

$^1H$ NMR (DMSO-d6) δ: 1.30 (d, J=6.8 Hz, 3H), 3.14-3.19 (m, 4H), 3.32 (br s, 4H), 3.79-3.84 (m, 1H), 7.01 (d, J=8.4 Hz, 2H), 7.42-7.47 (m, 3H), 7.56-7.65 (m, 2H), 7.90 (d, J=8.4 Hz, 1H), 7.97 (d, J=8.0 Hz, 1H), 8.05 (s, 1H), 8.26 (s, 1H), 12.70 (br s, 1H).

Synthesis of (R)-2-{4-[4-(3-benzyloxy4-oxazol-2-ylphenylethynyl)-phenyl]-piperazine-1-sulfonylamino}-proponic acid (I-8)

$^1H$ NMR (DMSO-d6) δ: 1.30 (d, J=6.8 Hz, 3H), 3.14-3.19 (m, 4H), 3.32 (br s, 4H), 3.79-3.84 (m, 1H), 5.34 (s, 2H), 7.01 (d, J=8.8 Hz, 2H), 7.22 (d, J=7.2 Hz, 1H), 7.31-7.45 (m, 7H), 7.55 (d, J=7.2 Hz, 2H), 7.90 (d, J=8.0 Hz, 2H), 8.25 (s, 1H), 12.69 (br s, 1H).

Synthesis of (R)-2-[4-(4-{3,5-dimethyl4-[(pyridine 4-carbonyl)-amino]-phenylethynyl}-phenyl)-piperazine-1-sulfonylamino]-proponic acid (I-9)

$^1H$ NMR (DMSO-d6) δ: 1.30 (d, J=7.2 Hz, 3H), 2.19 (s, 6H), 3.05-3.40 (m, 8H), 3.81 (m, 1H), 7.00 (d, J=8.7 Hz, 2H), 7.31 (s, 2H), 7.40 (d, J=8.4 Hz, 1H), 7.86-7.96 (m, 3H), 8.80 (d, J=5.4 Hz, 1H), 10.13 (s, 1H), 12.73 (br s, 1H).

Synthesis of (R)-2-(4-{4-[3-ethyl4-(4-methoxybenzoylamino)-phenylethynyl]-phenyl}-piperazine-1-sulfonylamino)-proponic acid (I-10)

$^1H$ NMR (DMSO-d6) δ: 1.14 (t, J=7.5 Hz, 3H), 1.30 (d, J=7.5 Hz, 3H), 2.09 (s, 3H), 2.64 (q, J=7.5 Hz, 2H), 3.05-3.40 (m, 8H), 3.81 (m, 1H), 3.84 (s, 3H), 7.00 (d, J=9.3 Hz, 1H), 7.07 (d, J=8.7 Hz, 1H), 7.36 (s, 1H), 7.41 (d, J=8.4 Hz, 2H), 7.91 (d, J=9.6 Hz, 1H), 7.97 (d, J=8.7 Hz, 1H), 9.76 (s, 1H), 12.72 (br s, 1H).

Synthesis of (R)-2-[4-(4-{3-cyano4-[(pyridine 4-carbonyl)-amino]-phenylethynyl}-phenyl)-piperazine-1-sulfonylamino]-proponic acid (I-11)

$^1H$ NMR (DMSO-d6) δ: 1.30 (d, J=7.2 Hz, 3H), 3.05-3.40 (m, 8H), 3.82 (m, 1H), 7.01 (d, J=9.3 Hz, 2H), 7.43 (d, J=8.7 Hz, 2H), 7.63 (d, J=9.0 Hz, 1H), 7.82-7.96 (m, 4H), 8.05 (d, J=2.1 Hz, 1H), 8.80-8.86 (m, 2H), 10.98 (s, 1H), 12.50 (br s, 1H).

Synthesis of (R)-2-[4-(4-{3-[(pyridine 4-carbonyl)-amino]-phenylethynyl}-phenyl)-piperazine-1-sulfonylamino]-proponic acid (I-12)

$^1H$ NMR (DMSO-d6) δ: 1.30 (d, J=7.2 Hz, 3H), 3.05-3.40 (m, 8H), 3.80 (m, 1H), 7.00 (d, J=9.0 Hz, 2H), 7.27 (m, 1H), 7.36-7.46 (m, 3H), 7.72 (m, 1H), 7.80-7.94 (m, 3H), 7.98 (s, 1H), 8.76-8.84 (m, 2H), 12.72 (br s, 1H).

Synthesis of (R)-2-{4-[4-(2-fluoro-4-methyl-5-oxazol-2-ylphenylethynyl)-phenyl]-piperazine-1-sulfonylamino}-proponic acid (I-13)

$^1H$ NMR (DMSO-d6) δ: 1.29 (d, J=6.8 Hz, 3H), 2.66 (s, 3H), 3.13-3.19 (m, 4H), 3.32 (br s, 4H), 3.78-3.84 (m, 1H), 7.01 (d, J=9.0 Hz, 2H), 7.43-7.46 (m, 4H), 7.93 (d, J=7.8 Hz, 1H), 8.06 (d, J=7.5 Hz, 1H), 8.27 (s, 1H), 12.73 (br s, 1H).

Synthesis of (R)-2-{4-[4-(3-dimethylcarbamoyl 5-oxazol-2-ylphenylethynyl)-phenyl]-piperazine-1-sulfonylamino}-proponic acid (I-14)

$^1H$ NMR (DMSO-d6) δ: 1.29 (d, J=6.8 Hz, 3H), 2.96 (s, 3H), 3.02 (s, 3H), 3.16 (br s, 4H), 3.31 (s, 4H), 3.76-3.82 (m, 1H), 7.01 (d, J=8.0 Hz, 2H), 7.46-7.48 (m, 3H), 7.64 (s, 1H), 7.91 (s, 1H), 8.09 (s, 1H), 8.30 (s, 1H).

Synthesis of (R)-2-(4-{4-[3-(5-methyloxazol-2-yl)-phenylethynyl]-phenyl}-piperazine-1-sulfonylamino)-proponic acid (I-15)

$^1H$ NMR (DMSO-d6) δ: 1.30 (d, J=6.8 Hz, 3H), 2.40 (s, 3H), 3.13-3.20 (m, 4H), 3.27-3.33 (m, 4H), 3.79-3.85 (m, 1H), 7.00-7.06 (m, 3H), 7.44-7.61 (m, 4H), 7.90-7.93 (m, 2H), 7.99 (s, 1H), 12.74 (br s, 1H).

Synthesis of (R)-2-(4-{4-[5-(4-fluorophenyl)-oxazol-2-ylethynyl]-phenyl}-piperazine-1-sulfonylamino)-proponic acid (I-16)

$^1$ H NMR (DMSO-d6) δ: 1.29 (d, J=7.1 Hz, 3H), 3.14-3.36 (m, 8H), 3.80 (br s, 1H), 7.04 (d, J=8.5 Hz, 2H), 7.32-7.40 (m, 2H), 7.54 (d, J=8.5 Hz, 2H), 7.80-7.95 (m, 4H).

Synthesis of (R)-2-{4-[4-(3-carbamoylphenylethynyl)-phenyl]-piperazine-1-sulfonylamino}-proponic acid (I-17)

$^1$ H NMR (DMSO-d6) δ: 1.30 (d, J=7.2 Hz, 3H), 3.11-3.19 (m, 4H), 3.30 (br s, 4H), 3.77-3.85 (m, 1H), 7.01 (d, J=8.4 Hz, 2H), 7.42-7.51 (m, 4H), 7.64 (d, J=8.0 Hz, 1H), 7.86 (d, J=8.0 Hz, 1H), 7.93 (d, J=8.0 Hz, 1H), 8.00 (s, 1H), 8.07 (s, 1H), 12.77 (br s, 1H).

Synthesis of (R)-2-{4-[4-(6-benzylcarbamoylpyridine 2-ylethynyl)-phenyl]-piperazine-1-sulfonylamino}-proponic acid (I-18)

$^1$ H NMR (DMSO-d6) δ: 1.30 (d, J=7.2 Hz, 3H), 3.11-3.19 (m, 4H), 3.31 (br s, 4H), 3.79-3.85 (m, 1H), 4.51 (d, J=6.0 Hz, 2H), 7.02 (d, J=8.8 Hz, 2H), 7.21-7.34 (m, 5H), 7.48 (d, J=8.8 Hz, 2H), 7.78 (dd, J=2.4, 8.0 Hz, 1H), 7.92 (d, J=8.0 Hz, 1H), 8.00-8.04 (m, 2H), 9.26 (t, J=6.4 Hz, 1H).

Synthesis of (R)-2-{4-[4-(2-benzylcarbamoylpyridin-4-ylethynyl)-phenyl]-piperazine-1-sulfonylamino}-proponic acid (I-19)

$^1$ H NMR (DMSO-d6) δ: 1.29 (d, J=7.2 Hz, 3H), 3.11-3.19 (m, 4H), 3.30 (br s, 4H), 3.78-3.84 (m, 1H), 4.50 (d, J=6.0 Hz, 2H), 7.02 (d, J=8.8 Hz, 2H), 7.22-7.33 (m, 5H), 7.50 (d, J=8.8 Hz, 2H), 7.66 (d, J=8.0 Hz, 1H), 7.92 (br s, 1H), 8.03 (s, 1H), 8.65 (d, J=4.8 Hz, 1H), 9.38 (t, J=6.4 Hz, 1H).

Synthesis of (R)-2-{4-[4-(3-benzylcarbamoylbenzyloxy)-phenyl]-piperazine-1-sulfonylamino}-proponic acid (I-20)

$^1$ H NMR (DMSO-d6) δ: 1.29 (d, J=7.2 Hz, 3H), 3.05 (br s, 4H), 3.11-3.14 (m, 4H), 3.77-3.86 (m, 1H), 4.48 (d, J=6.0 Hz, 2H), 5.09 (s, 2H), 6.92 (s, 4H), 7.23-7.33 (m, 5H), 7.46-7.60 (m, 2H), 7.83-7.88 (m, 2H), 7.96 (s, 1H), 9.08 (t, J=6.0 Hz, 1H), 12.72 (br s, 1H).

Synthesis of (R)-2-[4-(4-biphenyl3-ylethynylphenyl)-piperazine-1-sulfonylamino]-proponic acid (I-21)

$^1$ H NMR (DMSO-d6) δ: 1.30 (d, J=7.2 Hz, 3H), 3.05-3.40 (m, 8H), 3.80 (m, 1H), 7.01 (d, J=8.7 Hz, 2H), 7.36-7.54 (m, 7H), 7.64-7.80 (m, 4H), 7.92 (m, 1H), 12.80 (br s, 1H).

Synthesis of (R)-2-(4-{4-[3-(4-methylthiazol-2-yl)-phenylethynyl]-phenyl}-piperazine-1-sulfonylamino)-proponic acid (I-22)

$^1$ H NMR (DMSO-d6) δ: 1.30 (d, J = 7.1 Hz, 3H), 2.44 (s, 3H), 3.10-3.37 (m, 8H), 3.76-3.84 (m, 1H), 7.01 (d, J = 9.6 Hz, 2H), 7.39-7.60 (m, 5H), 7.89-7.91 (m, 2H), 8.00 (s, 1H).

Synthesis of (R)-2-(4-{4-[3-(4,5-dimethylthiazol-2-yl)-phenylethynyl]-phenyl}-piperazine-1-sulfonylamino)-proponic acid (I-23)

$^1$ H NMR (DMSO-d6) δ: 1.27 (d, J = 7.0 Hz, 3H), 2.32 (s, 3H), 2.39 (s, 3H), 3.09-3.33 (m, 8H), 3.68-3.78 (m, 1H), 6.99 (d, J = 8.8 Hz, 2H), 7.42-7.55 (m, 4H), 7.78-7.81 (m, 1H), 7.91 (t, J = 1.5 Hz, 1H).

Synthesis of (R)-2-{4-[4-(3-benzo[b]thiophen-2-ylphenylethynyl)-phenyl]-piperazine-1-sulfonylamino}-proponic acid (I-24)

$^1$ H NMR (DMSO-d6) δ: 1.30 (d, J = 7.1 Hz, 3H), 3.11-3.41 (m, 8H), 3.75-3.86 (m, 1H), 7.02 (d, J = 8.8 Hz, 2H), 7.35-7.53 (m, 6H), 7.76-8.02 (m, 6H).

Synthesis of (R)-2-(4-{4-[3-((E)-2-cyanovinyl)-phenylethynyl]-phenyl}-piperazine-1-sulfonylamino)-proponic acid (I-25)

$^1$ H NMR (DMSO-d6) δ: 1.30 (d, J = 7.1 Hz, 3H), 3.07-3.35 (m, 8H), 3.74-3.85 (m, 1H), 6.57 (d, J = 16.7 Hz,

1H), 7.00 (d, J = 8.6 Hz, 2H), 7.41 (d, J = 8.6 Hz, 2H), 7.47 (t, J = 7.6 Hz, 1H), 7.58 (d, J = 7.6 Hz, 1H), 7.65 (d, J = 7.6 Hz, 1H), 7.66 (d, J = 16.7 Hz, 1H), 7.82 (s, 1H), 7.89 (br s, 1H).

Synthesis of (S)-2-(4-{4-[3-(5-methyloxazol-2-yl)-phenylethynyl]-phenyl}-piperazine-1-sulfonylamino)-pentanoic acid (I-26)

$^1$ H NMR (DMSO-d6) δ: 0.88 (t, J = 7.6 Hz, 3H), 1.32-1.45 (m, 2H), 1.51-1.65 (m, 2H), 2.40 (s, 3H), 3.09-3.40 (m, 8H), 3.64-3.73 (m, 1H), 6.97-7.05 (m, 3H), 7.45 (d, J = 8.6 Hz, 2H), 7.52-7.62 (m, 2H), 7.86-7.95 (m, 2H), 7.99 (s, 1H).

Synthesis of (R)-3-methyl-2-(4-{4-[3-(5-methyloxazol-2-yl)-phenylethynyl]-phenyl}-piperazine-1-sulfonylamino)-butanoic acid (I-27)

$^1$ H NMR (DMSO-d6) δ: 0.91 (d, J = 6.6 Hz, 3H), 0.92 (d, J = 6.6 Hz, 3H), 1.91-2.03 (m, 1H), 2.40 (s, 3H), 3.08-3.54 (m, 9H), 6.96-7.05 (m, 3H), 7.45 (d, J = 8.6 Hz, 2H), 7.52-7.63 (m, 2H), 7.78-7.87 (m, 1H), 7.90 (d, J = 7.6 Hz, 1H), 7.99 (s, 1H).

Synthesis of (R)-2-(4-{4-[3-(4-fluoro3-methylbenzylcarbamoyl)-phenylethynyl]-phenyl}-piperazine-1-sulfonylamino)-proponic acid (I-28)

$^1$ H NMR (DMSO-d6) δ: 1.29 (d, J=7.2 Hz, 3H), 2.21 (d, J=1.5 Hz, 3H), 3.09-3.16 (m, 4H), 3.27-3.33 (m, 4H), 3.80 (m, 1H), 4.41 (d, J=5.6 Hz, 2H), 6.99 (d, J=8.9 Hz, 2H), 7.07 (t, J=9.2 Hz, 1H), 7.14-7.23 (m, 2H), 7.41 (d, J=8.7 Hz, 2H), 7.50 (t, J=7.6 Hz, 1H), 7.64 (d, J=8.1 Hz, 1H), 7.85 (d, J=7.8 Hz, 1H), 7.93 (d, J=9.2 Hz, 1H), 8.01 (s, 1H), 9.12 (t, J=6.0 Hz, 1H).

Synthesis of (R)-2-[4-(4-{(E)-2-[3-(4-fluoro3-methylbenzylcarbamoyl)-phenyl]-vinyl}-phenyl)-piperazine-1-sulfonylamino]-proponic acid (I-29)

$^1$ H NMR (DMSO-d6) δ: 1.30 (d, J=7.2 Hz, 3H), 2.22 (d, J=1.7 Hz, 3H), 3.15-3.28 (m, 8H), 3.79 (m, 1H), 4.44 (d, J=5.9 Hz, 2H), 6.98 (d, J=8.7 Hz, 2H), 7.05-7.26 (m, 5H), 7.44 (t, J=7.8 Hz, 1H), 7.49 (d, J=8.9 Hz, 2H), 7.69 (d, J=8.9 Hz, 1H), 7.71 (d, J=8.7 Hz, 1H), 7.85 (br s, 1H), 8.06 (s, 1H), 9.04 (t, J=6.0 Hz, 1H).

Synthesis of (R)-2-(4-{4-[3-(2-oxopropylcarbamoyl)-phenylethynyl]-phenyl}-piperazine-1-sulfonylamino)-proponic acid (I-30)

$^1$ H NMR (DMSO-d6) δ: 1.29 (d, J=7.2 Hz, 3H), 2.13 (s, 3H), 3.28-3.43 (m, 8H), 3.75 (m, 1H), 4.09 (d, J=5.6 Hz, 2H), 7.00 (d, J=9.0 Hz, 2H), 7.42 (d, J=8.7 Hz, 2H), 7.51 (t, J=7.8 Hz, 1H), 7.66 (d, J=7.6 Hz, 1H), 7.83 (d, J=7.8 Hz, 1H), 7.99 (s, 1H), 8.90 (t, J=5.5 Hz, 1H).

Synthesis of (R)-2-[4-(4-{3-[2-(4-chrolophenyl)-acetylamino]phenylethynyl}-phenyl)-piperazine-1-sulfonylamino]-proponic acid (I-31)

$^1$ H NMR (DMSO-d6) δ: 1.29 (d, J=7.2 Hz, 3H), 3.11-3.44 (m, 8H), 3.66 (s, 2H), 3.79 (m, 1H), 6.98 (d, J=8.9 Hz, 2H), 7.16 (d, J=7.8 Hz, 1H), 7.29-7.40 (m, 7H), 7.47 (d, J=8.4 Hz, 1H), 7.81 (t, J=1.7 Hz, 1H), 7.91 (d, J=7.0 Hz, 1H), 10.29 (s, 1H).

Synthesis of (R)-2-(4-{4-[3-(4-chrolobenzylcarbamoyl)-phenylethynyl]-phenyl}-piperazine-1-sulfonylamino)-proponic acid (I-32)

$^1$ H NMR (DMSO-d6) δ: 1.29 (d, J=7.2 Hz, 3H), 3.11-3.44 (m, 8H), 3.79 (m, 1H), 4.46 (d, J=6.0 Hz, 2H), 6.99 (d, J=9.0 Hz, 2H), 7.33-7.43 (m, 6H), 7.50 (t, J=7.8 Hz, 1H), 7.65 (dt, J=7.8, 1.2 Hz, 1H), 7.85 (dt, J=7.8, 1.4 Hz, 1H), 7.92 (d, J=8.4 Hz, 1H), 8.0 (t, J=1.4 Hz, 1H), 9.18 (t, J=1.4 Hz, 1H).

[Example 4]

Synthesis of (R)-2-(4-{4-[8-(morpholine-4-carbonyl)-naphthalen-2-ylethynyl]-phenyl}-piperazine-1-sulfonylamino)-proponic acid (I-33)

[Chemical formula 61]

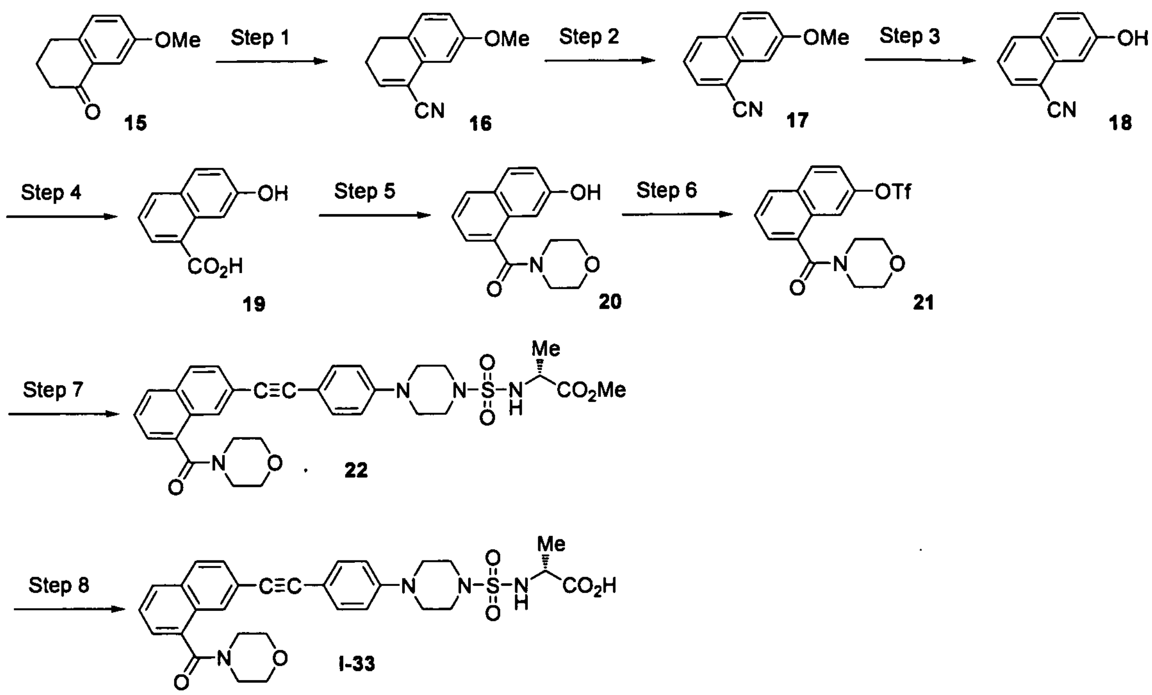


15 16 17 18 19 20 21 22 I-33

(Step 1)

A suspension of 7-methoxy-1-tetralone (15) (30.0 g, 170 mmol) and zinc iodide (1.09 g, 3.41 mmol) in benzene (75 mL) was added trimethylsilyl cyanide (25.0 mL, 187 mmol), and the mixture was stirred at room temperature for 17 hours. Then, to the reaction mixture were added pyridine (270 mL) and phosphorus oxychloride (75 mL) and the mixture was stirred at 130°C for 6 hours. The reaction mixture was cooled to room temperature, stirred at room temperature for 22 hours. The teaction mixture was poured into ice-3mol/L hydrochloric acid, and the mixture was extracted with ethyl acetate. The organic layer was washed with saturated sodium chloride aq., dried over anhydrous sodium sulfate, and concentrated under reduced pressure to give a pale brown compound (16) (27.1 g, yield 86.0%).

(Step 2)

To a solution of the compound (16) (27.0 g, 146 mmol) in 1, 4-dioxane was added 2, 3-dichrolo-5, 6-dicyanol, 4-benzoquinone (39.7 g, 175 mmol). The mixture was stirred under reflux at 125°C for 3 hours. The reaction mixture was cooled to room temperature, and extracted with ethyl acetate. The organic layer was washed with water, dried over anhydrous sodium sulfate, and concentrated under reduced pressure. The residue was subjected to silica gel chromatography, and the fractions eluted with n-hexane/ethyl acetate = 9/1 were collected, and recrystallized with n-hexane/diisopropyl ether to afford a colorless compound (17) (20.2 g, yield 75.4 %).

(Step 3)

A mixture of the compound (17) (10.0 g, 54.6 mmol) and pyridine hydrochloride (50.0 g, 433 mmol) was stirred at 190°C for 5 hours. The reaction mixture was cooled to room temperature, poured into ice-water (300 mL), and this was extracted with ethyl acetate. The organic layer was washed with water, dried over anhydrous sodium sulfate, and concentrated under reduced pressure. The residue was recrystallized from n-hexane/ethyl acetate to give a colorless compound (18) (8.75 g, yield 94.7 %).

(Step 4)

A suspension of the compound (18) (4.00 g, 23.6 mmol) and potassium hydroxide (20.0 g, 307 mmol) in ethylene glycol (80 mL) was stirred at 200°C for 1.5 hours. The reaction mixture was cooled to room temperature, and 2mol/L hydrochloric acid (150 mL) was added to the reaction mixture.. The precipitated crystals were collected by filtration, and washed with water to give a colorless compound (19) (4.00 g, yield 89.9 %).

(Step 5)

To a solution of the compound (19) (0.300 g, 1.59 mmol) in methylene chloride (5 mL) were added 1-hydroxybenzotriazole (0.452 g, 3.35 mmol) and 1-ethyl-3-(3-dimethylaminopropyl) carbodiimide hydrochloride (0.642 g, 3.35 mmol) at room temperature, and the mixture was stirred for 10 minites. To the reaction mixture was added morpholine (0.292 mL, 3.35 mmol), and the mixture was stirred 15minutes. To the reaction mixture was added an saturated sodium bicarbonate aq., and the mixture was extracted with ethyl acetate. The organic layer was washed with saturated sodium chloride aq., dried over anhydrous magnesium sulfate, and concentrated under reduced pressure. The residue was washed with diethyl ether to afford a colorless compound (20) (0.352 g, yield 85.8%).

(Step 6)

To a suspension of the compound (20) (0.200 g, 0.777 mmol) in methylene chloride (2 mL) were pyridine (0.377 mL, 4.66 mmol) and trifluoromethanesulfonic acid anhydride (0.157 mL, 0.932 mmol) under ice cooling, and the mixture was stirred at room temperature for 30 minutes. To the reaction mixture was added 5% citric acid aq., and the mixture was extracted with ethyl acetate. The organic layer was washed with saturated sodium chloride aq., dried over anhydrous magnesium sulfate, and concentrated under reduced pressure. The residue was subjected to silica gel chromatography, and the fractions eluted with n-hexane/ethyl acetate = 1/2 were collected, and evaporated to dryness to give a yellow compound (21) (0.277 g, yield 91.6%).

(Step 7)

A solution of compound (21) (0.274 g, 0.704 mmol) and compound (8) (0.247 g, 0.704 mmol) in N, N-dimethylformamide (2.7 mL) was degassed well, and the atmosphere was replaced with nitrogen gas. Then, to the mixture were added bis(triphenylphosphine) palladium (II) chloride (0.025 g, 0.035 mmol), copper (I) iodide (0.013 g, 0.036 mmol), and triethylamine (0.196 ml, 1.41 mmol), this was degassed again under nitrogen atmosphere, and stirred at 70°C for 30 minutes. The reaction mixture was cooled to room temperature, neutralized with 5% citric acid aq., and the mixture was extracted with ethyl acetate. The organic layer was washed with saturated sodium chloride aq., dried over anhydrous magnesium sulfate, and concentrated under reduced pressure. The residue was subjected to silica gel chromatography, the fractions eluted with n-hexane/ethyl acetate = 1/4 were collected, and recrystallized from methanol to afford a pale brown compound (22) (0.254 g, yield 61.1 %).

(Step 8)

To a solution of the compound (22) (0.217 g, 0.367 mmol) in dimethyl sulfoxide (3 mL) was added 1 mol/L sodium hydroxide aq. (1.1 mL) at room temperature, and the mixture was stirred for 30 minutes. The reaction mixture was poured into ice water-5% citric acid aq., and the mixture was extracted with ethyl acetate. The organic layer was washed with water, dried over anhydrous magnesium sulfate, and concentrated under reduced pressure. The residue was recrystallized from acetone / water to give (R)-2-(4-{4-[8-(morpholine-4-carbonyl)-naphthalen-2-ylethynyl]-phenyl}-piperazine-1-sulfonylamino)-proponic acid (I-33) (0.170 g, yield 80.3%).
$^{1}$H NMR (DMSO-d6) δ: 1.32 (d, J = 7.1 Hz, 3H), 3.08-3.62 (m, 13H), 3.74-3.87 (m, 4H), 7.04 (d, J = 9.1 Hz, 2H), 7.48-7.69 (m, 5H), 7.91 (s, 1H), 8.05 (d, J = 8.8 Hz, 2H).

[Example 5]

According to the same manner as that described Example4, the following compounds were synthesized.

Synthesis of (R)-2-{4-[4-(7-methoxynaphthalen-2-ylethynyl)-phenyl]-piperazine-1-sulfonylamino}-proponic acid (I-34)

$^{1}$H NMR (DMSO-d6) δ: 1.30 (d, J=8.0 Hz, 3H), 3.12-3.21 (m, 4H), 3.75-3.81 (m, 1H), 3.88 (s, 1H), 7.01 (d, J=8.4 Hz, 2H), 7.19 (d, J=8.4 Hz, 1H), 7.34 (s, 1H), 7.40 (d, J=8.0 Hz, 2H), 7.45 (d, J=12.0 Hz, 2H), 7.84 (d, J=8.0 Hz,

2H), 8.00 (s, 1H).

Synthesis of 3-{4-[4-((R)-1-carboxyethylsulfamoyl)-piperazin-1-yl]-phenylethynyl}-naphthalene 1-carboxylic acid (I-35)

1 H NMR (DMSO-d6) δ: 1.30 (d, J=6.8 Hz, 3H), 3.15-3.20 (m, 8H), 3.78-3.81 (m, 1H), 7.02 (d, J=8.8 Hz, 2H), 7.48 (d, J=8.4 Hz, 2H), 7.62-7.69 (m, 2H), 7.82-7.86 (m, 1H), 8.03 (d, J=7.6 Hz, 1H), 8.14 (s, 1H), 8.33 (s, 1H), 8.82 (d, J=7.6 Hz, 1H).

Synthesis of (R)-2-{4-[4-(4-carbamoylnaphthalen-2-ylethynyl)-phenyl]-piperazine-1-sulfonylamino}-proponic acid (I-36)

1 H NMR (DMSO-d6) δ: 1.30 (d, J=6.8 Hz, 3H), 3.13-3.20 (m, 4H), 3.32 (br s, 4H), 3.79-3.85 (m, 1H), 7.02 (d, J=8.4 Hz, 2H), 7.46 (d, J=8.0 Hz, 2H), 7.58-7.68 (m, 4H), 7.90 (d, J=8.8 Hz, 1H), 7.97-7.99 (m, 1H), 8.09 (s, 1H), 8.19 (s, 1H), 8.27-8.29 (m, 1H), 12.75 (br s, 1H).

Synthesis of (R)-2-{4-[4-(4-cyanonaphthalen-2-ylethynyl)-phenyl]-piperazine-1-sulfonylamino}-proponic acid (I-37)

1 H NMR (DMSO-d6) δ: 1.31 (d, J=6.8 Hz, 3H), 3.17 (br s, 4H), 3.32 (br s, 4H), 3.79-3.85 (m, 1H), 7.03 (d, J=8.4 Hz, 2H), 7.48 (d, J=8.4 Hz, 2H), 7.76-7.91 (m, 3H), 8.13 (br s, 2H), 8.27 (s, 1H), 8.49 (s, 1H), 12.72 (br s, 1H).

Synthesis of (R)-2-{4-[4-(8-cyanonaphthalen-2-ylethynyl)-phenyl]-piperazine-1-sulfonylamino}-proponic acid (I-38)

1 H NMR (DMSO-d6) δ: 1.30 (d, J=6.8 Hz, 3H), 3.16-3.18 (m, 8H), 3.77-3.80 (m, 1H), 7.02 (d, J=8.0 Hz, 2H), 7.52 (d, J=8.0 Hz, 2H), 7.68 (t, J=7.2 Hz, 2H), 7.77 (d, J=8.8 Hz, 1H), 8.15 (bs, 2H), 8.21 (d, J=7.6 Hz, 1H), 8.33 (d, J=8.4 Hz, 1H).

Synthesis of (R)-2-{4-[4-(8-carbamoylnaphthalen-2-ylethynyl)-phenyl]-piperazine-1-sulfonylamino}-proponic acid (I-39)

1 H NMR (DMSO-d6) δ: 1.28 (d, J=6.8 Hz, 3H), 3.15-3.18 (m, 8H), 3.72-3.74 (m, 1H), 7.00 (d, J=8.0 Hz, 2H), 7.48 (d, J=8.0 Hz, 2H), 7.54-7.70 (m, 4H), 7.98-8.06 (m, 3H), 8.45 (s, 1H).

Synthesis of (R)-2-{4-[4-(1-chrolo-8-cyanonaphthalen-2-ylethynyl)-phenyl]-piperazine-1-sulfonylamino}-proponic acid (I-40)

1 H NMR (DMSO-d6) δ: 1.31 (d, J=7.2 Hz, 3H), 3.15-3.21 (m, 8H), 3.82 (bs, 1H), 7.04 (d, J=8.4 Hz, 2H), 7.50 (d, J=8.8 Hz, 2H), 7.74 (t, J=7.6 Hz, 1H), 7.82 (d, J=7.6, 1H), 7.90 (bs, 1H), 8.10 (d, J=8.8 Hz, 1H), 8.29 (d, J=7.6 Hz, 1H), 8.37 (d, J=8.4 Hz, 1H).

Synthesis of (R)-2-{4-[4-(8-carbamoyl-1-chrolonaphthalen-2-ylethynyl)-phenyl]-piperazine-1-sulfonylamino}-proponic acid (I-41)

1 H NMR (DMSO-d6) δ: 1.30 (d, J=7.2 Hz, 3H), 3.14-3.20 (m, 4H), 3.81 (bs, 1H), 7.03 (d, J=8.8 Hz, 2H), 7.48 (d, J=8.4 Hz, 2H), 7.55-7.62 (m, 3H), 7.70 (d, J=8.4 Hz, 1H), 7.90-8.05 (m, 4H).

Synthesis of (R)-2-{4-[4-(5-chrolo-4-cyano-6-hydroxynaphthalen-2-ylethynyl)-phenyl]-piperazine-1-sulfonylamino}-proponic acid (I-42)

1 H NMR (DMSO-d6) δ: 1.31 (d, J=7.2 Hz, 3H), 3.15-3.21 (m, 8H), 3.81-3.84 (m, 1H), 7.02 (d, J=8.4 Hz, 2H), 7.46 (d, J=8.4 Hz, 2H), 7.90 (d, J=8.8 Hz, 1H), 7.96 (d, J=8.8 Hz, 1H), 8.19 (s, 1H), 8.39 (s, 1H).

Synthesis of 6-{4-[4-((R)-1-carboxyethylsulfamoyl)-piperazin-1-yl]-phenylethynyl}-naphthalene 1-carboxylic acid (I-43)

1 H NMR (DMSO-d6) δ: 1.31 (d, J=7.2 Hz, 3H), 3.15-3.21 (m, 8H), 3.80-3.86 (m, 1H), 7.02 (d, J=8.8 Hz, 2H), 7.47 (d, J=8.4 Hz, 2H), 7.64 (d, J=7.6 Hz, 1H), 7.70 (d, J=9.2 Hz, 1H), 7.90 (d, J=6.8 Hz, 1H), 8.16-8.22 (m, 3H), 8.88 (d, J=8.4 Hz, 1H).

Synthesis of (R)-2-{4-[4-(8-methylcarbamoylnaphthalen-2-ylethynyl)-phenyl]-piperazine-1-sulfonylamino}-proponic acid (I-44)

$^{1}$H NMR (DMSO-d6) δ: 1.30 (d, J=6.8 Hz, 3H), 2.88 (d, J=4.0 Hz, 3H), 3.14-3.19 (m, 4H), 3.32 (br s, 4H), 3.79-3.84 (m, 1H), 7.01 (d, J=8.8 Hz, 2H), 7.48 (d, J=8.4 Hz, 2H), 7.54-7.65 (m, 3H), 7.90 (d, J=8.8 Hz, 1H), 7.98-8.03 (m, 2H), 8.36 (s, 1H), 8.52 (d, J=4.0 Hz, 1H), 12.69 (br s, 1H).

Synthesis of (R)-2-{4-[4-(8-dimethylcarbamoylnaphthalen-2-ylethynyl)-phenyl]-piperazine-1-sulfonylamino}-proponic acid (I-45)

$^{1}$H NMR (DMSO-d6) δ: 1.30 (d, J=6.8 Hz, 3H), 2.78 (s, 3H), 3.15 (s, 7H), 3.30 (br s, 4H), 3.79-3.84 (m, 1H), 7.01 (d, J=8.4 Hz, 2H), 7.48 (d, J=7.2 Hz, 3H), 7.56-7.64 (m, 2H), 7.81 (s, 1H), 7.88-7.91 (m, 1H), 8.00 (t, J=8.0 Hz, 2H), 12.56 (br s, 1H).

Synthesis of (R)-2-{4-[4-(5-carbamoylnaphthalen-2-ylethynyl)-phenyl]-piperazine-1-sulfonylamino}-proponic acid (I-46)

$^{1}$H NMR (DMSO-d6) δ: 1.29 (s, 3H), 3.16 (bs, 8H), 3.79 (bs, 1H), 7.01 (d, J=7.6 Hz, 2H), 7.46 (d, J=7.6 Hz, 2H), 7.57-7.68 (m, 4H), 8.01-8.05 (m, 2H), 8.17 (s, 1H), 8.32 (d, J=7.6 Hz, 1H).

Synthesis of 7-{4-[4-((R)-1-carboxyethylsulfamoyl)-piperazin-1-yl]-phenylethynyl}-naphthalene 1-carboxylic acid (I-47)

$^{1}$H NMR (DMSO-d6) δ: 1.31 (d, J=7.2 Hz, 3H), 3.14-3.21 (m, 4H), 3.82 (bs, 1H), 7.02 (d, J=7.6 Hz, 2H), 7.49 (d, J=8.4 Hz, 2H), 7.61-7.67 (m, 2H), 7.92-7.93 (m, 1H), 7.99 (s, 1H).

Synthesis of (R)-2-{4-[4-(8-dimethylaminonaphthalen-2-ylethynyl)-phenyl]-piperazine-1-sulfonylamino}-proponic acid (I-48)

$^{1}$H NMR (DMSO-d6) δ: 1.29 (d, J=7.2 Hz, 3H), 2.82 (s, 6H), 3.09-3.35 (m, 8H), 3.74-3.85 (m, 1H), 6.99 (d, J=8.8 Hz, 2H), 7.14 (d, J=7.3 Hz, 1H), 7.42-7.47 (m, 3H), 7.50-7.57 (m, 2H), 7.86-7.95 (m, 2H), 8.23 (s, 1H), 12.72 (br s, 1H).

Synthesis of (R)-2-{4-[4-(8-acetylaminonaphthalen-2-ylethynyl)-phenyl]-piperazine-1-sulfonylamino}-proponic acid (I-49)

$^{1}$H NMR (DMSO-d6) δ: 1.30 (d, J=7.0 Hz, 3H), 2.21 (s, 3H), 3.10-3.38 (m, 8H), 3.75-3.86 (m, 1H), 7.02 (d, J=8.9 Hz, 2H), 7.45-7.62 (m, 4H), 7.73-7.82 (m, 2H), 7.89-7.98 (m, 2H), 8.28 (s, 1H), 9.98 (s, 1H).

Synthesis of (R)-2-{4-[4-(8-cyanomethylnaphthalen-2-ylethynyl)-phenyl]-piperazine-1-sulfonylamino}-proponic acid (I-50)

$^{1}$H NMR (DMSO-d6) δ: 1.30 (d, J=7.5 Hz, 3H), 3.05-3.40 (m, 8H), 3.81 (m, 1H), 4.55 (s, 2H), 7.03 (d, J=9.0 Hz, 2H), 7.48 (d, J=9.0 Hz, 2H), 7.52-7.70 (m, 3H), 7.92 (m, 1H), 7.96 (d, J=7.8 Hz, 1H), 8.02 (d, J=8.4 Hz, 1H), 8.19 (s, 1H), 12.75 (br s, 1H).

Synthesis of (R)-2-{4-[4-(8-isopropylcarbamoylnaphthalen-2-ylethynyl)-phenyl]-piperazine-1-sulfonylamino}-proponic acid (I-51)

$^{1}$H NMR (DMSO-d6) δ: 1.22 (d, J=6.5 Hz, 6H), 1.30 (d, J=7.2 Hz, 3H), 3.10-3.38 (m, 8H), 3.75-3.85 (m, 1H), 4.19 (tt, J=6.5 Hz, 1H),
7.01 (d, J=8.7 Hz, 2H), 7.47 (d, J=8.7 Hz, 2H), 7.54-7.63 (m, 3H), 7.90 (br s, 1H), 7.97-8.05 (m, 2H), 8.33 (s, 1H), 8.47 (d, J=7.4 Hz, 1H).

Synthesis of (R)-2-{4-[4-(5-chrolo-6-methoxynaphthalen-2-ylethynyl)-phenyl]-piperazine-1-sulfonylamino}-proponic acid (I-52)

$^{1}$H NMR (DMSO-d6) δ: 1.30 (d, J=7.2 Hz, 3H), 3.15-3.19 (m, 4H), 3.81 (bs, 1H), 4.02 (s, 3H), 7.01 (d, J=8.8 Hz, 2H), 7.45 (d, J=8.4 Hz, 2H), 7.62 (d, J=9.2 Hz, 1H), 7.68 (d, J=8.8 Hz, 1H), 7.87-7.91 (m, 1H), 8.00 (d, J=9.6 Hz, 1H), 8.09 (d, J=9.6 Hz, 1H), 8.15 (s, 1H).

Synthesis of (R)-2-{4-[4-(5-acetyl-6-methoxynaphthalen-2-ylethynyl)-phenyl]-piperazine-1-sulfonylamino}-proponic acid (I-53)

$^1$H NMR (DMSO-d6) δ: 1.30 (d, J=7.2 Hz, 3H), 2.58 (s, 3H), 3.15-3.20 (m, 4H), 3.81 (bs, 1H), 3.98 (s, 3H), 7.00 (d, J=8.8 Hz, 2H), 7.44 (d, J=8.4 Hz, 2H), 7.54-7.65 (m, 3H), 7.85 (bs, 1H), 8.07 (d, J=9.2 Hz, 2H), 8.12 (s, 1H).

Synthesis of (S)-2-{4-[4-(8-carbamoylnaphthalen-2-ylethynyl)-phenyl]-piperazine-1-sulfonylamino}-3-methylpentanoic acid (I-54)

$^1$H NMR (DMSO-d6) δ: 0.82-0.89 (m, 6H), 1.13-1.22 (m, 1H), 1.44-1.52 (m, 1H), 1.67-1.77 (m, 1H), 3.12-3.21 (m, 4H), 3.33-3.38 (m, 4H), 3.50-3.54 (m, 1H), 7.00 (d, 8.4 Hz, 2H), 7.48 (d, J=8.4 Hz, 2H), 7.54-7.70 (m, 5H), 7.95-8.08 (m, 3H), 8.45 (s, 1H).

Synthesis of (S)-2-{4-[4-(8-carbamoylnaphthalen-2-ylethynyl)-phenyl]-piperazine-1-sulfonylamino}-4-methylpentanoic acid (I-55)

$^1$H NMR (DMSO-d6) δ: 0.84-0.91 (m, 6H), 1.40-1.56 (m, 2H), 1.70-1.75 (m, 1H), 3.13-3.20 (m, 4H), 3.30 (br s, 4H), 3.68-3.73 (m, 1H), 7.00 (d, 8.4 Hz, 2H), 7.48 (d, J=8.4 Hz, 2H), 7.54-7.70 (m, 4H), 7.94-8.07 (m, 4H), 8.45 (s, 1H), 12.73 (br s, 1H).

Synthesis of (R)-2-(4-{4-[8-(2-fluoroethylcarbamoyl)-naphthalen-2-ylethynyl]-phenyl}-piperazine-1-sulfonylamino)-proponic acid (I-56)

$^1$H NMR (DMSO-d6) δ: 1.30 (d, J=7.2 Hz, 3H), 3.10-3.19 (m, 4H), 3.31 (br s, 4H), 3.62-3.83 (m, 3H), 4.56 (d, J=4.8 Hz, 1H), 4.68 (d, J=4.8 Hz, 1H), 7.01 (d, J=8.4 Hz, 2H), 7.47 (d, J=8.4 Hz, 2H), 7.56-7.70 (m, 3H), 7.90-8.06 (m, 3H), 8.36 (s, 1H), 8.84 (t, J=4.8 Hz, 1H), 13.02 (br s, 1H).

Synthesis of (R)-2-{4-[4-(8-morpholin-4-ylnaphthalen-2-ylethynyl)-phenyl]-piperazine-1-sulfonylamino}-proponic acid (I-57)

$^1$H NMR (DMSO-d6) δ: 1.30 (d, J=7.2 Hz, 3H), 3.02 (br s, 4H), 3.12-3.21 (m, 4H), 3.30 (br s, 4H), 3.76-3.83 (m, 1H), 3.89 (br s, 4H), 7.00 (d, J=8.4 Hz, 2H), 7.21 (d, J=8.0 Hz, 1H), 7.46-7.50 (m, 3H), 7.57 (d, J=8.0 Hz, 1H), 7.64 (d, J=8.4 Hz, 1H), 7.81-3.93 (m, 2H), 8.25 (s, 1H).

Synthesis of (S)-2-{4-[4-(8-carbamoylnaphthalen-2-ylethynyl)-phenyl]-piperazine-1-sulfonylamino}-3-methylbutanoic acid (I-58)

$^1$H NMR (DMSO-d6) δ: 0.92 (d, J=6.0 Hz, 6H), 1.95-1.99 (m, 1H), 3.12-3.21 (m, 4H), 3.30 (br s, 4H), 3.48-3.53 (m, 1H), 7.01 (d, 8.4 Hz, 2H), 7.48 (d, J=8.4 Hz, 2H), 7.55-7.70 (m, 4H), 7.84-8.07 (m, 4H), 8.45 (s, 1H), 12.77 (br s, 1H).

Synthesis of (S)-2-(4-{4-[(E)-2-(8-carbamoylnaphthalen-2-yl)-vinyl]-phenyl}-piperazine-1-sulfonylamino)-pentanoic acid (I-59)

$^1$H NMR (DMSO-d6) δ: 0.89 (t, J=5.4 Hz, 3H), 1.35-1.46 (m, 2H), 1.56-1.61 (m, 2H), 3.12-3.21 (m, 4H), 3.26 (br s, 4H), 3.64-3.70 (m, 1H), 6.99 (d, J=8.8 Hz, 2H), 7.27 (s, 2H), 7.42-7.62 (m, 5H), 7.89-8.01 (m, 5H), 8.28 (s, 1H), 12.69 (br s, 1H).

Synthesis of (R)-2-{4-[4-(8-carbamoylnaphthalen-2-ylethynyl)-phenyl]-piperazine-1-sulfonylamino}-4-methylpentanoic acid (I-60)

$^1$H NMR (DMSO-d6) δ: 0.89 (d, J=6.3 Hz, 3H), 0.90 (d, J=6.6 Hz, 3H), 1.35-1.58 (m, 2H), 1.73 (m, 1H), 3.06-3.50 (m, 8H), 3.69 (m, 1H), 7.00 (d, J=9.0 Hz, 2H), 7.47 (d, J=8.4 Hz, 2H), 7.52-7.72 (m, 4H), 7.88-8.12 (m, 4H), 8.43 (s, 1H), 12.74 (br s, 1H).

Synthesis of (R)-2-{4-[4-(8-methylcarbamoylnaphthalen-2-ylethynyl)-phenyl]-piperazine-1-sulfonylamino}-proponic acid (I-61)

$^1$H NMR (DMSO-d6) δ: 1.30 (d, J=7.2 Hz, 3H), 2.60 (d, J=4.5 Hz, 3H), 3.05-3.50 (m, 2H), 3.80 (m, 1H), 3.89

(s, 2H), 7.01 (d, J=9.0 Hz, 2H), 7.40-7.50 (m, 4H), 7.56 (m, 1H), 7.82 (dd, J=2.7, 6.9 Hz, 1H), 7.92 (d, J=8.7 Hz, 2H), 8.07 (m, 1H), 8.22 (s, 1H).

Synthesis of (R)-2-(4-{4-[8-(2-tert-butoxycarbonylaminoethylcarbamoyl)-naphthalen-2-ylethynyl]-phenyl}-piperazine-1-sulfonylamino)-proponic acid (I-62)

$^1$H NMR (DMSO-d6) δ: 1.30 (d, J=7.2 Hz, 3H), 1.38 (s, 9H), 3.05-3.50 (m, 12H), 3.80 (m, 1H), 6.96 (m, 1H), 7.01 (d, J=9.0 Hz, 2H), 7.48 (d, J=8.7 Hz, 2H), 7.52-7.64 (m, 2H), 7.70 (d, J=7.2 Hz, 1H), 7.92 (m, 1H), 7.99 (d, J=8.7 Hz, 1H), 8.03 (d, J=8.7 Hz, 1H), 8.38 (s, 1H), 8.58 (m, 1H).

Synthesis of (R)-2-[4-(4-{8-[2-(tert-butoxycarbonylmethylamino)-ethylcarbamoyl]-naphthalen-2-ylethynyl}-phenyl)-piperazine-1-sulfonylamino]-proponic acid (I-63)

$^1$H NMR (DMSO-d6) δ: 1.30 (d, J=7.2 Hz, 3H), 1.37 (s, 9H), 2.89 (s, 3H), 3.05-3.55 (m, 10H), 3.80 (m, 1H), 7.15 (d, J=9.0 Hz, 2H), 7.47 (d, J=9.0 Hz, 2H), 7.50-7.68 (m, 3H), 7.86-8.06 (m, 3H), 8.40 (s, 1H), 8.66 (m, 1H).

Synthesis of (R)-2-(4-{4-[8-(2-methylaminoethylcarbamoyl)-naphthalen-2-ylethynyl]-phenyl}-piperazine-1-sulfonylamino)-proponic acid dihydrochloride (I-64)

$^1$H NMR (DMSO-d6) δ: 1.30 (d, J=7.2 Hz, 3H), 2.63 (t, J=5.1 Hz, 3H), 3.06-3.36 (m, 10H), 3.50-3.90 (m, 3H), 7.03 (d, J=9.0 Hz, 2H), 7.48 (d, J=9.0 Hz, 2H), 7.56-7.66 (m, 2H), 7.86 (d, J=7.2 Hz, 1H), 7.90-8.12 (m, 3H), 8.47 (s, 1H), 8.88-9.00 (m, 3H).

Synthesis of (R)-2-(4-{4-[8-(2-aminoethylcarbamoyl)-naphthalen-2-ylethynyl]-phenyl}-piperazine-1-sulfonylamino)-proponic acid dihydrochloride (I-65)

$^1$H NMR (DMSO-d6) δ: 1.30 (d, J=7.2 Hz, 3H), 3.30-3.90 (m, 13H), 7.02 (d, J=9.0 Hz, 2H), 7.48 (d, J=8.4 Hz, 2H), 7.55-7.65 (m, 2H), 7.84 (d, J=6.9 Hz, 1H), 7.90-8.20 (m, 6H), 8.45 (s, 1H), 8.45 (t, J=6.0 Hz, 1H).

Synthesis of (R)-2-(4-{4-[8-(methyl imino-methyl)-naphthalen-2-ylethynyl]-phenyl}-piperazine-1-sulfonylamino)-proponic acid (I-66)

$^1$H NMR (DMSO-d6) δ: 1.30 (d, J = 7.4 Hz, 3H), 3.09-3.42 (m, 8H), 3.74-3.86 (m, 1H), 4.01 (s, 3H), 7.02 (d, J = 8.8 Hz, 2H), 7.48 (d, J = 8.8 Hz, 2H), 7.56-7.67 (m, 2H), 7.85 (d, J = 7.4 Hz, 1H), 7.91 (br s, 1H), 8.00-8.05 (m, 2H), 8.81 (s, 1H), 8.90 (s, 1H).

Synthesis of (R)-2-(4-{4-[8-((E)-2-cyanovinyl)-naphthalen-2-ylethynyl]-phenyl}-piperazine-1-sulfonylamino)-proponic acid (I-67)

$^1$H NMR (DMSO-d6) δ: 1.30 (d, J = 7.7 Hz, 3H), 3.09-3.41 (m, 8H), 3.74-3.87 (m, 1H), 6.56 (d, J = 16.2 Hz, 1H), 7.03 (d, J = 8.8 Hz, 2H), 7.48 (d, J = 8.8 Hz, 2H), 7.61-7.64 (m, 2H), 7.86-8.10 (m, 4H), 8.52 (s, 1H), 8.64 (d, J = 16.2 Hz, 1H).

Synthesis of (R)-2-(4-{4-[8-((E)-2-carbamoylvinyl)-naphthalen-2-ylethynyl]-phenyl}-piperazine-1-sulfonylamino)-proponic acid (I-68)

$^1$H NMR (DMSO-d6) δ: 1.30 (d, J = 7.1 Hz, 3H), 3.10-3.34 (m, 8H), 3.74-3.83 (m, 1H), 6.66 (d, J = 15.7 Hz, 1H), 7.01 (d, J = 8.6 Hz, 2H), 7.25 (s, 1H), 7.50 (d, J = 8.6 Hz, 2H), 7.56-7.66 (m, 2H), 7.76-7.89 (m, 3H), 7.97-8.02 (m, 2H), 8.20 (d, J = 15.7 Hz, 1H), 8.33 (s, 1H).

Synthesis of (R)-2-(4-{4-[8-((Z)-2-cyanovinyl)-naphthalen-2-ylethynyl]-phenyl}-piperazine-1-sulfonylamino)-proponic acid (I-69)

$^1$H NMR (DMSO-d6) δ: 1.30 (d, J = 7.2 Hz, 3H), 3.10-3.39 (m, 8H), 3.74-3.86 (m, 1H), 6.15 (d, J = 11.9 Hz, 1H), 7.02 (d, J = 9.1 Hz, 2H), 7.47 (d, J = 9.1 Hz, 2H), 7.63-7.69 (m, 2H), 7.87-7.98 (m, 2H), 8.02-8.10 (m, 2H), 8.20 (s, 1H), 8.31 (d, J = 11.9 Hz, 1H).

Synthesis of (R)-2-{4-[4-(8-vinylnaphthalen-2-ylethynyl)-phenyl]-piperazine-1-sulfonylamino}-proponic acid (I-70)

$^1$H NMR (DMSO-d6) δ: 1.29 (d, J = 7.2 Hz, 3H), 3.08-3.35 (m, 8H), 3.73-3.85 (m, 1H), 5.51 (dd, J = 10.8, 1.5 Hz, 1H), 5.87 (dd, J = 17.1, 1.5 Hz, 1H), 7.00 (d, J = 9.0 Hz, 2H), 7.46 (d, J = 9.0 Hz, 2H), 7.50-7.65 (m, 5H), 7.72 (d, J = 7.2 Hz, 1H), 7.86-7.97 (m, 3H), 8.30 (s, 1H), 12.73 (br s, 1H).

Synthesis of (R)-2-[4-(4-{8-[(E)-2-(1H-tetrazol-5-yl)-vinyl]-naphthalen-2-ylethynyl}-phenyl)-piperazine-1-sulfonylamino)-proponic acid (I-71)

$^1$H NMR (DMSO-d6) δ: 1.31 (d, J = 7.1 Hz, 3H), 3.10-3.36 (m, 8H), 3.75-3.87 (m, 1H), 7.03 (d, J = 8.1 Hz, 2H), 7.43-7.52 (m, 3H), 7.60-7.68 (m, 2H), 7.93 (d, J = 9.1 Hz, 1H), 8.00-8.05 (m, 2H), 8.11 (d, J = 8.1 Hz, 1H), 8.43-8.50 (m, 2H).

Synthesis of (R)-2-(4-{4-[8-((E)-2-chrolovinyl)-naphthalen-2-ylethynyl]-phenyl}-piperazine-1-sulfonylamino)-proponic acid (I-72)

$^1$H NMR (DMSO-d6) δ: 1.30 (d, J = 7.2 Hz, 3H), 3.09-3.38 (m, 8H), 3.74-3.87 (m, 1H), 7.02 (d, J = 9.1 Hz, 2H), 7.12 (d, J = 13.3 Hz, 1H), 7.48 (d, J = 9.1 Hz, 2H), 7.53 (t, J = 7.2 Hz, 1H), 7.61 (dd, J = 8.4, 1.3 Hz, 1H), 7.71 (d, J = 7.2 Hz, 1H), 7.81 (d, J = 13.3 Hz, 1H), 7.89-7.99 (m, 3H), 8.30 (d, J = 1.3 Hz, 1H).

Synthesis of (R)-2-{4-[4-(5-chrolo-6-methoxymethoxynaphthalen-2-ylethynyl)-phenyl]-piperazine-1-sulfonylamino}-proponic acid (I-73)

$^1$H NMR (DMSO-d6) δ: 1.31 (d, J=7.2 Hz, 3H), 3.15-3.21 (m, 4H), 3.46 (s, 3H), 3.82 bs, 1H), 5.44 (s, 2H), 7.01 (d, J=8.4 Hz, 2H), 7.62 (d, J=8.4 Hz, 2H), 7.70 (d, J=9.2 Hz, 1H), 7.89 (d, J=8.8 Hz, 1H), 7.89 (bs, 1H), 7.96 (d, J=9.2 Hz, 1H), 8.11 (d, J=8.8 Hz, 1H), 8.15 (s, 1H).

Synthesis of (R)-2-{4-[4-(5-chrolo-6-hydroxynaphthalen-2-ylethynyl)-phenyl]-piperazine-1-sulfonylamino}-proponic acid (I-74)

$^1$H NMR (DMSO-d6) δ: 1.31 (d, J=6.8 Hz, 3H), 3.15-3.17 (m, 4H), 3.81-3.83 (m, 1H), 7.00 (d, J=8.4 Hz, 2H), 7.33 (d, J=9.2 Hz, 1H), 7.44 (d, J=8.4 Hz, 2H), 7.64 (d, J=8.8 Hz, 1H), 7.81 (d, J=8.8 Hz, 1H), 7.89-7.90 (m, 1H), 8.02 (d, J=8.8 Hz, 1H), 8.07 (s, 1H).

Synthesis of (R)-2-{4-[4-(4-cyano-5-fluoro-6-methoxynaphthalen-2-ylethynyl)-phenyl]-piperazine-1-sulfonylamino}-proponic acid (I-75)

$^1$H NMR (DMSO-d6) δ: 1.31 (d, J=7.2 Hz, 3H), 3.16-3.21 (m, 4H), 4.04 (bs, 1H),7.02 (d, J=8.4 Hz, 2H), 7.46 (d, J=8.4 Hz, 2H), 7.77 (d, J=8.4 Hz, 1H), 7.88 (bs, 1H), 7.97 (d, J=9.2 Hz, 1H), 8.21 (s, 1H), 8.44 (s, 1H).

Synthesis of (R)-2-{4-[4-(8-chrolonaphthalen-2-ylethynyl)-phenyl]-piperazine-1-sulfonylamino}-proponic acid (I-76)

$^1$H NMR (DMSO-d6) δ: 1.30 (d, J=7.2 Hz, 3H), 3.11-3.21 (m, 4H), 3.78 (bs, 1H), 7.03 (d, J=8.7 Hz, 2H), 7.50 (d, J=8.7Hz, 2H), 7.56 (d, J=8.4 Hz, 1H), 7.70 (dd, J=1.2Hz, 8.4Hz, 1H), 7.75 (d, J=7.2 Hz, 1H), 7.90 (bs, 1H), 8.06 (d, J=8.7 Hz, 1H), 8.26 (s, 1H).

Synthesis of (R)-2-{4-[4-(8-trifluoromethylnaphthalen-2-ylethynyl)-phenyl]-piperazine-1-sulfonylamino}-proponic acid (I-77)

$^1$H NMR (DMSO-d6) δ: 1.31 (d, J=7.2 Hz, 3H), 3.16-3.21 (m, 4H), 3.82 (bs, 1H), 7.02 (d, J=8.4 Hz, 2H), 7.51 (d, J=8.4 Hz, 2H), 7.69 (t, J=8.0 Hz, 1H), 7.76 (d, J=8.0 Hz, 1H), 7.92 (bs, 1H), 8.04 (d, J=7.2 Hz, 1H), 8.14 (d, J=6.8Hz, 1H), 8.17 (s, 1H), 8.29 (d, J=8.0Hz, 1H).

Synthesis of (R)-2-{4-[4-(5-chrolonaphthalen-2-ylethynyl)-phenyl]-piperazine-1-sulfonylamino}-proponic acid (I-78)

$^1$H NMR (DMSO-d6) δ: 1.30 (d, J=7.2 Hz, 3H), 3.15-3.20 (m, 4H), 3.81 (bs, 1H), 7.01 (d, J=8.8 Hz, 2H), 7.47 (d, J=8.8 Hz, 2H), 7.55 (t, J=8.0 Hz, 1H), 7.73 (t, J=6.8 Hz, 1H), 7.92 (bs, 1H), 8.17 (d, J=7.2 Hz, 1H), 8.22 (s, 1H).

Synthesis of (R)-2-{4-[4-(5-trifluoromethylnaphthalen-2-ylethynyl)-phenyl]-piperazine-1-sulfonylamino}-proponic acid (I-79)

$^1$H NMR (DMSO-d6) δ: 1.31 (d, J=7.2 Hz, 3H), 3.15-3.20 (m, 4H), 3.81 (bs, 1H), 7.03 (d, J=8.4 Hz, 2H), 7.48 (d, J=8.4 Hz, 2H), 7.71 (t, J=8.4 Hz, 1H), 7.80 (d, J=9.2 Hz, 1H), 7.93 (bs, 1H), 8.01 (d, J=7.2 Hz, 1H), 8.08 (d, J=7.6 Hz, 1H), 8.28 (d, J=8.4 Hz, 1H), 8.22 (s, 1H).

Synthesis of (R)-2-{4-[4-(8-isopropylsulfamoylnaphthalen-2-ylethynyl)-phenyl]-piperazine-1-sulfonylamino}-proponic acid (I-80)

$^1$H NMR (DMSO-d6) δ: 0.89 (d, J=6.4 Hz, 6H), 1.30 (d, J=7.2 Hz, 3H), 3.13-3.17 (m, 4H), 3.38 (q, J=6.8 Hz, 1H), 3.79 (d, J=6.8 Hz, 1H), 7.03 (d, J=8.8 Hz, 2H), 7.49 (d, J=8.8 Hz, 2H), 7.66 (t, J=8.0 Hz, 1H), 7.20 (d, J=8.8 Hz, 1H), 7.86 (bs, 1H), 8.09 (t, J=8.8 Hz, 2H), 8.18 (d, J=7.6 Hz, 1H), 8.22 (d, J=8.4 Hz, 1H), 8.77 (s, 1H).

Synthesis of (R)-2-{4-[4-(8-sulfamoylnaphthalen-2-ylethynyl)-phenyl]-piperazine-1-sulfonylamino}-proponic acid (I-81)

$^1$H NMR (DMSO-d6) δ: 1.29 (d, J=7.2 Hz, 3H), 3.15-3.21 (m, 4H), 3.73-3.76 (m, 1H), 7.03 (d, J=8.8 Hz, 2H), 7.49 (d, J=8.8 Hz, 2H), 7.64-7.77 (m, 5H), 8.10 (d, J=7.6 Hz, 1H), 8.16-8.21 (m, 2H), 8.75 (s, 1H).

Synthesis of (R)-2-{4-[4-(8-methylsulfanylnaphthalen-2-ylethynyl)-phenyl]-piperazine-1-sulfonylamino}-proponic acid (I-82)

$^1$H NMR (DMSO-d6) δ: 1.30 (d, J=7.2 Hz, 3H), 2.62 (s, 2H), 3.15-3.20 (m, 4H), 3.80-3.83 (m, 1H), 7.01 (d, J=8.8 Hz, 2H), 7.46-7.51 (m, 4H), 7.62 (d, J=8.8 Hz, 1H), 7.75 (d, J=8.0 Hz, 1H), 8.22 (s, 1H).

Synthesis of (R)-2-{4-[4-(8-methanesulfonylnaphthalen-2-ylethynyl)-phenyl]-piperazine-1-sulfonylamino}-proponic acid (I-83)

$^1$H NMR (DMSO-d6) δ: 1.31 (d, J=7.2 Hz, 3H), 3.14-3.20 (m, 4H), 3.80-3.83 (m, 1H), 7.03 (d, J=8.0 Hz, 2H), 7.52 (d, J=8.0 Hz, 2H), 7.74-7.79 (m, 2H), 7.92-7.94 (m, 1H), 8.18 (d, J=8.0 Hz, 1H), 8.27 (d, J=8.0 Hz, 1H), 8.35 (d, J=8.0 Hz, 1H), 8.72 (s, 1H).

Synthesis of (R)-2-{4-[4-(8-methanesulfinylnaphthalen-2-ylethynyl)-phenyl]-piperazine-1-sulfonylamino}-proponic acid (I-84)

$^1$H NMR (DMSO-d6) δ: 1.30 (d, J=7.2 Hz, 3H), 2.86 (s, 3H), 3.16-3.21 (m, 4H), 3.80-3.83 (m, 1H), 7.02 (d, J=8.8 Hz, 2H), 7.10 (d, J=8.8 Hz, 1H), 7.71 (d, J=8.8 Hz, 1H), 7.77 (t, J=7.6 Hz, 1H), 7.88-7.94 (m, 1H), 8.07-8.16 (m, 4H).

Synthesis of (R)-2-{4-[4-(8-aminonaphthalen-2-ylethynyl)-phenyl]-piperazine-1-sulfonylamino}-proponic acid (I-85)

$^1$H NMR (DMSO-d6) δ: 1.31 (d, J=6.8 Hz, 3H), 3.12-3.17 (m, 4H), 3.82 (t, J=7.2 Hz, 1H), 6.69 (d, J=7.2 Hz, 1H), 7.01 (d, J=8.8 Hz, 2H), 7.07 (d, J=8.0 Hz, 1H), 7.23 (t, J=8.0 Hz, 1H), 7.42-7.45 (m, 3H), 7.72 (d, J=8.4 Hz, 1H), 7.93 (d, J=8.8 Hz, 1H), 8.30 (s, 1H).

Synthesis of (R)-2-{4-[4-(5-bromo-8-tert-butoxycarbonylaminonaphthalen-2-ylethynyl)-phenyl]-piperazine-1-sulfonylamino}-proponic acid (I-86)

$^1$H NMR (DMSO-d6) δ: 1.30 (d, J=7.2 Hz, 3H), 1.51 (s, 9H), 3.11-3.22 (m, 4H), 3.79 (bs, 1H), 7.03 (d, J=9.0 Hz, 2H), 7.48 (d, J=9.0 Hz, 2H), 7.62 (d, J=8.4 Hz, 1H), 7.75 (t, J=8.7 Hz, 1H), 7.86 (d, J=8.4Hz, 1H), 8.12 (d, J=9.0 Hz, 1H), 8.32 (s, 1H), 9.49 (s, 1H).

Synthesis of (R)-2-{4-[4-(8-amino-5-bromonaphthalen-2-ylethynyl)-phenyl]-piperazine-1-sulfonylamino}-proponic acid dihydrochloride (I-87)

$^1$H NMR (DMSO-d6) δ: 1.30 (d, J=7.2 Hz, 3H), 3.11-3.21 (m, 4H), 3.29-3.32 (m, 4H), 3.79 (bs, 1H), 6.82 (d, J=8.1 Hz, 1H), 7.03 (d, J=8.7 Hz, 2H), 7.46 (d, J=8.7 Hz, 2H), 7.62 (d, J=8.1 Hz, 1H), 7.67 (d, J=8.4 Hz, 1H), 7.95 (d, J=9.0Hz, 1H), 8.00 (d, J=9.0 Hz, 1H), 8.34 (s, 1H).

Synthesis of (R)-2-{4-[4-(8-benzylsulfanylnaphthalen-2-ylethynyl)-phenyl]-piperazine-1-sulfonylamino}-proponic acid (I-88)

$^1H$ NMR (DMSO-d6) δ: 1.31 (d, J=7.2 Hz, 3H), 3.15-3.22 (m, 4H), 3.82 (bs, 1H), 4.34 (s, 2H), 7.01 (d, J=8.8 Hz, 2H), 7.22-7.37 (m, 5H), 7.45-7.50 (m, 3H), 7.61 (d, J=8.0 Hz, 2H), 7.81 (d, J=8.0Hz, 1H), 7.92 (bs, 1H), 7.96 (d, J=8.4 Hz, 1H), 8.30 (s, 1H).

Synthesis of (R)-2-{4-[4-(8-phenylmethanesulfonylnaphthalen-2-ylethynyl)-phenyl]-piperazine-1-sulfonylamino}-proponic acid (I-89)

$^1H$ NMR (DMSO-d6) δ: 1.32 (d, J=7.2 Hz, 3H), 3.17-3.22 (m, 4H), 3.83 (t, J=8.0 Hz, 1H), 4.82 (s, 2H), 7.04-7.06 (m, 4H), 7.20-7.29 (m, 3H), 7.53 (d, J=8.8 Hz, 2H), 7.63 (d, J=8.0Hz, 1H), 7.74 (d, J=8.4 Hz, 1H), 7.96 (d, J=8.8 Hz, 1H), 8.32 (d, J=8.4 Hz, 1H), 8.62 (s, 1H).

Synthesis of (R)-2-{4-[4-(8-phenylmethanesulfinylnaphthalen-2-ylethynyl)-phenyl]-piperazine-1-sulfonylamino}-proponic acid (I-90)

$^1H$ NMR (DMSO-d6) δ: 1.31 (d, J=7.2 Hz, 3H), 3.16-3.21 (m, 4H), 3.81-3.84 (m, 1H), 4.23 (d, J=12.8 Hz, 1H), 4.40 (d, J=12.8 Hz, 1H), 6.93 (d, J=7.2 Hz, 2H), 7.04 (d, J=8.8 Hz, 2H), 7.17-7.25 (m, 3H), 7.51 (d, J=8.4 Hz, 2H), 7.59-7.69 (m, 3H), 7.94 (d, J=8.4 Hz, 1H), 8.07-8.11 (m, 3H).

Synthesis of (R)-2-{4-[4-(5-aminonaphthalen-2-ylethynyl)-phenyl]-piperazine-1-sulfonylamino}-proponic acid (I-91)

$^1H$ NMR (DMSO-d6) δ: 1.30 (d, J=7.2 Hz, 3H), 3.15-3.21 (m, 4H), 3.82 (t, J=7.2 Hz, 1H), 6.70 (d, J=7.2 Hz, 1H), 7.00 (d, J=8.8 Hz, 2H), 7.09 (d, J=8.0 Hz, 1H), 7.24 (d, J=8.0 Hz, 2H), 7.40-7.45 (m, 3H), 7.92-7.94 (m, 2H), 8.07 (d, J=8.8 Hz, 1H).

Synthesis of (S)-2-{4-[4-(8-carbamoylnaphthalen-2-ylethynyl)-phenyl]-piperazine-1-sulfonylamino}-pentanoic acid (I-92)

$^1H$ NMR (DMSO-d6) δ: 0.89 (t, J = 7.3 Hz, 3H), 1.31-1.46 (m, 2H), 1.53-1.68 (m, 2H), 3.08-3.50 (m, 8H), 3.59-3.67 (m, 1H), 7.03 (d, J = 9.1 Hz, 2H), 7.50 (d, J = 8.5 Hz, 2H), 7.55-7.75 (m, 4H), 7.98-8.14 (m, 3H), 8.47 (s, 1H).

Synthesis of (R)-2-{4-[4-(8-carbamoylnaphthalen-2-ylethynyl)-phenyl]-piperazine-1-sulfonylamino}-3-methylbutanoic acid (I-93)

$^1H$ NMR (DMSO-d6) δ: 0.93 (t, J = 6.5 Hz, 6H), 1.93-2.06 (m, 1H), 3.08-3.57 (m, 9H), 7.03 (d, J = 8.7 Hz, 2H), 7.50 (d, J = 8.7 Hz, 2H), 7.54-7.75 (m, 4H), 7.98-8.13 (m, 2H), 8.47 (s, 1H).

Synthesis of (R)-2-{4-[4-(8-acetylnaphthalen-2-ylethynyl)-phenyl]-piperazine-1-sulfonylamino}-proponic acid (I-94)

$^1H$ NMR (DMSO-d6) δ: 1.32 (d, J = 7.4 Hz, 3H), 2.77 (s, 3H), 3.09-3.45 (m, 8H), 3.74-3.86 (m, 1H), 5.79 (s, 1H), 7.04 (d, J = 8.9 Hz, 2H), 7.50 (d, J = 8.9 Hz, 2H), 7.62-7.72 (m, 2H), 8.06 (d, J = 8.5 Hz, 1H), 8.16-8.27 (m, 2H), 8.81 (s, 1H).

Synthesis of (R)-2-{4-[4-(8-{1-[(E)-methoxyimino]-ethyl}-naphthalen-2-ylethynyl)-phenyl]-piperazine-1-sulfonylamino}-proponic acid (I-95)

$^1H$ NMR (DMSO-d6) δ: 1.52 (d, J = 7.1 Hz, 3H), 2.39 (s, 3H), 3.27-3.36 (m, 4H), 3.36-3.45 (m, 4H), 4.09 (s, 3H), 4.11-4.18 (m, 1H), 5.07 (d, J = 8.8 Hz, 1H), 6.95 (d, J = 8.5 Hz, 2H), 7.45-7.54 (m, 4H), 7.59 (dd, J = 8.5, 1.6 Hz, 1H), 7.81-7.87 (m, 2H), 8.19 (s, 1H).

Synthesis of (R)-2-{4-[4-(8-{1-[(Z)-methoxyimino]-ethyl}-naphthalen-2-ylethynyl)-phenyl]-piperazine-1-sulfonylamino}-proponic acid (I-96)

$^1H$ NMR (DMSO-d6) δ: 1.32 (d, J = 7.1 Hz, 3H), 2.27 (s, 3H), 3.10-3.26 (m, 4H), 3.27-3.46 (m, 4H), 3.70 (s, 3H), 3.77-3.87 (m, 1H), 7.03 (d, J = 8.9 Hz, 2H), 7.43 (d, J = 6.9 Hz, 1H), 7.50 (d, J = 8.8 Hz, 2H), 7.56-7.69 (m, 3H), 7.88-8.05 (m, 3H).

Synthesis of (R)-2-{4-[4-(8-morpholin-4-ylmethylnaphthalen-2-ylethynyl)-phenyl]-piperazine-1-sulfonylamino}-proponic acid (I-97)

$^1$H NMR (DMSO-d6) δ: 1.32 (d, J = 7.1 Hz, 3H), 2.43-2.50 (m, 4H), 3.15-3.36 (m, 8H), 3.57-3.62 (m, 4H), 3.77-3.88 (m, 1H), 3.92 (s, 2H), 7.04 (d, J = 8.8 Hz, 2H), 7.48-7.54 (m, 4H), 7.60 (dd, J = 8.5, 1.4 Hz, 1H), 7.87-7.98 (m, 3H), 8.40 (s, 1H).

(R)-2-{4-[4-(8-ethylnaphthalen-2-ylethynyl)-phenyl]-piperazine-1-sulfonylamino}-proponic acid (I-98)

$^1$H NMR (DMSO-d6) δ: 1.32 (d, J = 7.1 Hz, 3H), 1.34 (t, J = 7.6 Hz, 3H), 3.06-3.37 (m, 10H), 3.77-3.86 (m, 1H), 7.04 (d, J = 8.8 Hz, 2H), 7.43-7.57 (m, 4H), 7.58-7.64 (m, 1H), 7.81 (d, J = 8.2 Hz, 1H), 7.87-7.95 (m, 1H), 7.96 (d, J = 8.8 Hz, 1H), 8.22 (s, 1H).

Synthesis of (R)-2-{4-[4-(8-methoxynaphthalen-2-ylethynyl)-phenyl]-piperazine-1-sulfonylamino}-proponic acid (I-99)

$^1$H NMR (DMSO-d6) δ: 1.32 (d, J = 7.4 Hz, 3H), 3.13-3.37 (m, 8H), 3.77-3.88 (m, 1H), 4.02 (s, 3H), 7.02-7.06 (m, 3H), 7.47-7.53 (m, 4H), 7.60 (dd, J = 8.4, 1.5 Hz, 1H), 7.89-8.00 (m, 2H), 8.27 (d, J = 1.1 Hz, 1H).

Synthesis of (R)-2-(4-{4-[8-(1-hydroxy-1-methylethyl)-naphthalen-2-ylethynyl]-phenyl}-piperazine-1-sulfonylamino)-proponic acid (I-100)

$^1$H NMR (DMSO-d6) δ: 1.49 (d, J = 7.1 Hz, 3H), 1.88 (s, 6H), 3.20-3.34 (m, 8H), 4.07-4.15 (m, 1H), 5.02 (d, J = 8.8 Hz1H), 6.87 (d, J = 8.8 Hz, 2H), 7.35-7.63 (m, 5H), 7.74 (d, J = 8.2 Hz, 1H), 7.80 (d, J = 8.8 Hz, 1H), 9.03 (s, 1H).

Synthesis of (R)-2-{4-[4-(8-acetylnaphthalen-2-ylethynyl)-phenyl]-piperazine-1-sulfonylamino}-3-methylbutanoic acid (I-101)

$^1$H NMR (DMSO-d6) δ: 0.92 (t, J = 5.8 Hz, 6H), 1.91-2.03 (m, 1H), 2.74 (s, 3H), 3.08-3.37 (m, 8H), 3.45-3.54 (m, 1H), 7.02 (d, J = 8.8 Hz, 2H), 7.48 (d, J = 8.8 Hz, 2H), 7.62-7.67 (m, 2H), 7.85 (d, J = 8.8 Hz, 1H), 8.04 (d, J = 8.8 Hz, 1H), 8.17 (d, J = 8.2 Hz, 1H), 8.21 (d, J = 7.4 Hz, 1H), 8.79 (s, 1H).

Synthesis of (R)-2-{4-[4-(6-methoxy3, 4-dihydronaphthalen-2-ylethynyl)-phenyl]-piperazine-1-sulfonylamino}-proponic acid (I-102)

$^1$H NMR (DMSO-d6) δ: 1.30 (d, J = 7.1 Hz, 3H), 2.21 (s, 3H), 3.10-3.37 (m, 8H), 3.67-3.76 (m, 1H), 5.06 (s, 1H), 5.52 (s, 1H), 7.03 (d, J = 9.1 Hz, 2H), 7.41 (d, J = 6.6 Hz, 1H), 7.48-7.62 (m, 5H), 7.89 (d, J = 8.0 Hz, 1H), 7.99 (d, J = 8.8 Hz, 1H), 8.11 (s, 1H).

Synthesis of (R)-2-(4-{4-[8-(1-fluoro-1-methylethyl)-naphthalen-2-ylethynyl]-phenyl}-piperazine-1-sulfonylamino)-proponic acid (I-103)

$^1$H NMR (DMSO-d6) δ: 1.32 (d, J = 7.1 Hz, 3H), 1.96 (d, J = 22.0 Hz, 6H), 3.10-3.41 (m, 8H), 3.76-3.84 (m, 1H), 7.04 (d, J = 8.8 Hz, 2H), 7.47-7.67 (m, 4H), 7.88-8.04 (m, 4H), 8.54 (s, 1H).

Synthesis of (R)-2-{4-[4-(8-ethylcarbamoylnaphthalen-2-ylethynyl)-phenyl]-piperazine-1-sulfonylamino}-proponic acid (I-104)

$^1$H NMR (DMSO-d6) δ: 1.19 (t, J=3.2 Hz, 3 H), 1.30 (d, J=7.2 Hz, 3H), 3.15 (m, 4H), 3.29-3.41 (m, 6H), 3.80 (m, 1H), 7.00 (d, J=8.7 Hz, 2H), 7.47 (d, J=8.9 Hz, 2H), 7.53-7.63 (m, 3H), 7.92 (d, J=8.9 Hz, 1H), 7.92 (d, J=8.5 Hz, 1H), 8.01 (d, J=8.5 Hz, 1H), 8.33 (s, 1H), 8.59 (t, J=5.5 Hz, 1H).

Synthesis of (S)-2-{4-[4-(8-carbamoylnaphthalen-2-ylethynyl)-phenyl]-piperazine-1-sulfonylamino}-proponic acid (I-105)

$^1$H NMR (DMSO-d6) δ: 1.30 (d, J=7.1 Hz, 3H), 3.16 (m, 4H), 3.31 (m, 4H), 3.80 (m, 1H), 7.00 (d, J=9.0 Hz, 2H), 7.47 (d, J=8.7 Hz, 2H), 7.55 (t, J=7.6 Hz, 1H), 7.60 (dd, J=8.5, 1.4 Hz, 1H), 7.66-7.69 (m, 2H), 7.93-8.08 (m, 4H), 8.44 (s, 1H), 12.74 (br s, 1H).

Synthesis of (R)-2-{4-[4-(8-benzylcarbamoylnaphthalen-2-ylethynyl)-phenyl]-piperazine-1-sulfonylamino}-proponic acid (I-106)

$^1$H NMR (DMSO-d6) δ: 1.29 (d, J=7.3 Hz, 3H), 3.15-3.38 (m, 8H), 3.77 (m, 1H), 4.56 (d, J=6.1 Hz, 2H), 7.01 (d, J=9.0 Hz, 2H), 7.28 (m, 1H), 7.35-7.47 (m, 6H), 7.55-7.63 (m, 3H), 7.69 (dd, J=7.0, 1.1 Hz, 1H), 7.99 (d, J=8.5 Hz, 1H), 8.03 (d, J=8.2 Hz, 1H), 8.31 (s, 1H), 9.18 (t, J=6.1 Hz, 1H).

Synthesis of (R)-2-{4-[4-(8-cyclopropylcarbamoylnaphthalen-2-ylethynyl)-phenyl]-piperazine-1-sulfonylamino}-proponic acid (I-107)

$^1$H NMR (DMSO-d6) δ: 0.58-0.63 (m, 2H), 0.72-0.78 (m, 2H), 1.30 (d, J=7.2 Hz, 3H), 2.97 (m, 1H), 3.13-3.35 (m, 8H), 3.81 (m, 1H), 7.01 (d, J=8.9 Hz, 2H), 7.48 (d, J=8.7 Hz, 2H), 7.54 (t, J=7.5 Hz, 1H), 7.58-7.63 (m, 2H), 7.91 (d, J=8.9 Hz, 1H), 7.99 (d, J=8.7 Hz, 1H), 8.01 (d, J=8.4 Hz, 1H), 8.34 (s, 1H), 8.62 (d, J=4.5 Hz, 1H), 12.71 (br s, 1H).

Synthesis of (R)-2-(4-{4-[8-(5-methyloxazol-2-yl)-naphthalen-2-ylethynyl]-phenyl}-piperazine-1-sulfonylamino)-proponic acid (I-108)

$^1$H NMR (DMSO-d6) δ: 1.30 (d, J=7.3 Hz, 3H), 2.46 (d, J=1.1 Hz, 3H), 3.12-3.17 (m, 4H), 3.30-3.35 (m, 4H), 3.80 (m, 1H), 7.01 (d, J=9.0 Hz, 2H), 7.19 (d, J=1.2 Hz, 1H), 7.48 (d, J=8.9 Hz, 2H), 7.63-7.68 (m, 2H), 7.94 (d, J=8.9 Hz, 1H), 8.04 (d, J=8.5 Hz, 1H), 8.09(d, J=7.9 Hz, 1H), 8.19 (d, J=7.3 Hz, 1H), 9.50 (s, 1H), 12.73 (br s, 1H).

Synthesis of (R)-2-{4-[4-(8-oxazol-2-ylnaphthalen-2-ylethynyl)-phenyl]-piperazine-1-sulfonylamino}-proponic acid (I-109)

$^1$H NMR (DMSO-d6) δ: 1.30 (d, J=7.2 Hz, 3H), 3.09-3.22 (m, 4H), 3.27-3.48 (m, 4H), 3.80 (m, 1H), 7.02 (d, J=9.0 Hz, 2H), 7.48 (d, J=8.9 Hz, 2H), 7.58 (s, 1H), 7.66-7.71 (m, 2H), 7.93 (d, J=8.7 Hz, 1H), 8.07 (d, J=8.9 Hz, 1H), 8.13(d, J=7.9 Hz, 1H), 8.24 (dd, J=7.4, 1.0 Hz, 1H), 8.37 (s, 1H), 9.46 (s, 1H).

Synthesis of (S)-2-{4-[4-(8-acetylnaphthalen-2-ylethynyl)-phenyl]-piperazine-1-sulfonylamino}-pentanoic acid (I-110)

$^1$H NMR (DMSO-d6) δ: 0.88 (t, J=7.3 Hz, 3H), 1.33-1.43 (m, 2H), 1.54-1.63 (m, 2H), 2.74 (s, 3H), 3.09-3.20 (m, 4H), 3.25-3.46 (m, 4H), 3.66 (m, 1H), 7.01 (d, J=8.9 Hz, 2H), 7.47 (d, J=8.9 Hz, 2H), 7.61-7.66 (m, 2H), 8.03 (d, J=8.7 Hz, 1H), 8.16 (d, J=8.1 Hz, 1H), 8.20(d, J=7.6 Hz, 1H), 8.77 (s, 1H).

Synthesis of (R)-2-(4-{4-[8-(1H-pyrrol-2-yl)-naphthalen-2-ylethynyl]-phenyl}-piperazine-1-sulfonylamino)-proponic acid (I-111)

$^1$H NMR (DMSO-d6) δ: 1.20 (d, J=6.7 Hz, 3H), 3.12-3.52 (m, 8H), 3.84 (m, 1H), 6.26 (s, 1H), 6.37 (s, 1H), 6.96-6.99 (m, 3H), 7.43 (d, J=8.7 Hz, 2H), 7.53-7.61 (m, 4H), 7.86 (d, J=6.6 Hz, 1H), 7.97 (d, J=8.2 Hz, 1H), 8.33 (s, 1H).

[Example 6]

Synthesis of (R)-2-{4-[4-(8-furan-2-yl-5,-6-dihydronaphthalen-2-ylethynyl)-phenyl]-piperazine-1-sulfonylamino}-proponic acid (I-112)

[Chemical formula 62]

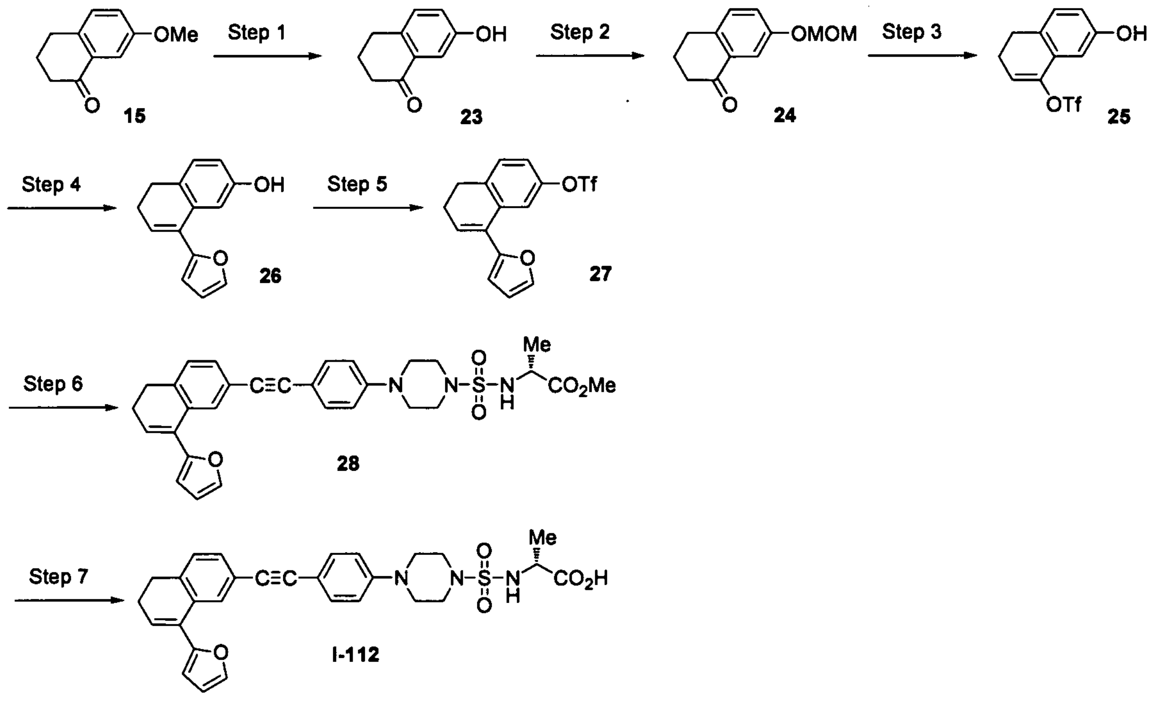


(Step 1)

A mixture of 7-methoxy-1-tetralone (15) (10.0 g, 56.7 mmol) and pyridine hydrochloride (50.0 g, 433 mmol) was stirred at 170°C for 7 hours. The reaction mixture was cooled to room temperature, and ice-water was added to the reaction mixture . The precipitated crystals were collected by filtration, and washed with water, and dried to give a pale yellow compound (23) (8.49 g, yield 92.3 %).

(Step 2)

To a suspension of sodium hydride (1.20 g, 30.0 mmol) in tetrahydrofuran (40 mL) was added a solution of the compound (23) (4.00 g, 24.7 mmol) in N, N-dimethylformamide (20 mL) under ice cooling, and this was stirred at room temperature for 20 minutes. To the reaction mixture was added chloromethyl ethyl ether (2.25 mL, 29.6 mmol) under ice cooling, and this was stirred at room temperature for 1 hour. To the reaction mixture was added saturated sodium bicarbonate aq., and the mixture was extracted with ethyl acetate. The organic layer was washed with saturated sodium chloride aq., dried over anhydrous magnesium sulfate, and concentrated under reduced pressure. The residue was subjected to silica gel chromatography, and the fractions eluted with n-hexane/ethyl acetate = 4/1 were collected, and evaporated to drynessto give a yellow compound (24) (4.69 g, yield 92.1%).

(Step 3)

To a suspension of the compound (24) (4.69 g, 22.7 mmol) and 2, 6-di-tert-butyl-4-methylpyridine (7.00 g, 34.1 mmol) in methylene chloride (50 mL) was added trifluoromethanesulfonic acid anhydride (5.36 mL, 31.8 mmol) under ice cooling, and the mixture was stirred at room temperature for 40 minutes. To the reaction mixture was added 0.2 mol/L hydrochloric acid, and the mixture was extracted with ethyl acetate. The organic layer was washed with saturated sodium bicarbonate aq. and saturated sodium chloride aq., dried over anhydrous magnesium sulfate, and concentrated under reduced pressure. The residue was subjected to silica gel chromatography, the fractions eluted with n-hexane/ethyl acetate = 5/1 were collected, and evaporated to dryness to afford a yellow compound (25) (3.12 g, yield 46.7%).

(Step 4)

To a solution of the compound (25) (0.527 g, 1.79 mmol), furan-2-boronic acid (0.401 g, 3.58 mmol) and barium hydroxide octahydrate (0.847 g, 2.69 mmol) in dimethoxyethane (70 mL)-water (30 mL) was degassed well, and the

atmosphere was replaced with nitrogen gas. Then, to the mixture was added tetrakis (triphenylphosphine) palladium (0) (0.207 g, 0.179 mmol), this was degassed again under nitrogen atmosphere, and stirred at 80°C for 1 hour. The reaction mixture was cooled to room temperature, water was added to it, and it was extracted with ethyl acetate. The organic layer was washed with saturated sodium chloride aq., dried over anhydrous magnesium sulfate, and concentrated under reduced pressure. The residue was subjected to silica gel chromatography, the fractions eluted with n-hexane/ethyl acetate = 2/1 were collected, and evaporated to dryness to give a brown compound (26) (0.0993 g, yield 26.1%).

(Step 5)

To a solution of the compound (26) (0.0954 g, 0.449 mmol) in methylene chloride (1 mL) was trifluoromethanesulfonic acid anhydride (0.113 mL, 0.673 mmol) under ice cooling, and the mixture was stirred at room temperature for 30 minutes. To the reaction mixture was added 5% citric acid aq., and the mixture was extracted with ethyl acetate. The organic layer was washed with saturated sodium chloride aq., dried over anhydrous magnesium sulfate, and concentrated under reduced pressure to give a brown compound (27) (0.141 g, yield 91.3%).

(Step 6)

To a solution of the compound (27) (0.139 g, 0.403 mmol) and the compound (8) (0.142 g, 0.403 mmol) in N, N-dimethylformamide (1.4 mL) was degassed well, and the atmosphere was replaced with nitrogen gas. Then, to the mixture were added bis(triphenylphosphine) palladium (II) chloride (0.014 g, 0.020 mmol), copper (I) iodide (0.0076 g, 0.040 mmol), and triethylamine (0.112 ml, 0.806 mmol), this was degassed again under nitrogen atmosphere, and stirred at 70°C for 18 hours. The reaction mixture was cooled to room temperature, neutralized with 5% citric acid aq, and extracted with ethyl acetate. The organic layer was washed with saturated sodium chloride aq., dried over anhydrous magnesium sulfate, and concentrated under reduced pressure. The residue was subjected to silica gel chromatography, the fractions eluted with chloroform/ethyl acetate = 9/1 were collected, and evaporated to dryness to afford a brown compound (28) (0.106 g, yield 48.4%).

(Step 7)

To a solution of the compound (28) (0.105 g, 0.192 mmol) in tetrahydrofuran (1 ml)-water (1 mL) was added 1 mol/L sodium hydroxide aq. (0.768 mL) at room temperature, and the mixture was stirred for 18 hours. The reaction mixture was poured into ice water-5% citric acid aq., and extracted with ethyl acetate. The organic layer was washed with water, dried over anhydrous sodium sulfate, and concentrated under reduced pressure. The residue was subjected to silica gel chromatography, the fractions eluted with chloroform/methanol/water=6/1/0.1 were collected, and concentrated under reduced pressure. The precipitated crystals were washed with ethyl acetate / n-hexane to afford (R)-2-{4-[4-(8-furan2-yl-5, 6-dihydronaphthalen-2-ylethynyl)-phenyl]-piperazine-1-sulfonylamino}-proponic acid (I-112) (0.0094 g, yield 9.2%).

$^1$ H NMR (DMSO-d6) δ: 1.29 (d, J=7.3 Hz, 3H), 2.32-2.39 (m, 2H), 2.76 (t, J=7.8 Hz, 2H), 3.08-3.16 (m, 4H), 3.25-3.30 (m, 4H), 3.79 (m, 1H), 6.51 (t, J=4.9 Hz, 1H), 6.57-6.70 (m, 2H), 6.96 (d, J=9.0 Hz, 2H), 7.28 (d, J=8.4 Hz, 1H), 7.34-7.40 (m, 4H), 7.74 (s, 1H), 7.92 (d, J=7.9 Hz, 1H), 12.72 (br s, 1H).

[Example 7]

Synthesis of (R)-2-[4-(4-{4-[(Z)-benzyloxyimino]-chroman-6-ylethynyl}-phenyl)-piperazine-1-sulfonylamino]-proponic acid (I-113)

[Chemical formula 63]

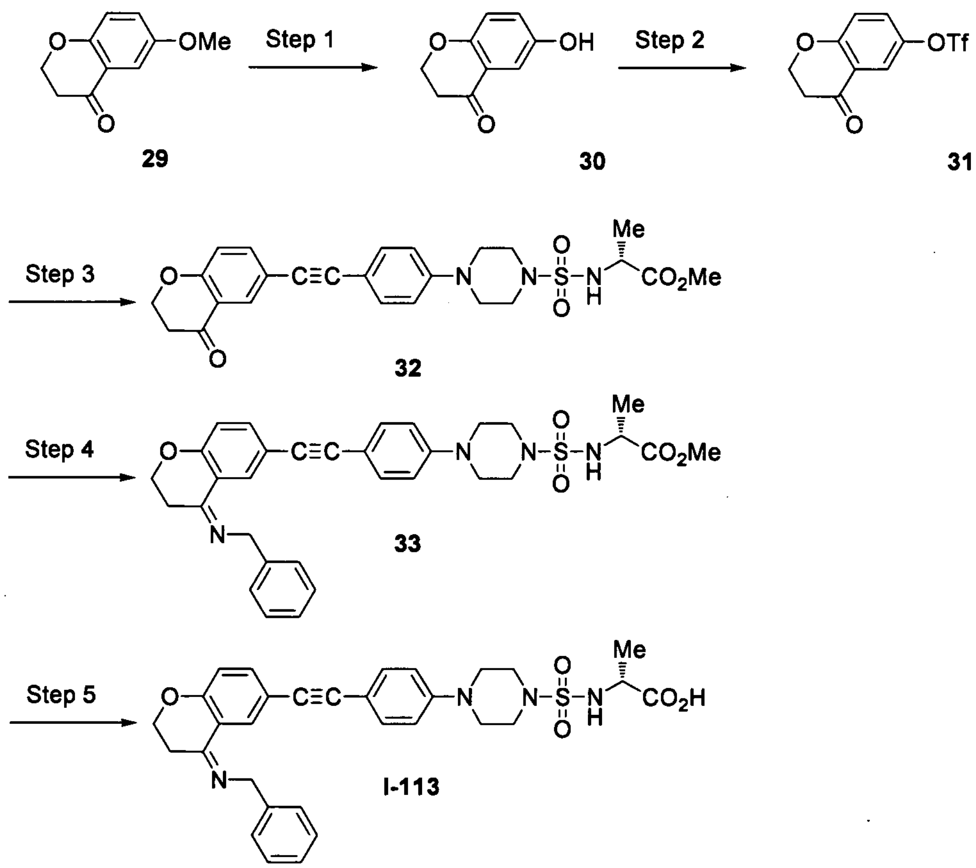


29 30 31 32 33 I-113

(Step 1)

A mixture of 6-methoxy chromanone (29) (0.500 g, 2.81 mmol) and pyridine hydrochloride (2.50 g, 21.6 mmol) was stirred at 170°C for 5 hours. The reaction mixture was cooled to room temperature, ice-water was added to the reaction mixture , and the mixture was extracted with ethyl acetate. The organic layer was washed with water, dried over anhydrous sodium sulfate, and concentrated to give a curde brown compound (30).

(Step 2)

To a suspension of the crude compound (30) in methylene chloride (5 mL) were added pyridine (1.40 mL, 17 mmol) and trifluoromethanesulfonic acid anhydride (0.57 mL, 3.4 mmol) under ice cooling, and the reaction mixture was stirred at room temperature for 20 minutes. To the reaction mixture was added 5% citric acid aq., and the mixture was extracted with ethyl acetate. The organic layer was washed with saturated sodium chloride aq., dried over anhydrous sodium sulfate, and concentrated under reduced pressure. The residue was subjected to silica gel chromatography, the fractions eluted with n-hexane/ethyl acetate = 5/1 were collected, and evaporated to dryness to give a brown compound (31) (0.590 g, total yield 72.0%).

(Step 3)

A solution of the compound (31) (0.260 g, 0.883 mmol) and the compound (8) (0.300 g, 0.854 mmol) in N, N-dimethylformamide (2.5 mL) was degassed well, and the atmosphere was replaced with nitrogen gas. Then, to the mixture were added bis(triphenylphosphine) palladium (II) chloride (0.030 g, 0.043 mmol), copper (I) iodide (0.0076 g, 0.089 mmol), and triethylamine (0.240 ml, 1.72 mmol), this was degassed again under nitrogen atmosphere, and stirred at 70°C for 2 hours. The reaction mixture was cooled to room temperature, neutralized with 5% citric acid aq., and extracted with ethyl acetate. The organic layer was washed with saturated sodium chloride aq., dried over anhydrous

magnesium sulfate, and concentrated under reduced pressure. The residue was subjected to silica gel chromatography, the fractions eluted with chloroform/n-hexane/ethyl acetate = 2/3/2 were collected, and recrystallized with n-hexane/ethyl acetate to afford a pale yellow compound (32) (0.176 g, yield 40.1 %).

(Step 4)

To a solution of the compound (32) (0.070 g, 0.14 mmol) in ethanol (1.4 mL) were added pyridine (0.1 mL) and O-benzyl hydroxyamine hydrochloride (0.067 g, 0.42 mmol) at room temperature, and the mixture was stirred at 80 °C for 1.5 hours. The reaction mixture was cooled to room temperature, and concentrated under reduced pressure. To the residue was added water, and the mixture was extracted with ethyl acetate. The organic layer was washed with water, dried over anhydrous sodium sulfate, and concentrated under reduced pressure. The residue was subjected to silica gel chromatography, the fractions eluted with n-hexane/ethyl acetate = 2/1 were collected, and evaporated to dryness to give a colorless compound (33) (0.055 g, yield 65%).

(Step 5)

To a solution of the compound (33) (0.054 g, 0.090 mmol) in dimethyl sulfoxide (1 mL) was added 1mol/L sodium hydroxide aq. (0.27 mL) at room temperature, and the mixture was stirred for 20 minutes. The reaction mixture was poured into ice water-5% citric acid aq., and the mixture was extracted with ethyl acetate. The organic layer was washed with water, dried over anhydrous sodium sulfate, and concentrated under reduced pressure. The residue was recrystallized from n-hexane/ethyl acetate to afford (R)-2-[4-(4-{4-[(Z)-benzyloxyimino]-chroman-6-ylethynyl}-phenyl)-piperazine-1-sulfonylamino]-proponic acid (I-113) (0.034 g, yield 65%).
$^{1}$H NMR (DMSO-d6) δ: 1.30 (d, J = 7.6 Hz, 3H), 2.92 (t, J = 6.6 Hz, 2H), 3.07-3.32 (m, 8H), 3.75-3.84 (m, 1H), 4.23 (t, J = 6.6 Hz, 2H), 5.23 (s, 2H), 6.93-7.00 (m, 3H), 7.29-7.44 (m, 8H), 7.83-7.95 (m, 2H).

[Example 8]

Synthesis of (R)-2-(4-{4-[3-((E)-2-cyanovinyl)-1-methyl-1H-indol-5-ylethynyl]-phenyl}-piperazine-1-sulfonylamino)-proponic acid (I-114)

[Chemical formula 64]

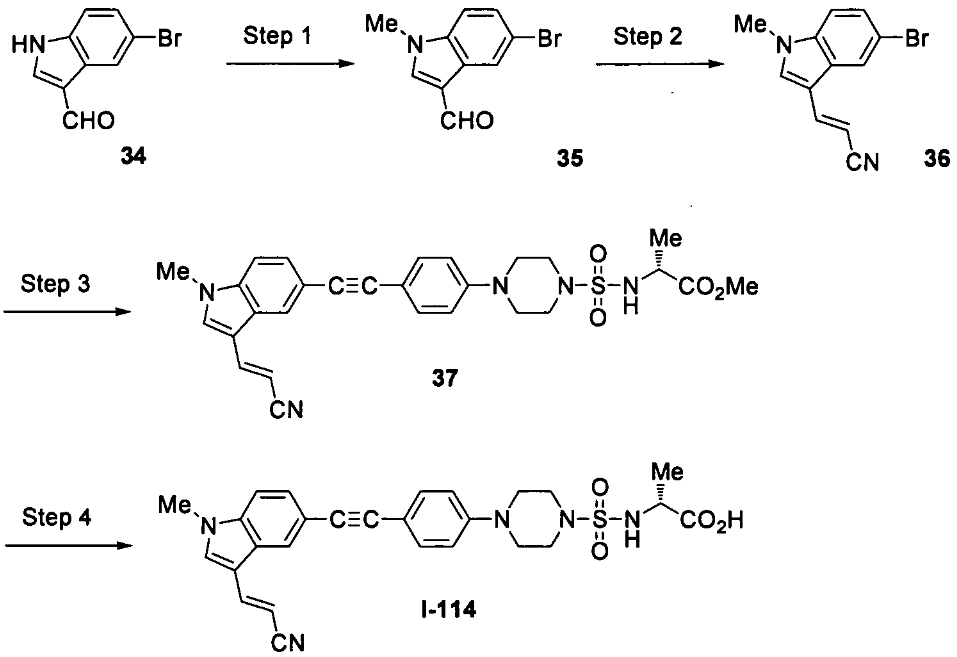


34 35 36 37 I-114

(Step 1)

To a solution of 5-bromo-1H-indole-3-carbaldehyde (34) (2.00 g, 8.93 mmol) in N,N-dimethylformamide (20 mL) were added cesium carbonate (3.49 g, 10.7 mmol) and methyl iodide (0.833 mL, 13.4 mmol) at room temperature, and the mixture was stirred at room temperature for 3 days. Water was added to the reaction mixture , which was extracted with ethyl acetate. The organic layer was washed with saturated sodium chloride aq., dried over anhydrous sodium sulfate, and concentrated under reduced pressure to give a brown compound (35) (1.87 g, yield 88.0%).

(Step 2)

To a suspension of sodium hydride (0.100 g, 2.50 mmol) in tetrahydrofuran (10 mL) were added a solution of the compound (35) (0.500 g, 2.10 mmol) and diethylcyanomethyl phosphonate (0.410 mL, 2.53 mmol) in tetrahydrofuran (5 mL) under ice cooling, and this was stirred at room temperature for 40 minutes. To the reaction mixture were added water and hydrochloric acid, the mixture was extracted with ethyl acetate. The organic layer was washed with water, dried over anhydrous sodium sulfate, and concentrated under reduced pressure. The residue was recrystallized with n-hexane/ethyl acetate to give a colorless compound (36) (0.183 g, yield 33.4%).

(Step 3)

To a solution of the compound (36) (0.170 g, 0.651 mmol) and the compound (8) (0.229 g, 0.652 mmol) in N, N-dimethylformamide (2 mL) was degassed well, and the atmosphere was replaced with nitrogen gas. Then, to the mixture were added bis(triphenylphosphine) palladium (II) chloride (0.023 g, 0.033 mmol), copper (I) iodide (0.012 g, 0.065 mmol), and triethylamine (0.180 ml, 1.29 mmol), this was degassed again under nitrogen atmosphere, and stirred at 70°C for 15 hours. The reaction mixture was cooled to room temperature, neutralized with 5% citric acid aq., and extracted with ethyl acetate. The organic layer was washed with saturated sodium chloride aq., dried over anhydrous sodium sulfate, and concentrated under reduced pressure. The residue was subjected to silica gel chromatography, and the fractions eluted with chloroform/n-hexane/ethyl acetate=2/3/2 were collected, and recrystallized with n-hexane/ethyl acetate to give a pale yellow compound (37) (0.107 g, yield 30.9 %).

(Step 4)

To a solution of the compound (37) (0.100 g, 0.188 mmol) in dimethyl sulfoxide (2 mL) was added 1 mol/L sodium hydroxide aq. (0.56 mL) at room temperature, and the mixture was stirred for 15 minutes. The reaction mixture was poured into ice water-5% citric acid aq., and extracted with ethyl acetate. The organic layer was washed with water,

dried over anhydrous sodium sulfate, and concentrated under reduced pressure. The residue was subjected to silica gel chromatography, and the fractions eluted with chloroform/methanol/water =32/9/1 were collected, and recrystallized with n-hexane/acetone to afford (R)-2-(4-{4-[3-((E)-2-cyanovinyl)-1-methyl-1H-indol-5-ylethynyl]-phenyl}-piperazine-1-sulfonylamino)-proponic acid(I-114) (0.0593 g, yield 60.9%).

$^1$H NMR (DMSO-d6) δ: 1.28 (d, J = 7.2 Hz, 3H), 3.09-3.32 (m, 8H), 3.72-3.81 (m, 1H), 3.83 (s, 3H), 6.12 (d, J = 16.9 Hz, 1H), 6.99 (d, J = 9.3 Hz, 2H), 7.36-7.43 (m, 3H), 7.55 (d, J = 8.7 Hz, 1H), 7.71 (d, J = 16.9 Hz, 1H), 7.86 (br s, 1H), 7.91 (s, 1H), 8.11 (s, 1H).

[Example 9]

Synthesis of (R)-2-{4-[4-(4-isopropylcarbamoyl-2-methylquinolin-6-ylethynyl)-phenyl]-piperazine-1-sulfonylamino}-proponic acid (I-115)

[Chemical formula 65]

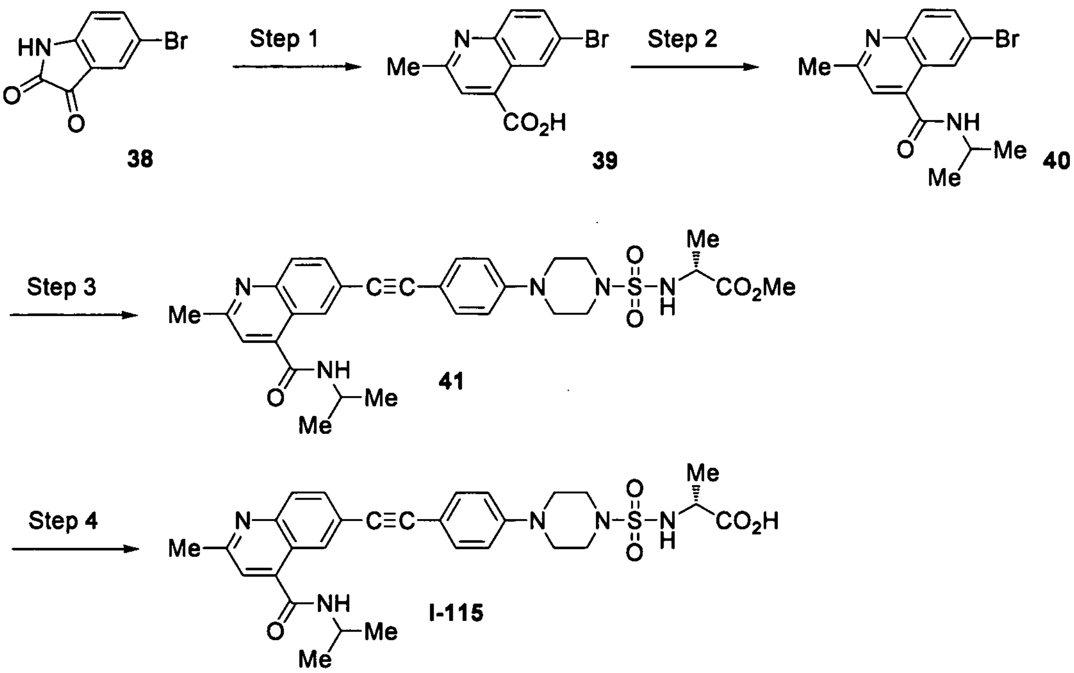


38 39 40 41 I-115

(Step 1)

To a solution of potassium hydroxide (6.70 g, 101 mmol) in ethanol (15 mL)-water (15 mL) were added 5-bromoisatine (38) (5.00 g, 22.1 mmol) and acetone (1.63 mL, 22.2 mmol), and this was stirred at 100°C for 9 hours. The reaction mixture was poured into ice water-5% citric acid aq.. The precipitated crystals were collected by filtration, and washed with water, and dried to give a pale brown compound (39) (3.65 g, yield 62.0 %).

(Step 2)

To a solution of the compound (39) (1.00 g, 3.76 mmol) in tetrahydrofuran (20 mL) were added oxalyl chloride (0.46 mL, 4.13 mmol) and a small amount of N, N-dimethylformamide, and this was stirred at room temperature for 3 hours. Then, to the reaction mixture was added isopropylamine (1.50 mL, 17.5 mmol), and the mixture was stirred for 1 hour. The reaction mixture was poured into water-5% citric acid aq., and extracted with ethyl acetate. The organic layer was washed with saturated sodium bicarbonate aq., and saturated sodium chloride aq., dried over anhydrous

sodium sulfate, and concentrated under reduced pressure. The residue was recrystallized from n-hexane / acetone to give a pale brown compound (40) (0.260 g, yield 22.6%).

(Step 3)

A solution of the compound (40) (0.219 g, 0.713 mmol) and the compound (8) (0.250 g, 0.711 mmol) in N, N-dimethylformamide (3 mL) was degassed well, and the atmosphere was replaced with nitrogen gas. Then, to the mixture were added bis(triphenylphosphine) palladium (II) chloride (0.025 g, 0.036 mmol), copper (I) iodide (0.014 g, 0.074 mmol), and triethylamine (0.200 ml, 1.43 mmol), this was degassed again under nitrogen atmosphere, and stirred at 70°C for 15 hours. The reaction mixture was cooled to room temperature, neutralized with 5% citric acid aq., and extracted with ethyl acetate. The organic layer was washed with saturated sodium chloride aq., dried over anhydrous sodium sulfate, and concentrated under reduced pressure. The residue was subjected to silica gel chromatography, the fractions eluted with ethyl acetate were collected, and recrystallized with n-hexane / acetone to afford a yellow compound (41) (0.230 g, yield 57.4 %).

(Step 4)

To a solution of the compound (41) (0.180 g, 0.312 mmol) in dimethyl sulfoxide (3.74 mL) was added 1 mol/L sodium hydroxide aq. (1.25 mL) at room temperature, and the mixture was stirred for 40 minutes. The reaction mixture was poured into ice water-5% citric acid aq., and the mixture was extracted with ethyl acetate. The organic layer was washed with water, dried over anhydrous sodium sulfate, and concentrated under reduced pressure. The residue was recrystallized from acetone to give (R)-2-{4-[4-(4-isopropylcarbamoyl-2-methylquinolin-6-ylethynyl)-phenyl]-piperazine-1-sulfonylamino}-proponic acid (I-115) (0.114 g, yield 64.9%).
$^1$H NMR (DMSO-d6) δ: 1.22 (d, J=6.6 Hz, 6H), 1.30 (d, J=7.5 Hz, 3H), 2.69 (s, 3H), 3.05-3.40 (m, 8H), 3.80 (m, 1H), 4.18 (m, 1H), 7.00 (d, J=9.0 Hz, 2H), 7.46 (s, 1H), 7.47 (d, J=8.7 Hz, 2H), 7.79 (dd, J=2.1, 8.7 Hz, 1H), 7.92 (m, 1H), 7.95 (d, J=8.7 Hz, 1H), 8.18 (d, J=1.8 Hz, 1H), 8.68 (d, J=7.8 Hz, 1H), 12.75 (br s, 1H).

[Example 10]

According to the same manner as Example 6 to 9, the following compounds were synthesized.

Synthesis of (R)-2-{4-[4-(6-methoxy-5, 6, 7, 8-tetrahydronaphthalen-2-ylethynyl)-phenyl]-piperazine-1-sulfonylamino}-proponic acid (I-116)

$^1$H NMR (DMSO-d6) δ: 1.30 (d, J=7.2 Hz, 3H), 1.72-1.80 (m, 1H), 1.94-1.97 (m, 1H), 2.67-2.73 (m, 2H), 2.78-2.84 (m, 1H), 3.00 (dd, J=4.0 Hz, 16.8 Hz, 1H), 3.15-3.20 (m, 8H), 3.58 (bs, 1H), 3.80 (bs, 1H), 6.97 (d, J=8.8 Hz, 2H), 7.08 (d, J=8.0 Hz, 1H), 7.21 (d, J=5.2 Hz, 2H), 7.37 (d, J=8.8 Hz, 2H), 7.88 (bs, 1H).

Synthesis of (R)-2-[4-(4-benzofuran-2-ylethynylphenyl)-piperazine-1-sulfonylamino]-proponic acid (I-117)

$^1$H NMR (DMSO-d6) δ: 1.30 (d, J=7.2 Hz, 3H), 3.10-3.37 (m, 8H), 3.80 (br s, 1H), 7.03 (d, J=8.7 Hz, 2H), 7.26-7.32 (m, 2H), 7.36-7.42 (m, 1H), 7.48 (d, J=8.7 Hz, 2H), 7.55-7.60 (m, 1H), 7.64-7.68 (m, 1H), 7.92 (br s, 1H).

Synthesis of (R)-2-{4-[4-(3-methylbenzofuran-2-ylethynyl)-phenyl]-piperazine-1-sulfonylamino]-proponic acid (I-118)

$^1$H NMR (DMSO-d6) δ: 1.30 (d, J=7.2 Hz, 3H), 2.34(s, 3H), 3.09-3.44 (m, 8H),
**[0212]** 3.78-3.87 (m, 1H), 7.01-7.06 (m, 2H), 7.28-7.34 (m, 1H), 7.36-7.43 (m, 1H), 7.46-7.55 (m, 3H), 7.62-7.66 (m, 1H), 7.90-7.96 (m, 1H).

Synthesis of (R)-2-{4-[4-(6-methoxy-4-methylquinolin-2-ylethynyl)-phenyl]-piperazine-1-sulfonylamino}-proponic acid (I-119)

$^1$H NMR (DMSO-d6) δ: 1.31 (d, J=6.8 Hz, 3H), 2.84 (s, 3H), 3.16 (br s, 4H), 3.33 (br s, 4H), 3.80-3.86 (m, 1H), 3.96 (s, 3H), 7.03 (d, J=8.4 Hz, 2H), 7.39-7.42 (m, 2H), 7.50 (d, J=8.4 Hz, 2H), 7.89-7.93 (m, 2H), 8.73 (s, 1H), 12.67 (br s, 1H).

Synthesis of (R)-2-{4-[4-(4-methoxy-3-methylbenzofuran-2-ylethynyl)-phenyl]-piperazine-1-sulfonylamino]-proponic acid (I-120)

$^1$H NMR (DMSO-d6) δ: 1.30 (d, J=7.2 Hz, 3H), 2.43 (s, 3H), 3.08-3.47 (m, 8H), 3.80 (br s, 1H), 3.89 (s, 3H), 6.79 (d, J=8.0 Hz, 1H), 7.02 (d, J=8.7 Hz, 2H), 7.10 (d, J=8.0 Hz, 1H), 7.29 (t, J=8.0 Hz, 1H), 7.46 (d, J=8.7 Hz, 2H), 7.93 (br s, 1H).

Synthesis of (R)-2-{4-[4-(5-ethyl-4-methoxy-3-methylbenzofuran-2-ylethynyl)-phenyl]-piperazine-1-sulfonylamino]-proponic acid (I-121)

$^1$H NMR (DMSO-d6) δ: 1.20 (t, J=7.6 Hz, 3H), 1.30 (d, J=7.2 Hz, 3H), 2.45 (s, 3H), 2.69 (q, J=7.6 Hz, 2H), 3.09-3.44 (m, 8H), 3.78-3.86 (m, 4H), 7.03 (d, J=8.7 Hz, 2H), 7.23-7.27 (m, 2H), 7.47 (d, J=8.7 Hz, 2H), 7.94 (br s, 1H).

Synthesis of (R)-2-[4-(4-dibenzofuran-2-ylethynylphenyl)-piperazine-1-sulfonylamino]-proponic acid (I-122)

$^1$H NMR (DMSO-d6) δ: 1.30 (d, J=7.2 Hz, 3H), 3.09-3.39 (m, 8H), 3.79 (br s, 1H), 7.00 (d, J=8.7 Hz, 2H), 7.40-7.76 (m, 7H), 7.88 (br s, 1H), 8.21 (d, J=7.2 Hz, 1H), 8.36 (s, 1H).

Synthesis of 5-{4-[4-((R)-1-carboxyethylsulfamoyl)-piperazin-1-yl]-phenylethynyl}-7-methoxybenzofuran-2-carboxylic acid (I-123)

$^1$H NMR (DMSO-d6) δ: 1.30 (d, J=7.2 Hz, 3H), 3.11-3.62 (m, 8H), 3.75-3.86 (m, 1H), 4.00 (s, 3H), 7.00 (d, J=8.7 Hz, 2H), 7.19 (s, 1H), 7.43 (d, J=8.7 Hz, 2H), 7.50 (s, 1H), 7.63 (s, 1H), 7.90-7.97 (m, 1H).

Synthesis of (R)-2-{4-[4-(2-carbamoyl7-methoxybenzofuran-5-ylethynyl)-phenyl]-piperazine-1-sulfonylamino}-proponic acid (I-124)

$^1$H NMR (DMSO-d6) δ: 1.29 (d, J=7.2 Hz, 3H), 3.09-3.37 (m, 8H), 3.74-3.86 (m, 1H), 3.98 (s, 3H), 6.99 (d, J=8.8 Hz, 2H), 7.14 (d, J=1.4 Hz, 1H), 7.41 (d, J=8.8 Hz, 2H), 7.48 (d, J=1.4 Hz, 1H), 7.51 (s, 1H), 7.69 (br s, 1H), 7.92 (d, J=9.0 Hz, 1H), 8.11 (br s, 1H).

Synthesis of (R)-2-{4-[4-(5-oxo-5, 6, 7, 8-tetrahydronaphthalen-2-ylethynyl)-phenyl]-piperazine-1-sulfonylamino}-proponic acid (I-125)

$^1$H NMR (DMSO-d6) δ: 1.30 (d, J=7.2 Hz, 3H), 1.98-2.10 (m, 2H), 2.61 (t, J=6.0 Hz, 2H), 2.95 (t, J=6.6 Hz, 2H), 3.05-3.40 (m, 8H), 3.80 (m, 1H), 7.01 (d, J=8.7 Hz, 2H), 7.38-7.52 (m, 4H), 7.86 (d, J=7.8 Hz, 1H), 7.91 (m, 1H), 12.74 (br s, 1H).

Synthesis of (R)-2-{4-[4-(4-ethoxy-3-methylbenzofuran-2-ylethynyl)-phenyl]-piperazine-1-sulfonylamino]-proponic acid (I-126)

$^1$H NMR (DMSO-d6) δ: 1.30 (d, J=7.2 Hz, 3H), 1.41 (t, J=6.8 Hz, 3H), 2.43 (s, 3H), 3.10-3.41 (m, 8H), 3.74-3.86 (m, 1H), 4.14 (q, J=6.8 Hz, 2H), 6.76 (d, J=8.0 Hz, 1H), 7.01 (d, J=8.5 Hz, 2H), 7.07 (d, J=8.0 Hz, 1H), 7.27 (t, J=8.0 Hz, 1H), 7.46 (d, J=8.5 Hz, 2H), 7.89 (br s, 1H).

Synthesis of (R)-2-[4-(4-{5-[(Z)-hydroxyimino]-5, 6, 7, 8-tetrahydronaphthalen-2-ylethynyl}-phenyl)-piperazine-1-sulfonylamino]-proponic acid (I-127)

$^1$H NMR (DMSO-d6) δ: 1.29 (d, J=7.2 Hz, 3H), 1.65-1.85 (m, 2H), 2.60-2.76 (m, 4H), 3.00-3.40 (m, 8H), 3.80 (m, 1H), 6.98 (d, J=8.4 Hz, 2H), 7.29 (d, J=8.4 Hz, 1H), 7.32 (s, 1H), 7.38 (d, J=8.7 Hz, 1H), 7.76-7.94 (m, 2H), 11.21 (s, 1H).

Synthesis of (R)-2-{4-[4-(8-oxo-5, 6, 7, 8-tetrahydronaphthalen-2-ylethynyl)-phenyl]-piperazine-1-sulfonylamino}-proponic acid (I-128)

$^1$H NMR (DMSO-d6) δ: 1.30 (d, J=7.2 Hz, 3H), 2.00-2.10 (m, 2H), 2.63 (t, J=6.9 Hz, 2H), 2.97 (d, J=5.7 Hz, 2H), 3.05-3.40 (m, 8H), 3.81 (m, 1H), 6.99 (d, J=9.0 Hz, 2H), 7.36-7.48 (m, 3H), 7.65 (dd, J=1.8, 7.8 Hz, 1H), 7.86-7.94 (m, 2H), 12.72 (br s, 1H).

Synthesis of (R)-2-{4-[4-(4, 6-dimethoxy-3-methylbenzofuran-2-ylethynyl)-phenyl]-piperazine-1-sulfonylamino]-proponic acid (I-129)

$^1$ H NMR (DMSO-d6) δ: 1.30 (d, J=7.2 Hz, 3H), 2.37 (s, 3H), 3.10-3.40 (m, 8H), 3.77-3.90 (m, 7H), 6.40 (d, J=1.8 Hz, 1H), 6.72 (d, J=1.8 Hz, 1H), 7.01 (d, J=8.4 Hz, 2H), 7.44 (d, J=8.4 Hz, 2H), 7.92 (br s, 1H).

Synthesis of (R)-2-[4-(4-{8-[(Z)-hydroxyimino]-5, 6, 7, 8-tetrahydronaphthalen-2-ylethynyl}-phenyl)-piperazine-1-sulfonylamino]-proponic acid (I-130)

$^1$ H NMR (DMSO-d6) δ: 1.29 (d, J=7.2 Hz, 3H), 1.75-1.85 (m, 2H), 2.60-2.80 (m, 4H), 3.00-3.40 (m, 8H), 3.79 (m, 1H), 6.97 (d, J=9.0 Hz, 2H), 7.21 (d, J=8.1 Hz, 1H), 7.35 (dd, J=1.8, 7.8 Hz, 1H), 7.40 (d, J=8.7 Hz, 2H), 7.84-7.96 (m, 2H), 11.18 (s, 1H).

Synthesis of (R)-2-{4-[4-(1-carbamoylmethyl-1H-indol-6-ylethynyl)-phenyl]-piperazine-1-sulfonylamino}-proponic acid (I-131)

$^1$ H NMR (DMSO-d6) δ: 1.30 (d, J=6.8 Hz, 3H), 3.13-3.19 (m, 4H), 3.28 (br s, 4H), 3.77-3.85 (m, 1H), 4.83 (s, 2H), 6.47 (s, 1H), 6.99 (d, J=8.4 Hz, 2H), 7.14 (d, J=8.4 Hz, 1H), 7.26 (s, 1H), 7.39-7.42 (m, 3H), 7.54-7.58 (m, 3H), 7.92 (d, J=8.8 Hz, 1H), 12.74 (br s, 1H).

Synthesis of (R)-2-{4-[4-(1-carbamoylmethyl-1H-indol-5-ylethynyl)-phenyl]-piperazine-1-sulfonylamino}-proponic acid (I-132)

$^1$ H NMR (DMSO-d6) δ: 1.30 (d, J=6.8 Hz, 3H), 3.13-3.19 (m, 4H), 3.27 (br s, 4H), 3.78-3.84 (m, 1H), 4.82 (s, 2H), 6.46 (s, 1H), 6.98 (d, J=7.6 Hz, 2H), 7.25-7.41 (m, 6H), 7.59 (s, 1H), 7.73 (s, 1H), 7.93 (d, J=8.4 Hz, 1H), 12.73 (br s, 1H).

Synthesis of (R)-2-{4-[4-(4,7-dimethoxy-3-methylbenzofuran-2-ylethynyl)-phenyl]-piperazine-1-sulfonylamino}-proponic acid (I-133)

$^1$ H NMR (DMSO-d6) δ: 1.30 (d, J=7.1 Hz, 3H), 2.40 (s, 3H), 3.09-3.44 (m, 8H), 3.77-3.89 (m, 7H), 6.65 (d, J=8.8 Hz, 1H), 6.88 (d, J=8.8 Hz, 1H), 7.02 (d, J=9.0 Hz, 2H), 7.47 (d, J=9.0 Hz, 2H), 7.91 (br s, 1H).

Synthesis of (R)-2-{4-[4-(6-methoxy-3-methylbenzofuran-2-ylethynyl)-phenyl]-piperazine-1-sulfonylamino}-proponic acid (I-134)

$^1$ H NMR (DMSO-d6) δ: 1.30 (d, J=7.1 Hz, 3H), 2.30 (s, 3H), 3.09-3.43 (m, 8H), 3.75-3.84 (m, 4H), 6.93 (dd, J=8.7, 2.1 Hz, 1H), 7.02 (d, J=9.1 Hz, 2H), 7.14 (d, J=2.1 Hz, 1H), 7.45 (d, J=9.1 Hz, 2H), 7.50 (d, J=8.7 Hz, 1H), 7.89 (br s, 1H).

Synthesis of (R)-2-{4-[4-(5-methoxy-3-methylbenzofuran-2-ylethynyl)-phenyl]-piperazine-1-sulfonylamino}-proponic acid (I-135)

$^1$ H NMR (DMSO-d6) δ: 1.30 (d, J=7.2 Hz, 3H), 2.32 (s, 3H), 3.09-3.46 (m, 8H), 3.74-3.85 (m, 4H), 6.96 (dd, J=8.9, 2.7 Hz, 1H), 7.02 (d, J=9.1 Hz, 2H), 7.13 (d, J = 2.7 Hz, 1H), 7.42 (d, J=8.9 Hz, 1H), 7.47 (d, J=9.1 Hz, 2H), 7.90 (br s, 1H).

Synthesis of (R)-2-[4-(4-{5-[(Z)-methoxyimino]-5, 6, 7, 8-tetrahydronaphthalen-2-ylethynyl}-phenyl)-piperazine-1-sulfonylamino]-proponic acid (I-136)

$^1$ H NMR (DMSO-d6) δ: 1.30 (d, J=7.5 Hz, 3H), 1.72-1.84 (m, 2H), 2.62-2.76 (m, 4H), 3.05-3.40 (m, 8H), 3.81 (m, 1H), 3.92 (s, 3H), 6.99 (d, J=9.0 Hz, 2H), 7.32 (d, J=8.4 Hz, 1H), 7.35 (s, 1H), 7.40 (d, J=8.7 Hz, 2H), 7.85 (d, J=8.4 Hz, 1H), 7.92 (d, J=8.7 Hz, 1H), 12.69 (br s, 1H).

Synthesis of (R)-2-[4-(4-{5-[(Z)-ethoxymino]-5, 6, 7, 8-tetrahydronaphthalen-2-ylethynyl}-phenyl)-piperazine-1-sulfonylamino]-proponic acid (I-137)

$^1$ H NMR (DMSO-d6) δ: 1.27 (t, J=6.9 Hz, 3H), 1.30 (d, J=7.2 Hz, 3H), 1.70-1.84 (m, 2H), 2.62-2.78 (m, 4H),

3.05-3.40 (m, 8H), 3.80 (m, 1H), 4.19 (q, J=6.9 Hz, 2H), 6.99 (d, J=9.0 Hz, 2H), 7.24 (d, J=8.1 Hz, 1H), 7.38-7.46 (m, 3H), 7.88-7.98 (m, 2H), 12.72 (br s, 1H).

Synthesis of (R)-2-{4-[4-(3-dimethylcarbamoyl-1-methyl-1H-indol-5-ylethynyl)-phenyl]-piperazine-1-sulfonylamino}-proponic acid (I-138)

$^1$ H NMR (DMSO-d6) δ: 1.30 (d, J=7.2 Hz, 3H), 3.15-3.19 (m, 4H), 3.79-3.85 (m, 4H), 6.99 (d, J=8.4 Hz, 2H), 7.34 (d, J=8.8 Hz, 1H), 7.41 (d, J=8.4 Hz, 1H), 7.52 (d, J=8.4 Hz, 1H), 7.90-7.92 (m, 1H), 7.99 (s, 1H).

Synthesis of (R)-2-[4-(4-{8-[(Z)-methoxymino]-5, 6, 7, 8-tetrahydronaphthalen-2-ylethynyl}-phenyl)-piperazine-1-sulfonylamino]-proponic acid (I-139)

$^1$ H NMR (DMSO-d6) δ: 1.29 (d, J=6.9 Hz, 3H), 1.70-1.82 (m, 2H), 2.62-2.78 (m, 4H), 3.05-3.40 (m, 8H), 3.80 (m, 1H), 3.93 (s, 3H), 6.99 (d, J=9.0 Hz, 2H), 7.24 (d, J=7.8 Hz, 2H), 7.38-7.46 (m, 3H), 7.86-7.96 (m, 2H), 12.70 (br s, 1H).

Synthesis of (R)-2-[4-(4-{8-[(Z)-isobutyl]-5, 6, 7, 8-tetrahydronaphthalen-2-ylethynyl}-phenyl)-piperazine-1-sulfonylamino]-proponic acid (I-140)

$^1$ H NMR (DMSO-d6) δ: 0.93 (d, J=6.6 Hz, 6H), 1.30 (d, J=7.2 Hz, 3H), 1.70-1.85 (m, 2H), 2.01 (m, 1H), 2.65-2.80 (m, 4H), 3.05-3.40 (m, 8H), 3.80 (m, 1H), 3.92 (d, J=6.6 Hz, 2H), 6.99 (d, J=8.7 Hz, 2H), 7.24 (d, J=8.1 Hz, 1H), 7.36-7.46 (m, 3H), 7.86-7.96 (m, 2H), 12.80 (br s, 1H).

Synthesis of (R)-2-{4-[4-(1, 2-dimethyl1H-indol-3-ylethynyl)-phenyl]-piperazine-1-sulfonylamino}-proponic acid (I-141)

$^1$ H NMR (DMSO-d6) δ: 1.30 (d, J=6.8 Hz, 3H), 2.53 (s, 3H), 3.13-3.19 (m, 4H), 3.24-3.29 (m, 4H), 3.71 (s, 3H), 3.79-3.85 (m, 1H), 6.98 (d, J=8.4 Hz, 2H), 7.09-7.19 (m, 2H), 7.40 (d, J=8.8 Hz, 2H), 7.47 (d, J=8.0 Hz, 1H), 7.57 (d, J=8.0 Hz, 1H), 7.92 (d, J=8.8 Hz, 1H).

Synthesis of (R)-2-{4-[4-(2-oxo-1, 2, 3, 4-tetrahydroquinolin-7-ylethynyl)-phenyl]-piperazine-1-sulfonylamino}-proponic acid (I-142)

$^1$ H NMR (DMSO-d6) δ: 1.30 (d, J=7.2 Hz, 3H), 2.46 (t, J=7.2 Hz, 2H), 2.89 (t, J=7.2 Hz, 2H), 3.10-3.19 (m, 4H), 3.28 (br s, 4H), 3.78-3.82 (m, 1H), 6.94-7.20 (m, 5H), 7.39 (d, J=8.4 Hz, 2H), 7.92 (d, J=8.4 Hz, 1H), 10.17 (s, 1H), 12.76 (br s, 1H).

Synthesis of (R)-2-(4-{4-[1-(2-methoxyethyl)-2-oxo-1, 2, 3, 4-tetrahydroquinolin-7-ylethynyl]-phenyl}-piperazine-1-sulfonylamino)-proponic acid (I-143)

$^1$ H NMR (DMSO-d6) δ: 1.30 (d, J=7.2 Hz, 3H), 2.55 (t, J=7.2 Hz, 2H), 2.86 (t, J=7.2 Hz, 2H), 3.11-3.19 (m, 4H), 3.25 (s, 3H), 3.29 (br s, 4H), 3.52 (t, J=7.2 Hz, 2H), 3.75-3.86 (m, 1H), 4.08 (t, J=7.2 Hz, 2H), 6.99 (d, J=8.4 Hz, 2H), 7.14 (d, J=8.0 Hz, 1H), 7.23 (d, J=8.0 Hz, 1H), 7.32 (s, 1H), 7.41 (d, J=8.4 Hz, 2H), 7.92 (d, J=8.4 Hz, 1H), 12.72 (br s, 1H).

Synthesis of (R)-2-(4-{4-[1-(2-methoxyethyl)-2-oxo-1, 2-dihydroquinolin-7-ylethynyl]-phenyl}-piperazine-1-sulfonylamino)-proponic acid (I-144)

$^1$ H NMR (DMSO-d6) δ: 1.31 (d, J=7.2 Hz, 3H), 3.12-3.20 (m, 4H), 3.25 (s, 3H), 3.31 (br s, 4H), 3.63 (t, J=7.2 Hz, 2H), 3.78-3.86 (m, 1H), 4.46 (t, J=7.2 Hz, 2H), 6.62 (d, J=9.6 Hz, 1H), 7.01 (d, J=8.4 Hz, 2H), 7.35 (d, J=8.0 Hz, 1H), 7.47 (d, J=8.4 Hz, 2H), 7.71 (s, 1H), 7.72 (d, J=8.0 Hz, 1H), 7.91-7.95 (m, 2H), 12.76 (br s, 1H).

Synthesis of (R)-2-{4-[4-(1-dimethylaminoxalyl-1, 2, 3, 4-tetrahydroquinolin-7-ylethynyl)-phenyl]-piperazine-1-sulfonylamino}-proponic acid (I-145)

$^1$ H NMR (DMSO-d6) δ: 1.29 (d, J=7.2 Hz, 3H), 1.90-1.99 (m, 2H), 2.84-2.89 (m, 2H), 2.94 (s, 3H), 3.00 (s, 3H), 3.16 (br s, 4H), 3.28 (br s, 4H), 3.56 (br s, 2H), 3.74-3.80 (m, 1H), 6.98 (d, J=8.4 Hz, 2H), 7.25 (s, 2H), 7.36-7.42 (m, 2H), 7.81 (br s, 1H), 8.11 (s, 1H).

Synthesis of (R)-2-{4-[4-(1-acetyl-1, 2, 3, 4-tetrahydroquinolin-7-ylethynyl)-phenyl]-piperazine-1-sulfonylamino}-proponic acid (I-146)

$^1$H NMR (DMSO-d6) δ: 1.30 (d, J=7.2 Hz, 3H), 1.85-1.91 (m, 2H), 2.20 (s, 3H), 2.73 (t, J=6.4 Hz, 2H), 3.11-3.19 (m, 4H), 3.28 (br s, 4H), 3.68 (t, J=6.4 Hz, 2H), 6.98 (d, J=8.4 Hz, 2H), 7.19 (s, 2H), 7.39 (d, J=8.4 Hz, 2H), 7.64 (br s, 2H).

Synthesis of (R)-2-[4-(4-{3-[(Z)-methoxyimino]indan-5-ylethynyl}-phenyl)-piperazine-1-sulfonylamino]-proponic acid (I-147)

$^1$H NMR (DMSO-d6) δ: 1.29 (d, J=7.2 Hz, 3H), 2.78-2.87 (m, 2H), 2.98-3.50 (m, 10H), 3.80 (m, 1H), 3.90 (s, 3H), 6.98 (d, J=9.0 Hz, 2H), 7.38-7.45 (m, 3H), 7.49 (dd, J=1.5, 7.8 Hz, 1H), 7.61 (s, 1H), 7.91 (d, J=8.1 Hz, 1H), 12.70 (br s, 1H).

Synthesis of (R)-2-{4-[4-(1-methoxyisoquinolin-7-ylethynyl)-phenyl]-piperazine-1-sulfonylamino}-proponic acid (I-148)

$^1$H NMR (DMSO-d6) δ: 1.30 (d, J=7.2 Hz, 3H), 3.05-3.50 (m, 8H), 3.80 (m, 1H), 4.07 (s, 3H), 7.01 (d, J=9.0 Hz, 2H), 7.41 (d, J=8.7 Hz, 1H), 7.47 (d, J=8.7 Hz, 2H), 7.80 (dd, J=1.5, 8.7 Hz, 1H), 7.91 (d, J=8.7 Hz, 1H), 7.92 (m, 1H), 8.03 (d, J=5.7 Hz, 1H), 8.24 (m, 1H).

Synthesis of (R)-2-{4-[4-(2-methoxyquinolin-6-ylethynyl)-phenyl]-piperazine-1-sulfonylamino}-proponic acid (I-149)

$^1$H NMR (DMSO-d6) δ: 1.30 (d, J=7.2 Hz, 3H), 2.66 (s, 3H), 3.05-3.50 (m, 8H), 3.80 (m, 1H), 7.01 (d, J=9.0 Hz, 2H), 7.40-7.48 (m, 3H), 7.75 (dd, J=1.8, 8.7 Hz, 1H), 7.89 (d, J=8.7 Hz, 1H), 7.92 (m, 1H), 8.11 (d, J=1.5 Hz, 1H), 8.24 (d, J=8.7 Hz, 1H).

Synthesis of (R)-2-[4-(4-{3-[(E)-methoxyimino]-2-oxo-2,3-dihydro-1H-indol-5-ylethynyl}-phenyl)-piperazine-1-sulfonylamino]-proponic acid (I-150)

$^1$H NMR (DMSO-d6) δ: 1.29 (d, J=7.5 Hz, 3H), 3.05-3.50 (m, 8H), 3.79 (m, 1H), 4.22 (s, 3H), 6.91 (d, J=8.1 Hz, 1H), 6.97 (d, J=9.0 Hz, 2H), 7.39 (d, J=8.7 Hz, 2H), 7.52 (dd, J=1.8, 8.1 Hz, 1H), 7.91 (m, 1H), 7.92 (d, J=1.5 Hz, 1H), 10.99 (m, 1H).

Synthesis of (R)-2-{4-[4-(4-methoxyquinolin-6-ylethynyl)-phenyl]-piperazine-1-sulfonylamino}-proponic acid (I-151)

$^1$H NMR (DMSO-d6) δ: 1.30 (d, J=7.2 Hz, 3H), 2.70 (s, 3H), 3.05-3.45 (m, 8H), 3.81 (m, 1H), 7.02 (d, J=8.7 Hz, 2H), 7.41 (d, J=4.5 Hz, 1H), 7.47 (d, J=8.7 Hz, 2H), 7.80 (dd, J=1.8, 8.7 Hz, 1H), 7.94 (d, J=9.0 Hz, 1H), 7.99 (d, J=8.7 Hz, 1H), 8.22 (d, J=1.8 Hz, 1H), 8.25 (d, J=4.2 Hz, 1H), 12.75 (br s, 1H).

Synthesis of (R)-2-[4-(4-{8-[(Z)-methoxyimino]-7-methyl-5, 6, 7, 8-tetrahydronaphthalen-2-ylethynyl}-phenyl)-piperazine-1-sulfonylamino]-proponic acid (I-152)

$^1$H NMR (DMSO-d6) δ: 1.09 (d, J=6.9 Hz, 3H), 1.29 (d, J=7.2 Hz, 3H), 1.65-1.90 (m, 2H), 2.67 (m, 1H), 2.91 (m, 1H), 3.05-3.50 (m, 9H), 3.79 (m, 1H), 3.93 (s, 3H), 6.98 (d, J=9.0 Hz, 2H), 7.23 (d, J=8.1 Hz, 1H), 7.39 (dd, J=1.8, 7.8 Hz, 1H), 7.41 (d, J=8.7 Hz, 2H), 7.92 (m, 1H), 7.92 (d, J=1.8 Hz, 1H), 12.78 (br s, 1H).

Synthesis of (R)-2-[4-(4-{8-[(Z)-methoxyimino]-7,7-dimethyl-5, 6, 7, 8-tetrahydronaphthalen-2-ylethynyl}-phenyl)-piperazine-1-sulfonylamino]-proponic acid (I-153)

$^1$H NMR (DMSO-d6) δ: 1.12 (s, 3H), 1.29 (d, J=7.2 Hz, 3H), 3.37 (s, 3H), 1.80-1.55 (m, 2H), 2.65-2.95 (m, 2H), 3.05-3.50 (m, 8H), 3.79 (m, 1H), 3.86-3.92 (m, 3H), 6.98 (d, J=9.0 Hz, 2H), 7.24 (m, 1H), 7.34-7.46 (m, 3H), 7.87 (d, J=1.5 Hz, 1H), 7.91 (m, 1H), 8.27 (d, J=1.2 Hz, 1H), 12.70 (br s, 1H).

Synthesis of (R)-2-{4-[4-(4-carbamoyl2-methylquinolin-6-ylethynyl)-phenyl]-piperazine-1-sulfonylamino}-proponic acid (I-154)

$^1$H NMR (DMSO-d6) δ: 1.30 (d, J=7.2 Hz, 3H), 2.69 (s, 3H), 3.05-3.50 (m, 8H), 3.80 (m, 1H), 7.00 (d, J=9.0 Hz, 2H), 7.48 (d, J=8.7 Hz, 2H), 7.52 (s, 1H), 7.79 (dd, J=2.1, 8.7 Hz, 1H), 7.88-7.98 (m, 3H), 8.2, 4-8.30 (m, 2H), 12.69 (br s, 1H).

Synthesis of (R)-2-{4-[4-(7,7-dimethyl8-oxo-5, 6, 7, 8-tetrahydronaphthalen-2-ylethynyl)-phenyl]-piperazine-1-sulfonylamino}-proponic acid (I-155)

$^1$H NMR (DMSO-d6) δ: 1.14 (s, 6H), 1.29 (d, J=7.2 Hz, 3H), 1.94 (t, J=6.3 Hz, 2H), 2.99 (t, J=6.3 Hz, 2H), 3.05-3.50 (m, 8H), 3.79 (m, 1H), 6.98 (d, J=9.0 Hz, 2H), 7.37 (d, J=7.8 Hz, 1H), 7.42 (d, J=9.0 Hz, 2H), 7.64 (dd, J=1.8, 7.8 Hz, 1H), 7.90 (m, 1H), 7.91 (d, J=1.8 Hz, 1H), 12.83 (br s, 1H).

Synthesis of (R)-2-{4-[4-(4-carbamoyl-2-ethyl-3-methylquinolin-6-ylethynyl)-phenyl]-piperazine-1-sulfonylamino}-proponic acid (I-156)

$^1$H NMR (DMSO-d6) δ: 1.30 (d, J=7.2 Hz, 3H), 1.32 (t, J=7.5 Hz, 3H), 2.40 (s, 3H), 2.98 (q, J=7.2 Hz, 2H), 3.05-3.50 (m, 8H), 3.80 (m, 1H), 7.00 (d, J=9.0 Hz, 2H), 7.48 (d, J=9.0 Hz, 2H), 7.72 (dd, J=1.8, 8.7 Hz, 1H), 7.79 (d, J=1.8 Hz, 1H), 7.92 (m, 1H), 7.93 (d, J=8.7 Hz, 1H), 8.04 (s, 1H), 8.20 (s, 1H), 12.72 (br s, 1H).

Synthesis of (R)-2-{4-[4-(4-isopropylcarbamoylquinolin-6-ylethynyl)-phenyl]-piperazine-1-sulfonylamino}-proponic acid (I-157)

$^1$H NMR (DMSO-d6) δ: 1.23 (d, J=6.6 Hz, 6H), 1.30 (d, J=7.2 Hz, 3H), 3.05-3.40 (m, 8H), 3.79 (m, 1H), 4.19 (m, 1H), 7.01 (d, J=9.0 Hz, 2H), 7.48 (d, J=8.7 Hz, 2H), 7.55 (d, J=4.5 Hz, 1H), 7.85 (dd, J=2.1, 8.7 Hz, 1H), 7.95 (d, J=8.1 Hz, 1H), 8.06 (d, J=8.7 Hz, 1H), 8.23 (d, J=1.8 Hz, 1H), 8.73 (d, J=7.8 Hz, 1H), 8.96 (d, J=4.5 Hz, 1H), 12.76 (br s, 1H).

Synthesis of (R)-2-{4-[4-(1-oxo-1,2-dihydroisoquinolin-7-ylethynyl)-phenyl]-piperazine-1-sulfonylamino}-proponic acid (I-158)

$^1$H NMR (DMSO-d6) δ: 1.30 (d, J=7.2 Hz, 3H), 3.05-3.45 (m, 8H), 3.80 (m, 1H), 6.58 (d, J=7.2 Hz, 1H), 7.01 (d, J=9.0 Hz, 2H), 7.22 (m, 1H), 7.45 (d, J=8.4 Hz, 2H), 7.68 (d, J=8.7 Hz, 1H), 7.76 (dd, J=1.8, 8.1 Hz, 1H), 7.92 (m, 1H), 8.23 (d, J=1.5 Hz, 1H), 11.39 (m, 1H), 12.76 (br s, 1H).

Synthesis of (R)-2-{4-[4-(4-methoxycarbamoyl-2-methylquinolin-6-ylethynyl)-phenyl]-piperazine-1-sulfonylamino}-proponic acid (I-159)

$^1$H NMR (DMSO-d6) δ: 1.30 (d, J=7.2 Hz, 3H), 3.69 (s, 1H), 3.05-3.40 (m, 8H), 3.80 (m, 1H), 3.82 (s, 1H), 7.00 (d, J=8.7 Hz, 2H), 7.48 (d, J=8.7 Hz, 2H), 7.53 (s, 1H), 7.82 (dd, J=2.1, 8.7 Hz, 1H), 7.93 (m, 1H), 7.97 (d, J=8.7 Hz, 1H), 8.17 (s, 1H), 11.99 (s, 1H), 12.68 (br s, 1H).

Synthesis of (R)-2-{4-[4-(11-isopropylcarbamoyl-7, 8, 9, 10-tetrahydro-6H-cyclohepta[b]quinolin-2-ylethynyl)-phenyl]-piperazine-1-sulfonylamino}-proponic acid (I-160)

$^1$H NMR (DMSO-d6) δ: 1.19 (d, J=6.3 Hz, 3H), 1.23 (d, J=6.6 Hz, 3H), 1.30 (d, J=7.5 Hz, 3H), 1.55-1.95 (m, 6H), 2.80-2.90 (m, 2H), 3.05-3.40 (m, 10H), 3.80 (m, 1H), 4.24 (m, 1H), 7.01 (d, J=8.7 Hz, 2H), 7.46 (d, J=8.7 Hz, 2H), 7.66-7.76 (m, 2H), 7.86-8.00 (m, 2H), 8.64 (d, J=7.8 Hz, 1H), 12.73 (br s, 1H).

Synthesis of (R)-2-[4-(4-{3-[(E)-isobutoxyimino]indan-5-ylethynyl}-phenyl)-piperazine-1-sulfonylamino]-proponic acid (I-161)

$^1$H NMR (DMSO-d6) δ: 0.92 (d, J=6.6 Hz, 6H), 1.29 (d, J=6.9 Hz, 3H), 2.00 (m, 1H), 2.80-2.88 (m, 2H), 3.00-3.50 (m, 10H), 3.80 (m, 1H), 3.89 (d, J=6.9 Hz, 2H), 6.99 (d, J=9.0 Hz, 2H), 7.38-7.52 (m, 4H), 7.62 (s, 1H), 7.93 (d, J=7.5 Hz, 1H), 12.82 (br s, 1H).

Synthesis of (R)-2-[4-(4-{4-[(Z)-methoxyimino]-chroman-6-ylethynyl}-phenyl)-piperazine-1-sulfonylamino]-proponic acid (I-162)

$^1$H NMR (DMSO-d6) δ: 1.29 (d, J = 7.4 Hz, 3H), 2.86 (t, J = 6.0 Hz, 2H), 3.12-3.30 (m, 8H), 3.74-3.85 (m, 1H), 3.94 (s, 3H), 4.23 (t, J = 6.0 Hz, 2H), 6.95 (d, J = 9.1 Hz, 2H), 6.98 (d, J = 8.8 Hz, 1H), 7.38-7.43 (m, 3H), 7.87 (d, J = 1.9 Hz, 1H), 7.89 (br s, 1H).

Synthesis of (R)-2-[4-(4-{8-[(Z)-2-(tert-butoxycarbonylmethylamino)-ethoxyimono]-5, 6, 7, 8-tetrahydronaphthalen-2-ylethynyl}-phenyl)-piperazine-1-sulfonylamino]-proponic acid (I-163)

$^1$H NMR (DMSO-d6) δ: 1.29 (d, J = 7.4 Hz, 3H), 1.37 (s, 9H), 1.70-1.81 (m, 2H), 2.62-2.76 (m, 4H), 2.79-2.86 (m, 3H), 3.11-3.34 (m, 8H), 3.44-3.53 (m, 2H), 3.76-3.83 (m, 1H), 4.24 (t, J = 4.9 Hz, 2H), 6.99 (d, J = 8.0 Hz, 2H), 7.24 (d, J = 8.0 Hz, 1H), 7.40 (d, J = 8.0 Hz, 3H), 7.86-7.98 (m, 2H).

Synthesis of (R)-2-[4-(4-{8-[(Z)-2-methylaminoethoxyimino]-5, 6, 7, 8-tetrahydronaphthalen-2-ylethynyl}-phenyl)-piperazine-1-sulfonylamino]-proponic acid dihydrochloride (I-164)

$^1$H NMR (DMSO-d6) δ: 1.30 (d, J = 7.1 Hz, 3H), 1.72-1.84 (m, 2H), 2.60 (t, J = 5.2 Hz, 3H), 2.72-2.80 (m, 3H), 3.10-3.33 (m, 12H), 3.75-3.87 (m, 1H), 4.37-4.43 (m, 2H), 6.99 (d, J = 8.8 Hz, 2H), 7.26 (d, J = 8.2 Hz, 1H), 7.39-7.46 (m, 3H), 7.90-7.98 (m, 2H), 8.89 (br s, 2H).

Synthesis of (R)-2-[4-(4-{4-[(Z)-isobutoxyimino]-chroman-6-ylethynyl}-phenyl)-piperazine-1-sulfonylamino]-proponic acid (I-165)

$^1$H NMR (DMSO-d6) δ: 0.92 (d, J = 6.9 Hz, 6H), 1.28 (d, J = 7.2 Hz, 3H), 1.93-2.07 (m, 1H), 2.87 (t, J = 6.1 Hz, 2H), 3.06-3.31 (m, 8H), 3.72-3.85 (m, 1H), 3.93 (d, J = 6.9 Hz, 2H), 4.22 (t, J = 6.1 Hz, 2H), 6.90-7.00 (m, 3H), 7.36-7.42 (m, 3H), 7.82-7.94 (m, 2H).

Synthesis of (R)-2-[4-(4-{4-[(Z)-ethoxyimino]-chroman-6-ylethynyl}-phenyl)-piperazine-1-sulfonylamino]-proponic acid (I-166)

$^1$H NMR (DMSO-d6) δ: 1.27 (d, J = 7.2 Hz, 3H), 1.29 (d, J = 6.9 Hz, 3H), 2.86 (t, J = 6.0 Hz, 2H), 3.09-3.32 (m, 8H), 3.74-3.85 (m, 1H), 4.16-4.26 (m, 4H), 6.92-7.01 (m, 3H), 7.37-7.43 (m, 3H), 7.87-7.95 (m, 2H).

Synthesis of (S)-2-[4-(4-{4-[(Z)-methoxyimino]-chroman-6-ylethynyl}-phenyl)-piperazine-1-sulfonylamino]-pentanoic acid (I-167)

$^1$H NMR (DMSO-d6) δ: 0.88 (t, J = 7.0 Hz, 3H), 1.30-1.46 (m, 2H), 1.54-1.64 (m, 2H), 2.86 (t, J = 6.0 Hz, 2H), 3.08-3.32 (m, 8H), 3.62-3.73 (m, 1H), 3.94 (s, 3H), 4.23 (t, J = 6.0 Hz, 2H), 6.93-7.00 (m, 3H), 7.38-7.43 (m, 3H), 7.86-7.94 (m, 2H).

Synthesis of (R)-2-{4-[4-(8-isopropenylnaphthalen-2-ylethynyl)-phenyl]-piperazine-1-sulfonylamino}-proponic acid (I-168)

$^1$H NMR (DMSO-d6) δ: 1.31 (d, J = 7.1 Hz, 3H), 2.43 (t, J = 8.1 Hz, 1H), 2.82 (t, J = 8.0 Hz, 1H), 3.08-3.47 (m, 8H), 3.76-3.78 (m, 1H), 3.77 (s, 3H), 6.74-6.85 (m, 4H), 6.98 (d, J = 9.1 Hz, 2H), 7.10 (d, J = 8.5 Hz, 1H), 7.34 (d, J = 8.8 Hz, 2H), 7.82-7.94 (m, 1H).

Synthesis of (R)-2-{4-[4-(8-thiophen-2-yl-5, 6-dihydronaphthalen-2-ylethynyl)-phenyl]-piperazine-1-sulfonylamino}-proponic acid (I-169)

$^1$H NMR (DMSO-d6) δ: 1.28 (d, J=7.2 Hz, 3H), 2.33-2.40 (m, 2H), 2.80 (t, J=7.9 Hz, 2H), 3.10-3.15 (m, 4H), 3.22-3.29 (m, 4H), 3.78 (m, 1H), 6.33 (t, J=4.7 Hz, 1H), 6.94 (d, J=8.9 Hz, 2H), 7.11-7.15 (m, 2H), 7.27-7.30 (m, 2H), 7.34-7.37 (m, 3H), 7.54 (dd, J=4.7, 1.5 Hz, 1H), 7.91 (d, J=7.9 Hz, 1H).

Synthesis of 2-(7-{4-[4-((R)-1-carboxyethylsulfamoyl)-piperazin-1-yl]-phenylethynyl}-3,4-dihydronaphthalene-1-yl)-pyrrole-1-carboxylic acid tert-butyl ester (I-170)

$^1$H NMR ($CDCl_3$) δ: 1.23 (s, 9H), 1.50 (d, J=7.2 Hz, 3H), 2.36-2.45 (m, 2H), 2.85 (t, J=7.9 Hz, 2H), 3.32-3.35 (m, 4H), 3.49-3.54 (m, 4H), 4.11 (m, 1H), 5.03 (d, J=9.5 Hz, 1H), 6.14 (t, J=4.7 Hz, 1H), 6.18 (dd, J=3.2, 2.0 Hz, 1H), 6.24 (t, J=3.3 Hz, 1H), 6.82 (d, J=1.4 Hz, 1H), 7.05-7.12 (m, 3H), 7.25-7.28 (m, 1H), 7.42-7.45 (m, 3H).

Synthesis of (R)-2-(4-{4-[8-(1H-pyrrole2-yl)-5,6-dihydronaphthalen-2-ylethynyl]-phenyl}-piperazine-1-sulfonylamino)-proponic acid (I-171)

$^1$ H NMR (DMSO-d6) δ: 1.26 (d, J=7.4 Hz, 3H), 2.26-2.33 (m, 2H), 2.72-2.79 (m, 2H), 3.11-3.44 (m, 8H), 3.69 (m, 1H), 6.09-6.10 (m, 2H), 6.21 (t, J=4.9 Hz, 1H), 6.79 (dd, J=4.8, 2.4 Hz, 1H), 6.95 (d, J=8.9 Hz, 2H), 7.25 (d, J=7.9 Hz, 1H), 7.31-7.39 (m, 4H), 10.96 (s, 1H).

[Example 11]

Synthesis of (R)-2-(4-{4-[2-(4-chrolobenzylcarbamoyl)-pyridin-4-ylethynyl]-phenyl}-piperazine-1-sulfonylamino)-proponic acid (III-1)

[Chemical formula 66]

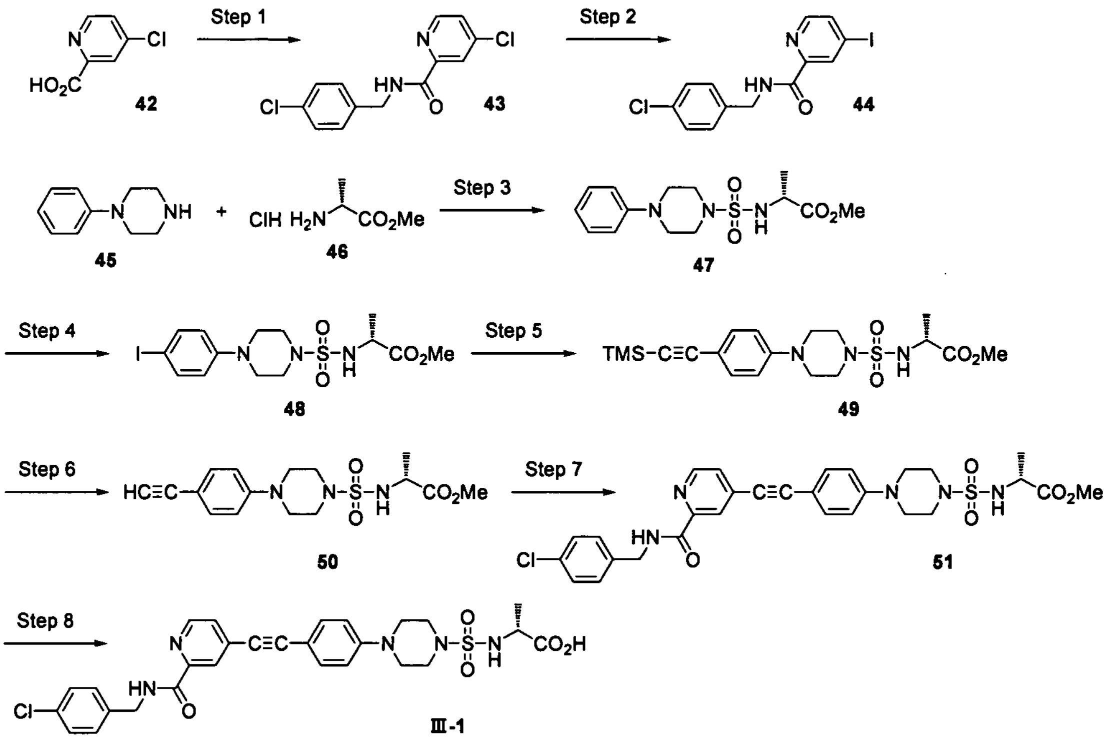


(Step 1)

To a solution of 4-chrolopyridine-2-carboxylic acid (42) (0.800 g, 5.08 mmol) in N, N-dimethylformamide (2 mL) was added 1-hydroxybenzotriazole (0.755 g, 5.59 mmol) and 1-ethyl-3-(3-dimethylaminopropyl) carbodiimide hydrochloride (1.46 g,7.62 mmol) at room temperature, and the mixture was stirred for 15 minites. Then 4-chrolobenzylamine (0.744 mL, 6.09 mmol) was added to the reaction mixture , which was stirred for 3 hours. To the reaction mixture was added 5% citric acid aq., and the mixture was extracted with ethyl acetate. The organic layer was washed with saturated sodium bicarbonate aq., and saturated sodium chloride aq., dried over anhydrous magnesium sulfate, and concentrated under reduced pressure.

The residue was subjected to silica gel chromatography, the fractions eluted with n-hexane/ethyl acetate = 1/1 were collected, and evaporated to dryness to give a colorless compound (43) (1.13 g, yield 79%).

(Step 2)

To a solution of the compound (43) (0.400 g, 1.42 mmol) in acetonitrile (12 mL) were added acetyl chloride (0.304 mL, 4.27 mmol) and sodium iodide (4.27 g, 28.5 mmol) at room temperature, the mixture was stirred under reflux

for 12 hours. After the reaction mixture was cooled to room temperature, saturated sodium bicarbonate aq. was added to the reaction mixture , which was extracted with ethyl acetate. The organic layer was washed with sodium thiosulfate aq., and saturated sodium chloride aq., dried over anhydrous magnesium sulfate, and concentrated under reduced pressure to afford a crude pale yellow compound (44) (0.560 g).

(Step 3)

To a solution of 2-chloroethanol (2.47 mL, 37.0 mmol) in acetonitrile (40 mL) was added chlorosulfonyl isocyanate (3.18 mL, 36.5 mmol) under ice cooling, and this was stirred at room temperature for 2 hours. Separately, to a solution of D-alanine methyl ester hydrochloride in acetonitrile (40 mL) were added N-methylmorpholine (19.7 mL, 179 mmol) under ice cooling, and the mixture was stirred for 3 hours. The former reaction mixture was added to the latter which was stirred at the room temperature for 20 hours. To the reaction mixture were added 1-phenylpiperazine (45) (5.60 g, 34.5 mmol) and chlorotrimethylsilane (2.30 mL, 18.1 mmol), and the mixture was stirred at 80°C for 5 hours. The reaction mixture was poured into ice-water, neutralized with 5% citric acid aq., and extracted with ethyl acetate. The organic layer was washed with saturated sodium bicarbonate aq., and saturated sodium chloride aq., dried over anhydrous sodium sulfate, and concentrated under reduced pressure. The residue was subjected to silica gel chromatography, the fractions eluted with n-hexane/ethyl acetate= 1/2 were collected, and evaporated to dryness to afford a colorless compound (47) (6.26 g, yield 55 %).

(Step 4)

To a solution of sodium bicarbonate (2.21 g, 26.3 mmol) in Water (43 mL) was added methylene chloride(43 mL), the compound (47) (4.30 g, 13.1 mmol) and iodine(3.50 g, 13.8 mmol), and the mixture was stirred at room temperature for 3.5 hours. To the reaction mixture was added 1mol/L sodium thiosulfate aq.(10 mL), and the reaction mixture was extracted with chloroform. The organic layer was washed with water, dried over anhydrous sodium sulfate, and concentrated under reduced pressure. The residue was washed with n-hexane to give a colorless compound (48) (6.24 g) quantitatively.

(Step 5)

To a solution of the compound (48) (6.24 g, 13.8 mmol) in tetrahydrofuran (125 mL) was added trimethylsilylacetylene (2.92 mL, 20.7 mmol), and this was degassed under nitrogen atmosphere. Then, to the mixture were added bis(triphenylphosphine) palladium (II) chloride (0.48 g, 0.68 mmol), copper (I) iodide (0.26 g, 1.37 mmol), and triethylamine (3.84 mL, 27.6 mmol) , and this was degassed again under nitrogen atmosphere, and stirred at room temperature for 15 hours. The reaction mixture was poured into ice-water, neutralized with 5% citric acid aq., and extracted with ethyl acetate. The organic layer was washed with saturated sodium bicarbonate aq., and saturated sodium chloride aq., dried over anhydrous sodium sulfate, and concentrated under reduced pressure. The residue was subjected to silica gel chromatography, the fractions eluted with n-hexane/ethyl acetate = 3/1 were collected, and evaporated to dryness to give a pale brown compound (49) (4.32 g, yield 74%).

(Step 6)

To a solution of the compound (49) (1.80 g, 4.25 mmol) in methanol (18 mL) was added potassium carbonate (0.76 g, 5.50 mmol) at room temperature, and the mixture was stirred at room temperature for 1.5 hours. The reaction mixture was poured into ice water-5% citric acid aq., and extracted with ethyl acetate. The organic layer was washed with water, dried over anhydrous sodium sulfate, and concentrated under reduced pressure. The residue was recrystallized from n-hexane/ethyl acetate to afford a pale yellow compound (50) (1.12 g, yield 75%).

(Step 7)

A solution of the compound (44) (0.200 g, 0.537 mmol) and the compound (50) (0.179 g, 0.510 mmol) in N, N-dimethylformamide (2 mL) was degassed well, and the atmosphere was replaced with nitrogen gas. Then, to the mixture were added bis(triphenylphosphine) palladium (II) chloride (0.019 g,0.027 mmol), copper (I) iodide (0.010 g, 0.054 mmol), and triethylamine (0.15 mL, 0.71 mmol), this was degassed again under nitrogen atmosphere, and stirred at room temperature for 1 hours. The reaction mixture was neutralized with 5% citric acid aq, and extracted with ethyl acetate. The organic layer was washed with saturated sodium bicarbonate aq, and saturated sodium chloride aq., dried over anhydrous sodium sulfate, and concentrated under reduced pressure. The residue was subjected to silica gel chromatography, the fractions eluted with n-hexane/ethyl acetate = 1/1 were collected, and evaporated to drynes to give a pale

yellow compound (51) (0.245 g, yield 77%).

(Step 8)

To a solution of the compound (51) (0.181 g, 0.303 mmol) in dimethyl sulfoxide (3.6 mL) was added 1 mol/L sodium hydroxide aq. (1.2 mL) at room temperature, and the mixture was stirred for 30 minutes. The reaction mixture was poured into ice water-5% citric acid aq., and extracted with ethyl acetate. The organic layer was washed with water, dried over anhydrous sodium sulfate, and concentrated under reduced pressure. The residue was recrystallized from n-hexane/ethyl acetate to afford (R)-2-(4-{4-[2-(4-chrolobenzylcarbamoyl)-pyridin-4-ylethynyl]-phenyl}-piperazine-1-sulfonylamino)-proponic acid (III-1) (0.130 g, yield 74%).

$^{1}H$ NMR (DMSO-d6) δ: 1.30 (d, J=7.1 Hz, 3H), 2.00 (m, 1H), 3.14-3.39 (m, 8H), 3.80 (m, 1H), 4.48 (d, J=6.2 Hz, 2H), 7.02 (d, J=8.9 Hz, 2H), 7.33-7.39 (m, 4H), 7.50 (d, J=8.7 Hz, 2H), 7.66 (dd, J=5.0, 1.5 Hz, 1H), 7.91 (d, J=9.1 Hz, 1H), 8.02 (s, 1H), 8.65 (d, J=5.0 Hz, 1H), 9.44 (t, J=6.5 Hz, 1H).

[Example 12]

Synthesis of (R)-2-(4-{4-[3-(cyanomethylcarbamoyl)-4-(2-methoxyethoxy)-phenylethynyl]-phenyl}-piperazine-1-sulfonylamino)-3-methylbutanoic acid (III-2)

[Chemical formula 67]

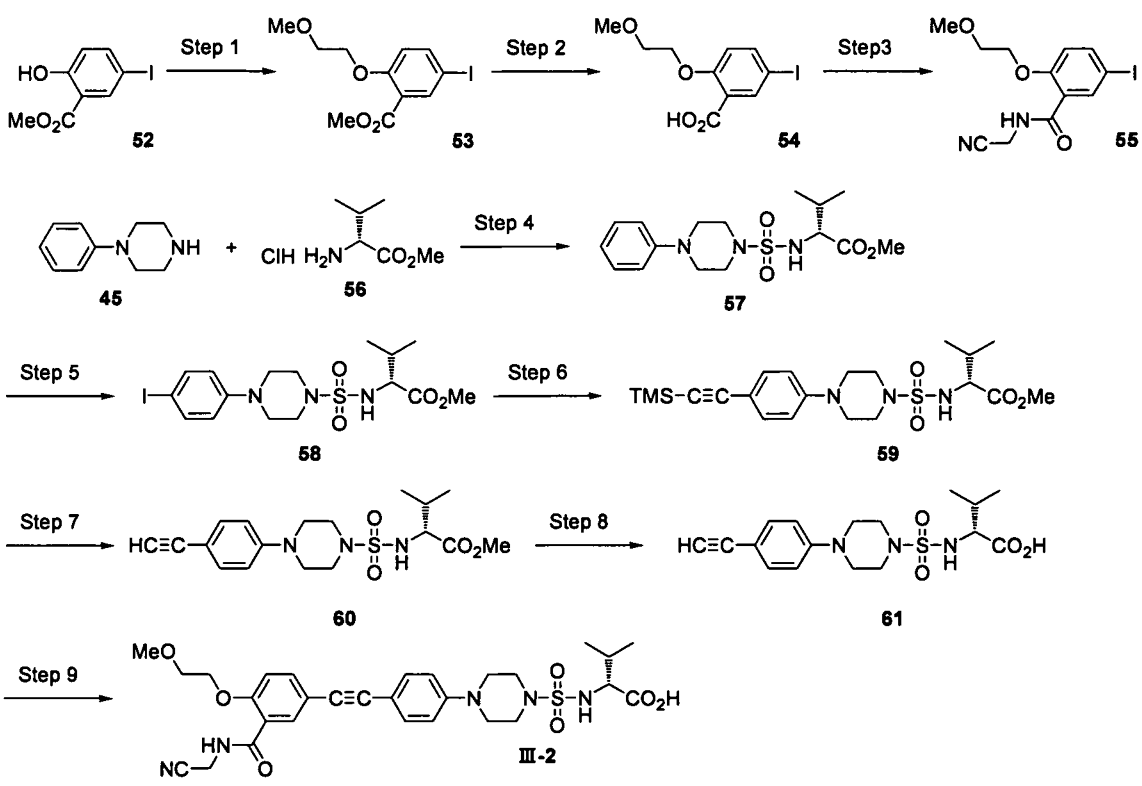


(Step 1)

To a solution of the 2-hydroxy-5-iodobenzoic acid methyl ester (52) (2.00 g, 7.19 mmol) in N, N-dimethylformamide (30 mL) were added potassium carbonate (1.49 g, 10.8 mmol) and 2-bromoethyl methyl ether (0.743 mL, 7.91

mmol) at room temperature, and the mixture was stirred for 20 hours. To the reaction mixture was added 2 mol/L hydrochloric acid, and the mixture was extracted with ethyl acetate. The organic layer was washed with saturated sodium bicarbonate aq., and saturated sodium chloride aq., dried over anhydrous sodium sulfate, and concentrated under reduced pressure. The residue was subjected to silica gel chromatography, the fractions eluted with n-hexane/ethyl acetate = 2/1 were collected, and evaporated to dryness to give a pale yellow compound (53) (1.27 g, yield 53%).

(Step 2)

The compound (54) was prepared from the compound (53) according to the procedure for Step 8 in Example 11.

(Step 3)

To a solution of the compound (54) (0.100 g, 0.310 mmol) in N, N-dimethylformamide (1 mL) were added 1-hydroxybenzotriazole (0.013 g, 0.093 mmol), 1-ethyl-3-(3-dimethylaminopropyl) carbodiimide hydrochloride (0.089 g, 0.47 mmol) and aminoacetonitrile (0.744 mL, 6.09 mmol) at room temperature, and the mixture was stirred for 4 hours. To the reaction mixture was added 5% citric acid aq., and the mixture was extracted with ethyl acetate. The organic layer was washed with saturated sodium bicarbonate aq., and saturated sodium chloride aq., dried over anhydrous sodium sulfate, and concentrated under reduced pressure. The residue was subjected to silica gel chromatography, the fractions eluted with n-hexane/ethyl acetate =1/1 were collected, and concentrated under reduced pressure to give a colorless compound (55) (0.104 g, yield 93%).

(Step 4)

The compound (57) was prepared from the compound (45) and the compound (56) according to the procedure for Step 3 in Example 11.

(Step 5)

The compound (58) was prepared from the compound (57) according to the procedure for Step 4 in Example 11.

(Step 6)

The compound (59) was prepared from the compound (58) according to the procedure for Step 5 in Example 11.

(Step 7)

The compound (60) was prepared from the compound (59) according to the procedure for Step 6 in Example 11.

(Step 8)

To a solution of the compound (60) (3.00 g, 7.91 mmol) in dimethyl sulfoxide (36 mL) was added 2 mol/L sodium hydroxide aq. (12 mL) at room temperature, and the mixture was stirred at 50°C for 4 hours. The reaction mixture was cooled to room temperature. To the reaction mixture was added 5% citric acid aq. (100 mL) and water(50 mL), and the precipitated crystals were collected by filtration to give a colorless compound (61) (2.60 g, yield 90%).

(Step 9)

A solution of the compound (61) (0.105 g, 0.288 mmol) and the compound (55) (0.104 g, 0.288 mmol) in N, N-dimethylformamide (1.5 mL) was degassed well, and the atmosphere was replaced with nitrogen gas. Then, bis(triphenylphosphine) palladium (II) chloride (0.010 g, 0.014 mmol), copper (I) iodide (0.0055 g, 0.029 mmol), and triethylamine (0.080 mL, 0.58 mmol) were added, this was degassed again under nitrogen atmosphere, and stirred at room temperature for 1 hours.

The reaction mixture was neutralized with 5% citric acid aq., and extracted with ethyl acetate. The organic layer was washed with saturated sodium bicarbonate aq., and saturated sodium chloride aq., dried over anhydrous sodium sulfate, and concentrated under reduced pressure. The residue was subjected to silica gel chromatography, the fractions eluted with chloroform / methanol =4 / 1 were collected, and concentrated under reduced pressure to afford (R)-2-(4-{4-[3-(cyanomethylcarbamoyl)-4-(2-methoxyethoxy)-phenylethynyl]-phenyl}-piperazine-1-sulfonylamino) -3-methylbutanoic acid (III-2) (0.110 g, yield 64%).

$^1$ H NMR ($CDCl_3$) δ: 0.94 (d, J=6.9 Hz, 3H), 1.06 (d, J=6.9 Hz, 3H), 2.13-2.19 (m, 1H), 3.18-3.25 (m, 4H), 3.32-3.34 (m, 4H), 3.52 (s, 3H), 3.79-3.84 (m, 2H), 4.27-4.30 (m, 2H), 4.37 (d, J=5.7 Hz, 2H), 5.12-5.15 (m, 1H), 6.83 (d, J=8.7 Hz, 2H), 6.95 (d, J=8.7 Hz, 1H),7.38 (d, J=8.7 Hz, 2H), 7.57 (dd, J=2.1, 8.7 Hz, 1H), 8.30 (d, J=2.1 Hz, 1H), 8.71 (t, J=5.7Hz, 1H).

[Example 13]

Synthesis of (R)-2-(4-{4-[2-(3-chrolobenzylcarbamoyl)-4-fluorophenylethynyl]-phenyl}-piperazine-1-sulfonylamino)-proponic acid (III-3)

[Chemical formula 68]

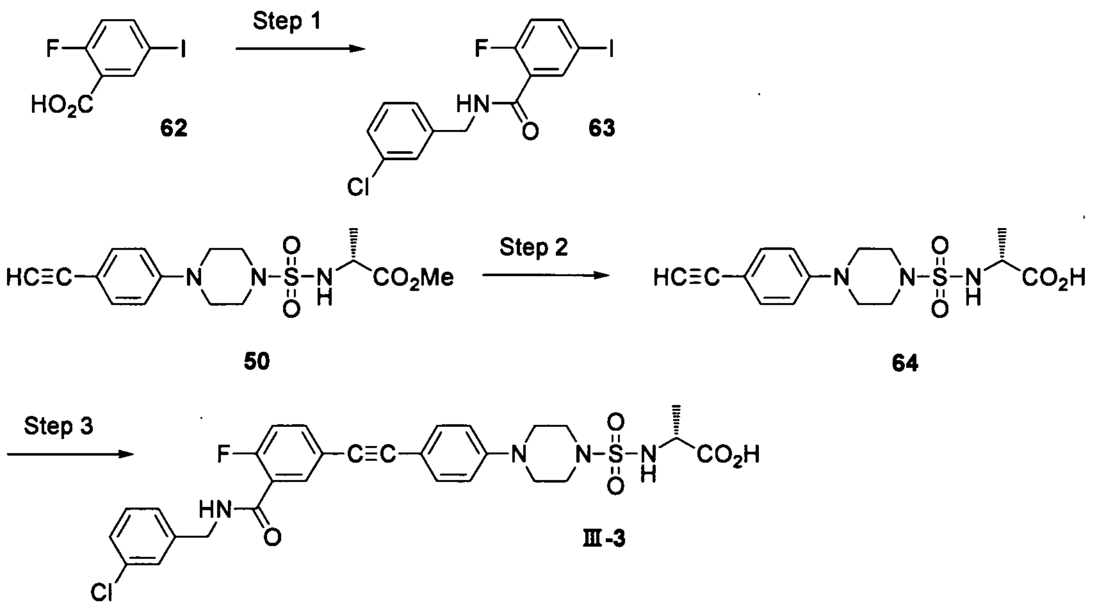


62 63 50 64 III-3

(Step 1)

To a solution of 2-fluoro-5-iodo benzoic acid (62) (0.250 g, 0.940 mmol) in N, N-dimethylformamide (3 mL) were added 1-hydroxybenzotriazole (0.038 g, 0.28 mmol) and 1-ethyl-3-(3-dimethylaminopropyl) carbodiimide hydrochloride (0.270 g, 1.41 mmol) at room temperature, and the mixture was stirred for 15 minites. Then, 4-chrolobenzylamine (0.744 mL, 6.09 mmol) was added to the reaction mixture , which was stirred at room temperature for 16 hours. To the reaction mixture was added 2mol/L hydrochloric acid, and the mixture was extracted with ethyl acetate. The organic layer was washed with saturated sodium bicarbonate aq., and saturated sodium chloride aq., dried over anhydrous sodium sulfate, and concentrated under reduced pressure. The residue was recrystallized from n-hexane/ethyl acetate to give a colorless compound (63) (0.288 g, yield 79 %).

(Step 2)

The compound (64) was prepared from the compound (50) according to the procedure for Step 8 in Example 2.

(Step 3)

A solution of the compound (64) (0.050 g, 0.15 mmol) and the compound (63) (0.052 g, 0.13 mmol) in N, N-dimethylformamide (1 mL) was degassed well, and the atmosphere was replaced with nitrogen gas. Then, bis(triphenylphosphine)palladium (II) chloride (0.005 g, 0.007 mmol), copper (I) iodide (0.003 g, 0.015 mmol), and triethylamine (0.041 mL, 0.30 mmol) were added, this was degassed again under nitrogen atmosphere, and stirred at room temperature for 1 hours. The reaction mixture was neutralized with 5% citric acid aq., and extracted with ethyl acetate. The organic layer was washed with saturated sodium bicarbonate aq., and saturated sodium chloride aq., dried over anhydrous sodium sulfate, and concentrated under reduced pressure. The residue was subjected to silica gel chromatography, the

fractions eluted with chloroform / methanol=4/1 were collected, and evaporated to dryness to afford (R)-2-(4-{4-[2-(3-chrolobenzylcarbamoyl)-4-fluorophenylethynyl]-phenyl}-piperazine-1-sulfonylamino)-proponic acid (III-3) (0.030 g, yield 34%).

$^1$H NMR (DMSO-d6) δ: 1.25 (d, J=7.2 Hz, 3H), 3.12-3.39 (m, 8H), 3.55-3.61 (m, 1H), 4.47 (d, J=5.7 Hz, 2H), 6.98 (d, J=8.7 Hz, 2H), 7.29-7.43 (m, 8H), 7.65-7.68 (m, 1H), 7.72-7.75 (m, 1H), 9.10 (t, J=5.7 Hz, 1H).

[Example 14]

Synthesis of (R)-2-{4-[4-(5-benzylcarbamoyl-1-methyl-6-oxo-1,6-dihydropyridin-3-ylethynyl)-phenyl]-piperazine-1-sulfonylamino}-proponic acid (III-4)

[Chemical formula 69]

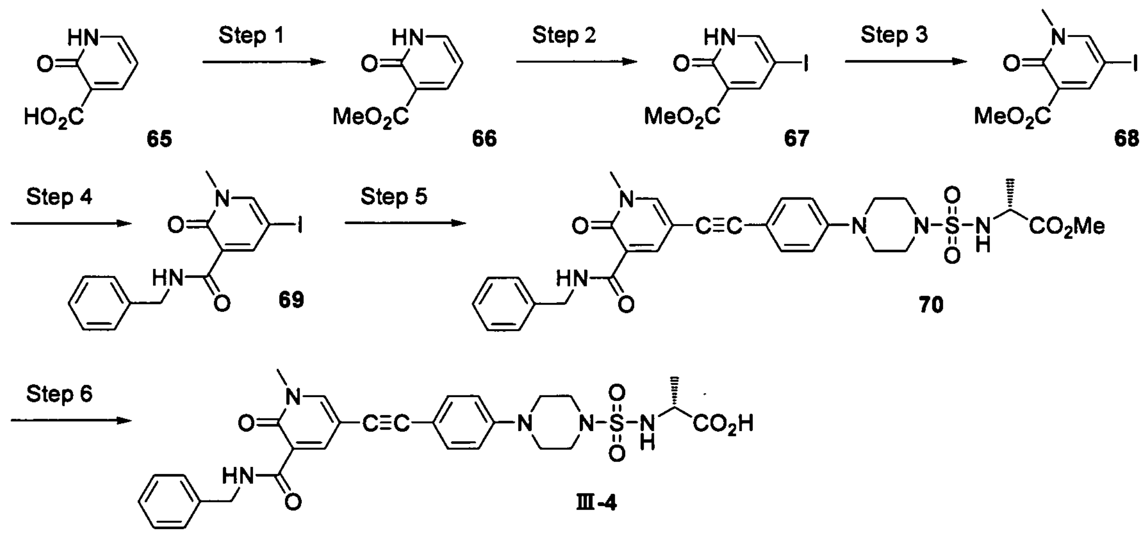


65, 66, 67, 68, 69, 70, III-4

(Step 1)

To a solution of 2-hydroxynicotinic acid (65) (13.9 g, 100 mmol) in methanol (140 mL) were added concentrated sulfuric acid (4.2 mL) and toluene (38 mL) at room temperature, and the mixture was stirred under reflux for 26 hours. The reaction mixture was cooled to room temperature and neutralized with potassium carbonate aq. After the mixture was concentrated under reduced pressure, saturated ammonium chloride aq. was added to the residue, which was extracted with chloroform. The organic layer was washed with saturated sodium chloride aq., dried over anhydrous magnesium sulfate, and concentrated under reduced pressure to give a compound (66) (8.10 g, yield 53 %).

(Step 2)

To a solution of the compound (66) (8.10 g, 52.9 mmol) in methylene chloride (200 mL) was added N-iodosuccinimide (15.5 g, 68.8 mmol) at room temperature, and the mixture was stirred under reflux for 23 hours. The reaction mixture was cooled to room temperature, and concentrated under reduced pressure. To the residue was added ethyl acetate, and this was refluxed with stirring for 2 hours. The reaction mixture was cooled to room temperature, the precipitated crystals were collected by filtration, washed with ethyl acetate to afford the compound (67) (11.3 g, yield 77 %).

(Step 3)

To a solution of the compound (67) (0.500 g, 1.79 mmol) in N, N-dimethylformamide (5 mL) were added sodium hydride (60% oin oil) (0.079 g, 2.0 mmol) and methyl iodide (0.167 mL, 2.69 mmol) at room temperature, and the mixture was stirred for 5 hours. To the reaction mixture was added 5% citric acid aq., and the mixture was extracted with ethyl acetate. The organic layer was washed with saturated sodium chloride aq., dried over anhydrous sodium sulfate, and

concentrated under reduced pressure. The residue was recrystallized from ethyl acetate to give a colorless compound (68) (0.352 g, yield 67%).

(Step 4)

To a solution of the compound (68) (0.300 g, 1.02 mmol) in methanol (3 mL) was added benzylamine (0.559 mL, 5.12 mmol) at room temperature, the mixture was stirred at 150°C for 2 hours under microwave irradiation. The reaction mixture was cooled to room temperature, and concentrated under reduced pressure. The residue was recrystallized from acetone/ethyl acetate to give a colorless compound (69) (0.240 g, yield 64%).

(Step 5)

A solution of the compound (69) (0.119 g, 0.323 mmol) and the compound (50) (0.114 g, 0.323 mmol) in N, N-dimethylformamide (1.2 mL) was degassed well, and the atmosphere was replaced with nitrogen gas. Then, bis(triphenylphosphine) palladium (II) chloride (0.011 g, 0.016 mmol), copper (I) iodide (0.0061 g, 0.032 mmol), and triethylamine (0.090 mL, 0.65 mmol) were added, this was degassed again under nitrogen atmosphere, and stirred at room temperature for 14 hours. The reaction mixture was neutralized with 5% aqueous citric acid aq., and extracted with ethyl acetate. The organic layer was washed with saturated sodium bicarbonate aq., and saturated sodium chloride aq., dried over anhydrous sodium sulfate, and concentrated under reduced pressure. The residue was subjected to silica gel chromatography, the fractions eluted with n-hexane/ethyl acetate =1/2 were collected, and crystallized with ethyl acetate to give a pale yellow compound (70) (0.077 g, yield 40%).

(Step 6)

To a solution of the compound (70) (0.064 g, 0.11 mmol) in dimethyl sulfoxide (1.2 mL) was added 2 mol/L sodium hydroxide aq. (0.16 mL) at room temperature, and the mixture was stirred for 30 minutes. The reaction mixture was poured into ice water-5% citric acid aq., and extracted with ethyl acetate. The organic layer was washed with water, dried over anhydrous sodium sulfate, and concentrated under reduced pressure. The residue was recrystallized from n-hexane/ethyl acetate to afford (R)-2-{4-[4-(5-benzylcarbamoyl-1-methyl-6-oxo-1, 6-dihydropyridin-3-ylethynyl)-phenyl]-piperazine-1-sulfonylamino}-proponic acid (III-4) (0.050 g, yield 80%).

$^{1}$H NMR (DMSO-d6) δ: 1.30 (d, J=7.2 Hz, 3H), 3.14-3.19 (m, 4H), 3.29-3.33 (m, 4H), 3.57 (s, 3H), 3.78-3.83 (m, 1H), 4.54 (d, J=5.6 Hz, 2H), 6.99 (d, J=8.4 Hz, 2H), 7.26-7.40 (m, 8H), 7.90 (d, J=8.4 Hz, 1H), 8.33 (d, J=2.0 Hz, 1H), 8.43 (d, J=2.0 Hz, 1H), 10.02 (t, J=5.6 Hz, 1H), 12.71 (s, 1H).

[Example 15]

Synthesis of (R)-2-{4-[4-(2-benzyl-1-oxo-1,2-dihydroisoquinolin-7-ylethynyl)-phenyl]-piperazine-1-sulfonylamino}-proponic acid (III-5)

[Chemical formula 70]

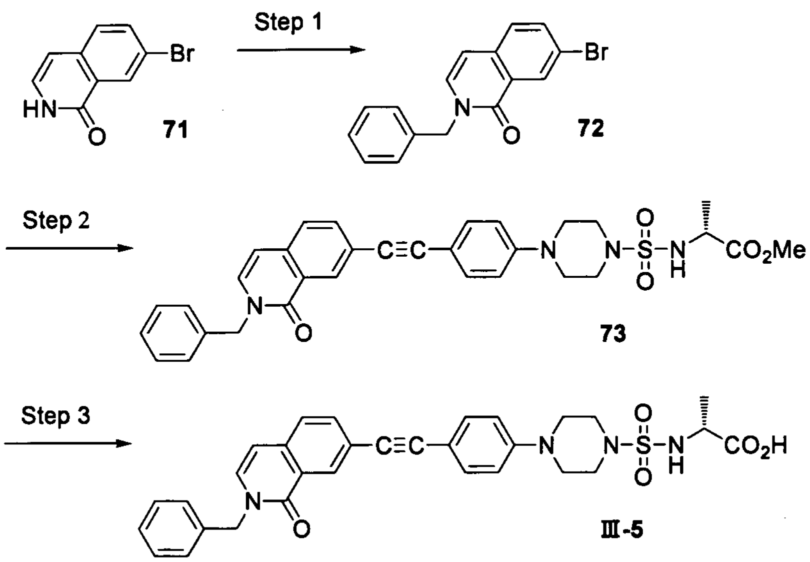


(Step 1)

To a solution of the 7-bromo-2H-isoquinolin-1-one (71) (0.250 g, 1.12 mmol) in N,N-dimethylformamide (2.5 mL) were added cesium carbonate (0.545 g, 1.67 mmol) and benzyl bromide (0.146 mL,1.23 mmol) at room temperature, and the mixture was stirred for 1 hour. To the reaction mixture was added 10% citric acid aq., and the mixture was extracted with ethyl acetate. The organic layer was washed with saturated sodium chloride aq., dried over anhydrous sodium sulfate, and concentrated under reduced pressure. The residue was crystallized with diisopropyl ether to give a colorless compound (72) (0.303 g, yield 76%).

(Step 2)

A solution of the compound (72) (0.120 g, 0.382 mmol) and the compound (50) (0.134 g, 0.382 mmol) in N, N-dimethylformamide (1.2 mL) was degassed well, and the atmosphere was replaced with nitrogen gas. Then, bis(triphenylphosphine) palladium (II) chloride (0.013 g, 0.019 mmol), copper (I) iodide (0.0072 g, 0.038 mmol), and triethylamine (0.11 ml, 0.76 mmol) were added, this was degassed again under nitrogen atmosphere, and stirred at 70°C for 7 hours. The reaction mixture was cooled to room temperature, saturated ammonium chloride aq. was added to the reaction mixture, which was extracted with ethyl acetate. The organic layer was washed with saturated sodium chloride aq., dried over anhydrous sodium sulfate, and concentrated under reduced pressure. The residue was subjected to silica gel chromatography, the fractions eluted with n-hexane/ethyl acetate =1/1 were collected, and crystallized with n-hexane/ethyl acetate to afford a colorless compound (73) (0.104 g, yield 47%).

(Step 3)

To a solution of the compound (73) (0.101 g, 0.173 mmol) in dimethyl sulfoxide (0.8 mL) was added 2 mol/L sodium hydroxide aq. (0.26 mL) at room temperature, and the mixture was stirred at for 19 hours. The reaction mixture was poured into ice water-5% citric acid aq., and extracted with ethyl acetate. The organic layer was washed with water, dried over anhydrous sodium sulfate, and concentrated under reduced pressure. The residue was recrystallized from n-hexane/ethyl acetate to give (R)-2-{4-[4-(2-benzyl1-oxo-1,2-dihydroisoquinolin-7-ylethynyl)-phenyl]-piperazine-1-sulfonylamino}-proponic acid (III-5) (0.095 g, yield 96%).

$^{1}$H NMR (DMSO-d6) δ: 1.30 (d, J=7.2 Hz, 3H), 3.14-3.19 (m, 4H), 3.29-3.33 (m, 4H), 3.78-3.83 (m, 1H), 5.20 (s, 2H), 6.69 (d, J=8.4 Hz, 1H), 7.01 (d, J=8.4 Hz, 2H), 7.28-7.37 (m, 5H), 7.45 (d, J=8.4 Hz, 2H), 7.61-7.92 (m, 4H), 8.28 (s, 1H), 12.71 (br s, 1H).

[Example 16]

Synthesis of (R)-2-(4-{4-[3-(4-chrolobenzyl)-2, 4-dioxo-1, 2, 3, 4-tetrahydroquinazolin-6-ylethynyl]-phenyl}-piperazine-1-sulfonylamino)-3-methylbutanoic acid (III-6)

[Chemical formula 71]

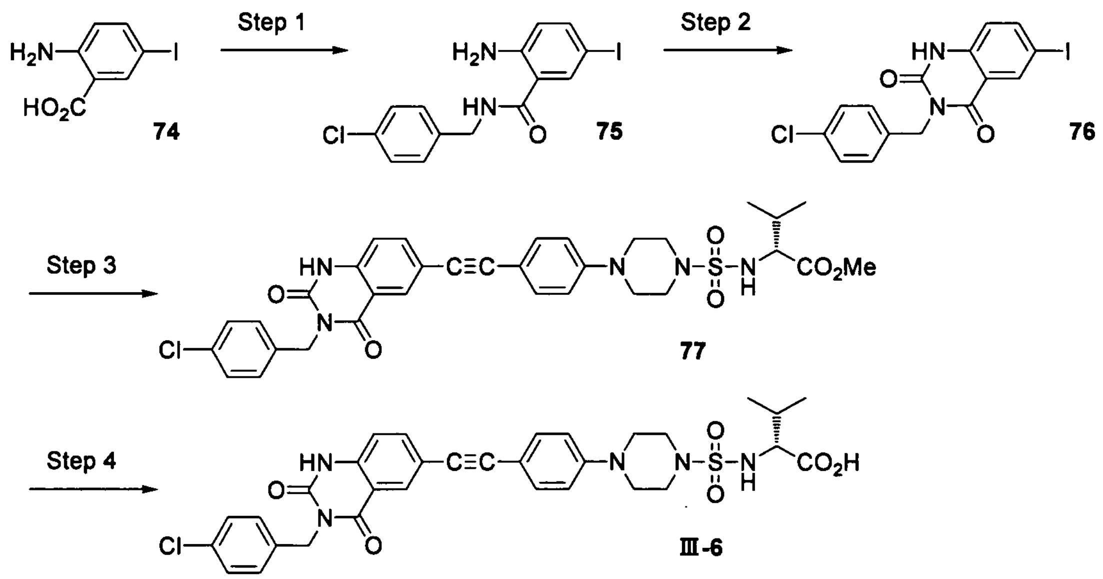


74 75 76 77 III-6

(Step 1)

To a solution of 5-iodoanthranilic acid (74) (1.0 g, 3.8 mmol) in methylene chloride (10 mL) were added 1-hydroxybenzotriazole (0.15 g, 1.1 mmol) and 1-ethyl-3-(3-dimethylaminopropyl)carbodiimide hydrochloride (0.73 g, 3.8 mmol) and 4-chrolobenzylamine (0.65 mL, 5.2 mmol) at room temperature, and the mixture was stirred at room temperature for 24 hours. Water was added to the reaction mixture, which was extracted with chloroform. The organic layer was washed with 5% citric acid aq., saturated sodium bicarbonate aq., and saturated sodium chloride aq., dried over anhydrous sodium sulfate, and concentrated under reduced pressure. The residue was recrystallized from n-hexane/acetone to give a pale yellow compound (75) (1.4 g, yield 95 %).

(Step 2)

To a solution of the compound (75) (1.25 g,3.23 mmol) in tetrahydrofuran (14 mL) was added N, N'-carbonyl bis-1H-imidazole (2.00 g, 12.3 mmol) at room temperature, and the mixture was stirred at 60°C for 2.5 hours. After the reaction mixture was cooled to room temperature, ice-2 mol/L hydrochloric acid was added to the reaction mixture, which was extracted with ethyl acetate. The organic layer was washed with saturated sodium bicarbonate aq., and saturated sodium chloride aq., dried over anhydrous sodium sulfate, and concentrated under reduced pressure. The residue was recrystallized from n-hexane/acetone to afford a pale yellow compound (76) (0.85 g, yield 64 %).

(Step 3)

A solution of the compound (76) (0.100 g, 0.24 mmol) and the compound (19) (0.090 g, 0.24 mmol) in N,N-dimethylformamide (1 mL) was degassed well, and the atmosphere was replaced with nitrogen gas. Then, bis(triphenylphosphine) palladium (II) chloride (0.0083 g, 0.012 mmol), copper (I) iodide (0.0045 g, 0.024 mmol), and triethylamine (0.066 mL, 0.47 mmol) were added, and this was degassed again under nitrogen atmosphere, and stirred for 2 hours. The reaction mixture was poured into ice-water-5% acid aq., and extracted with ethyl acetate. The organic layer was washed with saturated sodium bicarbonate aq., and saturated sodium chloride aq., dried over anhydrous sodium sulfate,

and concentrated under reduced pressure. The residue was subjected to silica gel chromatography, the fractions eluted with n-hexane/ethyl acetate/chloroform=2/1/1 were collected, and evaporated to dryness to give a pale yellow compound (77) (0.110 g, yield 68 %).

(Step 4)

To a solution of the compound (77) (0.100 g, 0.15 mmol) in dimethyl sulfoxide (2.4 mL) was added 1 mol/L sodium hydroxide aq. (0.6 mL) at room temperature, and the mixture was stirred at 50°C for 2 hours. The reaction mixture was cooled to room temperature, poured into ice water-5% citric acid aq. and extracted with ethyl acetate. The organic layer was washed with water, dried over anhydrous sodium sulfate, and concentrated under reduced pressure. The residue was recrystallized from n-hexane/acetone to give (R)-2-(4-{4-[3-(4-chrolobenzyl)-2, 4-dioxo-1, 2, 3, 4-tetrahydroquinazolin-6-ylethynyl]-phenyl}-piperazine-1-sulfonylamino)-3-methylbutanoic acid (III-6) (0.095 g, yield 97%).
$^1$ H NMR (DMSO-d6) δ: 0.91 (d, J=6.3 Hz, 3H), 0.92 (d, J=6.3 Hz, 3H), 1.97 (m, 1H), 3.05-3.40 (m, 8H), 3.50 (m, 1H), 5.07 (s, 2H), 6.99 (d, J=8.7 Hz, 2H), 7.22 (d, J=8.7 Hz, 1H), 7.32-7.40 (m, 4H), 7.42 (d, J=8.4 Hz, 2H), 7.78 (dd, J=1.2, 8.4 Hz, 1H), 7.83 (d, J=9.9 Hz, 1H), 7.99 (d, J=1.2 Hz, 1H), 11.74 (s, 1H), 12.72 (br s, 1H).
According to the same manner as Example11 to 16, the following compounds were synthesized.

Synthesis of (R)-2-{4-[4-(5-tert-butoxycarbonylaminonaphthalen-2-ylethynyl)-phenyl]-piperazine-1-sulfonylamino}-proponic acid (III-7)

$^1$ H NMR (DMSO-d6) δ: 1.30 (d, J=7.2 Hz, 3H), 1.50 (s, 9H), 3.15-3.20 (m, 4H), 3.81 (bs, 1H), 7.02 (d, J=8.8 Hz, 2H), 7.45-7.52 (m, 3H), 7.57-7.61 (m, 2H), 7.73 (d, J=8.0 Hz, 1H), 7.90 (bs, 1H), 8.06 (d, J=8.8 Hz, 1H), 8.10 (s, 1H).

Synthesis of (R)-2-{4-[4-(5-acetylaminonaphthalen-2-ylethynyl)-phenyl]-piperazine-1-sulfonylamino}-proponic acid (III-8)

$^1$ H NMR (DMSO-d6) δ: 1.30 (d, J=6.8 Hz, 3H), 3.15-3.20 (m, 4H), 3.80-3.85 (m, 1H), 7.02 (d, J=8.8 Hz, 2H), 7.46 (d, J=8.4 Hz, 3H), 7.53 (t, J=8.0 Hz, 2H), 7.61 (d, J=8.8 Hz, 1H), 7.71-7.78 (m, 2H), 7.93 (d, J=8.8 Hz, 1H), 8.08-8.13 (m, 2H).

Synthesis of (R)-2-{4-[4-(5-acetylnaphthalen-2-ylethynyl)-phenyl]-piperazine-1-sulfonylamino}-proponic acid (III-9)

$^1$ H NMR (DMSO-d6) δ: 1.30 (d, J=7.2 Hz, 3H), 2.74 (s, 1H), 3.12-3.20 (m, 4H), 3.80-3.85 (m, 1H), 7.02 (d, J=8.8 Hz, 2H), 7.46 (d, J=8.4 Hz, 3H), 7.64-7.69 (m, 2H), 7.93 (d, J=7.6 Hz, 1H), 8.15-8.21 (m, 3H), 8.63 (d, J=5.2 Hz, 1H).

Synthesis of (R)-2-{4-[4-(8-acetyl-5-chrolonaphthalen-2-ylethynyl)-phenyl]-piperazine-1-sulfonylamino}-proponic acid (III-10)

$^1$ H NMR (DMSO-d6) δ: 1.31 (d, J=7.6 Hz, 3H), 2.74 (s, 3H), 3.15-3.19 (m, 4H), 3.80-3.83 (m, 1H), 7.02 (d, J=8.0 Hz, 2H), 7.50 (d, J=8.4 Hz, 2H), 7.83 (t, J=8.0 Hz, 2H), 7.93 (d, J=8.4 Hz, 1H), 8.18 (d, J=7.6 Hz, 1H), 8.29 (d, J=9.2 Hz, 1H), 8.80 (s, 1H).

Synthesis of (R)-2-{4-[4-(5-isopropylcarbonylnaphthalen-2-ylethynyl)-phenyl]-piperazine-1-sulfonylamino}-proponic acid (III-11)

$^1$ H NMR (DMSO-d6) δ: 1.21 (d, J=6.4 Hz, 6H), 1.30 (d, J=7.2 Hz, 3H), 3.15-3.20 (m, 4H), 3.77-3.85 (m, 1H), 4.13-4.20 (m, 1H), 7.02 (d, J=8.8 Hz, 2H), 7.46 (d, J=8.8 Hz, 2H), 7.62 (d, J=9.2 Hz, 2H), 7.93 (d, J=8.8 Hz, 1H), 8.00-8.02 (m, 1H), 8.16-8.18 (m, 2H), 8.44 (d, J=7.6 Hz, 1H).

Synthesis of (R)-2-[4-(4-{3-[5-(4-fluorophenyl)-oxazol-2-yl]-phenylethynyl}-phenyl)-piperazine-1-sulfonylamino]-proponic acid (III-12)

$^1$ H NMR (DMSO-d6) δ: 1.30 (d, J=7.2 Hz, 3H), 3.13-3.17 (m, 4H), 3.79-3.83 (m, 1H), 7.02 (d, J=8.8 Hz, 2H), 7.37 (t, J=8.8 Hz, 2H), 7.47 (d, J=8.4 Hz, 2H), 7.59 (t, J=8.4 Hz, 1H), 7.66 (d, J=7.6 Hz, 1H), 7.86 (s, 1H), 7.91-7.96 (m, 3H), 8.07 (d, J=7.6 Hz, 1H), 8.19 (s, 1H).

Synthesis of (R)-2-[4-(4-{3-[5-(4-fluorophenyl)-thiazol-2-yl]-phenylethynyl}-phenyl)-piperazine-1-sulfonylamino]-proponic acid (III-13)

$^1$H NMR (DMSO-d6) δ: 1.30 (d, J=7.2 Hz, 3H), 3.15-3.21 (m, 4H), 3.80-3.83 (m, 1H), 7.01 (d, J=8.4 Hz, 2H), 7.34 (t, J=8.4 Hz, 2H), 7.47 (d, J=8.4 Hz, 2H), 7.56 (t, J=8.4 Hz, 1H), 7.62 (d, J=7.2 Hz, 1H), 7.78-7.81 (m, 2H), 7.91-7.96 (m, 2H), 8.04 (s, 1H), 8.34 (s, 1H).

Synthesis of (R)-2-[4-(4-{3-[5-(4-fluorophenyl)-oxazol-2-yl]-phenylethynyl}-phenyl)-piperazine-1-sulfonylamino]-proponic acidmethyl ester (III-14)

$^1$H NMR (DMSO-d6) δ: 1.31 (d, J=6.8 Hz, 3H), 3.12-3.15 (m, 4H), 3.66 (s, 3H), 3.93 (t, J=8.0 Hz, 1H), 7.02 (d, J=8.8 Hz, 2H), 7.35-7.39 (m, 2H), 7.48 (d, J=8.4 Hz, 2H), 7.59 (d, J=8.0 Hz, 1H), 7.66 (d, J=6.4 Hz, 1H), 7.86 (s, 1H), 7.94-7.98 (m, 2H), 8.07-8.11 (m, 2H), 8.19 (s, 1H).

Synthesis of (R)-2-[4-(4-{3-[5-(4-fluorobenzyl)-oxazol-2-yl]-phenylethynyl}-phenyl)-piperazine-1-sulfonylamino]-proponic acid (III-15)

$^1$H NMR (DMSO-d6) δ: 1.30 (d, J=7.2 Hz, 3H), 3.09-3.19 (m, 4H), 3.78-3.83 (m, 1H), 4.14 (s, 2H), 7.01 (d, J=8.4 Hz, 2H), 7.08 (s, 1H), 7.18 (d, J=8.8 Hz, 2H), 7.37-7.40 (m, 2H), 7.45 (d, J=8.4 Hz, 2H), 7.54 (t, J=8.0 Hz, 1H), 7.61 (d, J=8.0 Hz, 1H), 7.89 (d, J=8.0 Hz, 2H), 7.97 (s, 1H).

Synthesis of (R)-2-[4-(4-{3-[3-(4-fluorophenyl)-[1, 2, 4]-oxadiazol-5-yl]-phenylethynyl}-phenyl)-piperazine-1-sulfonylamino]-proponic acid (III-16)

$^1$H NMR (DMSO-d6) δ: 1.30 (d, J=6.8 Hz, 3H), 3.15-3.20 (m, 4H), 3.74-3.83 (m, 1H), 7.03 (d, J=8.0 Hz, 2H), 7.44-7.50 (m, 4H), 7.70 (t, J=7.6 Hz, 1H), 7.85 (d, J=8.0 Hz, 1H), 7.93 (d, J=8.8 Hz, 1H), 8.16-8.19 (m, 3H), 8.25 (s, 1H).

Synthesis of (R)-2-(4-{4-[8-(isobutoxyiminomethyl)-naphthalen-2-ylethynyl]-phenyl}-piperazine-1-sulfonylamino)-proponic acid (III-17)

$^1$H NMR (DMSO-d6) δ: 1.02 (d, J=6.6 Hz, 6H), 1.33 (d, J=7.1 Hz, 3H), 2.06-2.17 (m, 1H), 3.12-3.37 (m, 8H), 3.78-3.89 (m, 1H), 4.04 (d, J=6.6 Hz, 2H), 7.05 (d, J=8.9 Hz, 2H), 7.49 (d, J=8.9 Hz, 2H), 7.58-7.68 (m, 2H), 7.87 (d, J=6.9 Hz, 1H), 7.91-7.98 (m, 1H), 8.04 (d, J=8.5 Hz, 2H), 8.88 (s, 1H), 8.92 (s, 1H).

Synthesis of (R)-2-(4-{4-[8-(2, 2, 2-trifluoroacetyl)-naphthalen-2-ylethynyl]-phenyl}-piperazine-1-sulfonylamino)-proponic acid (III-18)

$^1$H NMR (DMSO-d6) δ: 1.33 (d, J=7.1 Hz, 3H), 3.13-3.23 (m, 4H), 3.31-3.38 (m, 4H), 3.78-3.89 (m, 1H), 7.05 (d, J=8.7 Hz, 2H), 7.53 (d, J=8.7 Hz, 2H), 7.76-7.83 (m, 2H), 7.97 (d, J=8.8 Hz, 1H), 8.18 (d, J=8.8 Hz, 1H), 8.27-8.33 (m, 1H), 8.44 (d, J=8.2 Hz, 1H), 8.84 (s, 1H), 12.68-12.88 (m, 1H).

(R)-2-{4-[4-(8-pentanoylnaphthalen-2-ylethynyl)-phenyl]-piperazine-1-sulfonylamino)-proponic acid (III-19)

$^1$H NMR ($CDCl_3$) 0.97 (t, J=7.3 Hz, 3H), 1.38-1.54 (m, 2H), 1.51 (d, J=7.3 Hz, 3H), 1.79 (dt, J=16.8, 6.1 Hz, 2H), 3.07 (t, J=7.5 Hz, 1H), 3.27-3.34 (m, 4H), 3.35-3.40 (m, 4H), 4.07-4.19 (m, 1H), 5.01 (d, J=8.8 Hz, 2H), 6.90 (d, J=8.7 Hz, 2H), 7.44-7.53 (m, 3H), 7.59 (dd, J=8.4, 1.5 Hz, 1H), 7.81 (d, J=8.4 Hz, 1H), 7.88 (dd, J=7.2, 1.1 Hz, 1H), 7.94 (d, J=8.1 Hz, 1H), 8.76 (s, 1H).

Synthesis of (R)-2-(4-{4-[3-(2-oxopropyl)-phenylethynyl]-phenyl}-piperazine-1-sulfonylamino)-proponic acid (III-20)

$^1$H NMR (DMSO-d6) δ: 1.28 (d, J=7.1 Hz, 3H), 2.18 (s, 3H), 3.14-3.21 (m, 4H), 3.27-3.33 (m, 4H), 3.63-3.72 (m, 1H), 3.83 (s, 2H), 7.01 (d, J=8.8 Hz, 2H), 7.20 (d, J=6.9 Hz, 1H), 7.33-7.43 (m, 5H).

Synthesis of (R)-2-{4-[4-(3-phenylcarbamoylphenylethynyl)-phenyl]-piperazine-1-sulfonylamino}-proponic acid (III-21)

$^1$H NMR (DMSO-d6) δ: 1.32 (d, J=7.1 Hz, 3H), 3.14-3.22 (m, 4H), 3.29-3.35 (m, 4H), 3.78-3.88 (m, 1H), 7.04 (d, J=8.8 Hz, 2H), 7.14 (t, J=7.3 Hz, 1H), 7.39 (t, J=8.0 Hz, 2H), 7.47 (d, J=8.8 Hz, 2H), 7.59 (t, J=7.8 Hz, 1H), 7.74 (d, J=7.7 Hz, 1H), 7.81 (d, J=7.7 Hz, 2H), 7.93-7.99 (m, 2H), 8.12 (s, 1H), 10.37 (s, 1H).

Synthesis of (R)-2-(4-{4-[3-((Z)-1-fluoro-2-phenylvinyl)-phenylethynyl]-phenyl}-piperazine-1-sulfonylamino)-proponic acid (III-22)

$^1$H NMR (DMSO-d6) δ: 1.33 (d, J=7.4 Hz, 3H), 3.14-3.22 (m, 4H), 3.29-3.36 (m, 4H), 3.78-3.89 (m, 1H), 6.87 (s, 1H), 7.04 (d, J=9.1 Hz, 3H), 7.31-7.38 (m, 2H), 7.43-7.56 (m, 6H), 7.69-7.77 (m, 3H), 7.90 (s, 1H), 7.93-7.99 (m, 1H).

Synthesis of (R)-2-{4-[4-(3-benzoxazol-2-ylphenylethynyl)-phenyl]-piperazine-1-sulfonylamino}-proponic acid (III-23)

$^1$H NMR (DMSO-d6) δ: 1.32 (d, J=7.1 Hz, 3H), 3.14-3.22 (m, 4H), 3.30-3.40 (m, 4H), 3.79-3.88 (m, 1H), 7.05 (d, J=9.1 Hz, 2H), 7.44-7.53 (m, 4H), 7.68 (t, J=7.7 Hz, 1H), 7.78 (d, J=8.0 Hz, 1H), 7.82-7.90 (m, 2H), 7.93-7.99 (m, 1H), 8.21 (d, J=8.2 Hz, 1H), 8.30 (s, 1H).

Synthesis of (S)-2-{4-[4-(8-acetylnaphthalen-2-ylethynyl)-phenyl]-piperazine-1-sulfonylamino}-3-hydroxyproponic acid (III-24)

$^1$H NMR (DMSO-d6) δ: 2.74 (s, 3H), 3.17-3.22 (m, 4H), 3.29-3.34 (m, 4H), 3.62 (d, J=5.2 Hz, 2H), 3.78-3.82 (m, 1H), 5.04 (br s, 1H), 7.01 (d, J=8.4 Hz, 2H), 7.48 (d, J=8.4 Hz, 2H), 7.60-7.66 (m, 2H), 7.77 (d, J=8.4 Hz, 1H), 8.03 (d, J=8.4 Hz, 1H), 8.16-8.22 (m, 2H), 8.79 (s, 1H), 12.75 (br s, 1H).

Synthesis of (S)-2-(4-{4-[(E)-2-(acetylnaphthalen-2-yl)-vinyl]-phenyl}-piperazine-1-sulfonylamino)-pentanoic acid (III-25)

$^1$H NMR (DMSO-d6) δ: 0.89 (t, J=7.2 Hz, 3H), 1.35-1.44 (m, 2H), 1.56-1.63 (m, 2H), 2.74 (s, 3H), 3.13-3.19 (m, 4H), 3.26 (br s, 4H), 3.69 (br s, 1H), 6.99 (d, J=8.4 Hz, 2H), 7.22-7.34 (m, 2H), 7.53-7.57 (m, 3H), 7.87-7.99 (m, 3H), 8.09-8.12 (m, 2H), 8.63 (s, 1H).

Synthesis of (R)-2-{4-[4-(1-benzyloxyisoquinolin-7-ylethynyl)-phenyl]-piperazine-1-sulfonylamino}-proponic acid (III-26)

$^1$H NMR (DMSO-d6) δ: 1.30 (d, J=7.2 Hz, 3H), 3.14-3.19 (m, 4H), 3.29-3.32 (m, 4H), 3.79-3.83 (m, 1H), 5.59 (s, 2H), 7.00 (d, J=8.4 Hz, 2H), 7.34-7.58 (m, 8H), 7.82-7.95 (m, 3H), 8.05 (d, J=5.6 Hz, 1H), 8.26 (s, 1H), 12.74 (br s, 1H).

Synthesis of (R)-2-{4-[4-(2-benzyl1-oxo-1,2-dihydroisoquinolin-7-ylethynyl)-phenyl]-piperazine-1-sulfonylamino}-3-methylbutanoic acid (III-27)

$^1$H NMR (DMSO-d6) δ: 0.92 (d, J=6.4 Hz, 6H), 1.93-2.02 (m, 1H), 3.12-3.22 (m, 4H), 3.28-3.32 (m, 4H), 3.78-3.83 (m, 1H), 5.20 (s, 2H), 6.69 (d, J=8.4 Hz, 1H), 7.00 (d, J=8.4 Hz, 2H), 7.27-7.37 (m, 5H), 7.45 (d, J=8.4 Hz, 2H), 7.61-7.85 (m, 4H), 8.29 (s, 1H), 12.73 (br s, 1H).

(R)-2-{4-[4-(5-benzylcarbamoyl-6-oxo-1, 6-dihydropyridin-3-ylethynyl)-phenyl]-piperazine-1-sulfonylamino}-proponic acid (III-28)

$^1$H NMR (DMSO-d6) δ: 1.30 (d, J=7.2 Hz, 3H), 3.14-3.19 (m, 4H), 3.29-3.33 (m, 4H), 3.78-3.83 (m, 1H), 4.54 (d, J=5.6 Hz, 2H), 6.98 (d, J=8.4 Hz, 2H), 7.61-7.42 (m, 5H), 7.55-7.66 (m, 2H), 7.90 (d, J=8.4 Hz, 1H), 8.00 (s, 1H), 8.33 (d, J=2.0 Hz, 1H), 10.00 (t, J=5.6 Hz, 1H), 12.79 (br s, 1H), 12.87 (s, 1H).

Synthesis of (R)-2-{4-[4-(5-benzylcarbamoyl-6-oxo-1, 6-dihydropyridin-3-ylethynyl)-phenyl]-piperazine-1-sulfonylamino}-3-methylbutanoic acid (III-29)

$^1$H NMR (DMSO-d6) δ: 0.92 (d, J=6.4 Hz, 6H), 1.94-2.02 (m, 1H), 3.12-3.22 (m, 4H), 3.28-3.32 (m, 4H), 3.78-3.83 (m, 1H), 4.54 (d, J=5.6 Hz, 2H), 6.98 (d, J=8.4 Hz, 2H), 7.26-7.43 (m, 7H), 7.82 (d, J=8.4 Hz, 1H), 8.00 (s, 1H), 8.33 (d, J=2.0 Hz, 1H), 10.00 (t, J=5.6 Hz, 1H), 12.87 (s, 1H).

Synthesis of (R)-2-{4-[4-(5-benzylcarbamoyl-1-methyl-6-oxo-1,6-dihydropyridin-3-ylethynyl)-phenyl]-piperazine-1-sulfonylamino}-3-methylbutanoic acid (III-30)

$^1$H NMR (DMSO-d6) δ: 0.91 (d, J=6.4 Hz, 6H), 1.94-2.00 (m, 1H), 3.12-3.22 (m, 4H), 3.28-3.32 (m, 4H), 3.49 (br s, 1H), 3.57 (s, 3H), 4.54 (d, J=5.6 Hz, 2H), 6.98 (d, J=8.4 Hz, 2H), 7.26-7.39 (m, 6H), 7.81 (br s, 2H), 8.33 (d, J=2.0 Hz, 1H), 8.44 (d, J=2.0 Hz, 1H), 10.03 (t, J=5.6 Hz, 1H).

Synthesis of (R)-2-{4-[4-(2-cyanomethyl-1-oxo-1, 2-dihydroisoquinolin-7-ylethynyl)-phenyl]-piperazine-1-sulfonylamino}-3-methylbutanoic acid (III-31)

$^1$H NMR (DMSO-d6) δ: 0.92 (br s, 6H), 1.93-2.02 (m, 1H), 3.12-3.22 (m, 4H), 5.09 (s, 2H), 6.76 (d, J=8.4 Hz, 1H), 7.00 (d, J=8.4 Hz, 2H), 7.45-7.82 (m, 6H), 8.28 (s, 1H).

Synthesis of (R)-2-(4-{4-[3-(cyanomethylcarbamoyl)-phenylethynyl]-phenyl}-piperazine-1-sulfonylamino)-3-methylbutanoic acid (III-32)

$^1$H NMR (DMSO-d6) δ: 0.88 (d, J=22.8 Hz, 6H), 1.93-2.02 (m, 1H), 3.12-3.22 (m, 4H), 4.32 (s, 2H), 6.99 (d, J=8.4 Hz, 2H), 7.42 (d, J=8.4 Hz, 2H), 7.54 (t, J=8.4 Hz, 2H), 7.70 (d, J=8.4 Hz, 1H), 7.85 (d, J=8.4 Hz, 1H), 7.89 (s, 1H), 9.35 (s, 1H).

Synthesis of (R)-2-(4-{4-[4-chrolo-3-(cyanomethylcarbamoyl)-phenylethynyl]-phenyl}-piperazine-1-sulfonylamino)-3-methylbutanoic acid (III-33)

$^1$H NMR (DMSO-d6) δ: 0.92 (t, J=6.0 Hz, 6H), 1.94-2.01 (m, 1H), 3.17-3.29 (m, 4H), 3.47-3.53 (m, 1H), 4.33 (d, J=5.6 Hz, 2H), 6.99 (d, J=8.4 Hz, 2H), 7.42 (d, J=8.4 Hz, 2H), 7.55-7.59 (m, 3H), 7.82 (d, J=8.4 Hz, 1H), 9.28 (t, J=5.6 Hz, 1H).

Synthesis of (R)-2-(4-{4-[3-(2-cyanoethylcarbamoyl)-4-(2-methoxyethoxy)-phenylethynyl]-phenyl}-piperazine-1-sulfonylamino)-3-methylbutanoic acid (III-34)

$^1$H NMR (DMSO-d6) δ: 0.90 (br s, 6H), 1.96-2.03 (m, 1H), 2.75-2.79 (m, 2H), 3.12-3.24 (m, 4H), 3.26 (s, 3H), 3.52-3.56 (m, 2H), 3.75 (s, 2H), 4.29 (s, 2H), 6.97 (d, J=8.4 Hz, 2H), 7.21 (d, J=8.4 Hz, 1H), 7.33-7.46 (m, 3H), 7.61 (d, J=8.4 Hz, 1H), 7.86 (d, J=8.4 Hz, 1H), 8.54 (t, J=5.6 Hz, 1H).

Synthesis of (R)-2-{4-[4-(3-benzylcarbamoylphenylethynyl)-phenyl]-piperazine-1-sulfonylamino}-3-methylbutanoic acid (III-35)

$^1$H NMR (DMSO-d6) δ: 0.92 (t, J=6.0 Hz, 6H), 1.95-2.02 (m, 1H), 3.12-3.22 (m, 4H), 3.35 (br s, 4H), 3.49-3.53 (m, 1H), 4.50 (d, J=5.6 Hz, 2H), 6.99 (d, J=8.4 Hz, 2H), 7.24-7.53 (m, 8H), 7.66 (d, J=8.4 Hz, 1H), 7.81-7.89 (m, 2H), 8.04 (s, 1H), 9.15 (t, J=5.6 Hz, 1H), 12.84 (br s, 1H).

Synthesis of (R)-2-[4-(4-{3-[(cyanophenylmethyl)-carbamoyl]-phenylethynyl}-phenyl)-piperazine-1-sulfonylamino]-3-methylbutanoic acid (III-36)

$^1$H NMR (DMSO-d6) δ: 0.86 (br s, 6H), 1.92 (br s, 1H), 3.11 (br s, 4H), 3.35 (br s, 4H), 6.37 (br s, 1H), 6.93 (br s, 2H), 7.37-7.99 (m, 12H), 9.80 (br s, 1H), 12.73 (br s, 1H).

(R)-3-methyl-2-[4-(4-{2-[4-(2-oxopyrrolidine -1-yl)-benzylcarbamoyl]-pyridin-4-ylethynyl}-phenyl)-piperazine-1-sulfonylamino]-butanoic acid (III-37)

$^1$H NMR (DMSO-d6) δ: 0.92 (t, J=6.0 Hz, 6H), 1.93-2.08 (m, 3H), 2.45-2.51 (m, 2H), 3.10-3.20 (m, 4H), 3.48-3.53 (m, 1H), 3.80 (t, J=6.8 Hz, 2H), 4.48 (d, J=5.6 Hz, 2H), 7.02 (d, J=8.4 Hz, 2H), 7.33 (d, J=8.4 Hz, 2H), 7.49-7.66 (m, 5H), 7.84 (d, J=8.4 Hz, 1H), 8.03 (s, 1H), 8.65 (d, J=5.6 Hz, 1H), 9.34 (t, J=5.6 Hz, 1H), 12.70 (br s, 1H).

Synthesis of (R)-2-(4-{4-[((E)-8-propenyl)-naphthalen-2-ylethynyl]-phenyl}-piperazine-1-sulfonylamino)-proponic acid (III-38)

$^1$H NMR (DMSO-d6) δ: 1:30 (d, J = 7.1 Hz, 3H), 1.99 (d, J = 6.1 Hz, 3H), 3.10-3.57 (m, 8H), 3.77-3.79 (m, 1H), 6.31-6.35 (m, 1H), 7.01 (d, J = 8.1 Hz, 2H), 7.27 (d, J = 15.7 Hz, 1H), 7.46-7.65 (m, 6H), 7.82 (d, J = 7.6 Hz, 1H), 7.93 (d, J = 8.1 Hz, 1H), 8.31 (s, 1H).

Synthesis of (R)-2-{4-[4-(3-acetyl-1H-indol-5-ylethynyl)-phenyl]-piperazine-1-sulfonylamino}-proponic acid (III-39)

$^1$H NMR (DMSO-d6) δ: 1.30 (d, J = 7.1 Hz, 3H), 2.47 (s, 3H), 3.09-3.43 (m, 8H), 3.77-3.84 (m, 1H), 6.99 (d, J = 8.6 Hz, 2H), 7.33 (d, J = 8.6 Hz, 1H), 7.42 (d, J = 8.6 Hz, 2H), 7.49 (d, J = 8.6 Hz, 1H), 7.86-7.95 (m, 1H), 8.31 (s,

1H), 8.37 (d, J = 2.5 Hz, 1H), 12.10 (s, 1H).

Synthesis of (R)-2-{4-[4-(3-acetyl-1-methyl-1H-indol-5-ylethynyl)-phenyl]-piperazine-1-sulfonylamino}-proponic acid (III-40)

$^1$H NMR (DMSO-d6) δ: 1.30 (d, J = 7.1 Hz, 3H), 2.45 (s, 3H), 3.10-3.42 (m, 8H), 3.78-3.84 (m, 1H), 3.88 (s, 3H), 6.99 (d, J = 8.6 Hz, 2H), 7.38-7.45 (m, 3H), 7.58 (d, J = 8.6 Hz, 1H), 7.88-7.96 (m, 1H), 8.32 (s, 1H), 8.39 (s, 1H).

Synthesis of (R)-2-{4-[4-(3-acetyl-1-isobutyl-1H-indol-5-ylethynyl)-phenyl]-piperazine-1-sulfonylamino}-proponic acid (III-41)

$^1$H NMR (DMSO-d6) δ: 0.88 (d, J = 6.6 Hz, 6H), 1.30 (d, J = 7.1 Hz, 3H), 2.12-2.23 (m, 1H), 2.46 (s, 3H), 3.09-3.44 (m, 8H), 3.76-3.86 (m, 1H), 4.07 (d, J = 7.6 Hz, 2H), 6.99 (d, J = 8.6 Hz, 2H), 7.37 (d, J = 9.1 Hz, 1H), 7.43 (d, J = 8.6 Hz, 2H), 7.64 (d, J = 9.1 Hz, 1H), 7.92 (d, J = 8.6 Hz, 1H), 8.33 (s, 1H), 8.41 (s, 1H).

Synthesis of (R)-2-(4-{4-[3-(2-methoxyacetyl)-1-methyl-1H-indol-5-ylethynyl]-phenyl}-piperazine-1-sulfonylamino)-proponic acid (III-42)

$^1$H NMR (DMSO-d6) δ: 1.30 (d, J = 7.1 Hz, 3H), 3.10-3.40 (m, 11H), 3.77-3.84 (m, 1H), 3.89 (s, 3H), 4.49 (s, 2H), 6.99 (d, J = 8.6 Hz, 2H), 7.40-7.46 (m, 3H), 7.60 (d, J = 8.6 Hz, 1H), 7.92 (d, J = 8.6 Hz, 1H), 8.32 (s, 1H), 8.43 (s, 1H).

Synthesis of (R)-2-{4-[4-(3-benzoyl-1-methyl-1H-indol-5-ylethynyl)-phenyl]-piperazine-1-sulfonylamino}-proponic acid (III-43)

$^1$H NMR (DMSO-d6) δ: 1.30 (d, J = 6.6 Hz, 3H), 3.10-3.41 (m, 8H), 3.77-3.85 (m, 1H), 3.90 (s, 3H), 7.00 (d, J = 8.6 Hz, 2H), 7.43-7.49 (m, 3H), 7.53-7.65 (m, 4H), 7.81 (d, J = 7.1 Hz, 2H), 7.89-7.96 (m, 1H), 8.09 (s, 1H), 8.42 (s, 1H).

Synthesis of (R)-2-{4-[4-(3-isobutyryl-1-methyl-1H-indol-5-ylethynyl)-phenyl]-piperazine-1-sulfonylamino}-proponic acid (III-44)

$^1$H NMR (DMSO-d6) δ: 1.14 (d, J = 6.6 Hz, 6H), 1.30 (d, J = 7.1 Hz, 3H), 3.10-3.43 (m, 9H), 3.77-3.85 (m, 1H), 3.88 (s, 3H), 6.99 (d, J = 8.6 Hz, 2H), 7.38-7.45 (m, 3H), 7.57 (d, J = 8.6 Hz, 1H), 7.87-7.95 (m, 1H), 8.36 (s, 1H), 8.45 (s, 1H).

Synthesis of (R)-2-{4-[4-(1-methyl-3-pentanoyl-1H-indol-5-ylethynyl)-phenyl]-piperazine-1-sulfonylamino}-proponic acid (III-45)

$^1$H NMR (DMSO-d6) δ: 0.91 (t, J = 7.3 Hz, 3H), 1.26-1.41 (m, 5H), 1.60-1.67 (m, 2H), 2.81 (t, J = 7.3 Hz, 2H), 3.09-3.31 (m, 8H), 3.75-3.83 (m, 1H), 3.87 (s, 3H), 6.99 (d, J = 8.6 Hz, 2H), 7.37-7.45 (m, 3H), 7.56 (d, J = 8.6 Hz, 1H), 7.74-8.00 (m, 1H), 8.34 (s, 1H), 8.41 (s, 1H).

Synthesis of (R)-2-{4-[4-(1-methyl-3-oxazol-2-yl-1H-indol-5-ylethynyl)-phenyl]-piperazine-1-sulfonylamino}-proponic acid(III-46)

$^1$H NMR (DMSO-d6) δ: 1.30 (d, J = 7.6 Hz, 3H), 3.09-3.46 (m, 8H), 3.76-3.86 (m, 1H), 3.90 (s, 3H), 6.99 (d, J = 8.1 Hz, 2H), 7.32 (s, 1H), 7.39-7.46 (m, 3H), 7.60 (d, J = 8.1 Hz, 1H), 7.86-7.96 (m, 1H), 8.09 (s, 1H), 8.13 (s, 1H), 8.31 (s, 1H).

Synthesis of (R)-2-[4-(4-{4-[(E)-cyanomethoxyimino]-chroman-6-ylethynyl}-phenyl)-piperazine-1-sulfonylamino]-proponic acid (III-47)

$^1$H NMR (DMSO-d6) δ: 1.30 (d, J = 7.6 Hz, 3H), 2.89-2.95 (m, 2H), 3.09-3.34 (m, 8H), 3.73-3.85 (m, 1H), 4.23-4.30 (m, 2H), 5.13 (s, 2H), 6.95-7.02 (m, 3H), 7.41 (d, J = 7.6 Hz, 2H), 7.48 (d, J = 8.1 Hz, 1H), 7.74-7.91 (m, 2H).

Synthesis of (R)-2-[4-(4-{4-[(E)-methoxyimino]-1, 2, 3, 4-tetrahydroquinolin-6-ylethynyl}-phenyl)-piperazine-1-sulfonylamino]-proponic acid (III-48)

$^1$H NMR (DMSO-d6) δ: 1.30 (d, J = 7.1 Hz, 3H), 2.68 (t, J = 6.3 Hz, 2H), 3.09-3.40 (m, 10H), 3.76-3.85 (m, 1H), 3.89 (s, 3H), 6.52 (s, 1H), 6.66 (d, J = 8.6 Hz, 1H), 6.96 (d, J = 8.6 Hz, 2H), 7.18 (d, J = 8.6 Hz, 1H), 7.35 (d, J =

8.6 Hz, 2H), 7.73 (s, 1H), 7.90 (d, J = 7.1 Hz, 1H).

Synthesis of (R)-2-[4-(4-{4-[(E)-carbamoylmethoxyimino]-chroman-6-ylethynyl}-phenyl)-piperazine-1-sulfonylamino]-proponic acid (III-49)

$^1$H NMR (DMSO-d6) δ: 1.28 (d, J = 7.1 Hz, 3H), 2.99 (t, J = 5.8 Hz, 2H), 3.08-3.31 (m, 8H), 3.68-3.76 (m, 1H), 4.25 (t, J = 5.8 Hz, 2H), 4.52 (s, 2H), 6.93-6.99 (m, 3H), 7.25 (s, 1H), 7.33 (s, 1H), 7.38-7.45 (m, 3H), 7.83 (s, 1H).

Synthesis of (R)-2-[4-(4-{4-[(E)-2-hydroxyethoxyimino]-chroman-6-ylethynyl}-phenyl)-piperazine-1-sulfonylamino]-proponic acid (III-50)

$^1$H NMR (DMSO-d6) δ: 1.30 (d, J = 7.1 Hz, 3H), 2.89 (t, J = 6.1 Hz, 2H), 3.09-3.31 (m, 8H), 3.68 (t, J = 6.1 Hz, 2H), 3.75-3.84 (m, 1H), 4.17 (t, J = 5.6 Hz, 2H), 4.23 (t, J = 5.6 Hz, 2H), 6.93-7.00 (m, 3H), 7.38-7.43 (m, 3H), 7.77-7.91 (m, 2H).

Synthesis of (R)-2-{4-[4-(3-cyano-2H-chromen-6-ylethynyl)-phenyl]-piperazine-1-sulfonylamino}-proponic acid (III-51)

$^1$H NMR (DMSO-d6) δ: 1.29 (d, J = 6.6 Hz, 3H), 3.08-3.32 (m, 8H), 3.79 (s, 1H), 4.94 (s, 2H), 6.92 (d, J = 8.1 Hz, 1H), 6.97 (d, J = 8.1 Hz, 2H), 7.38 (d, J = 8.1 Hz, 2H), 7.41-7.45 (m, 2H), 7.57 (s, 1H), 7.89 (br s, 1H).

(R)-2-[4-(4-{4-[(E)-methoxyimino]-1-methyl-1, 2, 3, 4-tetrahydroquinolin-6-ylethynyl}-phenyl)-piperazine-1-sulfonylamino]-proponic acid (III-52)

$^1$H NMR (DMSO-d6) δ: 1.23 (d, J = 6.6 Hz, 3H), 2.75-2.80 (m, 2H), 2.88 (s, 3H), 3.10-3.62 (m, 11H), 3.89 (s, 3H), 6.76 (d, J = 7.6 Hz, 1H), 6.95 (d, J = 9.1 Hz, 2H), 7.38-7.31 (m, 3H), 7.81 (s, 1H).

N-benzyl4-{4-[4-((R)-1-carboxyethylsulfamoyl)-piperazin-1-yl]-phenylethynyl}-phthalamic acid (III-53)

$^1$H NMR (DMSO-d6) δ: 1.29 (d, J = 7.1 Hz, 3H), 3.08-3.92 (m, 9H), 4.44 (d, J = 6.1 Hz, 2H), 7.00 (d, J = 8.6 Hz, 2H), 7.22-7.92 (m, 11H), 8.90-8.97 (m, 1H).

Synthesis of (R)-2-{4-[4-(2-benzyl-1, 3-dioxo-2,3-dihydro-1H-isoindol-5-ylethynyl)-phenyl]-piperazine-1-sulfonylamino}-3-methylbutanoic acid (III-54)

$^1$H NMR (DMSO-d6) δ: 0.90 (d, J = 6.1 Hz, 3H), 0.91 (d, J = 6.1 Hz, 3H), 1.89-2.01 (m, 1H), 3.08-3.56 (m, 9H), 4.77 (s, 2H), 7.01 (d, J = 8.6 Hz, 2H), 7.24-7.36 (m, 5H), 7.47 (d, J = 8.6 Hz, 2H), 7.76-7.83 (m, 1H), 7.87-7.94 (m, 3H).

Synthesis of (R)-2-(4-{4-[4-acetylamino3-(4-chrolobenzylcarbamoyl)-phenylethynyl]-phenyl}-piperazine-1-sulfonylamino)-3-methylbutanoic acid (III-55)

$^1$H NMR (DMSO-d6) δ: 0.91 (d, J = 6.6 Hz, 3H), 0.92 (d, J = 6.6 Hz, 3H), 1.92-2.01 (m, 1H), 2.10 (s, 3H), 3.09-3.35 (m, 8H), 3.47-3.53 (m, 1H), 4.47 (d, J = 6.1 Hz, 2H), 6.99 (d, J = 8.6 Hz, 2H), 7.36-7.43 (m, 6H), 7.62 (d, J = 9.1 Hz, 1H), 7.77-7.85 (m, 1H), 7.95 (s, 1H), 8.43 (d, J = 9.1 Hz, 1H), 9.43 (t, J = 6.1 Hz, 1H), 11.30 (s, 1H).

Synthesis of (R)-2-{4-[4-(3-benzylcarbamoyl-4-nitrophenylethynyl)-phenyl]-piperazine-1-sulfonylamino}-3-methylbutanoic acid (III-56)

$^1$H NMR (DMSO-d6) δ: 0.91 (d, J = 6.1 Hz, 3H), 0.92 (d, J = 6.1 Hz, 3H), 1.93-2.01 (m, 1H), 3.08-3.34 (m, 8H), 3.47-3.53 (m, 1H), 4.47 (d, J = 5.6 Hz, 2H), 7.02 (d, J = 8.6 Hz, 2H), 7.25-7.30 (m, 1H), 7.34-7.40 (m, 4H), 7.48 (d, J = 8.6 Hz, 2H), 7.72 (s, 1H), 7.76 (d, J = 8.1 Hz, 1H), 7.80-7.86 (m, 1H), 8.07 (d, J = 8.1 Hz, 1H), 9.23 (t, J = 5.6 Hz, 1H).

Synthesis of (R)-2-(4-{4-[5-(4-fluorobenzylcarbamoyl)-1-(2-methoxyethyl)-6-oxo-1,6-dihydropyridin-3-yllethynyl]-phenyl}-piperazine-1-sulfonylamino)-3-methylbutanoic acid (III-57)

$^1$H NMR (DMSO-d6) δ: 0.90 (d, J = 6.6 Hz, 3H), 0.92 (d, J = 6.6 Hz, 3H), 1.91-2.00 (m, 1H), 3.03-3.69 (m, 13H), 4.18-4.24 (m, 2H), 4.50 (d, J = 6.1 Hz, 2H), 6.98 (d, J = 8.6 Hz, 2H), 7.15 (t, J = 8.8 Hz, 2H), 7.34-7.42 (m, 4H), 7.73-7.85 (m, 1H), 8.27 (s, 1H), 8.32 (s, 1H), 9.95 (t, J = 6.1 Hz, 1H).

Synthesis of (R)-2-{4-[4-(3-isopropylaminoxalyl -1-methyl-1H-indol-5-ylethynyl)-phenyl]-piperazine-1-sulfonylamino}-proponic acid (III-58)

$^1$ H NMR (DMSO-d6) δ: 1.18 (d, J=6.6 Hz, 6H), 1.30 (d, J=7.2 Hz, 3H), 3.05-3.40 (m, 8H), 3.81 (m, 1H), 4.04 (m, 1H), 7.00 (d, J=8.7 Hz, 2H), 7.40-7.50 (m, 3H), 7.64 (d, J=8.4 Hz, 1H), 7.93 (d, J=9.9 Hz, 1H), 8.35 (d, J=0.9 Hz, 1H), 8.56 (d, J=8.4 Hz, 1H), 8.80 (s, 1H), 12.77 (br s, 1H).

Synthesis of (S)-2-(4-{4-[2-(4-chrolobenzylcarbamoyl)-pyridin-4-ylethynyl]-phenyl}-piperazine-1-sulfonylamino)-proponic acid (III-59)

$^1$ H NMR (DMSO-d6) δ: 1.30 (d, J=7.5 Hz, 3H), 3.05-3.50 (m, 8H), 3.81 (m, 1H), 4.48 (d, J=6.3 Hz, 2H), 7.03 (d, J=9.0 Hz, 2H), 7.35-7.40 (m, 4H), 7.50 (d, J=8.7 Hz, 2H), 7.67 (dd, J=1.5, 4.8 Hz, 1H), 7.92 (d, J=9.3 Hz, 1H), 8.02 (s, 1H), 8.66 (d, J=5.4 Hz, 1H), 9.45 (t, J=6.0 Hz, 1H), 12.78 (br s, 1H).

Synthesis of (S)-2-(4-{4-[2-(4-chrolobenzylcarbamoyl)-pyridin-4-ylethynyl]-phenyl}-piperazine-1-sulfonylamino)-pentanoic acid (III-60)

$^1$ H NMR (DMSO-d6) δ: 0.88 (t, J=7.2 Hz, 3H), 1.30-1.50 (m, 2H), 1.50-1.66 (m, 2H), 3.05-3.45 (m, 8H), 3.70 (m, 1H), 4.48 (d, J=6.3 Hz, 2H), 7.02 (d, J=8.4 Hz, 2H), 7.32-7.42 (m, 4H), 7.50 (d, J=8.7 Hz, 2H), 7.67 (m, 1H), 7.90 (m, 1H), 8.02 (s, 1H), 8.66 (d, J=5.4 Hz, 1H), 9.45 (t, J=6.6 Hz, 1H).

Synthesis of (R)-2-(4-{4-[2-(4-chrolobenzylcarbamoyl)-pyridin-4-ylethynyl]-phenyl}-piperazine-1-sulfonylamino)-4-methylpentanoic acid (III-61)

$^1$ H NMR (DMSO-d6) δ: 0.80-0.95 (m, 6H), 1.35-1.60 (m, 2H), 1.73 (m, 1H), 3.00-3.40 (m, 8H), 3.70 (m, 1H), 4.48 (d, J=6.6 Hz, 2H), 7.02 (d, J=9.0 Hz, 2H), 7.30-7.40 (m, 4H), 7.49 (d, J=9.0 Hz, 2H), 7.66 (dd, J=1.2, 5.1 Hz, 1H), 7.94 (d, J=8.7 Hz, 1H), 8.01 (d, J=0.9 Hz, 1H), 8.65 (d, J=5.1 Hz, 1H), 9.45 (t, J=6.6 Hz, 1H), 12.72 (br s, 1H).

Synthesis of (R)-2-{4-[4-(8-isopropylcarbamoylnaphthalen-2-ylethynyl)-phenyl]-piperazine-1-sulfonylamino}-3-methylbutanoic acid (III-62)

$^1$ H NMR (DMSO-d6) δ: 0.90-1.00 (m, 6H), 1.22 (d, J=6.6 Hz, 6H), 1.98 (m, 1H), 3.05-3.40 (m, 8H), 3.49 (m, 1H), 4.19 (m, 1H), 7.01 (d, J=8.4 Hz, 2H), 7.47 (d, J=8.7 Hz, 2H), 7.52-7.64 (m, 3H), 7.83 (d, J=9.9 Hz, 1H), 7.96-8.05 (m, 2H), 8.33 (s, 1H), 8.47 (d, J=8.1 Hz, 1H).

Synthesis of (R)-2-{4-[4-(2-benzyl-3-oxo-2, 3-dihydro-1H-isoindol-5-ylethynyl)-phenyl]-piperazine-1-sulfonylamino}-proponic acid (III-63)

$^1$ H NMR (DMSO-d6) δ: 1.30 (d, J=7.2 Hz, 3H), 3.05-3.45 (m, 8H), 3.81 (m, 1H), 4.40 (s, 2H), 4.74 (s, 2H), 7.01 (d, J=9.0 Hz, 2H), 7.2, 4-7.40 (m, 5H), 7.45 (d, J=8.7 Hz, 2H), 7.59 (d, J=8.1 Hz, 1H), 7.71 (d, J=9.0 Hz, 1H), 7.77 (s, 1H), 7.92 (d, J=9.3 Hz, 1H), 12.76 (br s, 1H).

Synthesis of (R)-2-{4-[4-(2-benzyl1-oxo-1, 2, 3, 4-tetrahydroisoquinolin-7-ylethynyl)-phenyl]-piperazine-1-sulfonylamino}-proponic acid (III-64)

$^1$ H NMR (DMSO-d6) δ: 1.30 (d, J=7.2 Hz, 3H), 2.98 (t, J=6.6 Hz, 2H), 3.05-3.40 (m, 9H), 3.50 (t, J=6.6 Hz, 2H), 3.80 (m, 1H), 4.73 (s, 2H), 7.00 (d, J=9.0 Hz, 2H), 7.24-7.40 (m, 6H), 7.44 (d, J=8.4 Hz, 2H), 7.60 (dd, J=1.8, 8.1 Hz, 1H), 7.91 (d, J=8.4 Hz, 1H), 7.99 (s, 1H), 12.70 (br s, 1H).

Synthesis of (R)-2-{4-[4-(2-benzyl-3-oxo-2, 3-dihydro1H-isoindol-5-ylethynyl)-phenyl]-piperazine-1-sulfonylamino}-3-methylbutanoic acid (III-65)

$^1$ H NMR (DMSO-d6) δ: 0.85-0.95 (m, 6H), 1.98 (m, 1H), 3.05-3.55 (m, 9H), 4.40 (s, 2H), 4.74 (s, 2H), 7.00 (d, J=9.0 Hz, 2H), 7.26-7.42 (m, 5H), 7.45 (d, J=8.4 Hz, 2H), 7.59 (d, J=7.8 Hz, 1H), 7.71 (d, J=7.5 Hz, 1H), 7.74 (m, 1H), 7.78 (s, 1H).

Synthesis of (R)-2-{4-[4-(2-benzyl1-oxo-1, 2, 3, 4-tetrahydroisoquinolin-7-ylethynyl)-phenyl]-piperazine-1-sulfonylamino}-3-methylbutanoic acid (III-66)

$^1$ H NMR (DMSO-d6) δ: 0.85-0.95 (m, 6H), 1.98 (m, 1H), 2.98 (t, J=6.0 Hz, 2H), 3.00-3.60 (m, 13H), 4.72 (s, 2H), 6.99 (d, J=8.4 Hz, 2H), 7.2, 4-7.40 (m, 6H), 7.43 (d, J=8.7 Hz, 2H), 7.60 (m, 1H), 7.62 (m, 1H), 7.99 (s, 1H).

Synthesis of (R)-2-(4-{4-[5-(4-chrolobenzylcarbamoyl)-4-methylthiazol-2-ylethynyl]-phenyl}-piperazine-1-sulfonylamino)-proponic acid (III-67)

$^1$ H NMR (DMSO-d6) δ: 1.29 (d, J=6.9 Hz, 3H), 2.57 (s, 3H), 3.05-3.50 (m, 8H), 3.80 (m, 1H), 4.42 (d, J=5.7 Hz, 2H), 7.03 (d, J=8.7 Hz, 2H), 7.34 (d, J=8.7 Hz, 2H), 7.41 (d, J=8.4 Hz, 2H), 7.50 (d, J=9.0 Hz, 1H), 7.92 (m, 1H), 8.88 (m, 1H), 12.72 (br s, 1H).

Synthesis of (S)-2-(4-{4-[2-(4-chrolobenzylcarbamoyl)-pyridin-4-ylethynyl]-phenyl}-piperazine-1-sulfonylamino)-butanoic acid (III-68)

$^1$ H NMR (DMSO-d6) δ: 0.93 (t, J=7.5 Hz, 3H), 1.50-1.80 (m, 2H), 3.05-3.60 (m, 8H), 3.62 (m, 1H), 4.49 (d, J=6.3 Hz, 2H), 7.03 (d, J=8.7 Hz, 2H), 7.30-7.45 (m, 4H), 7.50 (d, J=8.7 Hz, 2H), 7.67 (d, J=5.1 Hz, 1H), 7.88 (m, 1H), 8.02 (s, 1H), 8.66 (d, J=5.4 Hz, 1H), 9.46 (t, J=6.3 Hz, 1H).

Synthesis of (S)-2-(4-{4-[2-(4-chrolobenzylcarbamoyl)-pyridin-4-ylethynyl]-phenyl}-piperazine-1-sulfonylamino)-3-methylbutanoic acid (III-69)

$^1$ H NMR (DMSO-d6) δ: 0.85-1.00 (m, 6H), 1.97 (m, 1H), 3.00-3.60 (m, 9H), 4.48 (d, J=6.3 Hz, 2H), 7.02 (d, J=8.7 Hz, 2H), 7.30-7.45 (m, 4H), 7.50 (d, J=8.4 Hz, 2H), 7.67 (d, J=5.4 Hz, 1H), 7.85 (d, J=9.9 Hz, 1H), 8.02 (s, 1H), 8.66 (d, J=5.4 Hz, 1H), 9.46 (t, J=6.3 Hz, 1H).

Synthesis of (R)-2-(4-{4-[5-(4-chrolobenzylcarbamoyl)-pyridin-3-yllethynyl]-phenyl}-piperazine-1-sulfonylamino)-3-methylbutanoic acid (III-70)

$^1$ H NMR (DMSO-d6) δ: 0.91 (d, J=6.3 Hz, 3H), 0.92 (d, J=6.6 Hz, 3H), 1.97 (m, 1H), 3.05-3.40 (m, 8H), 3.49 (m, 1H), 4.48 (d, J=6.0 Hz, 2H), 7.01 (d, J=8.7 Hz, 2H), 7.32-7.44 (m, 4H), 7.44 (d, J=8.7 Hz, 2H), 7.84 (d, J=9.6 Hz, 1H), 8.33 (m, 1H), 8.82 (d, J=1.8 Hz, 1H), 8.96 (d, J=2.1 Hz, 1H), 9.31 (t, J=6.0 Hz, 1H), 12.74 (br s, 1H).

Synthesis of (R)-2-(4-{4-[4-amino3-(4-chrolobenzylcarbamoyl)-phenylethynyl]-phenyl}-piperazine-1-sulfonylamino)-3-methylbutanoic acid (III-71)

$^1$ H NMR (DMSO-d6) δ: 0.91 (d, J=6.9 Hz, 3H), 0.92 (d, J=6.6 Hz, 3H), 1.97 (m, 1H), 3.05-3.40 (m, 8H), 3.49 (m, 1H), 4.39 (d, J=5.4 Hz, 2H), 6.70 (d, J=8.4 Hz, 1H), 6.82 (s, 2H), 6.95 (d, J=8.7 Hz, 2H), 7.26 (dd, J=1.8, 8.4 Hz, 1H), 7.28-7.42 (m, 6H), 7.75 (d, J=1.8 Hz, 1H), 7.83 (d, J=9.6 Hz, 1H), 8.95 (t, J=5.4 Hz, 1H), 12.72 (br s, 1H).

Synthesis of (R)-2-(4-{4-[3-(4-fluorobenzyl)-2, 4-dioxo-1, 2, 3, 4-tetrahydroquinazolin-6-ylethynyl]-phenyl}-piperazine-1-sulfonylamino)-3-methylbutanoic acid (III-72)

$^1$ H NMR (DMSO-d6) δ: 0.91 (d, J=6.6 Hz, 3H), 0.92 (d, J=6.6 Hz, 3H), 1.97 (m, 1H), 3.05-3.50 (m, 8H), 3.50 (m, 1H), 5.07 (s, 2H), 6.99 (d, J=8.4 Hz, 2H), 7.00-7.26 (m, 3H), 7.34-7.48 (m, 4H), 7.77 (dd, J=1.8, 8.1 Hz, 1H), 7.84 (d, J=9.6 Hz, 1H), 7.99 (d, J=1.5 Hz, 1H), 11.73 (s, 1H), 12.75 (br s, 1H).

Synthesis of (R)-2-(4-{4-[3-(4-methoxybenzyl)-2, 4-dioxo-1, 2, 3, 4-tetrahydroquinazolin-6-ylethynyl] -phenyl}-piperazine-1-sulfonylamino)-3-methylbutanoic acid (III-73)

$^1$ H NMR (DMSO-d6) δ: 0.91 (d, J=6.6 Hz, 3H), 0.92 (d, J=6.6 Hz, 3H), 1.97 (m, 1H), 3.05-3.45 (m, 8H), 3.49 (m, 1H), 5.02 (s, 2H), 6.87 (d, J=8.4 Hz, 2H), 6.99 (d, J=8.7 Hz, 2H), 7.20 (d, J=8.1 Hz, 1H), 7.29 (d, J=8.4 Hz, 2H), 7.42 (d, J=8.7 Hz, 2H), 7.77 (d, J=8.1 Hz, 1H), 7.84 (d, J=8.4 Hz, 1H), 7.98 (s, 1H), 11.69 (s, 1H), 12.70 (br s, 1H).

Synthesis of (R)-2-(4-{4-[3-(4-chrolobenzylcarbamoyl)-4-methylaminophenylethynyl]-phenyl}-piperazine-1-sulfonylamino)-3-methylbutanoic acid (III-74)

$^1$H NMR (DMSO-d6) δ: 0.91 (d, J=6.9 Hz, 3H), 0.92 (d, J=6.6 Hz, 3H), 1.97 (m, 1H), 3.05-3.40 (m, 8H), 3.49 (m, 1H), 4.40 (d, J=6.0 Hz, 2H), 6.66 (d, J=9.0 Hz, 1H), 6.96 (d, J=8.7 Hz, 2H), 7.30-7.46 (m, 6H), 7.80 (d, J=1.5 Hz, 1H), 7.83 (m, 1H), 8.00 (m, 1H), 9.06 (t, J=5.7 Hz, 1H), 12.70 (br s, 1H).

Synthesis of (R)-2-(4-{4-[3-(4-methoxybenzylcarbamoyl)-4-methylaminophenylethynyl]-phenyl}-piperazine-1-sulfonylamino)-3-methylbutanoic acid (III-75)

$^1$H NMR (DMSO-d6) δ: 0.91 (d, J=6.6 Hz, 3H), 0.92 (d, J=6.6 Hz, 3H), 1.97 (m, 1H), 2.80 (d, J=4.8 Hz, 3H), 3.05-3.60 (m, 9H), 3.72 (s, 3H), 4.34 (d, J=6.0 Hz, 2H), 6.65 (d, J=9.0 Hz, 1H), 6.74 (d, J=8.7 Hz, 2H), 6.96 (d, J=8.4 Hz, 2H), 7.24 (d, J=8.7 Hz, 2H), 7.33 (d, J=8.4 Hz, 2H), 7.41 (d, J=8.7 Hz, 1H), 7.77 (s, 1H), 7.82 (d, J=8.4 Hz, 1H), 8.00 (m, 1H), 8.96 (m, 1H).

Synthesis of (S)-2-{4-[4-(8-ethylnaphthalen-2-ylethynyl)-phenyl]-piperazine-1-sulfonylamino}-proponic acid (III-76)

$^1$H NMR (DMSO-d6) δ: 1.27-1.35 (m, 6H), 3.05-3.40 (m, 10H), 3.81 (m, 1H), 7.01 (d, J=8.7 Hz, 2H), 7.38-7.52 (m, 4H), 7.56 (dd, J=1.2, 8.4 Hz, 1H), 7.77 (d, J=7.5 Hz, 1H), 7.86-7.98 (m, 2H), 8.18 (s, 1H), 12.75 (br s, 1H).

Synthesis of (S)-2-{4-[4-(8-ethylnaphthalen-2-ylethynyl)-phenyl]-piperazine-1-sulfonylamino}-pentanoic acid (III-77)

$^1$H NMR (DMSO-d6) δ: 0.89 (t, J=7.2 Hz, 3H), 1.31 (t, J=7.5 Hz, 3H), 1.32-1.48 (m, 2H), 1.50-1.68 (m, 2H), 3.09 (q, J=7.2 Hz, 2H), 3.05-3.40 (m, 8H), 3.69 (m, 1H), 7.01 (d, J=9.0 Hz, 2H), 7.38-7.52 (m, 4H), 7.56 (d, J=8.4 Hz, 1H), 7.77 (d, J=7.8 Hz, 1H), 7.86-7.96 (m, 2H), 8.18 (s, 1H), 12.71 (br s, 1H).

Synthesis of (R)-2-{4-[4-(8-ethylnaphthalen-2-ylethynyl)-phenyl]-piperazine-1-sulfonylamino}-3-methylbutanoic acid (III-78)

$^1$H NMR (DMSO-d6) δ: 0.91 (d, J=6.6 Hz, 3H), 0.93 (d, J=6.6 Hz, 3H), 1.31 (t, J=7.5 Hz, 3H), 1.97 (m, 1H), 3.09 (q, J=7.5 Hz, 2H), 3.05-3.40 (m, 8H), 3.50 (m, 1H), 7.00 (d, J=8.7 Hz, 2H), 7.38-7.50 (m, 4H), 7.56 (m, 1H), 7.77 (d, J=8.4 Hz, 1H), 7.84 (d, J=9.9 Hz, 1H), 7.93 (d, J=8.7 Hz, 1H), 8.18 (s, 1H), 12.75 (br s, 1H).

Synthesis of (R)-2-(4-{4-[3-(4-methoxyethyl)-2, 4-dioxo-1, 2, 3, 4-tetrahydroquinazolin-6-ylethynyl]-phenyl}-piperazine-1-sulfonylamino)-3-methylbutanoic acid (III-79)

$^1$H NMR (DMSO-d6) δ: 0.91 (d, J=6.9 Hz, 3H), 0.93 (d, J=6.6 Hz, 3H), 1.97 (m, 1H), 3.05-3.40 (m, 8H), 3.26 (s, 3H), 3.50 (m, 1H), 3.54 (t, J=6.0 Hz, 2H), 4.09 (t, J=6.0 Hz, 2H), 6.99 (d, J=9.0 Hz, 2H), 7.19 (d, J=8.4 Hz, 1H), 7.42 (d, J=8.4 Hz, 2H), 7.76 (dd, J=1.8, 8.4 Hz, 1H), 7.83 (d, J=8.7 Hz, 1H), 7.97 (d, J=1.8 Hz, 1H), 11.62 (s, 1H), 12.75 (br s, 1H).

Synthesis of (R)-2-(4-{4-[3-(4-methoxybenzyl)-1-methyl-2, 4-dioxo-1, 2, 3, 4-tetrahydroquinazolin-6-ylethynyl]-phenyl}-piperazine-1-sulfonylamino)-3-methylbutanoic acid (III-80)

$^1$H NMR (DMSO-d6) δ: 0.91 (d, J=6.3 Hz, 3H), 0.92 (d, J=6.6 Hz, 3H), 1.97 (m, 1H), 3.05-3.40 (m, 8H), 3.50 (m, 1H), 5.07 (s, 2H), 6.86 (d, J=8.7 Hz, 2H), 7.00 (d, J=9.3 Hz, 2H), 7.30 (d, J=8.7 Hz, 2H), 7.44 (d, J=9.0 Hz, 2H), 7.49 (d, J=9.3 Hz, 1H), 7.80 (m, 1H), 7.87 (dd, J=1.8, 8.7 Hz, 1H), 8.10 (d, J=2.1 Hz, 1H), 12.74 (br s, 1H).

Synthesis of (R)-2-(4-{4-[2-(3-chrolobenzylcarbamoyl)-pyridin-4-ylethynyl]-phenyl}-piperazine-1-sulfonylamino)-3-methylbutanoic acid (III-81)

$^1$H NMR (DMSO-d6) δ: 0.91 (d, J=6.9 Hz, 3H), 0.92 (d, J=6.6 Hz, 3H), 1.97 (m, 1H), 3.09-3.65 (m, 9H), 4.46 (d, J=6.6 Hz, 2H), 6.98 (d, J=9.0 Hz, 2H), 7.25-7.37 (m, 4H), 7.46 (d, J=9.0 Hz, 2H), 7.64 (dd, J=1.8, 5.4 Hz, 1H), 7.78 (m, 1H), 7.99 (d, J=1.8 Hz, 1H), 8.62 (d, J=5.4 Hz, 1H), 9.45 (t, J=6.6 Hz, 1H).

Synthesis of (R)-2-(4-{4-[3-benzylcarbamoyl-4-(2-methoxyethoxy)-phenylethynyl]-phenyl}-piperazine-1-sulfonylamino)-proponic acid (III-82)

$^1$H NMR (DMSO-d6) δ: 1.29 (d, J=7.2 Hz, 3H), 3.10-3.48 (m, 11H), 3.66 (t, J=4.2 Hz, 2H), 3.80 (m, 1H), 4.28

(t, J=4.2 Hz, 2H), 4.52 (d, J=6.0 Hz, 2H), 6.98 (d, J=9.0 Hz, 2H), 7.20-7.42 (m, 8H), 7.60 (dd, J=2.4, 8.4 Hz, 1H), 7.87 (d, J=2.4 Hz, 1H), 7.91 (m, 1H), 8.69 (t, J=6.0 Hz, 1H).

Synthesis of (R)-2-(4-{4-[3-(3-chrolobenzylcarbamoyl)-4-(2-methoxyethoxy)-phenylethynyl]-phenyl}-piperazine-1-sulfonylamino)-proponic acid (III-83)

$^1$H NMR (DMSO-d6) δ: 1.29 (d, J=6.9 Hz, 3H), 3.10-3.39 (m, 11H), 3.68 (t, J=4.2 Hz, 2H), 3.80 (m, 1H), 4.29 (t, J=4.2 Hz, 2H), 4.52 (d, J=6.0 Hz, 2H), 6.98 (d, J=9.6 Hz, 2H), 7.22 (d, J=8.7 Hz, 1H), 7.31-7.42 (m, 6H), 7.61 (dd, J=2.4, 8.7 Hz, 1H), 7.85 (d, J=2.4 Hz, 1H), 7.90 (m, 1H), 8.73 (t, J=6.0 Hz, 1H).

Synthesis of (R)-2-(4-{4-[3-(3-chrolobenzylcarbamoyl)-4-(2-methoxyethoxy)-phenylethynyl]-phenyl}-piperazine-1-sulfonylamino)-3-methylbutanoic acid (III-84)

$^1$H NMR (DMSO-d6) δ: 0.91 (d, J=6.9 Hz, 3H), 0.92 (d, J=6.6 Hz, 3H), 1.97 (m, 1H), 3.11-3.47 (m, 11H), 3.49 (dd, J=6.6, 9.3 Hz, 1H), 3.68 (t, J=4.5 Hz, 2H), 4.29 (t, J=4.5 Hz, 2H), 4.52 (d, J=6.0 Hz, 2H), 6.98 (d, J=9.0 Hz, 2H), 7.22 (d, J=8.7 Hz, 1H), 7.31-7.42 (m, 6H), 7.61 (dd, J=2.4, 8.7 Hz, 1H), 7.84 (m, 1H), 7.85 (d, J=2.4 Hz, 1H), 8.73 (t, J=6.0 Hz, 1H).

Synthesis of (R)-2-(4-{4-[3-carbamoyl-4-(2-methoxyethoxy)-phenylethynyl]-phenyl}-piperazine-1-sulfonylamino)-proponic acid (III-85)

$^1$H NMR (DMSO-d6) δ: 1.30 (d, J=7.2 Hz, 3H), 3.07-3.47 (m, 11H), 3.73 (t, J=4.4 Hz, 2H), 3.81 (m, 1H), 4.29 (t, J=4.4 Hz, 2H), 6.98 (d, J=8.8 Hz, 2H), 7.19 (d, J=8.4 Hz, 1H), 7.40 (d, J=8.8 Hz, 2H), 7.59 (dd, J=2.4, 8.4 Hz, 1H), 7.66 (brs, 1H), 7.69 (brs, 1H), 7.90 (d, J=8.8 Hz, 1H), 7.92 (d, J=2.0 Hz, 1H).

Synthesis of (R)-2-{4-[4-(8-propionylnaphthalen-2-ylethynyl)-phenyl]-piperazine-1-sulfonylamino}-proponic acid (III-86)

$^1$H NMR (DMSO-d6) δ8: 1.16 (t, J=7.2 Hz, 3H), 1.31 (d, J=7.2 Hz, 3H), 3.10-3.48 (m, 10H), 3.82 (m, 1H), 7.01 (d, J=8.8 Hz, 2H), 7.48 (d, J=8.4 Hz, 2H), 7.61-7.65 (m, 2H), 7.91 (d, J=9.2 Hz, 1H), 8.02 (d, J=8.4 Hz, 1H), 8.12-8.15 (m, 2H), 8.61 (s, 1H).

Synthesis of (R)-2-{4-[4-(8-propionylnaphthalen-2-ylethynyl)-phenyl]-piperazine-1-sulfonylamino}-3-methylbutanoic acid (III-87)

$^1$H NMR (DMSO-d6) δ: 0.91 (d, J=6.0 Hz, 3H), 0.93 (d, J=6.4 Hz, 3H), 1.16 (t, J=7.2 Hz, 3H), 1.97 (m, 1H), 3.10-3.60 (m, 11H), 7.01 (d, J=8.8 Hz, 2H), 7.48 (d, J=8.8 Hz, 2H), 7.61-7.65 (m, 2H), 7.73 (d, J=8.8 Hz, 1H), 8.03 (d, J=8.4 Hz, 1H), 8.12-8.15 (m, 2H), 8.61 (s, 1H).

Synthesis of (R)-2-(4-{4-[3-benzylcarbamoyl-4-(2-dimethylaminoethoxy)-phenylethynyl]-phenyl}-piperazine-1-sulfonylamino)-proponic acid (III-88)

$^1$H NMR (DMSO-d6) δ: 1.27 (d, J=6.4 Hz, 3H), 2.06 (s, 6H), 2.58 (t, J=4.4 Hz, 2H), 3.09-3.68 (m, 9H), 4.24 (t, J=4.4 Hz, 2H), 4.52 (d, J=5.6 Hz, 2H), 6.97 (d, J=8.8 Hz, 2H), 7.20-7.41 (m, 9H), 7.60 (dd, J=2.0, 8.4 Hz, 1H), 7.92 (d, J=2.0 Hz, 1H), 9.26 (t, J=5.6 Hz, 1H).

Synthesis of (R)-2-{4-[4-(3-carbamoyl-4-phenethyloxyphenylethynyl)-phenyl]-piperazine-1-sulfonylamino}-3-methylbutanoic acid (III-89)

$^1$H NMR (DMSO-d6) δ: 0.91 (d, J=6.9 Hz, 3H), 0.92 (d, J=6.6 Hz, 3H), 1.96 (m, 1H), 3.10-3.40 (m, 10H), 3.49 (dd, J=6.6, 9.9 Hz, 1H), 4.37 (t, J=6.9 Hz, 2H), 6.97 (d, J=9.0 Hz, 2H), 7.17-7.39 (m, 8H), 7.56 (dd, J=2.4, 8.7 Hz, 1H), 7.56 (m, 1H), 7.87-7.84 (m, 2H).

Synthesis of (R)-2-{4-[4-(3-benzylcarbamoyl-4-methylcarbamoylmethoxyphenylethynyl)-phenyl]-piperazine-1-sulfonylamino}-3-methylbutanoic acid (III-90)

$^1$H NMR (DMSO-d6) δ: 0.81 (d, J=6.8 Hz, 3H), 0.90 (d, J=6.8 Hz, 3H), 2.05 (m, 1H), 2.63 (d, J=4.4 Hz, 3H), 3.11-3.48 (m, 9H), 4.52 (d, J=6.4 Hz, 2H), 4.71 (s, 2H), 6.96 (d, J=8.8 Hz, 2H), 7.11 (d, J=8.8 Hz, 1H), 7.25 (m, 1H), 7.31-7.42 (m, 7H), 7.59 (dd, J=2.0, 8.8 Hz, 1H), 7.78 (d, J=2.0 Hz, 1H), 8.25 (m, 1H), 9.21 (t, J=6.4 Hz, 1H).

Synthesis of (R)-2-{4-[4-(3-benzyl4-oxo-3,4-dihydro-2H-benzo][1,3]-oxazin-6-ylethynyl)-phenyl]-piperazine-1-sulfonylamino}-3-methylbutanoic acid (III-91)

$^1$H NMR (DMSO-d6) δ: 0.91 (d, J=6.0 Hz, 3H), 0.93 (d, J=6.0 Hz, 3H), 1.97 (m, 1H), 3.11-3.48 (m, 8H), 3.51 (dd, J=6.4, 9.6 Hz, 1H), 4.71 (s, 2H), 5.37 (s, 2H), 6.99 (d, J=8.8 Hz, 2H), 7.11 (d, J=8.4 Hz, 1H), 7.26-7.44 (m, 7H), 7.65 (dd, J=2.0, 8.4 Hz, 1H), 7.83 (d, J=9.6 Hz, 1H), 7.90 (d, J=2.0 Hz, 1H).

Synthesis of (R)-2-(4-{4-[3-(cyanomethylcarbamoyl)-benzyl-4-fluorophenylethynyl]-phenyl}-piperazine-1-sulfonylamino)-3-methylbutanoic acid (III-92)

$^1$H NMR (DMSO-d6) δ: 0.91 (d, J=6.0 Hz, 3H), 0.94 (d, J=6.4 Hz, 3H), 1.97 (m, 1H), 3.08-3.54 (m, 9H), 4.33 (d, J=5.2 Hz, 2H), 7.00 (d, J=8.8 Hz, 2H), 7.36-7.43 (m, 3H), 7.70 (m, 1H), 7.78 (dd, J=2.0, 8.4 Hz, 1H), 7.82 (d, J=9.6 Hz, 1H), 9.12 (t, J=5.2 Hz, 1H).

Synthesis of (R)-2-(4-{4-[2-(cyanomethylcarbamoyl)-pyridin-4-ylethynyl]-phenyl}-piperazine-1-sulfonylamino)-3-methylbutanoic acid (III-93)

$^1$H NMR (DMSO-d6) δ: 0.91 (d, J=5.6 Hz, 3H), 0.93 (d, J=6.4 Hz, 3H), 1.97 (m, 1H), 3.08-3.53 (m, 9H), 4.32 (d, J=6.0 Hz, 2H), 7.02 (d, J=8.8 Hz, 2H), 7.51 (d, J=8.8 Hz, 2H), 7.70 (dd, J=1.4, 5.2 Hz, 1H), 7.84 (d, J=9.6 Hz, 1H), 8.05 (d, J=1.4 Hz, 1H), 8.68 (d, J=5.2 Hz, 1H), 9.51 (t, J=6.0 Hz, 1H).

Synthesis of (R)-2-(4-{4-[3-(cyanomethylcarbamoyl)-4-methoxyphenylethynyl]-phenyl}-piperazine-1-sulfonylamino)-3-methylbutanoic acid (III-94)

$^1$H NMR (DMSO-d6) δ: 0.91 (d, J=6.0 Hz, 3H), 0.93 (d, J=6.4 Hz, 3H), 1.97 (m, 1H), 3.09-3.52 (m, 9H), 3.94 (s, 3H), 4.30 (d, J=5.6 Hz, 2H), 6.98 (d, J=8.8 Hz, 2H), 7.21 (d, J=8.4 Hz, 1H), 7.40 (d, J=8.8 Hz, 2H), 7.65 (dd, J=2.0, 8.4 Hz, 1H), 7.82 (d, J=9.6 Hz, 1H), 7.88 (d, J=2.0 Hz, 1H), 8.86 (t, J=5.6 Hz, 1H).

Synthesis of (R)-2-{4-[4-(4-benzyl-5-oxo-2, 3, 4, 5-tetrahydrobenzo[f][1,4]oxazepin-7-ylethynyl)-phenyl]-piperazine-1-sulfonylamino}-3-methylbutanoic acid (III-95)

$^1$H NMR (DMSO-d6) δ: 0.91 (d, J=6.0 Hz, 3H), 0.93 (d, J=6.0 Hz, 3H), 1.98 (m, 1H), 3.10-3.58 (m, 11H), 4.27 (t, J=4.4 Hz, 2H), 4.77 (s, 2H), 6.99 (d, J=8.8 Hz, 2H), 7.05 (d, J=8.4 Hz, 1H), 7.26-7.43 (m, 7H), 7.58 (dd, J=2.0, 8.4 Hz, 1H), 7.83 (d, J=9.6 Hz, 1H), 7.84 (d, J=2.0 Hz, 1H).

Synthesis of (R)-2-(4-{4-[4-benzyloxy-3-(cyanomethylcarbamoyl)-phenylethynyl]-phenyl}-piperazine-1-sulfonylamino)-3-methylbutanoic acid (III-96)

$^1$H NMR (DMSO-d6) δ: 0.91 (d, J=6.4 Hz, 3H), 0.92 (d, J=6.0 Hz, 3H), 1.94-1.99 (m, 1H), 3.10-3.83 (m, 9H), 4.33 (d, J=5.6 Hz, 2H), 5.34 (s, 2H), 6.98 (d, J=8.8 Hz, 2H), 7.23 (d, J=8.8 Hz, 1H), 7.30-7.42 (m, 5H), 7.48 (d, J=7.2 Hz, 2H), 7.58 (dd, J=2.0, 8.8 Hz, 1H), 7.77 (d, J=2.0 Hz, 1H), 7.81 (d, J=9.6 Hz, 1H), 8.94 (t, J=5.6 Hz, 1H).

Synthesis of (R)-2-(4-{4-[3-(3-methanesulfonylaminopropylcarbamoyl)-4-(2-methoxyethoxy)-phenylethynyl]-phenyl}-piperazine-1-sulfonylamino)-3-methylbutanoic acid (III-97)

$^1$H NMR (DMSO-d6) δ: 0.91 (d, J=6.0 Hz, 3H), 0.93 (d, J=6.0 Hz, 3H), 1.72 (m, 2H), 1.97 (m, 1H), 3.02 (m, 2H), 3.02-3.58 (m, 14H), 3.74 (t, J=4.4 Hz, 2H), 4.28 (t, J=4.4 Hz, 2H), 6.96-7.10 (m, 3H), 7.20 (d, J=8.8 Hz, 1H), 7.40 (d, J=8.4 Hz, 2H), 7.58 (dd, J=2.0, 8.8 Hz, 1H), 7.81 (d, J=7.6 Hz, 1H), 7.85 (d, J=2.0 Hz, 1H), 8.24 (t, J=4.8 Hz, 1H).

Synthesis of (R)-2-(4-{4-[3-(cyanomethylcarbamoyl)-4-(3-methyl-2-butenyloxy)phenylethynyl]-phenyl}-piperazine-1-sulfonylamino)-3-methylbutanoic acid (III-98)

$^1$H NMR (DMSO-d6) δ: 0.90 (d, J=6.4 Hz, 3H), 0.92 (d, J=7.2 Hz, 3H), 1.73 (s, 6H), 1.91-2.08 (m, 1H), 3.05-3.48 (m, 9H), 4.33 (d, J=5.2 Hz, 2H), 4.76 (d, J=5.6 Hz, 2H), 5.47 (m, 1H), 6.98 (d, J=8.4 Hz, 2H), 7.19 (d, J=8.4 Hz, 1H), 7.40 (d, J=8.4 Hz, 2H), 7.61 (dd, J=2.4, 8.4 Hz, 1H), 7.73-7.82 (m, 1H), 7.82 (d, J=2.4 Hz, 1H), 8.75 (t, J=5.2 Hz, 1H).

Synthesis of (R)-2-(4-{4-[3-(cyanomethylcarbamoyl)-4-(3-methylbutoxy)phenylethynyl]-phenyl}-piperazine-1-sulfonylamino)-3-methylbutanoic acid (III-99)

$^1$H NMR (DMSO-d6) δ: 0.91-0.95 (m, 12H), 1.68-1.79 (m, 3H), 1.97 (s, 1H), 3.14-3.35 (m, 8H), 3.50 (m, 1H), 4.17 (t, J=6.4 Hz, 2H), 4.33 (d, J=5.2 Hz, 2H), 6.98 (d, J=8.4 Hz, 2H), 7.22 (d, J=8.8 Hz, 1H), 7.40 (d, J=8.4 Hz, 2H), 7.61 (dd, J=2.4, 8.8 Hz, 1H), 7.77 (d, J=2.4 Hz, 1H), 7.83 (d, J=10 Hz 1H), 8.67 (t, J=5.2 Hz, 1H).

Synthesis of (R)-2-(4-{4-[3-(cyanomethylcarbamoyl)-4-trifluoromethoxyphenylethynyl]-phenyl}-piperazine-1-sulfonylamino)-3-methylbutanoic acid (III-100)

$^1$H NMR (DMSO-d6) δ: 0.91 (d, J=6.4 Hz, 3H),0.92 (d, J=6.8 Hz, 3H), 1.98 (m, 1H), 3.07-3.56 (m, 9H), 4.33 (d, J=5.6 Hz, 2H), 7.00 (d, J=8.4 Hz, 2H), 7.43 (d, J=8.4 Hz, 2H), 7.51 (d, J=8.0 Hz, 1H), 7.72-7.74 (m, 2H), 7.81 (m, 1H), 9.29 (t, J=5.6 Hz, 1H).

Synthesis of (R)-2-(4-{4-[3-(cyanomethylcarbamoyl)-4-(tetrahydropyran-4-ylmethoxy)-phenylethynyl]-phenyl}-piperazine-1-sulfonylamino)-3-methylbutanoic acid (III-101)

$^1$H NMR (DMSO-d6) δ: 0.83 (d, J=6.8 Hz, 3H), 0.90 (d, J=6.4 Hz, 3H), 1.21-1.38 (m, 4H), 1.69 (m, 1H), 2.07 (m, 1H), 2.69 (m, 2H), 3.10-3.78 (m, 9H), 3.87 (m, 2H), 3.99 (d, J=6.8 Hz, 2H), 4.33 (d, J=5.6 Hz, 2H), 6.97 (d, J=8.8 Hz, 2H), 7.19 (d, J=8.4 Hz, 1H), 7.39 (d, J=8.8 Hz, 2H), 7.60 (dd, J=2.0, 8.4 Hz, 1H), 7.69 (d, J=2.0 Hz, 1H), 8.71 (t, J=5.6 Hz, 1H).

Synthesis of (R)-2-(4-{4-[3-[3-(3-isopropylureido)-propylcarbamoyl]-4-(2-methoxyethoxy)-phenylethynyl]-phenyl}-piperazine-1-sulfonylamino)-3-methylbutanoic acid (III-102)

$^1$H NMR (DMSO-d6) δ: 0.91 (d, J=6.4 Hz, 3H), 0.92 (d, J=6.8 Hz, 3H), 1.02 (d, J=6.4 Hz, 6H), 1.59 (m, 2H), 1.98 (m, 1H), 3.03-3.55 (m, 16H), 3.62 (m, 1H), 3.75 (t, J=4.4 Hz, 2H), 4.28 (t, J=4.4 Hz, 2H), 5.70 (d, J=7.6 Hz, 1H), 5.76 (t, J=5.2 Hz, 1H), 6.98 (d, J=8.8 Hz, 2H), 7.19 (d, J=8.4 Hz, 1H), 7.40 (d, J=8.8 Hz, 2H), 7.58 (dd, J=2.0, 8.4 Hz, 1H), 7.82 (d, J=8.8 Hz, 1H), 7.87 (d, J=2.0 Hz, 1H), 8.26 (t, J=5.6 Hz, 1H).

Synthesis of (R)-2-(4-{4-[3-(3-acetylaminopropylcarbamoyl)-4-(2-methoxyethoxy)-phenylethynyl]-phenyl}-piperazine-1-sulfonylamino)-3-methylbutanoic acid (III-103)

$^1$H NMR (DMSO-d6) δ: 0.85 (d, J=6.4 Hz, 3H), 0.91 (d, J=6.4 Hz, 3H), 1.63 (m, 2H), 1.81 (s, 3H), 2.00 (m, 1H), 3.10-3.39 (m, 16H), 3.74 (m, 2H), 4.28 (m, 2H), 6.97 (d, J=8.4 Hz, 2H), 7.19 (d, J=8.4 Hz, 1H), 7.39 (d, J=8.4 Hz, 2H), 7.58 (dd, J=2.4, 8.4 Hz, 1H), 7.86 (m, 2H), 8.24 (m, 1H), 8.31 (s, 1H).

Synthesis of (R)-2-(4-{4-[3-(3-ethoxycarbonylaminopropylcarbamoyl)-4-(2-methoxyethoxy)-phenylethynyl]-phenyl}-piperazine-1-sulfonylamino)-3-methylbutanoic acid (III-104)

$^1$H NMR (DMSO-d6) δ: 0.88 (d, J=6.8 Hz, 3H), 0.92 (d, J=6.8 Hz, 3H), 1.15 (t, J=7.2 Hz, 3H), 1.64 (m, 2H), 1.99 (m, 1H), 3.03-3.51 (m, 16H), 3.74 (m, 2H), 3.98 (q, J=7.2 Hz, 2H), 4.28 (m, 2H), 6.97 (d, J=7.6 Hz, 2H), 7.08 (m, 1H), 7.19 (d, J=8.8 Hz, 1H), 7.39 (d, J=7.6 Hz, 2H), 7.58 (dd, J=2.4, 8.8 Hz, 1H), 7.86 (d, J=2.4 Hz, 1H), 8.23 (m, 1H), 8.31 (s, 1H).

Synthesis of (R)-2-[4-(4-{4-(2-methoxyethoxy)-3-[3-(propane-2-sulfonylamino)-propylcarbamoyl]-phenylethynyl}-phenyl)-piperazine-1-sulfonylamino]-3-methylbutanoic acid (III-105)

$^1$H NMR (DMSO-d6) δ: 0.91 (d, J=5.6 Hz, 3H), 0.92 (d, J=6.0 Hz, 3H), 1.22 (d, J=6.4 Hz, 6H), 1.71 (m, 2H), 1.97 (m, 1H), 3.03 (m, 2H), 3.08-3.55 (m, 15H), 3.74 (t, J=4.4 Hz, 2H), 4.28 (t, J=4.4 Hz, 2H), 6.92-7.05 (m, 3H), 7.20 (d, J=8.4 Hz, 1H), 7.40 (d, J=8.4 Hz, 2H), 7.58 (dd, J=2.0, 8.4 Hz, 1H), 7.82 (d, J=9.2 Hz, 1H), 7.85 (d, J=2.0 Hz, 1H), 8.23 (t, J=5.2 Hz, 1H).

Synthesis of (R)-2-(4-{4-[3-(cyanomethylcarbamoyl)-4-(3-methoxypropoxy)-phenylethynyl]-phenyl}-piperazine-1-sulfonylamino)-3-methylbutanoic acid (III-106)

$^1$H NMR (DMSO-d6) δ: 0.90 (d, J=7.2 Hz, 3H), 0.92 (d, J=7.2 Hz, 3H), 1.95-2.06 (m, 3H), 3.08-3.53 (m, 14H), 4.21 (t, J=6.4 Hz, 2H), 4.33 (d, J=5.6 Hz, 2H), 6.98 (d, J=8.4 Hz, 2H), 7.20 (d, J=8.4 Hz, 1H), 7.40 (d, J=8.4 Hz, 2H),

7.62 (dd, J=2.0, 8.4 Hz, 1H), 7.77 (m, 1H), 7.82 (d, J=2.0 Hz, 1H), 8.77 (t, J=5.6 Hz, 1H).

Synthesis of (R)-2-(4-{4-[3-[1-(methanesulfonylpiperidin-4-ylmethyl)-carbamoyl]-4-(2-methoxyethoxy)-phenylethynyl]-phenyl}-piperazine-1-sulfonylamino)-3-methylbutanoic acid (III-107)

$^1$H NMR (DMSO-d6) δ: 0.91 (d, J=6.8 Hz, 3H), 0.92 (d, J=6.4 Hz, 3H), 1.25 (m, 2H), 1.65 (m, 1H), 1.81 (m, 2H), 1.97 (m, 1H), 2.69 (t, J=10.4 Hz, 2H), 2.84 (s, 3H), 3.07-3.59 (m, 16H), 3.71 (t, J=4.4 Hz, 2H), 4.28 (t, J=4.4 Hz, 2H), 6.98 (d, J=8.4 Hz, 2H), 7.19 (d, J=8.8 Hz, 1H), 7.40 (d, J=8.0 Hz, 2H), 7.58 (dd, J=2.4, 8.8 Hz, 1H), 7.78 (m, 1H), 7.85 (d, J=2.4 Hz, 1H), 8.27 (t, J=6.0 Hz, 1H).

Synthesis of (R)-2-(4-{4-[2-(thiophene2-carbonyl)-1, 2, 3, 4-tetrahydroisoquinolin-7-ylethynyl]-phenyl}-piperazine-1-sulfonylamino)-proponic acid (III-108)

$^1$H NMR ($CDCl_3$) δ: 1.50 (d, J=7.2 Hz, 3H), 2.97 (t, J=6.0 Hz, 2H), 3.27-3.29 (m, 4H), 3.35-3.40 (m, 4H), 3.95 (t, J=6.0 Hz, 2H), 4.10-4.16 (m, 1H), 4.86 (s, 2H), 4.94 (d, J=8.4 Hz, 1H), 6.85-6.91 (m, 3H), 7.06-7.49 (m, 7H).

Synthesis of (R)-2-{4-[4-(3-benzylcarbamoyl-4-methoxyphenylethynyl)-phenyl]-piperazine-1-sulfonylamino}-proponic acid (III-109)

$^1$H NMR ($CDCl_3$) δ: 1.48 (d, J=7.2 Hz, 3H), 3.21-3.30 (m, 4H), 3.31-3.34 (m, 4H), 3.93 (s, 3H), 4.09 (dd, J=7.2, 8.1 Hz, 1H), 4.69 (d, J=5.7 Hz, 2H), 5.13 (d, J=8.1 Hz, 1H), 6.83 (d, J=8.4 Hz, 2H),6.94 (d, J=8.7 Hz, 1H),7.25-7.40 (m, 7H), 7.57 (dd, J=2.1, 8.7 Hz, 1H), 8.19 (t, J=5.7 Hz, 1H), 8.36 (d, J=2.1 Hz, 1H).

Synthesis of (R)-2-(4-{4-[3-(4-fluorobenzylcarbamoyl)-4-methoxyphenylethynyl]-phenyl}-piperazine-1-sulfonylamino)-proponic acid (III-110)

$^1$H NMR ($CDCl_3$) δ: 1.48 (d, J=7.2 Hz, 3H), 3.22-3.28 (m, 4H), 3.36-3.38 (m, 4H), 3.94 (s, 3H), 4.11 (dd, J=7.2, 8.4 Hz, 1H), 4.65 (d, J=6.0 Hz, 2H), 5.13 (d, J=8.4 Hz, 1H), 6.86-7.07 (m, 5H), 7.29-7.35 (m, 2H), 7.40 (d, J=10.5 Hz, 2H), 7.58 (dd, J=2.7, 7.8 Hz, 1H), 8.15 (t, J=6.0 Hz, 1H), 8.36 (d, J=2. 7Hz, 1H).

Synthesis of (R)-2-(4-{4-[4-(3-trifluoromethoxyphenyl)-thiazol-2-ylethynyl]-phenyl}-piperazine-1-sulfonylamino)-proponic acid (III-111)

$^1$H NMR (DMSO-d6) δ: 1.29 (d, J=7.2 Hz, 3H), 3.09-3.21 (m, 4H), 3.21-3.40 (m, 4H), 3.77-3.83 (m, 1H), 7.04 (d, J=9.3 Hz, 2H), 7.37-7.41 (m, 1H), 7.50 (d, J=9.3 Hz, 2H), 7.62 (t, J=8.1 Hz, 1H), 7.90-8.05 (m, 3H), 8.43 (s, 1H).

Synthesis of (R)-2-(4-{4-[4-(4-trifluoromethoxyphenyl)-thiazol-2-ylethynyl]-phenyl}-piperazine-1-sulfonylamino)-proponic acid (III-112)

$^1$H NMR (DMSO-d6) δ: 1.29 (d, J=7.2 Hz, 3H), 3.10-3.39 (m, 8H), 3.78-3.83 (m, 1H), 7.03 (d, J=9.0 Hz, 2H), 7.46 (d, J=8.1Hz, 2H), 7.53 (d, J=9.0 Hz, 2H), 7.88-7.91 (m, 1H), 8.11 (d, J=8.1 Hz, 2H), 8.31 (s, 1H).

Synthesis of (R)-2-(4-{4-[4-(2-trifluoromethoxyphenyl)-thiazol-2-ylethynyl]-phenyl}-piperazine-1-sulfonylamino)-proponic acid (III-113)

$^1$H NMR (DMSO-d6) δ: 1.29 (d, J=7.2 Hz, 3H), 3.06-3.39 (m, 8H), 3.78-3.83 (m, 1H), 7.04 (d, J=9.0 Hz, 2H), 7.49-7.58 (m, 5H), 7.89-7.94 (m, 1H), 8.06 (s, 1H), 8.07-8.10 (m, 1H).

Synthesis of (R)-2-{4-[4-(4-bromothiazol-2-ylethynyl)-phenyl]-piperazine-1-sulfonylamino}-proponic acid (III-114)

$^1$H NMR (DMSO-d6) δ: 1.27 (d, J=7.2 Hz, 3H), 2.99-3.40 (m, 8H), 3.78-3.81 (m, 1H), 7.01 (d, J=9. 0Hz, 2H), 7.48 (d, J=9.0 Hz, 2H), 7.88-7.91 (m, 1H), 7.91 (s, 1H).

Synthesis of (R)-2-(4-{4-[4-(4-phenoxyphenyl)-thiazol-2-ylethynyl]-phenyl}-piperazine-1-sulfonylamino)-proponic acid (III-115)

$^1$H NMR (DMSO-d6) δ: 1.29 (d, J=7.5 Hz, 3H), 3.12-3.50 (m, 8H), 3.68-3.80 (m, 1H), 7.02-7.10 (m, 6H), 7.19 (t, J=6.9 Hz, 1H),7.41-7.47 (m,2 H), 7.53 (d, J=8.4 Hz, 2H), 7.80-7.89 (m, 1H), 8.00 (d, J=8.4 Hz, 2H), 8.15 (s, 1H).

Synthesis of (R)-2-{4-[4-(3-benzylcarbamoyl-4-isopropoxyphenylethynyl)-phenyl]-piperazine-1-sulfonylamino}-proponic acid (III-116)

$^1$ H NMR (DMSO-d6) δ: 1.25 (d, J=6.3 Hz, 3H), 1.25 (d, J=6.3 Hz, 6H), 2.98-3.47 (m, 8H), 3.71-3.78 (m, 1H), 4.48 (d, J=5.7 Hz, 2H), 4.73-4.77 (m, 1H), 6.96 (d, J=8.7 Hz, 2H), 7.15-7.34 (m, 8H), 7.53 (dd, J=2.4, 8.4 Hz, 1H), 7.74 (d, J=2.4 Hz, 1H), 7.87 (d, =9.3 Hz, 1H), 8.52 (t, J=6.0 Hz, 1H).

Synthesis of (R)-2-(4-{4-[4-isopropoxy3-(4-trifluoromethoxybenzylcarbamoyl)-phenylethynyl]-phenyl}-piperazine-1-sulfonylamino)-proponic acid (III-117)

$^1$ H NMR (DMSO-d6) δ: 1.26 (d, J=6.3 Hz, 3H), 1.24 (d, J=6.3 Hz, 6H), 3.10-3.57 (m, 8H), 3.60-3.78 (m, 1H), 4.59 (d, J=5.7 Hz, 2H), 4.65-4.74 (m, 1H), 6.96 (d, J=8.7 Hz, 2H), 7.17 (d, J=10.8 Hz, 1H), 7.31-7.49 (m, 4H), 7.53 (dd, J=2.4, 10.8 Hz, 1H), 7.71 (d, J=2.4 Hz, 1H), 7.83 (br s, 1H), 8.57 (t, J=5.7 Hz, 1H).

Synthesis of (R)-2-(4-{4-[3-(3-chrolobenzylcarbamoyl)-4-isopropoxyphenylethynyl]-phenyl}-piperazine-1-sulfonylamino)-proponic acid (III-118)

$^1$ H NMR (DMSO-d6) δ: 1.25 (d, J=8.4 Hz, 3H), 1.30 (d, J=6.0 Hz, 6H), 3.05-3.37 (m, 8H), 3.78-3.83 (m, 1H), 4.50 (d, J=5.7Hz, 2H), 4.74-4.83 (m, 1H), 6.98 (d,J =8.7 Hz, 2H), 7.19 (d, J=8.4 Hz, 1H), 7.34-7.42 (m, 6H), 7.56 (dd, J=1.2, 8.4 Hz, 1H), 7.72 (d, J=1.2 Hz, 1H), 7.92 (d, J=8.4 Hz, 1H), 8.59 (t, J=5.7 Hz, 1H), 12.71 (br s, 1H).

Synthesis of (R)-2-(4-{4-[3-(3-chrolobenzylcarbamoyl)-4-isopropoxyphenylethynyl]-phenyl}-piperazine-1-sulfonylamino)-3-methylbutanoic acid (III-119)

$^1$ H NMR (DMSO-d6) δ: 0.90 (d, J=6.3 Hz, 3H), 0.92 (d, J=6.3 Hz, 3H), 1.30 (d, J=6.0 Hz, 6H), 1.91-2.00 (m, 1H), 3.07-3.43 (m, 8H), 3.47-3.52 (m, 1H), 4.50 (d, J=6.3 Hz, 2H), 4.74-4.82 (m, 1H), 6.97 (d, J=8.7 Hz, 2H), 7.18 (d, J=9.0 Hz, 1H), 7.31-7.41 (m, 6H), 7.55 (dd, J=2.1, 8.7 Hz, 1H), 7.70 (d, J=2.1 Hz, 1H), 7.84 (d, J=9.0 Hz, 1H), 8.58 (t, J=6.3 Hz, 1H).

Synthesis of (R)-2-{4-[4-(3-carbamoyl-4-isopropoxyphenylethynyl)-phenyl]-piperazine-1-sulfonylamino}-proponic acid (III-120)

$^1$ H NMR (DMSO-d6) δ: 1.28-1.35 (m, 9H), 3.10-3.40 (m, 8H), 3.78-3.83 (m, 1H), 4.79-4.85 (m, 1H), 6.98 (d, J=9.0 Hz, 2H), 7.19 (d, J=8.4 Hz, 1H), 7.39 (d, J=9.0 Hz, 2H), 7.55-7.63 (m, 3H), 7.88-7.93 (m, 2H), 12.72 (br s, 1H).

Synthesis of (R)-2-{4-[4-(3-benzylcarbamoyl-4-isobutoxyphenylethynyl)-phenyl]-piperazine-1-sulfonylamino}-proponic acid (III-121)

$^1$ H NMR (DMSO-d6) δ: 0.89 (d, J=6.3 Hz, 6H), 1.30 (d, J=6.3 Hz, 3H), 1.96-2.04 (m, 1H), 3.12-3.39 (m, 8H), 3.78-3.83 (m, 1H), 3.88 (d, J=6.6 Hz, 2H), 4.49 (d, J=5.7 Hz, 2H), 6.98 (d, J=8.4 Hz, 2H), 7.15 (d, J=9.0 Hz, 1H), 7.26-7.41 (m, 7H), 7.55 (dd, J=2.1, 9.0 Hz, 1H), 7.75 (d, J=2.1 Hz, 1H), 7.90 (d, J=8.7 Hz, 1H), 8.54 (t, J=5.7 Hz, 1H), 12.71 (br s, 1H).

Synthesis of (R)-2-(4-{4-[3-(3-chrolobenzylcarbamoyl)-4-isobutoxyphenylethynyl]-phenyl}-piperazine-1-sulfonylamino)-proponic acid (III-122)

$^1$ H NMR (DMSO-d6) δ: 0.90 (d, J=6.6 Hz, 6H), 1.2, 4-1.31 (m, 3H), 1.99-2.06 (m, 1H), 3.12-3.39 (m, 8H), 3.78-3.83 (m, 1H), 3.88 (d, J=6.3 Hz, 2H), 4.49 (d, J=5.7 Hz, 2H), 6.98 (d, J=8.4 Hz, 2H), 7.15 (d, J=8.7 Hz, 1H), 7.31-7.41 (m, 6H), 7.55 (dd, J=1.8, 8.7 Hz, 1H), 7.71 (d, J=1.8 Hz, 1H), 7.91 (d, J=9.3 Hz, 1H), 8.61 (t, J=5.7 Hz, 1H), 12.72 (br s, 1H).

Synthesis of (R)-2-{4-[4-(3-benzylcarbamoyl-4-hydroxyphenylethynyl)-phenyl]-piperazine-1-sulfonylamino}-proponic acid (III-123)

$^1$ H NMR (DMSO-d6) δ: 1.26 (d, J=7.2 Hz, 3H),3.12-3.27 (m, 8H), 3.65-3.68 (m, 1H), 4.51 (d, J=5.7 Hz, 2H), 6.90-6.98 (m, 3H), 7.2, 4-7.35 (m, 7H), 7.47 (d, J=8.1 Hz, 1H), 8.08 (s, 1H), 9.62 (br s, 1H).

Synthesis of (R)-2-(4-{4-[3-carbamoyl-4-(2-cyclohexylethoxy)-phenylethynyl]-phenyl}-piperazine-1-sulfonylamino)-3-methylbutanoic acid (III-124)

$^1H$ NMR (DMSO-d6) δ: 0.84-1.76 (m, 19H), 1.91-2.00 (m, 1H), 3.10-3.32 (m, 8H), 3.47-3.52 (m, 1H), 4.17 (t, J=6.6 Hz, 2H), 6.98 (d, J=9.0 Hz, 2H), 7.18 (d, J=8.7 Hz, 1H), 7.38 (d, J=9.0 Hz, 2H), 7.55-7.63 (m, 3H), 7.82-7.84 (m, 2H).

Synthesis of (R)-2-(4-{4-[4-(benzylcarbamoylmethoxy)-3-carbamoylphenylethynyl]-phenyl}-piperazine-1-sulfonylamino)-3-methylbutanoic acid (III-125)

$^1H$ NMR (DMSO-d6) δ: 0.84-0.93 (m, 6H), 1.95-2.01 (m, 1H), 3.12-3.26 (m, 8H), 3.43-3.47 (m, 1H), 4.35 (d, J=6.0 Hz, 2H), 4.80 (s, 2H), 6.98 (d, J=9.0 Hz, 2H), 7.10 (d, J=8.7 Hz, 1H), 7.21-7.33 (m, 5H), 7.39 (d, J=9.0 Hz, 2H), 7.57 (dd, J=2.4, 8.7 Hz ,1H), 7.70 (s, 1H), 7.82 (d, J=2.4 Hz, 1H), 8.16 (s, 1H), 8.92 (t, J=6.0 Hz, 1H).

Synthesis of (R)-2-(4-{4-[3-(3-chrolobenzylcarbamoyl)-4-fluorophenylethynyl]-phenyl}-piperazine-1-sulfonylamino)-3-methylbutanoic acid (III-126)

$^1H$ NMR (DMSO-d6) δ: 0.86-0.92 (m, 6H), 1.97-1.99 (m, 1H), 3.08-3.39 (m, 8H), 4.47 (d, J=6.3 Hz, 2H), 6.99 (d, J=9.0 Hz, 2H), 7.32-7.43 (m, 8H), 7.63-7.66 (m, 1H), 7.71-7.73 (m, 1H), 9.04 (t, J=6.3 Hz, 1H).

Synthesis of (R)-2-(4-{4-[4-(2-methoxyethoxy)-3-(N'-phenylhydrazinocarbonyl)-phenylethynyl]-phenyl}-piperazine-1-sulfonylamino)-3-methylbutanoic acid (III-127)

$^1H$ NMR ($CDCl_3$) δ: 0.90 (d, J=6.6 Hz, 3H), 1.00 (d, J=6.6 Hz,3 H), 2.06-2.19 (m, 1H), 3.14-3.21 (m, 4H), 3.22-3.34 (m, 4H), 3.30 (s, 3H), 3.75-3.81 (m, 2H), 4.31-4.38 (m, 2H), 5.22 (b, 1H), 6.74 (d, J=9.0 Hz, 2H), 6.87-6.97 (m, 3H), 7.18-7.33 (m, 6H), 7.57 (dd, J=2.4, 8.4 Hz, 1H), 8.22 (s, 1H), 9.96 (br s, 1H).

Synthesis of (R)-2-(4-{4-[3-(cyanomethylcarbamoyl)-4-isopropoxyphenylethynyl]-phenyl}-piperazine-1-sulfonylamino)-3-methylbutanoic acid (III-128)

$^1H$ NMR (DMSO-d6) δ: 0.90-0.93 (m, 6H), 1.35 (d, J=6.0 Hz, 6H), 1.94-1.99 (m, 1H), 3.13-3.35 (m, 8H), 3.47-3.52 (m, 1H), 4.34 (d, J=5.7 Hz, 2H), 4.75-4.83 (m, 1H), 6.98 (d, J=8.7 Hz, 2H), 7.21 (d, J=8.1 Hz, 1H), 7.39 (d, J=8.7 Hz, 2H), 7.58 (d, J=8.1 Hz,1H), 7.78 (s, 1H), 7.85 (d, J=6.9 Hz, 1H), 8.59 (t, J=5.7 Hz, 1H), 12.82 (br s ,1H).

Synthesis of (R)-2-(4-{4-[4-(2-methoxyethoxy)-3-phenoxycarbamoylphenylethynyl]-phenyl}-piperazine-1-sulfonylamino)-3-methylbutanoic acid (III-129)

$^1H$ NMR (DMSO-d6) δ: 0.89-0.93 (m, 6H), 1.93-2.00 (m, 1H), 3.08-3.30 (m, 11H), 3.40-3.50 (m, 1H), 3.73-3.77 (m, 2H), 4.25-4.30 (m, 2H), 6.98 (d, J=8.7 Hz, 2H), 7.05 (t, J=7.2 Hz, 1H), 7.15 (d, J=8.7 Hz, 2H), 7.22 (d, J=8.7 Hz, 1H), 7.34-7.41 (m, 4H), 7.61-7.66 (m, 2H), 7.81 (d, J=8.7 Hz, 1H), 11.94 (s, 1H).

Synthesis of (R)-2-{4-[4-(4-isopropoxy3-prop-2-ynlcarbamoylphenylethynyl)-phenyl]-piperazine-1-sulfonylamino}-3-methylbutanoic acid (III-130)

$^1H$ NMR (DMSO-d6) δ: 0.89-0.93 (m, 6H), 1.34 (d, J=6.3 Hz ,6H), 1.93-1.99 (m, 1H), 3.15-3.50 (m, 10H), 4.09 (d, J=3.0 Hz, 2H), 4.75-4.86 (m, 1H), 6.98 (d, J=8.4 Hz, 2H), 7.21 (d, J=8.7 Hz, 1H), 7.40 (d, J=8.4 Hz, 2H), 7.57 (d, J=8.7 Hz, 1H), 7.81 (s, 1H), 7.80-7.90 (m, 1H), 8.43 (t, J=3.0 Hz, 1H).

Synthesis of (R)-2-(4-{4-[3-(cyanomethylcarbamoyl)-4-isobutoxyphenylethynyl]-phenyl}-piperazine-1-sulfonylamino)-3-methylbutanoic acid (III-131)

$^1H$ NMR (DMSO-d6) δ: 0.81-0.98 (m, 12H), 1.90-2.01 (m, 1H), 2.09-2.12 (m, 1H), 3.10-3.35 (m, 9H), 3.89 (d, J=5.7 Hz, 2H), 4.31 (d, J=5.4 Hz, 2H), 6.95 (d, J=8.4 Hz, 2H), 7.15 (d, J=8.7 Hz, 1H), 7.37 (d, J=8.4 Hz, 2H), 7.57 (d, J=8.7 Hz, 1H), 7.59 (br s, 1H), 7.70 (s, 1H), 8.68 (t, J=5.4 Hz, 1H).

Synthesis of (R)-2-(4-{4-[3-(cyanomethylcarbamoyl)-4-ethoxyphenylethynyl]-phenyl}-piperazine-1-sulfonylamino)-3-methylbutanoic acid (III-132)

$^1H$ NMR (DMSO-d6) δ: 0.84-0.91 (m, 6H), 1.39 (t, J=6.9 Hz, 3H), 1.92-2.01 (m, 1H), 3.10-3.40 (m, 8H), 3.47(bs, 1H), 4.23 (q, J=6.9 Hz, 2H), 4.33 (d, J=5.4 Hz, 2H), 6.98 (d, J=9.0 Hz, 2H), 7.19 (d, J=8.7Hz, 1H), 7.39 (d, J=9.0 Hz, 2H), 7.61 (dd, J=2.1, 8.7 Hz, 1H), 7.70 (br s, 1H), 7.81 (d, J=2.1 Hz, 1H), 8.70 (t, J=5.4 Hz, 1H).

Synthesis of (R)-2-(4-{4-[3-(cyanomethylcarbamoyl)-4-propoxyphenylethynyl]-phenyl}-piperazine-1-sulfonylamino)-3-methylbutanoic acid (III-133)

$^1$H NMR (DMSO-d6) δ: 0.84-1.08 (m, 9H), 1.74-1.86 (m, 2H), 1.94-2.01 (m, 1H), 3.10-3.32 (m, 8H), 3.48 (bs, 1H), 4.11 (t, J=6.3 Hz, 2H), 4.33 (d, J=5.7 Hz, 2H), 6.97 (d, J=9.0 Hz, 2H), 7.19 (d, J=8.7 Hz, 1H), 7.39 (d, J=9.0 Hz, 2H), 7.60 (dd, J=2.1, 8.7 Hz, 1H), 7.70 (br s, 1H), 7.77 (d, J=2.1 Hz, 1H), 8.69 (t, J=5.7 Hz, 1H).

Synthesis of (R)-2-(4-{4-[4-butoxy3-(cyanomethylcarbamoyl)-phenylethynyl]-phenyl}-piperazine-1-sulfonylamino)-3-methylbutanoic acid (III-134)

$^1$H NMR (DMSO-d6) δ: 0.84-0.96 (m, 9H), 1.37-1.45 (m, 2H), 1.72-1.82 (m, 2H), 1.94-2.01 (m, 1H), 3.10-3.32 (m, 8H), 3.47 (bs, 1H), 4.15(t, J=6.6 Hz, 2H), 4.33 (d, J=5.7 Hz, 2H), 6.97 (d, J=9.0 Hz, 2H), 7.19 (d, J=9.0 Hz ,1H), 7.39 (d ,J=9.0 Hz, 2H), 7.61 (dd, J=2.4, 9.0 Hz, 1H), 7.70 (br s, 1H), 7.77 (d, J=2.4 Hz, 1H), 8.68 (t ,J=5.7 Hz, 1H).

Synthesis of (R)-2-(4-{4-[3-(cyanomethylcarbamoyl)-4-(2-ethoxyethoxy)-phenylethynyl]-phenyl}-piperazine-1-sulfonylamino)-3-methylbutanoic acid (III-135)

$^1$H NMR (DMSO-d6) δ: 0.84-0.93 (m, 6H), 1.12 (t, J=6.9 Hz, 3H), 1.94-1.99 (m, 1H), 3.07-3.57 (m, 11H), 3.76-3.79 (m, 2H), 4.28-4.32 (m, 2H), 4.35 (d, J=5.4 Hz, 2H), 6.97 (d, J=9.0 Hz, 2H), 7.24 (d, J=8.4 Hz, 1H), 7.39 (d, J=9.0 Hz, 2H), 7.63 (dd, J=1.2, 8.4 Hz, 1H), 7.77 (br s, 1H), 7.86 (d, J=1.2 Hz, 1H), 8.73 (t, J=5.4 Hz, 1H).

Synthesis of (R)-2-(4-{4-[3-(cyanomethylcarbamoyl)-4-(2, 2, 2-trifluoroethoxy)-phenylethynyl]-phenyl}-piperazine-1-sulfonylamino)-3-methylbutanoic acid (III-136)

$^1$H NMR (DMSO-d6) δ: 0.86-0.93 (m, 6H), 1.95-2.01 (m, 1H), 3.10-3.47 (m, 9H), 4.32 (d, J=5.4 Hz, 2H), 4.89 (q, J=8.4 Hz, 2H), 6.98 (d, J=8.7 Hz, 2H), 7.30 (d, J=9.3 Hz, 1H), 7.40 (d, J=8.7 Hz, 2H), 7.64-7.66 (m, 2H), 7.77(br s, 1H), 8.84 (t, J=5.4 Hz, 1H).

Synthesis of (R)-2-(4-{4-[3-(cyanomethylcarbamoyl)-4-(2-isopropoxyethoxy)-phenylethynyl]-phenyl}-piperazine-1-sulfonylamino)-3-methylbutanoic acid (III-137)

$^1$H NMR (DMSO-d6) δ: 0.84-0.93 (m, 6H), 1.11 (d, J=6.3 Hz, 6H), 1.95-2.01 (m, 1H), 3.10-3.46 (m, 9H), 3.62-3.70 (m, 1H), 3.76-3.79 (m, 2H), 4.27-4.30 (m, 2H), 4.34 (d, J=5.7 Hz, 2H), 6.97 (d, J=9.0 Hz, 2H), 7.24 (d, J=8.7 Hz, 1H), 7.40 (d, J=9.0 Hz, 2H), 7.63 (dd, J=2.1, 8.7 Hz, 1H), 7.67 (br s, 1H), 7.87 (d, J=2.1 Hz, 1H), 8.71 (t, J=5.4 Hz, 1H).

Synthesis of (R)-2-(4-{4-[4-(2-tert-butoxyethoxy)-3-(cyanomethylcarbamoyl)-phenylethynyl]-phenyl}-piperazine-1-sulfonylamino)-3-methylbutanoic acid (III-138)

$^1$H NMR (DMSO-d6) δ: 0.84-0.93 (m, 6H), 1.17 (s, 9H), 1.94-2.01 (m, 1H), 3.12-3.45 (m, 9H), 3.70-3.73 (m, 2H), 4.25-4.28 (m, 2H), 4.34 (d, J=5.7 Hz, 2H), 6.97 (d, J=9.0 Hz, 2H), 7.25 (d, J=9.0 Hz, 1H), 7.40 (d, J=9.0 Hz, 2H), 7.63 (dd, J=2.4, 9.0 Hz, 1H), 7.64 (br s, 1H), 7.88 (d, J=2.4 Hz, 1H), 8.69 (t, J=5.7 Hz, 1H).

Synthesis of (R)-2-(4-{4-[3-(2-methanesulfonylaminoethylcarbamoyl)-4-(2-methoxyethoxy)-phenylethynyl]-phenyl}-piperazine-1-sulfonylamino)-3-methylbutanoic acid (III-139)

$^1$H NMR (DMSO-d6) δ: 0.88-0.93 (m, 6H), 1.93-2.01 (m, 1H), 2.91 (s, 3H), 3.10-3.43 (m, 13H), 3.73-3.76 (m, 2H), 4.27-4.30 (m, 2H), 6.98 (d, J=9.0 Hz, 2H), 7.14 (t, J=6.0 Hz, 1H), 7.20 (d, J=5.7 Hz, 1H), 7.39 (d, J=9.0 Hz, 2H), 7.59 (dd, J=2.1, 6.0 Hz, 1H), 7.68 (br s, 1H), 7.88 (d, J=2.1 Hz, 1H), 8.36 (t, J=6.0 Hz, 1H)

Synthesis of (R)-2-(4-{4-[3-(4-methanesulfonylaminobutylcarbamoyl)-4-(2-methoxyethoxy)-phenylethynyl]-phenyl}-piperazine-1-sulfonylamino)-3-methylbutanoic acid (III-140)

$^1$H NMR (DMSO-d6) δ: 0.84-0.93 (m, 6H), 1,53-1.55 (m, 4H), 1.94-2.01 (m, 1H), 2.88 (s, 3H), 2.95-2.99 (m, 2H), 3.10-3.35 (m, 11H), 3.72-3.75 (m, 2H), 4.26-4.29 (m, 2H), 6.94-6.99 (m, 3H), 7.19 (d, J=8.4 Hz, 1H), 7.39 (d, J=9.0 Hz, 2H), 7.57 (dd, J=2.4, 8.4 Hz, 1H), 7.61 (br s, 1H), 7.86 (d, J=2.4 Hz, 1H), 8.22 (t, J=6.0 Hz, 1H).

Synthesis of (R)-2-{4-[4-(1-benzyl-2-oxo-1, 2-dihydropyrimidin-5-ylethynyl)-phenyl]-piperazine-1-sulfonylamino}-3-methylbutanoic acid (III-141)

$^1$H NMR (DMSO-d6) δ: 0.84-0.93 (m, 6H), 1.91-2.00 (m, 1H), 3.11-3.48 (m, 9H), 3.72-5.07 (s, 2H), 6.98 (d, J=9.0 Hz, 2H), 7.31-7.38 (m, 7H), 7.77-7.80 (m, 1H), 8.68 (d, J=3.3 Hz, 1H), 8.74 (d, J=3.3 Hz, 1H).

Synthesis of (R)-2-{4-[4-(3-acetylphenylethynyl)-phenyl]-piperazine-1-sulfonylamino}-proponic acid (III-142)

$^1$H NMR (DMSO-d6) δ: 1.29 (d, J=7.3 Hz, 3H), 2.62 (s, 3H), 3.13-3.34 (m, 8H), 3.80 (m, 1H), 7.00 (d, J=9.0 Hz, 2H), 7.44 (d, J=8.9 Hz, 2H), 7.55 (t, J=7.8 Hz, 1H), 7.74 (dt, J=7.8, 1.2 Hz, 1H), 7.90-7.95 (m, 2H), 8.04 (t, J=1.5 Hz, 1H), 12.73 (br s, 1H).

Synthesis of (R)-2-(4-{4-[3-(1-isobutoxyimonoethyl)-phenylethynyl]-phenyl}-piperazine-1-sulfonylamino)-proponic acid (III-143)

$^1$H NMR (DMSO-d6) δ: 0.93 (d, J=6.7 Hz, 6H), 1.30 (d, J=7.2 Hz, 3H), 2.00 (m, 1H), 2.22 (s, 3H), 3.14-3.39 (m, 8H), 3.80 (m, 1H), 3.93 (d, J=6.7 Hz, 2H), 6.99 (d, J=8.9 Hz, 2H), 7.40-7.45 (m, 3H), 7.52 (dt, J=7.7, 1.4 Hz, 1H), 7.65 (dt, J=7.7, 1.5 Hz, 1H), 7.74 (t, J=1.5 Hz, 1H), 7.90 (d, J=6.9 Hz, 1H).

Synthesis of (R)-2-(4-{4-[2-(4-chrolobenzylcarbamoyl)-pyridin-4-ylethynyl]-phenyl}-piperazine-1-sulfonylamino)-3-methylbutanoic acid (III-144)

$^1$H NMR (DMSO-d6) δ: 0.90-0.93 (m, 6H), 1.97(m, 1H), 3.14-3.39 (m, 8H), 3.47 (m, 1H), 4.48 (d, J=6.4 Hz, 2H), 7.02 (d, J=8.6 Hz, 2H), 7.33-7.39 (m, 4H), 7.49 (d, J=8.6 Hz, 2H), 7.66 (d, J=4.4 Hz, 1H), 7.84 (d, J=9.7 Hz, 1H), 8.02 (s, 1H), 8.65 (d, J=5.4 Hz, 1H), 9.44 (t, J=6.1 Hz, 1H).

Synthesis of (R)-2-[4-(4-{2-[(4-chrolobenzyl)-methylcarbamoyl]-pyridin-4-ylethynyl}-phenyl)-piperazine-1-sulfonylamino]-proponic acid (III-145)

$^1$H NMR (DMSO-d6) δ: 1.29 (d, J=7.1 Hz, 3H), 2.88(d, J=6.2 Hz, 3H), 3.14-3.39 (m, 8H), 3.80 (m, 1H), 4.57 (s, 1H), 4.69 (s, 1H), 7.02 (d, J=8.7 Hz, 2H), 7.31-7.55 (m, 7H), 7.66 (s, 1H), 7.91 (d, J=8.6 Hz, 1H), 8.58 (dd, J=14.9, 4.8 Hz, 1H), 12.7 (br s, 1H).

(R)-2-{4-[4-(2-cyclopropylcarbamoylpyridin-4-ylethynyl)-phenyl]-piperazine-1-sulfonylamino}-proponic acid (III-146)

$^1$H NMR (DMSO-d6) δ: 1.29 (d, J=7.1 Hz, 3H), 2.88(d, J=6.2 Hz, 3H), 3.14-3.39 (m, 8H), 3.80 (m, 1H), 4.57 (s, 1H), 4.69 (s, 1H), 7.02 (d, J=8.7 Hz, 2H), 7.31-7.55 (m, 7H), 7.66 (s, 1H), 7.91 (d, J=8.6 Hz, 1H), 8.58 (dd, J=14.9, 4.8 Hz, 1H), 12.7 (br s, 1H).

Synthesis of (R)-2-{4-[4-(2-isobutylcarbamoylpyridin-4-ylethynyl)-phenyl]-piperazine-1-sulfonylamino}-proponic acid (III-147)

$^1$H NMR (DMSO-d6) δ: 0.88 (d, J=6.6 Hz, 6H), 1.29 (d, J=7.2 Hz, 3H), 1.88 (m, 1H), 3.14-3.39 (m, 10H), 3.79 (m, 1H), 7.02 (d, J=8.9 Hz, 2H), 7.49 (d, J=8.9 Hz, 2H), 7.64 (dd, J=5.0, 1.6 Hz, 1H), 7.93 (d, J=7.3 Hz, 1H), 8.00(s, 1H), 8.63 (d, J=7.3 Hz, 1H), 8.81 (t, J=6.3 Hz).

Synthesis of (R)-2-(4-{4-[2-(cyclohexylmethylcarbamoyl)-pyridin-4-ylethynyl]-phenyl}-piperazine-1-sulfonylamino)-proponic acid (III-148)

$^1$H NMR (DMSO-d6) δ: 0.91-0.97 (m, 2H), 1.14-1.20 (m, 4H), 1.29 (d, J=7.2, 3H), 1.65-1.69 (m, 5H), 3.14-3.39 (m, 10H), 3.80 (m, 1H), 7.02 (d, J=8.9 Hz, 2H), 7.49 (d, J=8.7 Hz, 2H), 7.64 (dd, J=5.0, 1.5 Hz, 1H), 7.94 (d, J=8.7 Hz, 1H), 7.99 (s, 1H), 8.63 (d, J=5.0 Hz, 1H), 8.78 (t, J=6.3 Hz, 1H), 12.7 (br s, 1H).

Synthesis of (R)-2-[4-(4-{2-[2-(4-chrolophenyl)-ethylcarbamoyl]-pyridin-4-ylethynyl}-phenyl)-piperazine-1-sulfonylamino]-3-methylbutanoic acid (III-149)

$^1$H NMR (DMSO-d6) δ: 0.90 (d, J=6.6 Hz, 3H), 0.92 (d, J=6.6 Hz, 3H), 1.97 (m, 1H), 2.86 (t, J=7.2 Hz, 2H), 3.09-3.41 (m, 8H), 3.46-3.57 (m, 3H), 7.02 (d, J=9.0 Hz, 2H), 7.26 (d, J=8.4 Hz, 2H), 7.33 (d, J=8.4 Hz, 2H), 7.49 (d,

J=8.7 Hz, 2H), 7.64 (dd, J=5.1, 1.6 Hz, 1H), 7.85 (d, J=9.8 Hz, 1H), 7.98 (s, 1H), 8.62 (d, J=5.0 Hz, 1H), 8.90 (t, J=5.9 Hz, 1H).

Synthesis of (R)-2-(4-{4-[2-(4-methoxybenzylcarbamoyl)-pyridin-4-ylethynyl]-phenyl}-piperazine-1-sulfonylamino)-3-methylbutanoic acid (III-150)

$^1$H NMR (DMSO-d6) δ: 0.90 (d, J=6.1 Hz, 3H), 0.91 (d, J=6.3 Hz, 3H), 1.95 (m, 1H), 3.10-3.32 (m, 8H), 3.49 (m, 1H), 3.70 (s, 3H), 4.42 (d, J=6.1 Hz, 2H), 6.86 (d, J=8.3 Hz, 2H), 7.00 (d, J=8.6 Hz, 2H), 7.25 (d, J=8.1 Hz, 2H), 7.48 (d, J=8.6 Hz, 2H), 7.63 (d, J=4.5 Hz, 1H), 7.81 (d, J=9.1 Hz, 1H), 8.01 (s, 1H), 8.62 (d, J=4.8 Hz, 1H), 9.25 (t, J=6.3 Hz, 1H).

Synthesis of (R)-2-(4-{4-[2-(2-chrolobenzylcarbamoyl)-pyridin-4-ylethynyl]-phenyl}-piperazine-1-sulfonylamino)-3-methylbutanoic acid (III-151)

$^1$H NMR (DMSO-d6) δ: 0.90 (d, J=6.2 Hz, 3H), 0.92 (d, J=6.7 Hz, 3H), 1.97 (m, 1H), 3.10-3.42 (m, 8H), 3.49 (m, 1H), 4.57 (d, J=6.6 Hz, 2H), 7.02 (d, J=8.7 Hz, 2H), 7.27-7.31 (m, 3H), 7.44-7.51(m, 3H), 7.68 (dd, J=5.0, 1.7 Hz, 1H), 7.84 (d, J=8.4 Hz, 1H), 8.02 (dd, J=1.7, 0.8 Hz, 1H), 8.68 (d, J=4.9 Hz, 1H), 9.39 (t, J=6.3 Hz, 1H).

Synthesis of (R)-2-(4-{4-[2-(4-fluorobenzylcarbamoyl)-pyridin-4-ylethynyl]-phenyl}-piperazine-1-sulfonylamino)-3-methylbutanoic acid (III-152)

$^1$H NMR (DMSO-d6) δ: 0.90 (d, J=6.7 Hz, 3H), 0.92 (d, J=6.7 Hz, 3H), 1.97 (m, 1H), 3.09-3.42 (m, 8H), 3.49 (m, 1H), 4.47 (d, J=6.4 Hz, 2H), 7.01 (d, J=9.0 Hz, 2H), 7.10-7.16 (m, 2H), 7.34-7.39 (m, 2H), 7.49 (d, J=9.0 Hz, 2H), 7.66 (dd, J=5.0, 1.7 Hz, 1H), 7.84 (d, J=10.1 Hz, 1H), 8.01 (dd, J=1.5, 0.8 Hz, 1H), 8.64 (dd, J=5.0, 0.6 Hz, 1H), 9.42 (t, J=6.3 Hz, 1H).

Synthesis of (R)-2-(4-{4-[2-(4-dimethylaminomethylbenzylcarbamoyl)-pyridin-4-ylethynyl]-phenyl}-piperazine-1-sulfonylamino)-3-methylbutanoic acid borane complex (III-153)

$^1$H NMR (DMSO-d6) δ: 0.90 (d, J=6.7 Hz, 3H), 0.92 (d, J=6.7 Hz, 3H), 1.97 (m, 1H), 2.39 (s, 6H), 3.09-3.42 (m, 8H), 3.49 (m, 1H), 3.86 (s, 2H), 4.52 (d, J=6.4 Hz, 2H), 7.01 (d, J=9.0 Hz, 2H), 7.33 (d, J=8.2 Hz, 2H), 7.39 (d, J=8.2 Hz, 2H), 7.49 (d, J=8.9 Hz, 2H), 7.66 (dd, J=5.0, 1.7 Hz, 1H), 7.84 (d, J=9.5 Hz, 1H), 8.02 (dd, J=1.6, 0.8 Hz, 1H), 8.65 (dd, J=5.1, 0.7 Hz, 1H), 9.42 (t, J=6.3 Hz, 1H)

Synthesis of (R)-2-(4-{4-[2-(4-tert-butylbenzylcarbamoyl)-pyridin-4-ylethynyl]-phenyl}-piperazine-1-sulfonylamino)-3-methylbutanoic acid (III-154)

$^1$H NMR (DMSO-d6) δ: 0.90 (d, J=6.6 Hz, 3H), 0.92 (d, J=6.6 Hz, 3H), 1.25 (s, 9H), 1.95 (m, 1H), 3.08-3.51 (m, 9H), 4.45 (d, J=6.1 Hz, 2H), 7.01 (d, J=8.7 Hz, 2H), 7.24 (d, J=8.2 Hz, 2H), 7.32 (d, J=8.4 Hz, 2H), 7.48 (d, J=8.5 Hz, 2H), 7.64 (dd, J=5.0, 1.5 Hz, 1H), 7.83 (d, J=8.7 Hz, 1H), 8.01 (s, 1H), 8.63 (d, J=5.2 Hz, 1H), 9.31 (t, J=6.3 Hz, 1H).

Synthesis of (R)-2-[4-(4-{2-[(6-chrolopyridin-3-yllmethyl)-carbamoyl]-pyridin-4-ylethynyl}-phenyl)-piperazine-1-sulfonylamino]-3-methylbutanoic acid (III-155)

$^1$H NMR (DMSO-d6) δ: 0.90 (d, J=6.6 Hz, 3H), 0.92 (d, J=6.6 Hz, 3H), 1.95 (m, 1H), 3.09-3.42 (m, 8H), 3.49 (dd, J=9.7, 6.5 Hz, 1H), 4.50 (d, J=6.3 Hz, 2H), 7.01 (d, J=9.0 Hz, 2H), 7.46-7.50 (m, 3H), 7.66 (dd, J=5.0, 1.7 Hz, 1H), 7.80 (dd, J=8.2, 2.4 Hz, 1H), 7.86 (d, J=9.6 Hz, 1H), 8.01 (dd, J=1.5, 0.8 Hz, 1H), 8.38 (d, J=2.1 Hz, 1H), 8.65 (d, J=5.0 Hz, 1H), 9.54 (t, J=6.3 Hz, 1H).

Synthesis of (R)-2-(4-{4-[2-(3,4-dichrolobenzylcarbamoyl)-pyridin-4-ylethynyl]-phenyl}-piperazine-1-sulfonylamino)-3-methylbutanoic acid (III-156)

$^1$H NMR (DMSO-d6) δ: 0.90 (d, J=6.6 Hz, 3H), 0.92 (d, J=6.6 Hz, 3H), 1.95 (m, 1H), 3.09-3.42 (m, 8H), 3.49 (dd, J=9.7, 6.5 Hz, 1H), 4.48 (d, J=6.3 Hz, 2H), 7.02 (d, J=8.9 Hz, 2H), 7.32 (dd, J=8.2, 2.0 Hz, 1H), 7.49 (d, J=8.9 Hz, 2H), 7.57-7.59 (m, 2H), 7.67 (dd, J=5.0, 1.7 Hz, 1H), 7.86 (d, J=9.9 Hz, 1H), 8.01 (dd, J=1.6, 0.8 Hz, 1H), 8.65 (dd, J=5.0, 0.8 Hz, 1H), 9.54 (t, J=6.3 Hz, 1H).

Synthesis of (R)-2-(4-{4-[2-(3, 5-dichrolobenzylcarbamoyl)-pyridin-4-ylethynyl]-phenyl}-piperazine-1-sulfonylamino)-3-methylbutanoic acid (III-157)

$^1H$ NMR (DMSO-d6) δ: 0.90 (d, J=6.6 Hz, 3H), 0.92 (d, J=6.6 Hz, 3H), 1.96 (m, 1H), 3.10-3.52 (m, 9H), 4.49 (d, J=6.3 Hz, 2H), 7.02 (d, J=9.0 Hz, 2H), 7.37 (d, J=1.8 Hz, 2H), 7.48-7.51 (m, 2H), 7.67 (dd, J=5.0, 1.5 Hz, 1H), 7.85 (d, J=9.5 Hz, 1H), 8.02 (s, 1H), 8.66 (d, J=4.9 Hz, 1H), 9.54 (t, J=6.3 Hz, 1H).

Synthesis of (R)-2-(4-{4-[2-(5-methyloxazol-2-yl)-pyridin-4-ylethynyl]-phenyl}-piperazine-1-sulfonylamino)-3-methylbutanoic acid (III-158)

$^1H$ NMR (DMSO-d6) δ: 1.30 (d, J=7.2 Hz, 3H), 2.42 (d, J=1.0 Hz, 3H), 3.10-3.41 (m, 8H), 3.80 (m, 1H), 7.02 (d, J=9.1 Hz, 2H), 7.10 (d, J=1.2 Hz, 1H), 7.50 (d, J=8.9 Hz, 2H), 7.54 (dd, J=5.0, 1.7 Hz, 1H), 7.90 (d, J=8.6 Hz, 1H), 8.03 (s, 1H), 8.66 (d, J=5.0 Hz, 1H).

Synthesis of (R)-2-(4-{4-[2-(4-dimethylaminomethylbenzylcarbamoyl)-pyridin-4-ylethynyl]-phenyl}-piperazine-1-sulfonylamino)-3-methylbutanoic acid (III-159)

$^1H$ NMR (DMSO-d6) δ: 0.90 (m, 6H), 1.98 (m, 1H), 2.23 (s, 6H), 3.14-3.70 (m, 11H), 4.49 (s, 2H), 7.00 (d, J=6.8 Hz, 2H), 7.29 (m, 4H), 7.48 (d, J=7.2 Hz, 2H), 7.65 (s, 1H), 8.02 (s, 1H), 8.64 (s, 1H), 9.35 (br s, 1H).

Synthesis of (R)-2-(4-{4-[2-(3-dimethylaminomethyl-4-fluorobenzylcarbamoyl)-pyridin-4-ylethynyl]-phenyl}-piperazine-1-sulfonylamino)-3-methylbutanoic acid (III-160)

$^1H$ NMR (DMSO-d6) δ: 0.89 (d, J=6.8 Hz, 3H), 0.92 (d, J=6.8 Hz, 3H), 1.98 (m, 1H), 2.19 (s, 6H), 3.11-3.21 (m, 4H), 3.30-3.31 (m, 4H), 3.49-3.50 (m, 3H), 4.48 (d, J=6.1 Hz, 2H), 7.00 (d, J=8.8 Hz, 2H), 7.11 (t, J=9.1 Hz, 1H), 7.29 (m, 1H), 7.38 (d, J=6.6 Hz, 1H), 7.48 (d, J=8.6 Hz, 2H), 7.65 (d, J=4.6 Hz, 1H), 8.03 (s, 1H), 8.64 (d, J=5.1 Hz, 1H), 9.40 (t, J=6.3 Hz, 1H).

Synthesis of (R)-2-[4-(4-{(E)-2-[2-(4-methoxybenzylcarbamoyl)-pyridin-4-yl]-vinyl}-phenyl)-piperazine-1-sulfonylamino]-3-methylbutanoic acid (III-161)

$^1H$ NMR (DMSO-d6) δ: 0.90 (d, J=6.3 Hz, 3H), 0.91 (d, J=6.3 Hz, 3H), 1.96 (m, 1H), 3.13-3.49 (m, 9H), 3.71 (s, 3H), 4.42 (d, J=6.3 Hz, 2H), 6.86 (d, J=8.3 Hz, 2H), 6.99 (d, J=8.6 Hz, 2H), 7.15 (d, J=16.4 Hz, 1H), 7.26 (d, J=8.8 Hz, 2H), 7.50-7.57 (m, 3H), 7.68 (d, J=5.1 Hz, 1H), 7.79 (br s, 1H), 8.13 (s, 1H), 8.53 (d, J=5.3 Hz, 1H), 9.17 (t, J=6.3 Hz, 1H).

Synthesis of (R)-2-(4-{4-[4-fluoro-3-(4-methoxybenzylcarbamoyl)-phenylethynyl]-phenyl}-piperazine-1-sulfonylamino)-3-methylbutanoic acid (III-162)

$^1H$ NMR (DMSO-d6) δ: 0.90 (d, J=6.2 Hz, 3H), 0.91 (d, J=6.2 Hz, 3H), 1.96 (m, 1H), 3.13-3.26 (m, 8H), 3.49 (m, 1H), 3.72 (s, 3H), 4.38 (d, J=5.8 Hz, 2H), 6.89 (d, J=8.3 Hz, 2H), 6.97 (d, J=8.6 Hz, 2H), 7.25 (d, J=8.1 Hz, 2H), 7.31 (t, J=9.5 Hz, 1H), 7.40 (d, J=8.3 Hz, 2H), 7.62 (m, 1H), 7.69 (d, J=6.1 Hz, 1H), 7.78 (m, 1H), 8.89 (t, J=5.2 Hz, 1H).

Synthesis of (R)-2-(4-{4-[4-fluoro3-(4-methanesulfonylbenzylcarbamoyl)-phenylethynyl]-phenyl}-piperazine-1-sulfonylamino)-3-methylbutanoic acid (III-163)

$^1H$ NMR (DMSO-d6) δ: 0.91 (d, J=6.2 Hz, 3H), 0.92 (d, J=6.2 Hz, 3H), 1.97 (m, 1H), 3.14-3.20 (m, 4H), 3.20 (s, 3H), 3.26-3.29 (m, 4H), 3.49 (m, 1H), 4.57 (d, J=6.1 Hz, 2H), 6.99 (d, J=8.3 Hz, 2H), 7.36 (t, J=9.7 Hz, 1H), 7.42 (d, J=8.3 Hz, 2H), 7.60 (d, J=8.3 Hz, 2H), 7.66 (m, 1H), 7.76 (d, J=5.8 Hz, 1H), 7.82 (d, J=9.4 Hz, 1H), 7.91 (d, J=7.8 Hz, 2H), 9.11 (t, J=5.2 Hz, 1H).

Synthesis of (R)-2-(4-{4-[4-fluoro-3-(4-fluorobenzylcarbamoyl)-phenylethynyl]-phenyl}-piperazine-1-sulfonylamino)-3-methylbutanoic acid (III-164)

$^1H$ NMR (DMSO-d6) δ: 0.91 (d, J=6.2 Hz, 3H), 0.92 (d, J=6.2 Hz, 3H), 1.97 (m, 1H), 3.14-3.50 (m, 9H), 4.45 (d, J=5.3 Hz, 2H), 6.99 (d, J=8.6 Hz, 2H), 7.16 (d, J=8.6 Hz, 1H), 7.18 (d, J=8.6 Hz, 1H), 7.32-7.42 (m, 5H), 7.64 (m, 1H), 7.72 (d, J=6.1 Hz, 1H), 7.80 (br s, 1H), 8.99 (t, J=5.6 Hz, 1H).

Synthesis of (R)-2-[4-(4-{4-fluoro3-[(thiophen-2-ylmethyl)-carbamoyl]-phenylethynyl}-phenyl)-piperazine-1-sulfonylamino]-3-methylbutanoic acid (III-165)

$^1$ H NMR (DMSO-d6) δ: 0.91 (d, J=6.1 Hz, 3H), 0.92 (d, J=6.1 Hz, 3H), 1.97 (m, 1H), 3.11-3.29 (m, 8H), 3.50 (m, 1H), 4.62 (d, J=5.8 Hz, 2H), 6.98-7.03 (m, 4H), 7.34 (t, J=9.4 Hz, 1H), 7.40-7.43 (m, 3H), 7.65 (m, 1H), 7.69 (d, J=6.6 Hz, 1H), 7.82 (d, J=9.1 Hz, 1H), 9.07 (t, J=5.9 Hz, 1H).

Synthesis of (R)-2-(4-{4-[4-fluoro-3-(4-sulfamoylbenzylcarbamoyl)-phenylethynyl]-phenyl}-piperazine-1-sulfonylamino)-3-methylbutanoic acid (III-166)

$^1$ H NMR (DMSO-d6) δ: 0.82 (d, J=6.6 Hz, 3H), 0.90 (d, J=6.8 Hz, 3H), 2.04 (m, 1H), 3.15-3.38 (m, 9H), 4.53 (d, J=5.3 Hz, 2H), 6.97 (d, J=8.6 Hz, 2H), 7.33-7.37 (m, 3H), 7.41 (d, J=7.8 Hz, 2H), 7.51 (d, J=7.8 Hz, 2H), 7.65 (m, 1H), 7.75 (d, J=6.1 Hz, 1H), 7.80 (d, J=7.8 Hz, 2H), 9.09 (t, J=5.2 Hz, 1H).

Synthesis of (R)-2-(4-{4-[2-(4-methoxybenzyl)-1-oxo-1, 2-dihydroisoquinolin-7-ylethynyl]-phenyl}-piperazine-1-sulfonylamino)-3-methylbutanoic acid (III-167)

$^1$ H NMR (DMSO-d6) δ: 0.91 (d, J=6.6 Hz, 3H), 0.92 (d, J=6.8 Hz, 3H), 1.97 (m, 1H), 3.14-3.45 (m, 9H), 3.72 (m, 3H), 5.11 (s, 2H), 6.67 (d, J=6.6 Hz, 2H), 6.90 (d, J=8.8 Hz, 2H), 7.00 (d, J=8.1 Hz, 2H), 7.31 (d, J=8.8 Hz, 2H), 7.45 (d, J=7.8 Hz, 2H), 7.59 (d, J=7.1 Hz, 1H), 7.67 (t, J=8.6 Hz, 1H), 7.75-7.80 (m, 2H), 8.28 (s, 1H).

Synthesis of (R)-2-[4-(4-{4-fluoro-3-[(pyridin-4-ylmethyl)-carbamoyl]-phenylethynyl}-phenyl)-piperazine-1-sulfonylamino]-3-methylbutanoic acid (III-168)

$^1$ H NMR (DMSO-d6) δ: 0.91 (d, J=6.3 Hz, 3H), 0.92 (d, J=6.3 Hz, 3H), 1.97 (m, 1H), 3.12-3.49 (m, 9H), 4.50 (d, J=5.8 Hz, 2H), 6.99 (d, J=8.8 Hz, 2H), 7.35-7.43 (m, 5H), 7.67 (m, 1H), 7.77 (d, J=6.6 Hz, 1H), 7.81 (br s, 1H), 8.53 (br s, 2H), 9.08 (t, J=5.8 Hz, 1H).

Synthesis of (R)-3-methyl-2-{4-[4-(1-oxo-2-pyridin-4-ylmethyl-1, 2-dihydroisoquinolin-7-ylethynyl)-phenyl]-piperazine-1-sulfonylamino}-butanoic acid (III-169)

$^1$ H NMR (DMSO-d6) δ: 0.91 (d, J=6.4 Hz, 3H), 0.92 (d, J=6.4 Hz, 3H), 1.97 (m, 1H), 3.11-3.48 (m, 9H), 5.23 (s, 2H), 6.74 (d, J=7.3 Hz, 2H), 7.00 (d, J=8.8 Hz, 2H), 7.23 (br s, 2H), 7.45 (d, J=8.8 Hz, 2H), 7.62 (d, J=7.1 Hz, 1H), 7.72 (d, J=8.1 Hz, 1H), 7.79-7.82 (m, 2H), 8.27 (s, 1H), 8.52 (br s, 2H).

Synthesis of (R)-2-(4-{4-[2-(4-methanesulfonylbenzyl)-1-oxo-1, 2-dihydroisoquinolin-7-ylethynyl]-phenyl}-piperazine-1-sulfonylamino)-3-methylbutanoic acid (III-170)

$^1$ H NMR (DMSO-d6) δ: 0.91 (d, J=6.3 Hz, 3H), 0.92 (d, J=6.3 Hz, 3H), 1.99 (m, 1H), 3.14-3.48 (m, 12H), 5.13 (s, 2H), 6.73 (d, J=7.3 Hz, 1H), 7.00 (d, J=9.4 Hz, 2H), 7.45 (d, J=8.6 Hz, 2H), 7.55 (d, J=7.8 Hz, 2H), 7.66 (d, J=7.3 Hz, 1H), 7.71 (t, J=8.1 Hz, 1H), 7.80 (m, 2H), 7.90 (d, J=7.8 Hz, 2H), 8.27 (s, 1H).

Synthesis of (R)-3-methyl-2-(4-{4-[2-(4-piperidin-1-ylbenzylcarbamoyl)-pyridin-4-ylethynyl]-phenyl}-piperazine-1-sulfonylamino)-butanoic acid (III-171)

$^1$ H NMR (DMSO-d6) δ: 0.90 (d, J=6.2 Hz, 3H), 0.92 (d, J=6.2 Hz, 3H), 1.51 (m, 2H), 1.59 (m, 4H), 1.96 (m, 1H), 3.07 (t, J=5.2, 4H), 3.12-3.50(m, 9H), 4.39 (d, J=6.1 Hz, 2H), 6.86 (d, J=8.6 Hz, 2H), 7.02 (d, J=8.6 Hz, 2H), 7.17 (d, J=8.3 Hz, 2H), 7.49 (d, J=8.5 Hz, 2H), 7.64 (dd, J=5.0, 1.5 Hz, 1H), 7.81 (d, J=8.7 Hz, 1H), 8.02 (s, 1H), 8.63 (d, J=5.3 Hz, 1H), 9.18 (t, J=6.6 Hz, 1H).

Synthesis of (R)-2-(4-{4-[5-(4-methoxybenzylcarbamoyl)-1-methyl-6-oxo-1, 6-dihydropyridin-3-yllethynyl]-phenyl}-piperazine-1-sulfonylamino)-3-methylbutanoic acid (III-172)

$^1$ H NMR (DMSO-d6) δ: 0.90 (d, J=6.2 Hz, 3H), 0.92 (d, J=6.2 Hz, 3H), 1.97 (m, 1H), 3.12-3.50(m, 9H), 3.56 (s, 3H), 3.73 (s, 3H), 4.45 (d, J=5.8 Hz, 2H), 6.90 (d, J=8.6 Hz, 2H), 6.98 (d, J=8.3 Hz, 2H), 7.25 (d, J=8.6 Hz, 2H), 7.38 (d, J=8.6 Hz, 2H), 7.81 (br s, 1H), 8.31 (d, J=2.3 H, 1H), 8.40 (d, J=2.3 Hz, 1H), 9.94 (t, J=5.8 Hz, 1H).

Synthesis of (R)-3-methyl-2-(4-{4-[2-(4-methylcarbamoylbenzylcarbamoyl)-pyridin-4-ylethynyl]-phenyl}-piperazine-1-sulfonylamino)-butanoic acid (III-173)

$^1$H NMR (DMSO-d6) δ: 0.90 (d, J=7.2 Hz, 3H), 0.91 (d, J=7.2 Hz, 3H), 1.97 (m, 1H), 2.77 (d, J=4.6 Hz, 3H), 3.12-3.50(m, 9H), 4.55 (d, J=5.8 Hz, 2H), 7.02 (d, J=9.4 Hz, 2H), 7.39 (d, J=7.8 Hz, 2H), 7.49 (d, J=9.4 Hz, 2H), 7.66 (d, J=5.1, 1H), 7.76-7.81 (m, 3H), 8.02 (s, 1H), 8.38 (br s, 1H), 8.66 (d, J=5.3 Hz, 1H), 9.45 (t, J=8.3 Hz, 1H).

Synthesis of (R)-2-(4-{4-[2-(4-isopropylcarbamoylbenzylcarbamoyl)-pyridin-4-ylethynyl]-phenyl}-piperazine-1-sulfonylamino)-3-methylbutanoic acid (III-174)

$^1$H NMR (DMSO-d6) δ: 0.90 (d, J=6.1 Hz, 3H), 0.91 (d, J=6.1 Hz, 3H), 1.15 (d, J=6.6 Hz, 6H), 1.96 (m, 1H), 3.12-3.50(m, 9H), 4.04 (m, 1H), 4.55 (d, J=5.6 Hz, 2H), 7.01 (d, J=8.3 Hz, 2H), 7.39 (d, J=8.1 Hz, 2H), 7.49 (d, J=8.6 Hz, 2H), 7.66 (d, J=4.3, 1H), 7.77-7.83 (m, 3H), 8.03 (s, 1H), 8.15 (d, J=6.8 Hz, 1H), 8.65 (d, J=4.8 Hz, 1H), 9.44 (t, J=6.1 Hz, 1H).

Synthesis of (R)-2-(4-{4-[2-(3-isopropylcarbamoylbenzylcarbamoyl)-pyridin-4-ylethynyl]-phenyl}-piperazine-1-sulfonylamino)-3-methylbutanoic acid (III-175)

$^1$H NMR (DMSO-d6) δ: 0.89 (br s, 6H), 1.14 (br s, 6H), 1.97 (br s, 1H), 3.15-3.50 (m, 9H), 4.01 (br s, 1H), 4.53 (br s, 2H), 7.00 (br s, 2H), 7.38-7.47 (m, 4H), 7.66-7.78 (m, 4H), 8.01 (br s, 1H), 8.20 (br s, 1H), 8.65 (br s, 1H), 9.41 (br s, 1H).

Synthesis of (R)-2-(4-{4-[2-(4-acetylaminobenzylcarbamoyl)-pyridin-4-ylethynyl]-phenyl}-piperazine-1-sulfonylamino)-3-methylbutanoic acid(III-176)

$^1$H NMR (DMSO-d6) δ: 0.90 (d, J=6.2 Hz, 3H), 0.91 (d, J=6.2 Hz, 3H), 1.97 (m, 1H), 2.02 (s, 3H), 3.12-3.52 (m, 9H), 4.44 (d, J=5.8 Hz, 2H), 7.01 (d, J=8.6 Hz, 2H), 7.25 (d, J=8.1 Hz, 2H), 7.48-7.51 (m, 4H), 7.64 (d, J=4.6, 1H), 7.82 (d, J=9.9 Hz, 1H), 8.02 (s, 1H), 8.63 (d, J=4.8 Hz, 1H), 9.29 (t, J=5.7 Hz, 1H), 9.91 (s, 1H).

Synthesis of (R)-2-{4-[4-(2-benzylcarbamoylpyridin-4-ylethynyl)-phenyl]-piperazine-1-sulfonylamino}-3-methylbutanoic acid (III-177)

[M+H]=576, Retention Time 2.28 min
Conditions (in the same condition as follows)

Column : Phenomenex Luna, C18(2), 4.6x50 mm, 5 mm
Solvent : Water/acetonitrile 90:10 - 0:100 (3min), 0:100 (1min) Flow:3.00 ml/min

Synthesis of (R)-2-(4-{4-[2-(3-methoxybenzylcarbamoyl)-pyridin-4-ylethynyl]-phenyl}-piperazine-1-sulfonylamino)-3-methylbutanoic acid (III-178)

[M+H]=606, Retention Time 2.28 min

Synthesis of (R)-2-(4-{4-[2-(3-chrolo4-fluorobenzylcarbamoyl)-pyridin-4-ylethynyl]-phenyl}-piperazine-1-sulfonylamino)-3-methylbutanoic acid (III-179)

[M+H]=628, Retention Time 2.48 min

Synthesis of (R)-2-(4-{4-[2-(4-dimethylcarbamoylbenzylcarbamoyl)-pyridin-4-ylethynyl]-phenyl}-piperazine-1-sulfonylamino)-3-methylbutanoic acid (III-180)

[M+H]=647, Retention Time 1.91 min

Synthesis of (R)-2-(4-{4-[2-(3-fluorobenzylcarbamoyl)-pyridin-4-ylethynyl]-phenyl}-piperazine-1-sulfonylamino)-3-methylbutanoic acid (III-181)

[M+H]=594, Retention Time 2.30 min

Synthesis of (R)-3-methyl-2-[4-(4-{2-[(pyridin-4-ylmethyl)-carbamoyl]-pyridin-4-ylethynyl}-phenyl)-piperazine-1-sulfonylamino]-butanoic acid (III-182)

[M+H]=577, Retention Time 1.30 min

Synthesis of (R)-3-methyl-2-[4-(4-{2-[(pyridine 2-ylmethyl)-carbamoyl]-pyridin-4-ylethynyl}-phenyl)-piperazine-1-sulfonylamino]-butanoic acid (III-183)

[M+H]=577, Retention Time 1.62 min

Synthesis of (R)-2-(4-{4-[2-(4-fluoro-3-methylbenzylcarbamoyl)-pyridin-4-ylethynyl]-phenyl}-piperazine-1-sulfonylamino)-3-methylbutanoic acid (III-184)

[M+H]=608, Retention Time 2.42 min

Synthesis of (R)-3-methyl-2-(4-{4-[2-(3-trifluoromethylbenzylcarbamoyl)-pyridin-4-ylethynyl]-phenyl}-piperazine-1-sulfonylamino)-butanoic acid (III-185)

[M+H]=644, Retention Time 2.50 min

Synthesis of (R)-3-methyl-2-(4-{4-[2-(3-methylbenzylcarbamoyl)-pyridin-4-ylethynyl]-phenyl}-piperazine-1-sulfonylamino)-butanoic acid (III-186)

[M+H]=590, Retention Time 2.44 min

Synthesis of (R)-3-methyl-2-[4-(4-{2-[(thiophen-2-ylmethyl)-carbamoyl]-pyridin-4-ylethynyl}-phenyl)-piperazine-1-sulfonylamino]-butanoic acid (III-187)

[M+H]=582, Retention Time 2.28 min

Synthesis of (R)-2-[4-(4-{2-[(furan-2-ylmethyl)-carbamoyl]-pyridin-4-ylethynyl}-phenyl)-piperazine-1-sulfonylamino]-3-methylbutanoic acid (III-188)

[M+H]=566, Retention Time 2.19 min

Synthesis of (R)-2-(4-{4-[2-(3. 4-difluorobenzylcarbamoyl)-pyridin-4-ylethynyl]-phenyl}-piperazine-1-sulfonylamino)-3-methylbutanoic acid (III-189)

[M+H]=612, Retention Time 2.69 min

Synthesis of (R)-3-methyl-2-[4-(4-{2-[(1-methyl1H-pyrazol-3-ylmethyl)-carbamoyl]-pyridin-4-ylethynyl}-phenyl)-piperazine-1-sulfonylamino]-butanoic acid (III-190)

[M+H]=580, Retention Time 1.92 min

Synthesis of (R)-2-(4-{4-[2-(4-fluoro-3-methoxymethylbenzylcarbamoyl)-pyridin-4-ylethynyl]-phenyl}-piperazine-1-sulfonylamino)-3-methylbutanoic acid (III-191)

[M+H]=638, Retention Time 2.33 min

Synthesis of (R)-2-(4-{4-[2-(3, 5-difluorobenzylcarbamoyl)-pyridin-4-ylethynyl]-phenyl}-piperazine-1-sulfonylamino)-3-methylbutanoic acid (III-192)

[M+H]=612, Retention Time 2.61 min

Synthesis of (R)-3-methyl-2-(4-{4-[2-(4-methylbenzylcarbamoyl)-pyridin-4-ylethynyl]-phenyl}-piperazine-1-sulfonylamino)-butanoic acid (III-193)

[M+H]=590, Retention Time 2.71 min

Synthesis of (R)-3-methyl-2-(4-{4-[2-(4-trifluoromethylbenzylcarbamoyl)-pyridin-4-ylethynyl]-phenyl}-piperazine-1-sulfonylamino)-butanoic acid (III-194)

[M+H]=644, Retention Time 2.77 min

Synthesis of (R)-2-(4-{4-[2-(4-isopropylbenzylcarbamoyl)-pyridin-4-ylethynyl]-phenyl}-piperazine-1-sulfonylamino)-3-methylbutanoic acid (III-195)

[M+H]=618, Retention Time 3.07 min

Synthesis of (R)-3-methyl-2-(4-{4-[2-(4-trifluoromethoxybenzylcarbamoyl)-pyridin-4-ylethynyl]-phenyl}-piperazine-1-sulfonylamino)-butanoic acid (III-196)

[M+H]=660, Retention Time 2.83 min

Synthesis of (R)-3-methyl-2-(4-{4-[2-(3-trifluoromethoxybenzylcarbamoyl)-pyridin-4-ylethynyl]-phenyl}-piperazine-1-sulfonylamino)-butanoic acid (III-197)

[M+H]=660, Retention Time 2.88 min

Synthesis of (R)-2-(4-{4-[2-(3, 4-dimethoxybenzylcarbamoyl)-pyridin-4-ylethynyl]-phenyl}-piperazine-1-sulfonylamino)-3-methylbutanoic acid (III-198)

[M+H]=636, Retention Time 2.18 min

Synthesis of (R)-2-[4-(4-{2-[(benzo [1, 3]dioxol-5-ylmethyl)-carbamoyl]-pyridin-4-ylethynyl}-phenyl)-piperazine-1-sulfonylamino]-3-methylbutanoic acid (III-199)

[M+H]=620, Retention Time 2.23 min

Synthesis of (R)-2-[4-(4-{2-[(2, 3-dihydrobenzofuran-5-ylmethyl)-carbamoyl]-pyridin-4-ylethynyl}-phenyl)-piperazine-1-sulfonylamino]-3-methylbutanoic acid (III-200)

[M+H]=618, Retention Time 2.44 min

Synthesis of (R)-3-methyl-2-(4-{4-[2-(4-sulfamoylbenzylcarbamoyl)-pyridin-4-ylethynyl]-phenyl}-piperazine-1-sulfonylamino)-butanoic acid (III-201)

[M+H]=655, Retention Time 1.91 min

Synthesis of (R)-3-methyl-2-[4-(4-{2-[(5-methylisoxazol-3-ylmethyl)-carbamoyl]-pyridin-4-ylethynyl}-phenyl)-piperazine-1-sulfonylamino]-butanoic acid (III-202)

[M+H]=581, Retention Time 2.05 min

Synthesis of (R)-3-methyl-2-[4-(4-{2-[(4-methylfurazan-3-ylmethyl)-carbamoyl]-pyridin-4-ylethynyl}-phenyl)-piperazine-1-sulfonylamino]-butanoic acid (III-203)

[M+H]=582, Retention Time 2.05 min

Synthesis of (R)-2-(4-{4-[2-(4-fluoro-3-trifluoromethylbenzylcarbamoyl)-pyridin-4-ylethynyl]-phenyl}-piperazine-1-sulfonylamino)-3-methylbutanoic acid (III-204)

[M+H]=662, Retention Time 2.76 min

Synthesis of (R)-2-[4-(4-{2-[(2, 3-dihydrbenzo [1,4]dioxin-6-ylmethyl)-carbamoyl]-pyridin-4-ylethynyl}-phenyl)-piperazine-1-sulfonylamino]-3-methylbutanoic acid (III-205)

[M+H]=634, Retention Time 2.19 min

Synthesis of (R)-2-(4-{4-[2-(4-methanesulfonylbenzylcarbamoyl)-pyridin-4-ylethynyl]-phenyl}-piperazine-1-sulfonylamino)-3-methylbutanoic acid (III-206)

[M+H]=654, Retention Time 1.98 min

Synthesis of (R)-3-methyl-2-(4-{2-[2-(4-morpholin-4-ylbenzylcarbamoyl)-pyridin-4-ylethynyl]-phenyl}-piperazine-1-sulfonylamino)-butanoic acid (III-207)

[M+H]=661, Retention Time 2.13 min

Synthesis of (R)-3-methyl-2-[4-(4-{2-[(5-methy 1[1, 2, 4]oxadiazol-3-ylmethyl)-carbamoyl]-pyridin-4-ylethynyl}-phenyl)-piperazine-1-sulfonylamino]-butanoic acid (III-208)

[M+H]=582, Retention Time 1.95 min

Synthesis of (R)-2-[4-(4-{2-[3-(3-methoxyphenoxy)-benzylcarbamoyl]-pyridin-4-ylethynyl}-phenyl)-piperazine-1-sulfonylamino]-3-methylbutanoic acid (III-209)

[M+H]=698, Retention Time 2.86 min

Synthesis of (R)-2-(4-{4-[2-(3-bromo4-fluorobenzylcarbamoyl)-pyridin-4-ylethynyl]-phenyl}-piperazine-1-sulfonylamino)-3-methylbutanoic acid (III-210)

[M+H]=672, Retention Time 2.79 min

Synthesis of (R)-2-(4-{4-[2-(4-fluoro-3-methoxybenzylcarbamoyl)-pyridin-4-ylethynyl]-phenyl}-piperazine-1-sulfonylamino)-3-methylbutanoic acid (III-211)

[M+H]=624, Retention Time 2.29 min

Synthesis of (R)-2-(4-{4-[2-(4-dimethylaminobenzylcarbamoyl)-pyridin-4-ylethynyl]-phenyl}-piperazine-1-sulfonylamino)-3-methylbutanoic acid (III-212)

[M+H]=619, Retention Time 1.86 min

Synthesis of (R)-2-[4-(4-{2-[(1, 5-dimethyl-1H-pyrazol-3-ylmethyl)-carbamoyl]-pyridin-4-ylethynyl}-phenyl)-piperazine-1-sulfonylamino]-3-methylbutanoic acid (III-213)

[M+H]=594, Retention Time 1.93 min

Synthesis of (R)-3-methyl-2-(4-{4-[2-(4-pyrazol-1-ylbenzylcarbamoyl)-pyridin-4-ylethynyl]-phenyl}-piperazine-1-sulfonylamino)-butanoic acid (III-214)

[M+H]=642, Retention Time 2.19 min

Synthesis of (R)-2-[4-(4-{2-[(2, 5-dimethyloxazol-4-ylmethyl)-carbamoyl]-pyridin-4-ylethynyl}-phenyl)-piperazine-1-sulfonylamino]-3-methylbutanoic acid (III-215)

[M+H]=595, Retention Time 2.01 min

Synthesis of (R)-2-(4-{4-[2-(4-bromobenzylcarbamoyl)-pyridin-4-ylethynyl]-phenyl}-piperazine-1-sulfonylamino)-3-methylbutanoic acid (III-216)

[M+H]=654, Retention Time 2.47 min

Synthesis of (R)-2-{4-[4-(2-carbamoylpyridin-4-ylethynyl)-phenyl]-piperazine-1-sulfonylamino}-3-methylbutanoic acid (III-217)

[M+H]=486, Retention Time 1.72 min

Synthesis of (R)-3-methyl-2-(4-{4-[2-(4-pyrrol-1-ylbenzylcarbamoyl)-pyridin-4-ylethynyl]-phenyl}-piperazine-1-sulfonylamino)-butanoic acid (III-218)

[M+H]=641, Retention Time 2.44 min

Synthesis of (R)-2-[4-(4-{2-[(biphenyl4-ylmethyl)-carbamoyl]-pyridin-4-ylethynyl}-phenyl)-piperazine-1-sulfonylamino]-3-methylbutanoic acid (III-219)

[M+H]=652, Retention Time 2.60 min

Synthesis of (R)-2-(4-{4-[2-(3-hydroxy4-methoxybenzylcarbamoyl)-pyridin-4-ylethynyl]-phenyl}-piperazine-1-sulfonylamino)-3-methylbutanoic acid (III-220)

[M+H]=622, Retention Time 2.01 min

Synthesis of (R)-3-methyl-2-(4-{4-[2-(4-phenoxybenzylcarbamoyl)-pyridin-4-ylethynyl]-phenyl}-piperazine-1-sulfonylamino)-butanoic acid (III-221)

[M+H]=668, Retention Time 2.59 min

Synthesis of (R)-3-methyl-2-(4-{4-[2-(4-trifluoromethylsulfanylbenzylcarbamoyl)-pyridin-4-ylethynyl]-phenyl}-piperazine-1-sulfonylamino)-butanoic acid (III-222)

[M+H]=676, Retention Time 2.61 min

Synthesis of (R)-2-[4-(4-{2-[4-(tert-butoxycarbonylaminomethyl)-benzylcarbamoyl]-pyridin-4-ylethynyl}-phenyl)-piperazine-1-sulfonylamino]-3-methylbutanoic acid (III-223)

[M+H]=705, Retention Time 2.34 min

Synthesis of (R)-2-[4-(4-{2-[3-(tert-butoxycarbonylaminomethyl)-benzylcarbamoyl]-pyridin-4-ylethynyl}-phenyl)-piperazine-1-sulfonylamino]-3-methylbutanoic acid (III-224)

[M+H]=705, Retention Time 2.35 min

Synthesis of (R)-2-(4-{4-[2-(4-fluoro-2-methoxymethylbenzylcarbamoyl)-pyridin-4-ylethynyl]-phenyl}-piperazine-1-sulfonylamino)-3-methylbutanoic acid (III-225)

[M+H]=638, Retention Time 2.36 min

Synthesis of (R)-2-(4-{4-[2-(3-iodobenzylcarbamoyl)-pyridin-4-ylethynyl]-phenyl}-piperazine-1-sulfonylamino)-3-methylbutanoic acid (III-226)

[M+H]=702, Retention Time 2.53 min

Synthesis of (R)-2-(4-{4-[2-(4-fluoro-2-methoxybenzylcarbamoyl)-pyridin-4-ylethynyl]-phenyl}-piperazine-1-sulfonylamino)-3-methylbutanoic acid (III-227)

[M+H]=624, Retention Time 2.39 min

Synthesis of (R)-3-methyl-2-(4-{4-[2-(4-ureidobenzylcarbamoyl)-pyridin-4-ylethynyl]-phenyl}-piperazine-1-sulfonylamino)-butanoic acid (III-228)

[M+H]=634, Retention Time 1.75 min

Synthesis of (R)-2-(4-{4-[2-(4-fluoro-2-methanesulfonylaminobenzylcarbamoyl)-pyridin-4-ylethynyl]-phenyl}-piperazine-1-sulfonylamino)-3-methylbutanoic acid (III-229)

[M+H]=687, Retention Time 2.18 min

Synthesis of (R)-2-(4-{4-[2-(3-fluoro-4-methylbenzylcarbamoyl)-pyridin-4-ylethynyl]-phenyl}-piperazine-1-sulfonylamino)-3-methylbutanoic acid (III-230)

[M+H]=608, Retention Time 2.45 min

Synthesis of (R)-2-{4-[4-(2-{[5-(4-chrolophenyl)-thiophen-2-ylmethyl]-carbamoyl}-pyridin-4-ylethynyl)-phenyl]-piperazine-1-sulfonylamino}-3-methylbutanoic acid (III-231)

[M+H]=692, Retention Time 2.73 min

Synthesis of (R)-2-(4-{4-[2-(2-dimethylcarbamoyl-4-fluorobenzylcarbamoyl)-pyridin-4-ylethynyl]-phenyl}-piperazine-1-sulfonylamino)-3-methylbutanoic acid (III-232)

[M+H]=665, Retention Time 2.05 min

Synthesis of (R)-2-(4-{4-[3-(4-fluorobenzyl)-1-methyl-2, 4-dioxo-1, 2, 3, 4-tetrahydroquinazolin-6-ylethynyl]-phenyl}-piperazine-1-sulfonylamino)-3-methylbutanoic acid (III-233)

Synthesis of (R)-2-(4-{4-[1-ethyl3-(4-fluorobenzyl)-2, 4-dioxo-1, 2, 3, 4-tetrahydroquinazolin-6-ylethynyl]-phenyl}-piperazine-1-sulfonylamino)-3-methylbutanoic acid (III-234)

Synthesis of (R)-2-(4-{4-[3-(4-fluorobenzyl)-2, 4-dioxo-1-propyl-1, 2, 3, 4-tetrahydroquinazolin-6-ylethynyl]-phenyl}-piperazine-1-sulfonylamino)-3-methylbutanoic acid (III-235)

Synthesis of (R)-2-{4-[4-(3-benzyloxy-2, 4-dioxo-1-propyl-1, 2, 3, 4-tetrahydroquinazolin-6-ylethynyl)-phenyl]-piperazine-1-sulfonylamino}-3-methylbutanoic acid (III-236)

Synthesis of (R)-2-(4-{4-[3-(4-fluorobenzyl)-4-oxo-3,-4-dihydroquinazolin-6-ylethynyl]-phenyl}-piperazine-1-sulfonylamino)-3-methylbutanoic acid (III-237)

Synthesis of (R)-3-methyl-2-[4-(4-{2-[(3-methyl-2-oxo-2,-3-dihydrobenzoxazol-5-ylmethyl)-carbamoyl]-pyridin-4-ylethynyl}-phenyl)-piperazine-1-sulfonylamino]-butanoic acid (III-238)

Synthesis of (R)-3-methyl-2-[4-(4-{2-[(3-methyl-2-oxo-2,3-dihydrobenzoxazol-6-ylmethyl)-carbamoyl]-pyridin-4-ylethynyl}-phenyl)-piperazine-1-sulfonylamino]-butanoic acid (III-239)

Synthesis of (R)-2-(4-{4-[2-(3-fluoro-4-isopropylcarbamoylbenzylcarbamoyl)-pyridin-4-ylethynyl]-phenyl}-piperazine-1-sulfonylamino)-3-methylbutanoic acid (III-240)

Synthesis of (R)-2-(4-{4-[2-(4-cyanobenzylcarbamoyl)-pyridin-4-ylethynyl]-phenyl}-piperazine-1-sulfonylamino)-3-methylbutanoic acid (III-241)

Synthesis of (R)-2-(4-{4-[4-methoxy-3-(5-methylcarbamoyloxazol-2-yl)-phenylethynyl]-phenyl}-piperazine-1-sulfonylamino)-3-methylbutanoic acid (III-242)

Example 17

Synthesis of (R)-2-{4-[4-(1-acetylisoquinolin-7-ylethynyl)-phenyl]-piperazine-1-sulfonylamino}-3-methylbutanoic acid (V-1)

[Chemical formula 71]

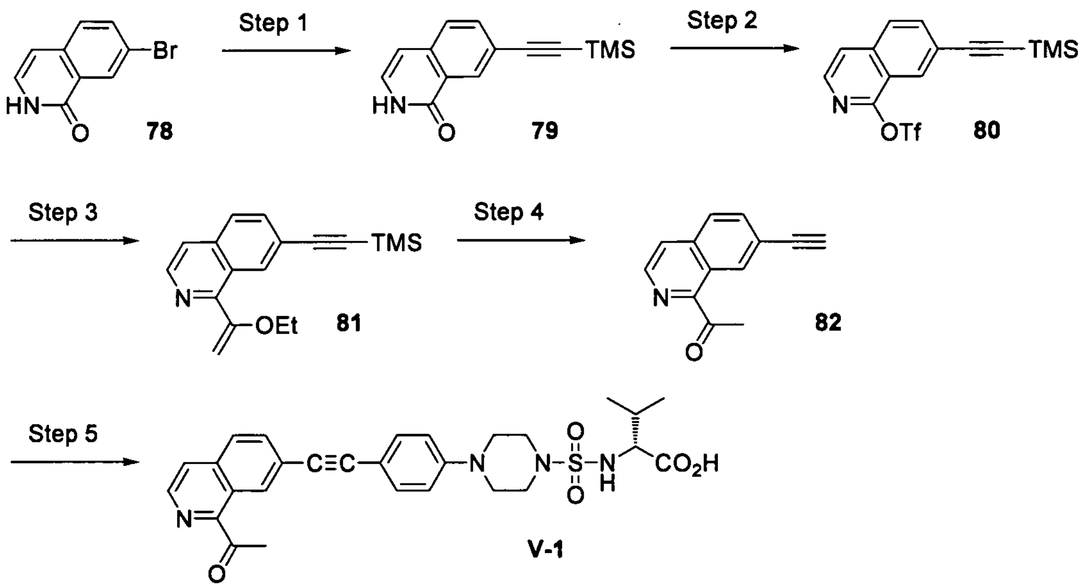


78
79
80
81
82
V-1

(Step 1)

To a solution of the compound (78) (3.00 g, 13.4 mmol) in N,N-dimethylformamide (20 mL) was added trimethylsilylacetylene (2.25 mL, 16.1 mmol), this was degassed under nitrogen atmosphere. Then, bis(triphenylphosphine) palladium (II) chloride (470 mg, 0.669 mmol), copper (I) iodide (255 mg,1.34 mmol), and triethylamine (4.64 mL, 33.5 mmol) were added, this was degassed again under nitrogen atmosphere, and stirred at 70 °C for 7 hours. The reaction mixture was poured into ice water, neutralized with 10% citric acid aq., and extracted with ethyl acetate. The organic layer was washed with saturated sodium bicarbonate aq., saturated sodium chloride aq., dried over anhydrous sodium sulfate, and concentrated under reduced pressure. The residue was subjected to silica gel chromatography, the fractions eluted with n-hexane/ethyl acetate = 3/2 were collected, and concentrated under reduced pressure to give a yellow compound (79) (1.29 g, yield 40%).

(Step 2)

To a suspension of the compound (79) (1.29 g, 5.34 mmol) in methylene chloride (27 mL) were added pyridine (1.30 mL, 16.0 mmol) and trifluoromethanesulfonic anhydride (1.08 mL, 6.41 mmol) under ice cooling, and the mixture was stirred at room temperature for 2 hours. 10% Citric acid aq.was added to the reaction mixture, which was extracted with ethyl acetate. The organic layer was washed with saturated sodium chloride aq., dried over anhydrous sodium sulfate, and concentrated under reduced pressure. The residue was subjected to silica gel chromatography, the fractions eluted with n-hexane/ethyl acetate=9/1 were collected, and concentrated under reduced pressure to give a colorless compound (80) (1.60 g, yield 80%).

(Step 3)

To a solution of the compound (80) (300 mg, 0.803 mmol) in toluene (2.5 mL) were added tetrakis (triphenylphosphine) palladium (0) (46 mg, 0.040 mmol) and tri-n-butyl(1-ethoxyvinyl)-tin (0.407 mL, 1.21 mmol), and the mixture was stirred at 150 °C for 30 minutes under microwave irradiation. The reaction mixture was poured into ice water, neutralized with 10% citric acid aq., and extracted with ethyl acetate. The organic layer was washed with saturated sodium bicarbonate aq. and saturated sodium chloride aq., dried over anhydrous sodium sulfate, and concentrated under reduced pressure. The residue was subjected to silica gel chromatography, the fractions eluted with n-hexane/ethyl acetate =4/1 were collected, and concentrated under reduced pressure to give a yellow compound (81) (94 mg, yield 39%).

(Step 4)

To a solution of the compound (81) (93 mg, 0.32 mmol) in tetrahydrofuran (3 mL) was added 2 mol/L hydrochloric acid (1.2 mL)and the mixture was stirred for 5 hours. Water was added to the reaction mixture, which was extracted with ethyl acetate. The organic layer was washed with saturated sodium chloride aq., dried over anhydrous sodium sulfate, and concentrated under reduced pressure. To a solution of the residue in methylene chloride (4 mL) was added 1mol/L solution of tetrabutylammonium fluoride in tetrahydrofuran (0.35mL) under ice cooling, and the mixture was stirred for 30 minutes. Water was added to the reaction mixture, which was extracted with ethyl acetate. The organic layer was washed with saturated sodium chloride solution, dried over anhydrous sodium sulfate, and concentrated under reduced pressure. The residue was subjected to silica gel chromatography, the fractions eluted with n-hexane/ethyl acetate =6/1 were collected, and evaporated to dryness to give a colorless compound (82) (28 mg, yield 46%).

(Step 5)

(R)-2-{4-[4-(1-acetylisoquinolin-7-ylethynyl)-phenyl]-piperazine-1-sulfonylamino}-3-methylbutanoic acid (V-1) (54 mg, yield 60%) was prepared from the compound (82) (28 mg, 0.14 mmol) and (R)-2-[4-(4-iodophenyl)- piperazine-1-sulfonylamino]-3-methylbutanoic acid according to the procedure for Step 1 in Example 17.

$^1$ H NMR (DMSO-d6) δ: 0.91 (d, J=6.1 Hz, 3H), 0.92 (d, J=6.1 Hz, 3H), 1.98 (m, 1H), 2.78 (s, 3H), 3.08-3.90 (m, 9H), 7.01 (d, J=8.1 Hz, 2H), 7.48 (d, J=8.1 Hz, 2H), 7.81 (m, 1H), 7.85 (d, J=8.6 Hz, 1H), 8.06-8.09 (m, 2H), 8.63 (d, J=5.6 Hz, 1H), 8.90 (s, 1H).

Example 18

Synthesis of (R)-2-[4-(4-{2-[(3-methyl-2-oxo-2,3-dihydrobenzoxazol-6-ylmethyl)-carbamoyl]-pyridin-4-ylethynyl}-phenyl)-piperazine-1-sulfonylamino]-proponic acid (V-2)

[Chemical formula 73]

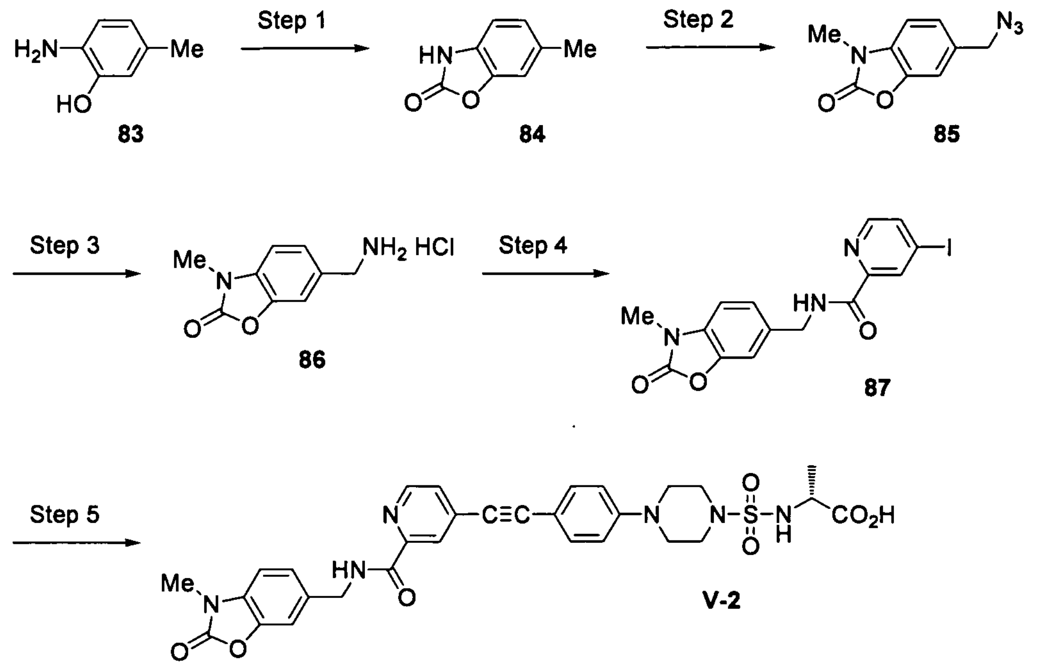


(Step 1)

To a suspension of the compound (83) (5.20 g, 42.2 mmol) in tetrahydrofuran (60 mL) was 1,1'-carbonyldiimidazole(8.20 g, 50.6 mmol), and the mixture was stirred at 75°C for 5 hours. After the reaction mixture was cooled to room temperature, 2mol/L hydrochloric acid was added to the mixture, which was extracted with ethyl acetate. The organic layer was washed with saturated sodium chloride aq., dried over anhydrous sodium sulfate, and concentrated under reduced pressure. The residue was recrystallized from acetone/n-hexane to give a pale brown compound (84) (3.76 g, yield 60%).

(Step 2)

To a solution of the compound (84) (3.76 g, 25.2 mmol) in N,N-dimethylformamide (30 mL) were added potassium carbonate (5.37 g ,37.8 mmol) and methyl iodide (1.83 mL,30.2 mmol) at room temperature, and the mixture was stirred at 50°C for 18 hours. After the reaction mixture was cooled to room temperature, 10% citric acid aq. was added to the mixture , which was extracted with ethyl acetate. The organic layer was washed with saturated sodium chloride aq., dried over anhydrous sodium sulfate, and concentrated under reduced pressure. To a solution of the residue in carbon tetrachloride (100 mL) were added N-bromosuccinimide (5.38 g, 30.2 mmol) and 2,2'-azobis (2-methylpropionitrile) (496 mg, 3.02 mmol) at room temperature, and the mixture was stirred under reflux for 2 hours. After the reaction mixture was cooled to room temperature, insoluble material was filtered off and the filtrate was evaporated to dryness under reduced pressure. To a solution of the residue in N, N-dimethylformamide (40 mL) was added sodium azide (3.76 g, 57.9 mmol) at room temperature, and the mixture was stirred at room temperature for 16 hours. 10% Citric acid aq. was added to the reaction mixture , which was extracted with ethyl acetate. The organic layer was washed with saturated sodium bicarbonate aq., and saturated sodium chloride aq., dried over anhydrous sodium sulfate, and concentrated

under reduced pressure to give the crude compound (85).

(Step 3)

To a solution of the crude compound (85) in tetrahydrofuran (100 mL) was added triphenylphosphine (7.30 g, 27.7 mmol) at room temperature, and the mixture was stirred for 16 hours. Next, water was added to the reaction mixture, which was stirred for 2 hours. After the mixture was concentrated under reduced pressure, 4 mol/L solution of hydrogen chloride in 1,4-dioxane was added to the residue, which was stirred at room temperature for 4 hours. The insoluble matter was filtered off to give the crude compound (86).

(Step 4)

To a solution of 4-iodopyridine-2-carboxylic acid 1/2 hydroiodide (200 mg, 0.639 mmol) in methylene chloride (2 mL), which was prepared according to the method described in the literature (Synthetic Communications, 1996, 26, 2017), were added 1-hydroxybenzotriazole (26 mg, 0.19 mmol) and 1-ethyl-3-(3-dimethylaminopropyl) carbodiimide hydrochloride (184 mg ,0.96 mmol), triethyiamine (0.22 mL, 1.6 mmol) and the crude compound (86) at room temperature, and the mixture was stirred for 24 hours. To the reaction mixture was added 10% citric acid aq., and the mixture was extracted with ethyl acetate. The organic layer was washed with saturated sodium bicarbonate aq., and saturated sodium chloride aq., dried over anhydrous magnesium sulfate, and concentrated under reduced pressure. The residue was subjected to silica gel chromatography, the fractions eluted with chloroform/ethyl acetate = 19/1 were collected, and evaporated to dryness to afford a colorless compound (87) (193 mg, yield 74%).

(Step 5)

(R)- 2-[4-(4- {2-[(3- methyl- 2- oxo- 2,3- dihydrobenzoxazol- 6- ylmethyl) carbamoyl]-pyridin- 4- ylethynyl}-phenyl)-piperazine-1-sulfonylamino]-proponic acid (V-2) (29 mg, yield 10%) was prepared from the compound (87) (193 mg, 0.471 mmol) and (R)-2-[4-(4-iodophenyl)-piperazine-1-sulfonylamino]-proponic acid according to the procedure for Step 1 in Example 17.

$^{1}$H NMR (DMSO-d6) δ: 1.28 (d, J=7.1 Hz, 3H), 3.12-3.78 (m, 12H), 4.51 (d, J=6.1 Hz, 2H), 7.02 (d, J=8.6 Hz, 2H), 7.20 (m, 2H), 7.31 (s, 1H), 7.49 (d, J=8.1 Hz, 2H), 7.66 (dd, J=1.3, 4.6 Hz, 1H), 8.02 (s, 1H), 8.64 (d, J=4.6 Hz, 1H), 9.40 (t, J=6.1 Hz, 1H).

Example 19

Synthesis of (R)-2-[4-(4-{2-[3-(4-methanesulfonylbenzyloxy)-phenyl]-thiazol-2-ylethynyl}-phenyl)-piperazine-1-sulfonylamino]-3-methylbutanoic acid (V-3)

[Chemical formula 74]

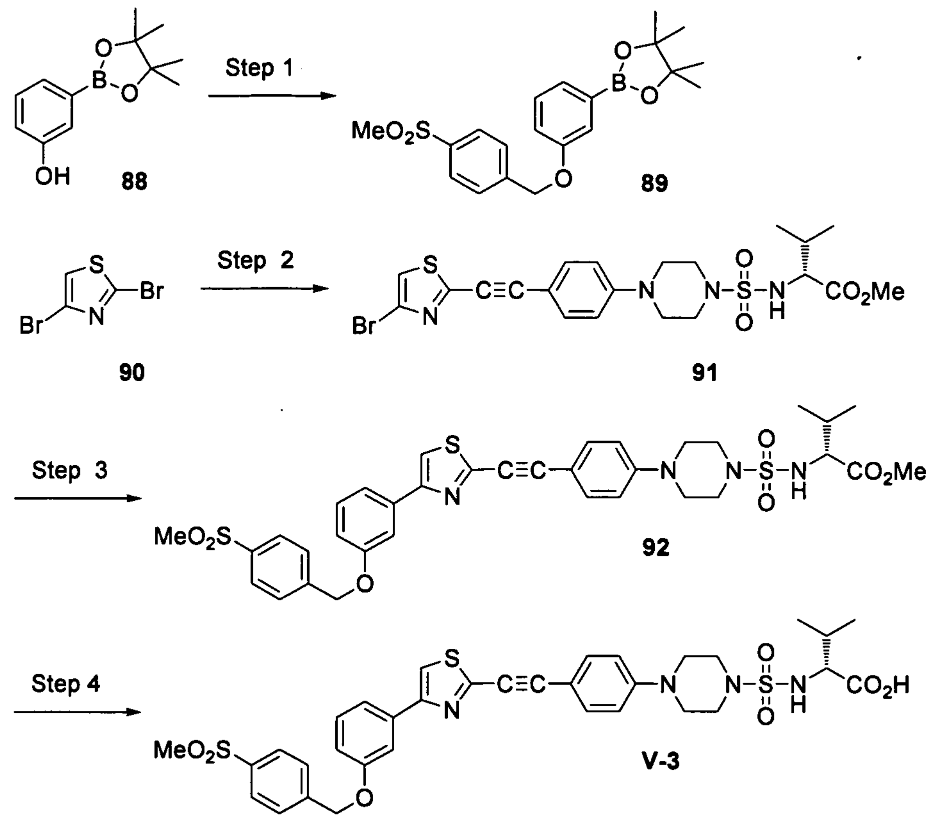


88 89 90 91 92 V-3

(Step 1)

To a suspension of the compound (88) (400 mg, 1.82 mmol) in N, N-dimethylformamide (8 mL) were added 1-(bromomethyl)-4-methylsulfonylbenzene (543 mg, 2.18 mmol) and potassium carbonate (327 mg, 2.36 mmol), and the mixture was stirred at 50°C for 6hours. After the reaction mixture was cooled to room temperature, 1 mol/L sodium hydroxide aq. was added to the mixture. which was extracted with ethyl acetate. The organic layer was washed with saturated sodium chloride aq., dried over anhydrous sodium sulfate, and concentrated under reduced pressure. The residue was subjected to silica gel chromatography, the fractions eluted with n-hexane/ethyl acetate = 8/1 were collected, and evaporated to dryness to afford a colorless compound (89) (474 mg, yield 67%)

(Step 2)

The pale yellow compound (91) (3.04 g, yield 85%) was prepared from the compound (90) and (R)-2-[4-(4-iodophenyl)-piperazine-1-sulfonylamino]-3-methylbutanoic acid methyl ester (1.76 g, 7.25 mmol) according to the procedure for Step 1 in Example 17.

(Step 3)

To a solution of the compound (91) (150 mg, 0.277 mmol) in 1,4-dioxane (2 mL) was added the compound (89) (140 mg, 0.360 mmol), tetrakis(triphenylphosphine)palladium (0) (16 mg, 0.014 mmol) and 2 mol/L potassium carbonate aq. (0.42 mL), and the mixture was stirred at 100°C for 15 minutes under microwave irradiation. After the reaction mixture was cooled to room temperature, 10% citric acid aq. was added to the mixture, which was extracted with ethyl acetate. The organic layer was washed with saturated sodium bicarbonate aq., saturated sodium chloride aq., dried over anhydrous sodium sulfate, and concentrated under reduced pressure. The residue was subjected to silica gel chromatography, the fractions eluted with n-hexane/ethyl acetate = 1/1 were collected, and concentrated under reduced pressure to give a yellow compound (92) (198 mg, yield 99%).

(Step 4)

To a solution of the compound (92) (183 mg, 0.253 mmol) in dimethyl sulfoxide (4 mL) was added 2 mol/L sodium hydroxide aq. (0.51 mL) at room temperature, and this was stirred for 20 hours. The reaction mixture was poured into ice water-5% citric acid aq. and extracted with ethyl acetate. The organic layer was washed with water, dried over anhydrous sodium sulfate, and concentrated under reduced pressure. The residue was recrystallized from n-hexane/ethyl acetate to afford (R)-2-[4-(4-{2-[3-(4-methanesulfonylbenzyloxy)-phenyl]-thiazol-2-ylethynyl}-phenyl)-piperazine-1-sulfonylamino]-3-methylbutanoic acid (V-3) (101 mg, yield 56%).

$^1$H NMR (DMSO-d6) δ: 0.90 (d, J=6.1 Hz, 3H), 0.93 (d, J=6.1 Hz, 3H), 2.00 (m, 1H), 3.20-3.48 (m, 11H), 3.50 (d, J=5.1 Hz, 1H), 5.34 (s, 2H), 7.01-7.07 (m, 3H), 7.40 (t, J=7.6 Hz, 1H), 7.52 (d, J=8.1 Hz, 2H), 7.61 (d, J=7.1 Hz, 1H), 7.68 (s, 1H), 7.77 (d, J=7.6 Hz, 2H), 7.89 (d, J=7.6 Hz, 2H), 8.26 (s, 1H).

Synthesis of (R)-2-[4-(4-{4-isobutoxy-3-[(thiophen-2-ylmethyl)carbamoyl]-phenylethynyl}-phenyl)-piperazine-1-sulfonylamino]-3-methylbutanoic acid (V-4)

$^1$H NMR (DMSO-d6) δ: 0.80-0.93 (m, 12H), 2.01 (br s, 2H), 3.15 (br s, 2H), 3.24 (br s, 4H), 3.87 (br s, 2H), 4.66 (br s, 2H), 6.97-7.15 (m, 5H), 7.39-7.57 (m, 4H), 7.75 (br s, 1H), 7.95 (br s, 1H), 8.60 (br s, 1H).

Synthesis of (R)-2-[4-(4-{4-isobutoxy3-[(pyridin-4-ylmethyl)carbamoyl]-phenylethynyl}-phenyl)-piperazine-1-sulfonylamino]-3-methylbutanoic acid (V-5)

$^1$H NMR (DMSO-d6) δ: 0.80-0.93 (m, 12H), 1.99-2.09 (m, 2H), 3.14 (br s, 2H), 3.23 (br s, 4H), 3.89 (d, J=6.4 Hz, 2H), 4.51 (d, J=5.6 Hz, 2H), 6.96 (d, J=8.8 Hz, 2H), 7.16 (d, J=8.8 Hz, 1H), 7.30-7.39 (m, 5H), 7.57 (dd, J=2.4, 8.8 Hz, 1H), 7.71 (d, J=2.4 Hz, 1H), 8.51 (br s, 2H), 8.69 (t, J=6.4 Hz, 1H).

Synthesis of (R)-2-(4-{4-[4-isobutoxy-3-(4-methanesulfonylbenzylcarbamoyl)-phenylethynyl]-phenyl}-piperazine-1-sulfonylamino)-3-methylbutanoic acid (V-6)

$^1$H NMR (DMSO-d6) δ: 0.80-0.92 (m, 12H), 1.99-2.09 (m, 2H), 3.14 (br s, 2H), 3.23 (br s, 7H), 3.88 (d, J=6.4 Hz, 2H), 4.59 (d, J=5.6 Hz, 2H), 6.96 (d, J=8.8 Hz, 2H), 7.15 (d, J=8.8 Hz, 1H), 7.38 (d, J=8.8 Hz, 2H), 7.54-7.62 (m, 3H), 7.72 (d, J=2.4 Hz, 1H), 7.89 (d, J=8.8 Hz, 2H), 8.71 (t, J=6.4 Hz, 1H).

Synthesis of 2-(4-{4-[2-(4-methoxybenzylcarbamoyl)-pyridin-4-ylethynyl]-phenyl}-piperazine-1-sulfonylamino)-2-methylproponic acid (V-7)

$^1$H NMR (DMSO-d6) δ: 1.39 (s, 6H), 3.16 (br s, 4H), 3.71 (s, 3H), 4.43 (d, J=6.4 Hz, 2H), 6.87 (d, J=8.8 Hz, 2H), 7.02 (d, J=8.8 Hz, 1H), 7.27 (d, J=8.8 Hz, 2H), 7.46-7.51 (m, 3H), 7.65 (d, J=2.4 Hz, 1H), 8.02 (s, 1H), 8.63 (d, J=2.4 Hz, 1H), 9.27 (t, J=6.4 Hz, 1H).

Synthesis of (R)-2-(4-{4-[2-(4-isobutyloxazol-2-yl)-pyridin-4-ylethynyl]-phenyl}-piperazine-1-sulfonylamino)-3-methylbutanoic acid (V-8)

$^1$H NMR (DMSO-d6) δ: 0.80-0.94 (m, 12H), 1.94-2.11 (m, 2H), 2.42 (d, J=6.8 Hz, 2H), 3.16 (m, 4H), 3.28 (m, 4H), 6.99 (d, J=8.8 Hz, 2H), 7.48-7.54 (m, 3H), 8.03 (d, J=2.4 Hz, 2H), 8.66 (d, J=2.4 Hz, 1H).

Synthesis of (R)-2-{4-[4-(4-methoxy-3-oxazol-2-ylphenylethynyl)-phenyl]-piperazine-1-sulfonylamino}-proponic acid (V-9)

$^1$H NMR (DMSO-d6) δ: 1.24 (d, J=6.9 Hz, 3H), 3.10-3.60 (m, 9H), 3.91(s, 3H), 6.98 (d, J=9.0 Hz, 2H), 7.25 (d, J=9.0 Hz, 1H), 7.39 (s, 1H), 7.41 (d, J=9.0 Hz, 2H), 7.63 (dd, J=2.4, 9.0 Hz, 1H), 7.93(d, J=2.4 Hz, 1H), 8.23 (s, 1H).

Synthesis of (R)-2-(4-{4-[4-methoxy-3-(5-methylcarbamoylmethyloxazol-2-yl)-phenylethynyl]-phenyl}-piperazine-1-sulfonylamino)-proponic acid (V-10)

$^1$H NMR (DMSO-d6) δ: 1.23 (d, J=6.6 Hz, 3H), 2.61 (d, J=4.5 Hz, 3H), 3.11-3.49 (m, 9H), 3.64 (s, 2H), 3.89 (s, 3H), 6.98 (d, J=8.7 Hz, 2H), 7.09 (s, 1H), 7.22 (d, J=9.0 Hz, 1H), 7.41 (d, J=8.7 Hz, 2H), 7.61 (dd, J=2.1, 9.0Hz, 1H), 7.86 (d, J=2.1 Hz, 1H), 8.04 (br s, 1H).

Synthesis of (R)-2-(4-{4-[4-(3-benzyloxyphenyl)-thiazol-2-ylethynyl]-phenyl}-piperazine-1-sulfonylamino)-proponic acid (V-11)

$^1$ H NMR (DMSO-d6) δ: 1.30 (d, J=7.2 Hz, 3H), 3.08-3.45 (m, 8H), 3.78-3.83 (m, 1H), 5.28 (s, 2H), 7.03 (d, J=8.7 Hz, 2H), 7.31-7.58 (m, 9H), 7.63 (s, 1H), 7.92 (d, J=8.4 Hz, 1H), 8.26 (s, 1H).

Synthesis of (R)-2-[4-(4-{3-[5-(benzylcarbamoylmethyl)-oxazol-2-yl]-4-methoxyphenylethynyl}-phenyl)-piperazine-1-sulfonylamino]-proponic acid (V-12)

$^1$ H NMR (DMSO-d6) δ: 1.24 (d, J=7.2 Hz, 3H), 3.11-3.60 (m, 9H), 3.73 (s, 2H), 3.88 (s, 3H), 4.31 (d, J=6.0Hz, 2H), 6.97 (d, J=9.0 Hz, 2H), 7.11 (s, 1H), 7.21-7.34 (m, H), 7.39 (d, J=9.0 Hz, 2H), 7.61 (dd, J=2.4, 9.0 Hz, 1H), 7.86(d, J=2.4 Hz, 1H), 8.66 (t, J=6.0 Hz, 1H).

Synthesis of (R)-2-(4-{4-[4-(3-acetylaminophenyl)-thiazol-2-ylethynyl]-phenyl}-piperazine-1-sulfonylamino)-proponic acid (V-13)

$^1$ H NMR (DMSO-d6) δ: 1.30 (d, J=7.2 Hz, 3H), 2.07 (s, 3H), 3.08-3.40 (m, 8H), 3.77-3.85 (m, 1H), 7.04 (d, J=8.7 Hz, 2H), 7.37 (t, J=8.4 Hz, 1H), 7.52 (d, J=8.7 Hz, 2H), 7.60 (d, J=8.4 Hz, 1H), 7.92 (d, 8.4 Hz, 1H), 8.13 (s, 1H), 8.22 (s, 1H), 10.05 (s, 1H).

(R)-2-(4-{4-[4-(3-benzylcarbamoylphenyl)-thiazol-2-ylethynyl]-phenyl}-piperazine-1-sulfonylamino)-proponic acid (V-14)

$^1$ H NMR (DMSO-d6) δ: 1.30 (d, J=7.2 Hz, 3H), 3.10-3.40 (m, 8H), 3.79-3.81 (m, 1H), 4.52(d, J=5.7 Hz, 2H), 7.04 (d, J=9.0 Hz, 2H), 7.20-7.28 (m, 1H), 7.31-7.36 (m, 4H), 7.54(d, J=9.0 Hz, 2H), 7.69 (d, J=7.8 Hz, 1H), 7.84 (br s, 1H), 7.90 (d, J=8.1 Hz, 1H), 8.14 (8.1 Hz, 1H), 8.51 (s, 1H), 9.19 (t, J=5.7 Hz, 1H).

Synthesis of (R)-2-(4-{4-[4-(5-fluoro-2-methoxyphenyl)-thiazol-2-ylethynyl]-phenyl}-piperazine-1-sulfonylamino)-proponic acid (V-15)

$^1$ H NMR (DMSO-d6) δ: 1.30 (d, J=7.2 Hz, 3H), 3.07-3.40 (m, 8H), 3.76-3.90 (m, 1H), 3.94 (s, 3H), 7.04 (d, J=9.0 Hz, 2H), 7.19-7.23 (m, 2H), 7.53 (d, J=9.0 Hz, 2H), 7.85-7.90 (m, 1H), 7.91 (br s, 1H), 8.28 (s, 1H).

Synthesis of (R)-2-[4-(4-{2-[5-(benzylcarbamoylmethyl)-oxazol-2-yl]-pyridin-4-ylethynyl}-phenyl)-piperazine-1-sulfonylamino]-proponic acid (V-16)

$^1$ H NMR (DMSO-d6) δ: 1.27 (d, J=6.0 Hz, 3H), 3.05-3.40 (m, 9H), 3.80 (s, 2H), 4.33 (d, J=5.7 Hz, 2H), 7.03 (d, J=6.0 Hz, 2H), 7.24-7.27 (m, 7H), 7.51 (d, J=6.0 Hz, 2H), 7.55-7.59 (m, 1H), 8.05 (s, 1H), 8.69 (t, J=5.7 Hz, 1H).

Synthesis of (R)-2-{4-[4-(2-{5-[(cyanomethylcarbamoyl)-methyl]-oxazol-2-yl}-pyridin-4-ylethynyl)-phenyl]-piperazine-1-sulfonylamino}-proponic acid (V-17)

$^1$ H NMR (DMSO-d6) δ: 1.23 (d, J=6.0 Hz, 3H), 3.15-3.50 (m, 9H), 3.83 (s, 2H), 4.19 (d, J=6.0 Hz, 2H), 7.02 (d, J=6.0 Hz, 2H), 7.25 (s, 1H), 7.50 (d, J=6.0 Hz, 2H), 7.55-7.58 (m, 1H), 8.05 (s, 1H), 8.65-8.70 (m, 1H), 8.93 (m, 1H).

Synthesis of (R)-2-[4-(4-{2-[5-(2-oxo-2-piperidin-1-ylethyl)-oxazol-2-yl]-pyridin-4-ylethynyl}-phenyl)-piperazine-1-sulfonylamino]-proponic acid (V-18)

$^1$ H NMR (DMSO-d6) δ: 1.28 (d, J=7.2 Hz, 3H), 1.45-1.59 (m, 10H), 3.20-3.52 (m, 8H), 3.73-3.80 (m, 1H), 4.00 (s, 2H), 7.03 (d, J=8.7 Hz, 2H), 7.22 (s, 1H), 7.51 (d, J=8.7 Hz, 2H), 7.56 (d, J=3.0 Hz, 1H), 8.04 (s, 1H), 8.67 (d, J=3.0 Hz, 1H).

Synthesis of (R)-2-(4-{4-[3-[5-(benzylcarbamoylmethyl)-oxazol-2-yl]-4-(2-methoxyethoxy)-phenylethynyl]-phenyl}-piperazine-1-sulfonylamino)-proponic acid (V-19)

$^1$ H NMR (DMSO-d6) δ: 1.25 (d, J=7.2 Hz, 3H), 3.11-3.75 (m, 9H), 3.31 (s, 3H), 3.69 (s, 2H), 3.72 (s, 2H), 4.22 (m, 2H), 4.31 (m, 2H), 6.95-7.01 (m, 2H), 7.12 (s, 1H), 7.22-7.45 (m, 8H), 7.57 (s, 1H), 7.89 (s, 1H), 8.65 (br s, 1H).

Synthesis of (R)-2-[4-(4-{4-[3-(4-methanesulfonylbenzyloxy)-phenyl]-thiazol-2-ylethynyl}-phenyl)-piperazine-1-sulfonylamino]-proponic acid (V-20)

$^{1}$H NMR (DMSO-d6) δ: 1.30 (d, J=6.0 Hz, 3H), 3.05-3.50 (m, 9H), 3.22 (s, 3H), 3.75-3.80 (m, 1H), 5.34 (s, 2H), 7.04 (d, J=6.0 Hz, 2H), 7.41 (t, J=6.0 Hz, 1H), 7.52-7.67 (m, 6H), 7.92 (m, 1H), 7.97 (d, J=6.0 Hz, 2H), 8.27 (s, 1H).

Synthesis of 4-[3-(2-{4-[4-((R)-1-carboxyethylsulfamoyl)-piperazin-1-yl]-phenylethynyl}-thiazol-4-yl)-phenoxymethyl]-benzoic acid (V-21)

$^{1}$H NMR (DMSO-d6) δ: 1.21 (d, J=5.7 Hz, 3H), 3.05-3.50 (m, 9H), 5.20 (s, 2H),6.98-7.04 (m, 3H), 7.38 (t, J=6.0 Hz, 1H), 7.44 (d, J=6.0 Hz, 2H), 7.50 (d, J=6.0 Hz, 2H), 7.56 (d, J=6.0 Hz, 1H), 7.64 (s, 1H), 7.91 (d, J=6.0 Hz, 2H), 8.24 (s, 1H).

4-[3-(2-{4-[4-((R)-1-carboxyethylsulfamoyl)-piperazin-1-yl]-phenylethynyl}-thiazol-4-yl)-phenoxymethyl]-benzoic acid methyl ester (V-22)

$^{1}$H NMR (DMSO-d6) δ: 1.24 (d, J=6.0 Hz, 3H), 3.10-3.57 (m, 9H), 3.86 (s, 3H), 5.30 (s, 2H),6.98-7.04 (m, 3H), 7.38 (t, J=6.0 Hz, 1H), 7.44 (d, J=6.0 Hz, 2H), 7.50 (d, J=6.0 Hz, 2H), 7.56 (d, J=6.0 Hz, 1H), 7.64 (s, 1H), 7.91 (d, J=6.0 Hz, 2H), 8.24 (s, 1H).

Synthesis of (R)-2-(4-{4-[3-(5-ethyloxazol-2-yl)-4-(2-methoxyethoxy)-phenylethynyl]-phenyl}-piperazine-1-sulfonylamino)-proponic acid (V-23)

$^{1}$H NMR (DMSO-d6) δ: 1.24 (t, J=7.5Hz, 3H), 1.28 (d, J=9.0Hz, 3H), 2.73 (q, J=7.5Hz, 2H), 3.09-3.60 (m, 9H), 3.33 (s, 3H), 3.70-3.73 (m, 2H), 4.21-4.25 (m, 2H), 6.98 (d, J=9.0 Hz, 2H), 7.21 (d, J=8.7 Hz, 1H), 7.40 (d, J=9.0 Hz, 2H), 7.57 (dd, J=1.8, 8.7 Hz, 1H), 7.90 (d, J=1.8 Hz, 1H).

Synthesis of (R)-2-(4-{4-[3-[5-(benzylcarbamoylmethyl)-oxazol-2-yl]-4-(2-methoxyethoxy)-phenylethynyl]-phenyl}-piperazine-1-sulfonylamino)-3-methylbutanoic acid (V-24)

$^{1}$H NMR (DMSO-d6) δ: 0.85-0.91 (m, 6H), 3.14-3.61 (m, 8H), 3.30 (s, 3H), 3.67-3.70 (m, 2H), 3.70 (s, 2H), 4.20-4.23 (m, 2H), 4.31 (d, J=6.0 Hz, 2H), 6.98 (d, J=8.7 Hz, 2H), 7.01 (s, 1H), 7.21-7.34 (m, 6H), 7.39 (d, J=8.7 Hz, 2H), 7.57 (d, J=8.4 Hz, 1H), 7.88 (s, 1H), 8.63 (t, J=6.0 Hz, 1H).

Synthesis of (R)-2-(4-{4-[4-ethoxy-3-(5-ethyloxazol-2-yl)-phenylethynyl]-phenyl}-piperazine-1-sulfonylamino)-proponic acid (V-25)

$^{1}$H NMR (DMSO-d6) δ: 1.18-1.26 (m, 6H), 1.36 (t, J=6.9 Hz, 3H), 2.72 (q, J=7.5 Hz, 2H), 3.12-3.37 (m, 8H), 3.37-3.63 (m, 1H), 4.15 (q, J=6.9 Hz, 2H), 6.97 (d, J=8.7 Hz, 2H), 6.99 (s, 1H), 7.19 (d, J=8.7 Hz, 1H), 7.39 (d, J=8.7 Hz, 2H), 7.56 (dd, J=2.1, 8.7 Hz, 1H), 7.88 (d, J=2.1 Hz, 1H).

Synthesis of (R)-2-(4-{4-[4-ethoxy-3-(5-ethyloxazol-2-yl)-phenylethynyl]-phenyl}-piperazine-1-sulfonylamino)-3-methylbutanoic acid (V-26)

$^{1}$H NMR (DMSO-d6) δ: 0.86-0.92 (m, 3H), 1.22-1.24 (m, 6H), 1.37 (t, J=6.0 Hz, 3H), 1.95-2.01 (m, 1H), 2.73 (q, J=6.0 Hz, 2H), 3.14-3.75 (m, 9H), 4.17 (q, J=6.0 Hz, 2H), 6.97 (d, J=8.7 Hz, 2H), 7.00 (s, 1H), 7.19 (d, J=8.7 Hz, 1H), 7.40 (d, J=8.7 Hz, 2H), 7.57 (dd, J=2.1, 8.7 Hz, 1H), 7.88 (d, J=2.1 Hz, 1H).

Synthesis of (R)-2-(4-{4-[4-(2-methoxyethoxy)-3-(5-methylcarbamoylmethyloxazol-2-yl)-phenylethynyl]-phenyl}-piperazine-1-sulfonylamino)-proponic acid (V-27)

$^{1}$H NMR (DMSO-d6) δ: 1.25 (d, J=6.0 Hz, 3H), 2.62 (d, J=3.0 Hz, 3H), 3.15-3,63 (m, 9H), 3.28 (s, 3H), 3.63 (s, 2H), 3.70-3.72 (m, 2H), 4.21-4.23 (m, 2H), 6.98 (d, J=6.0 Hz, 2H), 7.10 (s, 1H), 7.23 (d, J=9.0 Hz, 1H), 7.40 (d, J=6.0 Hz, 2H), 7.75 (d, J=9.0 Hz, 1H), 7.89 (s, 1H), 7.99-8.01 (m, 1H).

Synthesis of (R)-2-(4-{4-[4-(2-methoxyethoxy)-3-(5-methylcarbamoylmethyloxazol-2-yl)-phenylethynyl]-phenyl}-piperazine-1-sulfonylamino)-3-methylbutanoic acid (V-28)

$^1$H NMR (DMSO-d6) δ: 0.84 (d, J=6.0Hz, 3H), 0.90 (d, J=6.0Hz, 3H), 1.96-2.00 (m, 1H), 2.62 (d, J=6.0Hz, 3H), 3.00-3.50 (m, 8H), 3.25 (s, 3H), 3.63 (s, 2H), 3.70-3.72 (m, 2H), 4.21-4.22 (m, 2H), 6.97 (d, J=8.7Hz, 2H), 7.10 (s, 1H), 7.22 (d, J=6.0Hz, 1H), 7.40 (d, J=8.7Hz, 2H), 7.57 (d, J=6.0Hz, 1H), 7.89 (s, 1H), 7.97-8.05 (m, 1H).

Synthesis of (R)-2-{4-[4-(2-{5-[(cyanomethylcarbamoyl)-methyl]-oxazol-2-yl}-pyridin-4-ylethynyl)-phenyl]-piperazine-1-sulfonylamino}-3-methylbutanoic acid (V-29)

$^1$H NMR (DMSO-d6) δ: 0.83 (d, J=6.0 Hz, 3H), 0.89 (d, J=6.0 Hz, 3H), 3.11-3.46 (m, 9H), 3.83 (s, 2H), 4.19 (d, J=6.0 Hz, 2H), 7.01 (d, J=6.0 Hz, 2H), 7.25 (s, 1H), 7.50 (d, J=6.0 Hz, 2H), 7.56 (d, J=3.0 Hz, 1H), 8.05 (s, 1H), 8.66 (d, J=3.0 Hz, 1H), 8.85-9.01 (m, 1H).

Synthesis of (R)-2-[4-(4-{2-[4-(cyanomethylcarbamoyl)-benzylcarbamoyl]-pyridin-4-ylethynyl}-phenyl)-piperazine-1-sulfonylamino]-3-methylbutanoic acid (V-30)

$^1$H NMR (DMSO-d6) δ: 0.90 (d, J=6.6 Hz, 3H), 0.92 (d, J=6.6 Hz, 3H), 1.98 (m, 1H), 3.12-3.50 (m, 9H), 4.29 (d, J=5.3 Hz, 2H), 4.56 (d, J=6.6 Hz, 2H), 7.02 (d, J=9.1 Hz, 2H), 7.44 (d, J=8.1 Hz, 2H), 7.49 (d, J=8.6 H, 2H), 7.67 (d, J=5.6 Hz, 1H), 7.80-7.82 (m, 3H), 8.03 (s, 1H), 8.66 (d, J=4.8 Hz, 1H), 9.15 (t, J=5.1 Hz, 1H), 9.48 (t, J=6.6 Hz, 1H).

Synthesis of (R)-2-(4-{4-[2-(4-cyanobenzylcarbamoyl)-pyridin-4-ylethynyl]-phenyl}-piperazine-1-sulfonylamino)-3-methylbutanoic acid (V-31)

$^1$H NMR (DMSO-d6) δ: 0.90 (d, J=5.8 Hz, 3H), 0.92 (d, J=6.1 Hz, 3H), 1.98 (m, 1H), 3.14-3.56 (m, 9H), 4.58 (d, J=5.8 Hz, 2H), 7.01 (d, J=8.3 Hz, 2H), 7.48-7.51 (m, 4H), 7.66 (d, J=4.8 Hz, 1H), 7.76-7.82 (m, 3H), 8.02 (s, 1H), 8.66 (d, J=4.6 Hz, 1H), 9.15 (t, J=5.1 Hz, 1H), 9.54 (t, J=5.9 Hz, 1H).

Synthesis of (R)-2-(4-{4-[2-(3-cyanobenzylcarbamoyl)-pyridin-4-ylethynyl]-phenyl}-piperazine-1-sulfonylamino)-3-methylbutanoic acid (V-32)

$^1$H NMR (DMSO-d6) δ: 0.90 (d, J=6.3 Hz, 3H), 0.92 (d, J=6.3 Hz, 3H), 1.96 (m, 1H), 3.11-3.31 (m, 8H), 3.52 (m, 1H), 4.55 (d, J=6.1 Hz, 2H), 7.01 (d, J=8.8 Hz, 2H), 7.48 (d, J=8.3 Hz, 2H), 7.53 (t, J=7.8 Hz, 1H), 7.65-7.71 (m, 3H), 7.75 (s, 1H), 7.81 (d, J=5.3 Hz, 1H), 8.02 (s, 1H), 8.65 (d, J=5.1 Hz, 1H), 9.52 (t, J=6.1 Hz, 1H).

Synthesis of (S)-2-(4-{4-[2-(4-methoxybenzylcarbamoyl)-pyridin-4-ylethynyl]-phenyl}-piperazine-1-sulfonylamino)-pentanoic acid (V-33)

$^1$H NMR (DMSO-d6) δ: 0.87 (t, J=7.1 Hz, 3H), 1.37 (m, 2H), 1.58 (m, 2H), 3.13-3.53 (m, 9H), 3.71 (s, 3H), 4.42 (d, J=6.6 Hz, 2H), 6.87 (d, J=8.3 Hz, 2H), 7.01 (d, J=8.6 Hz, 2H), 7.26 (d, J=8.8 Hz, 2H), 7.49 (d, J=8.3 Hz, 2H), 7.64 (d, J=4.8 Hz, 1H), 7.86 (br s, 1H), 8.01 (s, 1H), 8.63 (d, J=4.8 Hz, 1H), 9.27 (t, J=6.2 Hz, 1H).

Synthesis of (S)-2-(4-{4-[2-(4-methoxybenzylcarbamoyl)-pyridin-4-ylethynyl]-phenyl}-piperazine-1-sulfonylamino)-3-methylbutanoic acid (V-34)

$^1$H NMR (DMSO-d6) δ: 0.91 (br s, 6H), 1.97 (br s, 1H), 3.14-3.71 (m, 12H), 4.21 (br s, 2H), 6.87 (d, J=6.6 Hz, 2H), 7.00 (d, J=6.8 Hz, 2H),7.26 (d, J=7.1 Hz, 2H), 7.48 (d, J=7.3 Hz, 2H), 7.63 (s, 1H), 7.81 (d, J=8.3 Hz, 1H), 8.01 (s, 1H), 8.62 (s, 1H), 9.26 (br s, 1H).

Synthesis of (R)-2-(4-{4-[2-(4-methoxybenzylcarbamoyl)-pyridin-4-ylethynyl]-phenyl}-piperazine-1-sulfonylamino)-proponic acid (V-35)

$^1$H NMR (DMSO-d6) δ: 1.29 (d, J=7.3 Hz, 3H), 3.15-3.31 (m, 8H), 3.71 (s, 3H), 3.70 (m, 1H), 4.43 (d, J=5.8 Hz, 2H), 6.87 (d, J=8.3 Hz, 2H), 7.01 (d, J=8.3 Hz, 2H), 7.26 (d, J=7.8 Hz, 2H), 7.49 (d, J=8.3 Hz, 2H), 7.64 (d, J=4.8 Hz, 1H), 7.88 (br s, 1H), 8.01 (s, 1H), 8.63 (d, J=4.3 Hz, 1H), 9.27 (t, J=5.9 Hz, 1H).

Synthesis of (R)-2-(4-{4-[2-(4-methoxybenzyl)-1-oxo-1, 2-dihydroisoquinolin-7-ylethynyl]-phenyl}-piperazine-1-sulfonylamino)-proponic acid (V-36)

$^1H$ NMR (DMSO-d6) δ: 1.30 (d, J=7.3 Hz, 3H), 3.14-3.29 (m, 8H), 3.71 (s, 3H), 3.80 (m, 1H), 5.11 (s, 2H), 6.66 (d, J=7.1 Hz, 1H), 6.89 (d, J=7.8 Hz, 2H), 7.00 (d, J=8.6 Hz, 2H), 7.30 (d, J=7.8 Hz, 2H), 7.45 (d, J=8.6 Hz, 2H), 7.58 (d, J=6.8 Hz, 1H), 7.67 (d, J=8.3 Hz, 1H), 7.76 (d, J=8.1 Hz, 1H), 7.89 (br s, 1H), 8.27 (s, 1H).

Synthesis of (S)-2-(4-{4-[2-(4-methoxybenzyl)-1-oxo-1, 2-dihydroisoquinolin-7-ylethynyl]-phenyl}-piperazine-1-sulfonylamino)-3-methylbutanoic acid (V-37)

$^1H$ NMR (DMSO-d6) δ: 0.89 (d, J=6.8 Hz, 3H), 0.91 (d, J=6.8 Hz, 3H), 1.96 (m, 1H), 3.14-3.27 (m, 8H), 3.49 (m, 1H), 3.71 (s, 3H), 5.11 (s, 2H), 6.67 (d, J=7.6 Hz, 1H), 6.89 (d, J=8.6 Hz, 2H), 6.99 (d, J=8.3 Hz, 2H), 7.30 (d, J=8.3 Hz, 2H), 7.44 (d, J=9.1 Hz, 2H), 7.57 (d, J=7.1 Hz, 1H), 7.67 (d, J=9.1 Hz, 1H), 7.76 (d, J=8.3 Hz, 1H), 8.27 (s, 1H).

Synthesis of (R)-2-(4-{4-[2-(5-ethyloxazol-2-yl)-pyridin-4-ylethynyl]-phenyl}-piperazine-1-sulfonylamino)-3-methylbutanoic acid (V-38)

$^1H$ NMR (DMSO-d6) δ: 0.90 (d, J=6.1 Hz, 3H), 0.91 (d, J=6.6 Hz, 3H), 1.24 (t, J=7.5 Hz, 3H), 1.96 (m, 1H), 2.77 (q, J=7.4 Hz, 2H), 3.10-3.19 (m, 4H), 3.30-3.59 (m, 5H), 7.01 (d, J=8.6 Hz, 2H), 7.11 (s, 1H), 7.49 (d, J=8.3 Hz, 2H), 7.53 (d, J=4.3 Hz, 1H), 7.80 (d, J=10.1 Hz, 1H), 8.03 (s, 1H), 8.65 (br s, 1H).

Synthesis of (R)-2-(4-{4-[2-(5-ethyloxazol-2-yl)-pyridin-4-ylethynyl]-phenyl}-piperazine-1-sulfonylamino)-proponic acid (V-39)

$^1H$ NMR (DMSO-d6) δ: 1.24 (t, J=7.6 Hz, 3H), 1.29 (d, J=7.1 Hz, 3H), 2.77 (q, J=7.6 Hz, 2H), 3.12-3.18 (m, 4H), 3.31-3.33 (m, 4H), 3.80 (m, 1H), 7.01 (d, J=8.6 Hz, 2H), 7.11 (s, 1H), 7.49 (d, J=8.6 Hz, 2H), 7.53 (d, J=4.8 Hz, 1H), 7.88 (br s, 1H), 8.03 (s, 1H), 8.65 (d, J=5.1 Hz, 1H).

Synthesis of (R)-2-[4-(4-{4-[3-(cyanomethylcarbamoyl)-phenyl]-thiazol-2-ylethynyl}-phenyl)-piperazine-1-sulfonylamino]-3-methylbutanoic acid (V-40)

$^1H$ NMR (DMSO-d6) δ: 0.91 (d, J=6.6 Hz, 3H), 0.93 (d, J=6.3 Hz, 3H), 1.98 (m, 1H), 3.13-3.36 (m, 8H), 3.51 (m, 1H), 4.36 (d, J=5.3 Hz, 2H), 7.04 (d, J=8.6 Hz, 2H), 7.54 (d, J=8.8 Hz, 2H), 7.62 (t, J=7.7 Hz, 1H), 7.83 (d, J=9.4 Hz, 1H), 7.88 (d, J=8.3 Hz, 1H), 8.18 (d, J=7.3 Hz, 1H), 8.32 (s, 1H), 8.49 (s, 1H), 9.35 (t, J=5.2 Hz, 1H).

Synthesis of (R)-methyl-2-[4-(4-{4-[3-(2-pyridin-4-ylacetylamino)-phenyl]-thiazol-2-ylethynyl}-phenyl)-piperazine-1-sulfonylamino]-butanoic acid (V-41)

$^1H$ NMR (DMSO-d6) δ: 0.92 (d, J=6.1 Hz, 3H), 0.93 (d, J=6.3 Hz, 3H), 1.98 (m, 1H), 3.13-3.20 (m, 4H), 3.33 (br s, 4H), 3.52 (m, 1H), 3.74 (s, 2H), 7.02 (d, J=8.6 Hz, 2H), 7.37-7.42 (m, 3H), 7.52 (d, J=8.3 Hz, 2H), 7.63 (d, J=8.6 Hz, 1H), 7.65 (d, J=8.1 Hz, 1H), 7.84 (d, J=9.4 Hz, 1H), 8.14 (s, 1H), 8.28 (s, 1H), 8.52 (br s, 2H), 10.4 (s, 1H).

Synthesis of (R)-2-(4-{4-[3-((Z)-isobutoxyiminomethyl)-1H-indol-5-ylethynyl]-phenyl}-piperazine-1-sulfonylamino)-proponic acid (V-42)

$^1H$ NMR (DMSO-d6) δ: 0.95 (d, J = 6.6 Hz, 6H), 1.30 (d, J = 7.1 Hz, 3H), 1.99-2.07 (m, 1H), 3.08-3.85 (m, 9H), 3.96 (d, J = 6.6 Hz, 2H), 6.97 (d, J = 8.6 Hz, 2H), 7.31 (d, J = 8.6 Hz, 1H), 7.39 (d, J = 8.6 Hz, 2H), 7.49 (d, J = 8.6 Hz, 1H), 7.83-7.90 (m, 2H), 8.09 (s, 1H), 8.16 (s, 1H), 11.83 (s, 1H).

(R)-2-(4-{4-[3-((E)-isobutoxyiminomethyl)-1H-indol-5-ylethynyl]-phenyl}-piperazine-1-sulfonylamino)-proponic acid (V-43)

$^1H$ NMR (DMSO-d6) δ: 0.94 (d, J = 6.6 Hz, 6H), 1.30 (d, J = 7.1 Hz, 3H), 1.95-2.06 (m, 1H), 3.09-3.83 (m, 9H), 3.88 (d, J = 6.6 Hz, 2H), 6.96 (d, J = 8.6 Hz, 2H), 7.31 (d, J = 8.6 Hz, 1H), 7.40 (d, J = 8.6 Hz, 2H), 7.45 (d, J = 8.6 Hz, 1H), 7.72 (d, J = 2.5 Hz, 1H), 7.82-7.91 (m, 1H), 8.11 (s, 1H), 8.36 (s, 1H), 11.67 (s, 1H).

Synthesis of (R)-2-(4-{4-[3-((E)-2-chrolovinyl)-1-methyl1H-indol-5-ylethynyl]-phenyl}-piperazine-1-sulfonylamino)-proponic acid (V-44)

$^1$H NMR (DMSO-d6) δ: 1.30 (d, J = 7.1 Hz, 3H), 3.08-3.37 (m, 8H), 3.77-3.83 (m, 4H), 6.92 (d, J = 14.1 Hz, 1H), 6.99 (d, J = 8.6 Hz, 2H), 7.06 (d, J = 14.1 Hz, 1H), 7.33 (d, J = 8.6 Hz, 1H), 7.41 (d, J = 8.6 Hz, 2H), 7.49 (d, J = 8.6 Hz, 1H), 7.59 (s, 1H), 7.85-7.92 (m, 1H), 8.01 (s, 1H).

Synthesis of (R)-2-{4-[4-(3-acetyl-1H-indol-5-ylethynyl)-phenyl]-piperazine-1-sulfonylamino}-3-methylbutanoic acid (V-45)

$^1$H NMR (DMSO-d6) δ: 0.93 (t, J = 6.1 Hz, 6H), 1.91-2.03 (m, 1H), 2.47 (s, 3H), 3.09-3.54 (m, 9H), 6.98 (d, J = 8.6 Hz, 2H), 7.34 (d, J = 8.6 Hz, 1H), 7.42 (d, J = 8.6 Hz, 2H), 7.49 (d, J = 8.6 Hz, 1H), 7.82 (d, J = 9.1 Hz, 1H), 8.32 (s, 1H), 8.36 (s, 1H), 12.09 (s, 1H).

Synthesis of (R)-2-{4-[4-(3-cyano-1H-indol-5-ylethynyl)-phenyl]-piperazine-1-sulfonylamino}-proponic acid (V-46)

$^1$H NMR (DMSO-d6) δ: 1.30 (d, J = 3.5 Hz, 3H), 3.05-3.36 (m, 8H), 3.80 (br s, 1H), 6.98 (d, J = 7.1 Hz, 2H), 7.37-7.60 (m, 4H), 7.72-7.92 (m, 2H), 8.30 (s, 1H), 12.40 (s, 1H).

Synthesis of (R)-2-{4-[4-(1-methyloxazol-5-yl-1H-indol-5-ylethynyl)-phenyl]-piperazine-1-sulfonylamino}-proponic acid (V-47)

$^1$H NMR (DMSO-d6) δ: 1.31 (d, J = 7.6 Hz, 3H), 3.10-3.31 (m, 8H), 3.79-3.84 (m, 1H), 3.87 (s, 3H), 7.00 (d, J = 8.6 Hz, 2H), 7.39 (d, J = 8.1 Hz, 1H), 7.43 (d, J = 8.6 Hz, 2H), 7.53 (s, 1H), 7.57 (d, J = 8.1 Hz, 1H), 7.87-7.93 (m, 2H), 8.02 (s, 1H), 8.36 (s, 1H).

Synthesis of (R)-2-[4-(4-{4-[(E)-isobutoxyimino]-1, 2, 3, 4-tetrahydroquinolin-6-ylethynyl}-phenyl)-piperazine-1-sulfonylamino]-proponic acid (V-48)

$^1$H NMR (DMSO-d6) δ: 0.92 (d, J = 6.6 Hz, 6H), 1.30 (d, J = 7.1 Hz, 3H), 1.95-2.03 (m, 1H), 2.71 (t, J = 6.3 Hz, 2H), 3.09-3.28 (m, 10H), 3.75-3.84 (m, 1H), 3.89 (d, J = 6.6 Hz, 2H), 6.50 (s, 1H), 6.65 (d, J = 8.1 Hz, 1H), 6.95 (d, J = 8.6 Hz, 2H), 7.18 (dd, J = 1.5, 8.1 Hz, 1H), 7.35 (d, J = 8.6 Hz, 2H), 7.72 (d, J = 1.5 Hz, 1H), 7.87 (br s, 1H).

Synthesis of (R)-2-[4-(4-{4-[(E)-benzyloxyimino]-1, 2, 3, 4-tetrahydroquinolin-6-ylethynyl}-phenyl)-piperazine-1-sulfonylamino]-proponic acid (V-49)

$^1$H NMR (DMSO-d6) δ: 1.30 (d, J = 7.6 Hz, 3H), 2.74 (t, J = 6.6 Hz, 2H), 3.09-3.28 (m, 10H), 3.76-3.83 (m, 1H), 5.18 (s, 2H), 6.53 (s, 1H), 6.66 (d, J = 8.1 Hz, 1H), 6.95 (d, J = 9.1 Hz, 2H), 7.18 (dd, J = 1.5, 8.1 Hz, 1H), 7.28-7.42 (m, 7H), 7.71 (d, J = 1.5 Hz, 1H), 7.87 (br s, 1H).

Synthesis of (R)-2-[4-(4-{4-[(E)-4-methoxybenzyloxyimino]-1, 2, 3, 4-tetrahydroquinolin-6-ylethynyl}-phenyl)-piperazine-1-sulfonylamino]-proponic acid (V-50)

$^1$H NMR (DMSO-d6) δ: 1.30 (d, J = 7.1 Hz, 3H), 2.70 (t, J = 6.3 Hz, 2H), 3.09-3.28 (m, 10H), 3.75 (s, 3H), 3.77-3.83 (m, 1H), 5.09 (s, 2H), 6.51 (s, 1H), 6.65 (d, J = 8.1 Hz, 1H), 6.91-6.98 (m, 4H), 7.18 (dd, J = 1.5, 8.1 Hz, 1H), 7.32-7.38 (m, 4H), 7.73 (d, J = 1.5 Hz, 1H), 7.82-7.90 (m, 1H).

Synthesis of (R)-2-{4-[4-(4-benzyl-5-oxo-2, 3, 4, 5-tetrahydro-1H-benzo[e][1,4]diazepin-7-ylethynyl)-phenyl]-piperazine-1-sulfonylamino}-proponic acid (V-51)

$^1$H NMR (DMSO-d6) δ: 1.30 (d, J = 7.1 Hz, 3H), 3.10-3.28 (m, 10H), 3.46-3.51 (m, 2H), 3.76-3.84 (m, 1H), 4.70 (s, 2H), 6.70 (d, J = 8.6 Hz, 1H), 6.75-6.77 (m, 1H), 6.96 (d, J = 9.1 Hz, 2H), 7.24-7.38 (m, 8H), 7.83 (s, 1H), 7.90 (d, J = 8.6 Hz, 1H).

Synthesis of (R)-2-{4-[4-(4-benzyl1-methyl-5-oxo-2, 3, 4, 5-tetrahydro1H-benzo[e][1,4]diazepin-7-ylethynyl)-phenyl]-piperazine-1-sulfonylamino}-proponic acid (V-52)

$^1$H NMR (DMSO-d6) δ: 1.29 (d, J = 7.2 Hz, 3H), 2.78 (s, 3H), 3.19-3.26 (m, 12H), 3.73-3.84 (m, 1H), 4.72 (s,

2H), 6.89-7.00 (m, 3H), 7.31-7.39 (m, 7H), 7.48 (dd, J = 2.1, 8.4 Hz, 1H), 7.58 (d, J = 2.1 Hz, 1H), 7.83-7.94 (m, 1H).

Synthesis of (R)-2-[4-(4-{4-[(E)-methoxyimino]-1, 2, 3, 4-tetrahydroquinolin-6-ylethynyl}-phenyl)-piperazine-1-sulfonylamino]-3-methylbutanoic acid (V-53)

$^1$H NMR (DMSO-d6) δ: 0.90 (t, J = 5.9 Hz, 6H), 1.89-2.02 (m, 1H), 2.67 (t, J = 6.4 Hz, 2H), 3.06-3.27 (m, 10H), 3.43-3.52 (m, 1H), 3.87 (s, 3H), 6.52 (s, 1H), 6.64 (d, J = 8.5 Hz, 1H), 6.94 (d, J = 8.8 Hz, 2H), 7.16 (dd, J = 8.5, 2.0 Hz, 1H), 7.33 (d, J = 8.8 Hz, 2H), 7.71 (d, J = 2.0 Hz, 1H), 7.74-7.83 (m, 1H).

Synthesis of (R)-2-{4-[4-(3-{1-[(Z)-methoxyimono]-ethyl}-1H-indol-5-ylethynyl)-phenyl]-piperazine-1-sulfonylamino}-3-methylbutanoic acid (V-54)

$^1$H NMR (DMSO-d6) δ: 0.93 (t, J = 5.8 Hz, 6H), 1.94-2.02 (m, 1H), 2.21 (s, 3H), 3.09-3.29 (m, 8H), 3.48-3.54 (m, 1H), 3.95 (s, 3H), 6.97 (d, J = 8.6 Hz, 2H), 7.29 (d, J = 8.1 Hz, 1H), 7.39-7.44 (m, 3H), 7.80-7.85 (m, 2H), 8.31 (s, 1H), 11.61 (s, 1H).

Synthesis of (R)-2-{4-[4-(3-{1-[(Z)-methoxyimino]-ethyl}-1H-indol-5-ylethynyl)-phenyl]-piperazine-1-sulfonylamino}-proponic acid (V-55)

$^1$H NMR (DMSO-d6) δ: 1.31 (d, J = 7.1 Hz, 3H), 2.21 (s, 3H), 3.08-3.33 (m, 8H), 3.77-3.87 (m, 1H), 3.95 (s, 3H), 6.97 (d, J = 8.1 Hz, 2H), 7.29 (d, J = 8.6 Hz, 1H), 7.38-7.45 (m, 3H), 7.82-7.93 (m, 2H), 8.31 (s, 1H), 11.61 (s, 1H).

Synthesis of (R)-2-{4-[4-(3-acetylbenzo[b]thiophen-5-ylethynyl)-phenyl]-piperazine-1-sulfonylamino}-proponic acid (V-56)

$^1$H NMR (DMSO-d6) δ: 1.29 (d, J = 7.1 Hz, 3H), 2.64 (s, 3H), 3.10-3.34 (m, 8H), 3.74-3.82 (m, 1H), 7.00 (d, J = 8.6 Hz, 2H), 7.46 (d, J = 8.6 Hz, 2H), 7.56 (d, J = 8.6 Hz, 1H), 8.12 (d, J = 8.6 Hz, 1H), 8.71 (s, 1H), 9.02 (s, 1H).

Synthesis of (R)-2-(4-{4-[3-(5-ethyloxazol-2-yl)-4-(2-methoxyethoxy)-phenylethynyl]-phenyl}-piperazine-1-sulfonylamino)-3-methylbutanoic acid (V-57)

$^1$H NMR (DMSO-d6) δ: 0.92 (t, J = 5.6 Hz, 6H), 1.25 (t, J = 7.3 Hz, 3H), 1.92-2.03 (m, 1H), 2.73 (q, J = 7.3 Hz, 2H), 3.10-3.39 (m, 11H), 3.47-3.54 (m, 1H), 3.72 (br s, 2H), 4.24 (br s, 2H), 6.96-7.03 (m, 3H), 7.22 (d, J = 8.6 Hz, 1H), 7.41 (d, J = 8.6 Hz, 2H), 7.57 (d, J = 8.6 Hz, 1H), 7.82 (d, J = 9.6 Hz, 1H), 7.91 (s, 1H).

Synthesis of (R)-2-(4-{4-[1-(4-methoxybenzyl)-3-methyl-2,4-dioxo-1, 2, 3, 4-tetrahydroquinazolin-6-ylethynyl]-phenyl}-piperazine-1-sulfonylamino)-3-methylbutanoic acid (V-58)

$^1$H NMR (DMSO-d6) δ: 0.91 (d, J=6.6 Hz, 3H), 0.92 (d, J=6.6 Hz, 3H), 1.97 (m, 1H), 3.05-3.40 (m, 8H), 3.49 (m, 1H), 3.72 (s, 3H), 5.32 (s, 2H), 6.89 (d, J=8.7 Hz, 2H), 6.98 (d, J=9.0 Hz, 2H), 7.27 (d, J=9.0 Hz, 2H), 7.34 (d, J=8.7 Hz, 1H), 7.42 (d, J=9.0 Hz, 2H), 7.75 (dd, J=2.1, 8.7 Hz, 1H), 7.83 (d, J=9.3 Hz, 1H), 7.95 (s, 1H), 8.10 (d, J=2.4 Hz, 1H), 12.74 (br s, 1H).

Synthesis of (R)-2-(4-{4-[3-(4-fluorobenzyl)-4-oxo-3,4-dihydroquinazolin-6-ylethynyl]-phenyl}-piperazine-1-sulfonylamino)-3-methylbutanoic acid (V-59)

$^1$H NMR (DMSO-d6) δ: 0.91 (d, J=6.6 Hz, 3H), 0.93 (d, J=6.6 Hz, 3H), 1.97 (m, 1H), 3.05-3.40 (m, 8H), 3.50 (m, 1H), 5.19 (s, 2H), 7.01 (d, J=9.0 Hz, 2H), 7.14-7.24 (m, 2H), 7.42-7.52 (m, 4H), 7.70 (d, J=8.7 Hz, 1H), 7.83 (d, J=9.3 Hz, 1H), 7.90 (dd, J=2.1, 8.4 Hz, 1H), 8.19 (d, J=2.4 Hz, 1H), 8.62 (s, 1H), 12.70 (br s, 1H).

Synthesis of (R)-2-(4-{4-[3-(4-fluorobenzyl)-1-methyl-2, 4-dioxo-1, 2, 3, 4-tetrahydroquinazolin-6-ylethynyl]-phenyl}-piperazine-1-sulfonylamino)-3-methylbutanoic acid (V-60)

$^1$H NMR (DMSO-d6) δ: 0:89-0.95 (m, 6H), 1.97 (m, 1H), 3.05-3.40 (m, 8H), 3.50 (m, 1H), 3.54 (s, 3H), 5.12 (s, 2H), 6.99 (d, J=9.3 Hz, 2H), 7.08-7.18 (m, 2H), 7.36-7.46 (m, 4H), 7.50 (d, J=9.0 Hz, 1H), 7.80 (m, 1H), 7.88 (dd, J=2.1, 8.7 Hz, 1H), 8.10 (d, J=2.1 Hz, 1H).

Synthesis of (R)-2-(4-{4-[1-ethyl-3-(4-fluorobenzyl)-2, 4-dioxo-1, 2, 3, 4-tetrahydroquinazolin-6-ylethynyl]-phenyl}-piperazine-1-sulfonylamino)-3-methylbutanoic acid (V-61)

$^1$H NMR (DMSO-d6) δ: 0.89-0.95 (m, 6H), 1.22 (d, J=6.9 Hz, 3H), 1.98 (m, 1H), 3.05-3.60 (m, 9H), 4.16 (q, J=6.9 Hz, 2H), 5.12 (s, 2H), 6.99 (d, J=9.3 Hz, 2H), 7.10-7.18 (m, 2H), 7.36-7.48 (m, 4H), 7.56 (d, J=9.0 Hz, 1H), 7.70 (m, 1H), 7.87 (dd, J=2.4, 9.0 Hz, 1H), 8.11 (d, J=2.4 Hz, 1H).

Synthesis of (R)-2-(4-{4-[3-(4-fluorobenzyl)-2, 4-dioxo-1-propyl-1, 2, 3, 4-tetrahydroquinazolin-6-ylethynyl]-phenyl}-piperazine-1-sulfonylamino)-3-methylbutanoic acid (V-62)

$^1$H NMR (DMSO-d6) δ: 0.87-0.98 (m, 9H), 1.55-1.70 (m, 2H), 1.97 (m, 1H), 3.05-3.55 (m, 9H), 4.00-4.15 (m, 2H), 5.11 (s, 2H), 6.99 (d, J=9.0 Hz, 2H), 7.08-7.18 (m, 2H), 7.34-7.46 (m, 4H), 7.54 (d, J=9.0 Hz, 1H), 7.80 (m, 1H), 7.84 (dd, J=2.1, 8.7 Hz, 1H), 8.09 (d, J=2.1 Hz, 1H).

Synthesis of (R)-2-{4-[4-(3-cyanomethyl-2, 4-dioxo-1, 2, 3, 4-tetrahydroquinazolin-6-ylethynyl)-phenyl]-piperazine-1-sulfonylamino}-3-methylbutanoic acid (V-63)

$^1$H NMR (DMSO-d6) δ: 0.85-0.96 (m, 6H), 1.98 (m, 1H), 3.05-3.40 (m, 8H), 3.49 (m, 1H), 4.91 (s, 2H), 6.99 (d, J=8.4 Hz, 2H), 7.23 (d, J=8.4 Hz, 1H), 7.43 (d, J=9.0 Hz, 2H), 7.72 (m, 1H), 7.80 (dd, J=1.8, 8.7 Hz, 1H), 8.00 (d, J=2.1 Hz, 1H), 11.94 (br s, 1H).

Synthesis of (R)-2-{4-[4-(3-benzyloxy2, 4-dioxo-1, 2, 3, 4-tetrahydroquinazolin-6-ylethynyl)-phenyl]-piperazine-1-sulfonylamino}-3-methylbutanoic acid (V-64)

$^1$H NMR (DMSO-d6) δ: 0.87-0.95 (m, 6H), 1.97 (m, 1H), 3.05-3.40 (m, 8H), 3.48 (m, 1H), 5.11 (s, 2H), 6.99 (d, J=9.0 Hz, 2H), 7.21 (d, J=8.7 Hz, 1H), 7.36-7.46 (m, 5H), 7.52-7.60 (m, 2H), 7.77 (dd, J=1.8, 8.4 Hz, 1H), 7.77 (m, 1H), 7.99 (d, J=2.1 Hz, 1H), 11.82 (s, 1H), 12.80 (br s, 1H).

Synthesis of (R)-2-(4-{4-[3-(3-fluorobenzyl)-2, 4-dioxo-1, 2, 3, 4-tetrahydroquinazolin-6-ylethynyl]-phenyl}-piperazine-1-sulfonylamino)-3-methylbutanoic acid (V-65)

$^1$H NMR (DMSO-d6) δ: 0.91 (d, J=6.6 Hz, 3H), 0.92 (d, J=6.6 Hz, 3H), 1.97 (m, 1H), 3.05-3.40 (m, 8H), 3.49 (m, 1H), 5.10 (s, 2H), 6.99 (d, J=8.7 Hz, 2H), 7.04-7.20 (m, 3H), 7.22 (d, J=8.7 Hz, 1H), 7.32-7.46 (m, 3H), 7.78 (dd, J=2.4, 9.0 Hz, 1H), 7.80 (m, 1H), 7.99 (d, J=2.1 Hz, 1H), 11.74 (s, 1H), 12.75 (br s, 1H).

Synthesis of (R)-2-(4-{4-[3-(4-fluorobenzyl)-2-methyl4-oxo-3,4-dihydroquinazolin-6-ylethynyl]-phenyl}-piperazine-1-sulfonylamino)-3-methylbutanoic acid (V-66)

$^1$H NMR (DMSO-d6) δ: 0.84-0.96 (m, 6H), 1.98 (m, 1H), 2.50 (s, 3H), 3.05-3.55 (m, 9H), 5.35 (s, 2H), 6.99 (d, J=9.0 Hz, 2H), 7.12-7.22 (m, 2H), 7.22-7.32 (m, 2H), 7.45 (d, J=8.4 Hz, 2H), 7.60 (d, J=8.4 Hz, 1H), 7.64 (m, 1H), 7.87 (dd, J=1.8, 8.4 Hz, 1H), 8.17 (d, J=2.1 Hz, 1H).

Synthesis of (R)-2-(4-{4-[3-(4-fluorophenyl)-2, 4-dioxo-1, 2, 3, 4-tetrahydroquinazolin-6-ylethynyl]-phenyl}-piperazine-1-sulfonylamino)-3-methylbutanoic acid (V-67)

$^1$H NMR (DMSO-d6) δ: 0.91 (d, J=6.6 Hz, 3H), 0.92 (d, J=6.9 Hz, 3H), 1.97 (m, 1H), 3.10-3.40 (m, 8H), 3.49 (m, 1H), 6.99 (d, J=8.7 Hz, 2H), 7.25 (d, J=8.4 Hz, 1H), 7.28-7.46 (m, 6H), 7.80 (dd, J=2.1, 8.4 Hz, 1H), 7.82 (m, 1H), 7.98 (d, J=1.8 Hz, 1H), 11.75 (s, 1H).

Synthesis of (R)-2-{4-[4-(3-cyanomethyl-1-methyl-2, 4-dioxo-1, 2, 3, 4-tetrahydroquinazolin-6-ylethynyl)-phenyl]-piperazine-1-sulfonylamino}-3-methylbutanoic acid (V-68)

$^1$H NMR (DMSO-d6) δ: 0.85-0.96 (m, 6H), 1.98 (m, 1H), 3.05-3.50 (m, 9H), 3.55 (s, 3H), 4.96 (s, 2H), 6.99 (d, J=8.7 Hz, 2H), 7.44 (d, J=8.7 Hz, 2H), 7.53 (d, J=9.0 Hz, 1H), 7.68 (m, 1H), 7.90 (dd, J=1.8, 8.7 Hz, 1H), 8.09 (d, J=2.1 Hz, 1H).

Synthesis of (R)-2-(4-{4-[3-(4-fluorobenzyl)-4-oxo-1, 2, 3, 4-tetrahydroquinazolin-6-ylethynyl]-phenyl}-piperazine-1-sulfonylamino)-3-methylbutanoic acid (V-69)

$^1$ H NMR (DMSO-d6) δ: 0.85-0.96 (m, 6H), 1.97 (m, 1H), 3.05-3.40 (m, 8H), 3.49 (m, 1H), 4.50-4.65 (m, 4H), 6.74 (d, J=8.4 Hz, 1H), 6.96 (d, J=8.4 Hz, 2H), 7.08-7.24 (m, 3H), 7.32-7.44 (m, 5H), 7.72-7.84 (m, 2H), 12.65 (br s, 1H).

Synthesis of (R)-2-{4-[4-(3-cyanomethyl-1-methyl-2, 4-dioxo-1, 2, 3, 4-tetrahydroquinazolin-6-ylethynyl)-phenyl]-piperazine-1-sulfonylamino}-proponic acid (V-70)

$^1$ H NMR (DMSO-d6) δ: 1.29 (d, J=7.2 Hz, 3H), 3.05-3.40 (m, 8H), 3.78 (m, 1H), 4.96 (s, 2H), 6.99 (d, J=9.0 Hz, 2H), 7.44 (d, J=8.7 Hz, 2H), 7.53 (d, J=8.7 Hz, 1H), 7.85 (m, 1H), 7.90 (dd, J=2.1, 8.7 Hz, 1H), 8.09 (d, J=2.1 Hz, 1H).

Synthesis of (R)-2-(4-{4-[3-(4-ffuorobenzyl)-1-methyl-2, 4-dioxo-1, 2, 3, 4-tetrahydroquinazolin-6-ylethynyl]-phenyl}-piperazine-1-sulfonylamino)-proponic acid (V-71)

$^1$ H NMR (DMSO-d6) δ: 1.30 (d, J=7.2 Hz, 3H), 3.05-3.40 (m, 8H), 3.80 (m, 1H), 5.11 (s, 2H), 6.99 (d, J=8.7 Hz, 2H), 7.08-7.18 (m, 2H), 7.34-7.46 (m, 4H), 7.50 (d, J=8.4 Hz, 1H), 7.88 (dd, J=2.4, 8.4 Hz, 1H), 7.90 (m, 1H), 8.09 (d, J=2.1 Hz, 1H), 12.72 (br s, 1H).

Synthesis of (R)-2-{4-[4-(3-cyclohexylmethyl-1-methyl-2, 4-dioxo-1, 2, 3, 4-tetrahydroquinazolin-6-ylethynyl)-phenyl]-piperazine-1-sulfonylamino}-3-methylbutanoic acid (V-72)

$^1$ H NMR (DMSO-d6) δ: 0.80-1.35 (m, 11H), 1.50-1.95 (m, 6H), 1.98 (m, 1H), 3.05-3.55 (m, 9H), 3.52 (s, 3H), 3.82 (d, J=6.9 Hz, 2H), 6.98 (d, J=8.4 Hz, 1H), 6.96 (d, J=8.4 Hz, 2H), 7.08-7.24 (m, 3H), 7.32-7.44 (m, 5H), 7.72-7.84 (m, 2H), 12.65 (br s, 1H).

Synthesis of (R)-2-[4-(4-{3-[2-(4-fluorophenyl)-ethyl]-1-methyl-2, 4-dioxo-1, 2, 3, 4-tetrahydroquinazolin-6-ylethynyl}-phenyl)-piperazine-1-sulfonylamino]-3-methylbutanoic acid (V-73)

$^1$ H NMR (DMSO-d6) δ: 0.85-0.95 (m, 6H), 1.98 (m, 1H), 2.88 (t, J=7.2 Hz, 2H), 3.05-3.55 (m, 9H), 3.54 (s, 3H), 4.14 (t, J=7.2 Hz, 2H), 7.00 (d, J=8.7 Hz, 2H), 7.07-7.18 (m, 2H), 7.24-7.32 (m, 2H), 7.44 (d, J=8.7 Hz, 2H), 7.49 (d, J=9.3 Hz, 1H), 7.78 (m, 1H), 7.87 (dd, J=2.1, 8.7 Hz, 1H), 8.06 (d, J=1.8 Hz, 1H).

Synthesis of (R)-2-(4-{4-[3-(3-fluorobenzyl)-2, 2-dimethyl4-oxo-1, 2, 3, 4-tetrahydroquinazolin-6-ylethynyl]-phenyl}-piperazine-1-sulfonylamino)-3-methylbutanoic acid (V-74)

$^1$ H NMR (DMSO-d6) δ: 0.88-0.96 (m, 6H), 1.42 (s, 6H), 1.97 (m, 1H), 3.05-3.40 (m, 8H), 3.49 (m, 1H), 4.74 (s, 2H), 6.72 (d, J=8.1 Hz, 1H), 6.97 (d, J=8.7 Hz, 2H), 7.00-7.18 (m, 3H), 7.20 (s, 1H), 7.32-7.45 (m, 4H), 7.77 (d, J=2.1 Hz, 1H), 7.78 (m, 1H).

Synthesis of (R)-2-{4-[4-(2, 4-dioxo-3-pyridin-4-ylmethyl-1, 2, 3, 4-tetrahydroquinazolin-6-ylethynyl)-phenyl]-piperazine-1-sulfonylamino}-3-methylbutanoic acid (V-75)

$^1$ H NMR (DMSO-d6) δ: 0.88-0.96 (m, 6H), 1.97 (m, 1H), 3.05-3.40 (m, 8H), 3.49 (m, 1H), 5.11 (s, 2H), 6.99 (d, J=8.7 Hz, 2H), 7.24 (d, J=8.4 Hz, 1H), 7.29 (d, J=5.1 Hz, 2H), 7.42 (d, J=8.7 Hz, 2H), 7.79 (dd, J=2.1, 8.7 Hz, 1H), 7.82 (m, 1H), 7.99 (d, J=2.1 Hz, 1H), 8.49 (d, J=5.7 Hz, 2H), 11.79 (s, 1H).

Synthesis of (R)-2-(4-{4-[3-(3,4-difluorobenzyl)-1-methyl-2, 4-dioxo-1, 2, 3, 4-tetrahydroquinazolin-6-ylethynyl]-phenyl}-piperazine-1-sulfonylamino)-3-methylbutanoic acid (V-76)

$^1$ H NMR (DMSO-d6) δ: 0.85-0.98 (m, 6H), 1.98 (m, 1H), 3.05-3.55 (m, 9H), 3.54 (s, 3H), 5.12 (s, 2H), 7.00 (d, J=9.3 Hz, 2H), 7.20 (m, 1H), 7.32-7.48 (m, 4H), 7.51 (d, J=9.3 Hz, 1H), 7.88 (m, 1H), 7.89 (dd, J=2.1, 8.7 Hz, 1H), 8.10 (d, J=1.8 Hz, 1H).

Synthesis of (R)-3-methyl-2-{4-[4-(1-methyl2, 4-dioxo-3-pyridin-4-ylmethyl-1, 2, 3, 4-tetrahydroquinazolin-6-ylethynyl)-phenyl]-piperazine-1-sulfonylamino}-3-methylbutanoic acid (V-77)

$^1$ H NMR (DMSO-d6) δ: 0.86-0.96 (m, 6H), 1.97 (m, 1H), 3.05-3.40 (m, 8H), 3.49 (m, 1H), 3.54 (s, 3H), 5.15

(s, 2H), 6.99 (d, J=8.4 Hz, 2H), 7.29 (d, J=5.4 Hz, 2H), 7.43 (d, J=8.4 Hz, 2H), 7.53 (d, J=8.4 Hz, 1H), 7.83 (d, J=9.3 Hz, 1H), 7.90 (dd, J=2.1, 8.7 Hz, 1H), 8.09 (d, J=1.8 Hz, 1H), 8.48 (d, J=5.7 Hz, 2H), 12.76 (br s, 1H).

Synthesis of (R)-2-[4-(4-{4-[3-(4-carbamoylbenzyloxy)-phenyl]-thiazol-2-ylethynyl}-phenyl)-piperazine-1-sulfonylamino]-3-methylbutanoic acid (V-78)

$^1$H NMR (DMSO-d6) δ: 0.88 (d, J=6.6 Hz, 3H), 0.92 (d, J=6.9 Hz, 3H), 1.99 (m, 1H), 3.05-3.55 (m, 9H), 5.25 (s, 2H), 7.00-7.05 (m, 3H), 7.34-7.44 (m, 2H), 7.50-7.68 (m, 7H), 7.90 (d, J=8.1 Hz, 2H), 7.98 (m, 1H), 8.27 (s, 1H).

Synthesis of (R)-3-methyl-2-[4-(4-{4-[3-(4-methylcarbamoylbenzyloxy)-phenyl]-thiazol-2-ylethynyl}-phenyl)-piperazine-1-sulfonylamino]-butanoic acid (V-79)

$^1$H NMR (DMSO-d6) δ: 0.90 (d, J=6.9 Hz, 3H), 0.92 (d, J=6.6 Hz, 3H), 1.97 (m, 1H), 2.78 (d, J=4.8 Hz, 3H), 3.05-3.40 (m, 8H), 3.49 (m, 1H), 5.24 (s, 2H), 6.88-7.08 (m, 3H), 7.38 (m, 1H), 7.46-7.60 (m, 5H), 7.64 (m, 1H), 7.82 (m, 1H), 7.85 (d, J=8.1 Hz, 2H), 8.27 (s, 1H), 8.44 (m, 1H), 12.70 (br s, 1H).

Synthesis of (R)-2-(4-{4-[2-(3-fluoro-4-isopropylcarbamoylbenzylcarbamoyl)-pyridin-4-ylethynyl]-phenyl}-piperazine-1-sulfonylamino)-proponic acid (V-80)

$^1$H NMR (DMSO-d6) δ: 1.13 (d, J=6.6 Hz, 6H), 1.30 (d, J=7.1 Hz, 3H), 3.08-3.84 (m, 9H), 4.03 (qd, J=7.1, 13.6 Hz, 1H), 4.53 (d, J=6.6 Hz, 2H), 7.02 (d, J=9.1 Hz, 2H), 7.17-7.21 (m, 2H), 7.48-7.52 (m, 3H), 7.67 (dd, J=1.5, 5.1 Hz, 1H), 7.85 (m, 1H), 8.02 (s, 1H), 8.06 (d, J=7.6 Hz, 1H), 8.67 (d, J=5.1 Hz, 1H), 9.50 (t, J=6.6 Hz, 1H).

Synthesis of (R)-2-[4-(4-{2-[(3-methyl-2-oxo-2,3-dihydrobenzoxazol-5-ylmethyl)-carbamoyl]-pyridin-4-ylethynyl}-phenyl)-piperazine-1-sulfonylamino]-proponic acid (V-81)

$^1$H NMR (DMSO-d6) δ: 1.27 (d, J=7.1 Hz, 3H), 3.08-3.70 (m, 12H), 4.53 (d, J=6.6 Hz, 2H), 7.01 (d, J=9.1 Hz, 2H), 7.11 (d, J=9.1 Hz, 1H), 7.22-7.26 (m, 2H), 7.48 (d, J=8.6 Hz, 2H), 7.65 (dd, J=1.3, 4.6 Hz, 1H), 8.02 (s, 1H), 8.64 (d, J=4.6 Hz, 1H), 9.39 (t, J=6.6 Hz, 1H).

Synthesis of (R)-3-methyl-2-(4-{4-[4-(5-methylisoxazol-3-ylmethyl)-5-oxo-2, 3, 4, 5-tetrahydrobenzo[f][1,4]-oxazepin-7-ylethynyl]-phenyl}-piperazine-1-sulfonylamino)-butanoic acid (V-82)

$^1$H NMR (DMSO-d6) δ: 0.91 (d, J=6.1 Hz, 3H), 0.92 (d, J=6.1 Hz, 3H), 1.97 (m, 1H), 2.38 (s, 3H), 3.06-3.72 (m, 11H), 4.37 (t, J=4.3 Hz, 2H), 4.76 (s, 2H), 6.20 (s, 1H), 6.98 (d, J=8.1 Hz, 2H), 7.05 (d, J=8.6 Hz, 1H), 7.40 (d, J=8.6 Hz, 2H), 7.58 (dd, J=1.0, 8.1 Hz, 1H), 7.82 (d, J=1.0 Hz, 1H), 7.82 (s, 1H).

Synthesis of (R)-2-(4-{4-[4-(3-methoxybenzyl)-5-oxo-2, 3, 4, 5-tetrahydrobenzo[f][1,4]-oxazepin-7-ylethynyl]-phenyl}-piperazine-1-sulfonylamino)-3-methylbutanoic acid (V-83)

$^1$H NMR (DMSO-d6) δ: 0.87 (d, J=6.1 Hz, 3H), 0.92 (d, J=6.1 Hz, 3H), 1.99 (m, 1H), 3.05-3.72 (m, 9H), 3.77 (s, 3H), 3.98 (t, J=9.1 Hz, 2H), 4.37 (t, J=9.1 Hz, 2H), 5.23 (s, 2H), 6.87 (m, 1H), 6.97 (d, J=9.1 Hz, 2H), 7.04 (m, 1H), 7.20 (s, 1H), 7.21 (d, J=9.6 Hz, 1H), 7.29 (t, J=7.6 Hz, 1H), 7.39 (d, J=8.1 Hz, 3H), 7.58 (dd, J=1.1, 8.1 Hz, 2H), 7.75 (s, 1H).

Synthesis of (R)-2-(4-{4-[4-(4-cyanobenzyl)-5-oxo-2, 3, 4, 5-tetrahydrobenzo[f][1,4]-oxazepin-7-ylethynyl]-phenyl}-piperazine-1-sulfonylamino)-3-methylbutanoic acid (V-84)

$^1$H NMR (DMSO-d6) δ: 0.82 (d, J=6.1 Hz, 3H), 0.89 (d, J=6.1 Hz, 3H), 2.01 (m, 1H), 3.08-3.75 (m, 11H), 4.39 (t, J=4.3 Hz, 2H), 5.35 (s, 2H), 6.94 (d, J=8.6 Hz, 2H), 7.26 (d, J=8.6 Hz, 1H), 7.35 (d, J=8.1 Hz, 2H), 7.69-7.72 (m, 3H), 7.81 (d, J=8.1 Hz, 2H), 8.05 (d, J=1.0 Hz, 1H).

Synthesis of (R)-3-methyl-2-[4-(4-{2-[(3-methyl-2-oxo-2, 3-dihydrobenzoxazol-5-ylmethyl)-carbamoyl]-pyridin-4-ylethynyl}-phenyl)-piperazine-1-sulfonylamino]-butanoic acid (V-85)

$^1$H NMR (DMSO-d6) δ: 0.90 (d, J=8.6 Hz, 3H), 0.92 (d, J=8.6 Hz, 3H), 1.99 (m, 1H), 3.08-3.38 (m, 11H), 3.51 (m, 1H), 4.51 (d, J=5.6 Hz, 2H), 6.98 (d, J=8.6 Hz, 2H), 7.11 (d, J=8.1 Hz, 1H), 7.20 (d, J=8.1 Hz, 1H), 7.28 (s, 1H), 7.46 (d, J=8.1 Hz, 2H), 7.60 (d, J=4.0 Hz, 1H), 8.02 (s, 1H), 8.61 (d, J=4.0 Hz, 1H), 9.37 (t, J=5.6 Hz, 1H).

Synthesis of (R)-2-(4-{4-[4-(3-acetylaminophenyl)-thiazol-2-ylethynyl]-phenyl}-piperazine-1-sulfonylamino)-3-methylbutanoic acid (V-86)

$^1H$ NMR (DMSO-d6) δ: 0.92 (d, J=6.1 Hz, 3H), 0.93 (d, J=6.1 Hz, 3H), 1.98 (m, 1H), 2.07 (s, 3H), 3.17-3.22 (m, 8H), 3.51 (dd, J=6.3, 9.4 Hz, 1H), 7.03 (d, J=8.6 Hz, 2H), 7.38 (t, J=7.6 Hz, 1H), 7.53 (d, J=8.6 Hz, 2H), 7.62 (d, J=7.6 Hz, 2H), 7.84 (d, J=9.6 Hz, 1H), 8.13 (s, 1H), 8.24 (s, 1H), 10.05 (s, 1H).

Synthesis of (R)-2-{4-[4-(1-cyanoisoquinolin-7-ylethynyl)-phenyl]-piperazine-1-sulfonylamino}-3-methylbutanoic acid (V-87)

$^1H$ NMR (DMSO-d6) δ: 0.87 (d, J=6.3 Hz, 3H), 0.92 (d, J=6.3 Hz, 3H), 2.00 (m, 1H), 3.20-3.92 (m, 9H), 7.03 (d, J=8.1 Hz, 2H), 7.53 (d, J=8.6 Hz, 2H), 7.82 (m, 1H), 7.99 (d, J=8.6 Hz, 1H), 8.20-8.26 (m, 3H), 8.72 (d, J=5.6 Hz, 1H).

Synthesis of (R)-2-{4-[4-(1-carbamoylisoquinolin-7-ylethynyl)-phenyl]-piperazine-1-sulfonylamino}-3-methylbutanoic acid (V-88)

$^1H$ NMR (DMSO-d6) δ: 0.91 (d, J=6.6 Hz, 3H), 0.92 (d, J=6.6 Hz, 3H), 1.97 (m, 1H), 3.04-3.85 (m, 9H), 7.01 (d, J=8.6 Hz, 2H), 7.49 (d, J=8.6 Hz, 2H), 7.81-7.86 (m, 3H), 8.01-8.07 (m, 2H), 8.29 (s, 1H), 8.55 (d, J=5.6 Hz, 1H), 9.12 (s, 1H).

7-{4-[4-((R)-1-carboxy-2-methylpropylsulfamoyl)-piperazin-1-yl]-phenylethynyl}-isoquinoline-1-carboxylic acid (V-89)

[M+H]=537, Retention Time 2.67 min

Conditions.( in the same condition as follows)
Column: Phenomenex Luna, C18(2), 4.6x50 mm, 5 mm
Solvent: Water/acetonitrile 90:10 - 0:100 (3min), 0:100 (1min)
Flow: 3.00 ml/min

Synthesis of (R)-2-(4-{4-[4-(4-methoxybenzyl)-5-oxo-2, 3, 4, 5-tetrahydrobenzo[f][1,4]-oxazepin-7-ylethynyl]-phenyl}-piperazine-1-sulfonylamino)-3-methylbutanoic acid (V-90)

[M+H]=647, Retention Time 1.51 min

Conditions.

Column: Phenomenex Luna, C18(2), 4.6x50 mm, 5 mm
Solvent: Water/acetonitrile 90:10 - 0:100 (3min), 0:100 (1min)
Flow:3.00 ml/min

According to the same manner as that described above, in the compounds represented by the general formula (I'):

[Chemical formula 75]

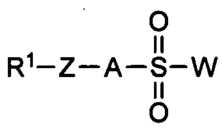

(I')

wherein $R^1$ is a group represented by:

[Chemical formula 76]

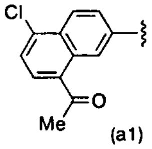
(a1),
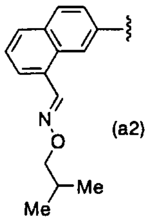
(a2),
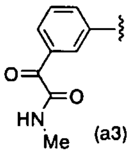
(a3),
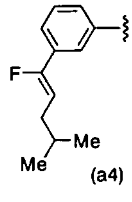
(a4),
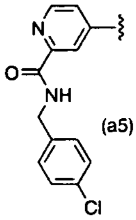
(a5),
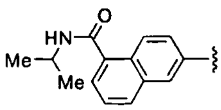
(a6),
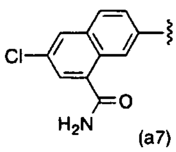
(a7),
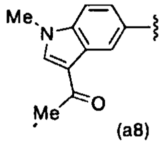
(a8),
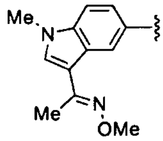
(a9),
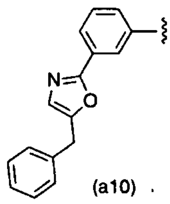
(a10),
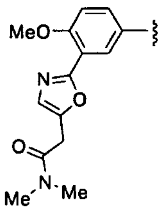
(a11),
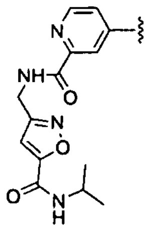
(a12),
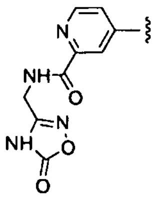
(a13),
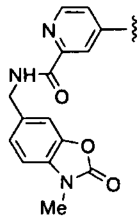
(a14),
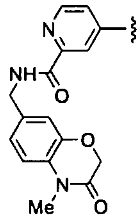
(a15),
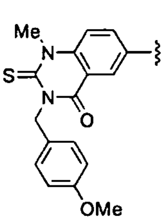
(a16),
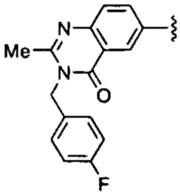
(a17),
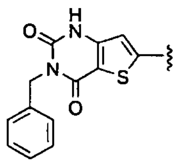
(a18),
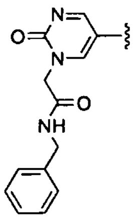
(a19), or
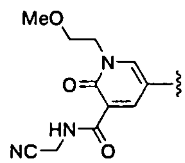
(a20)

Z is a group represented by:

[Chemical formula 77]

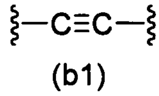
(b1),
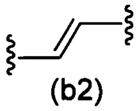
(b2),
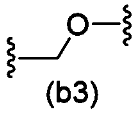
(b3), or
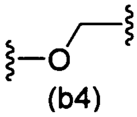
(b4)

A is a group represented by:

[Chemical formula 78]

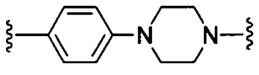
(c1)
,
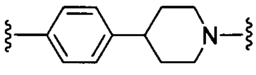
(c2)
or
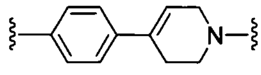
(c3)

W is a group represented by:

[Chemical formula 79]

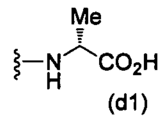
(d1)
,
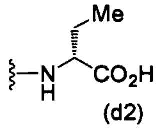
(d2)
,
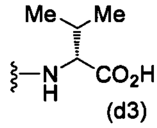
(d3)
,
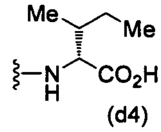
(d4)
,
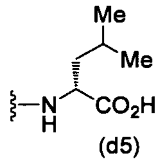
(d5)
,
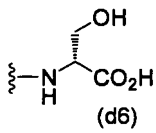
(d6)
,
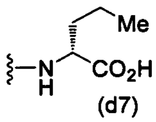
(d7)
,
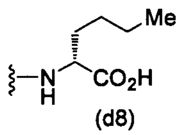
(d8)
,
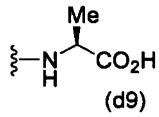
(d9)
,
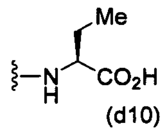
(d10)
,
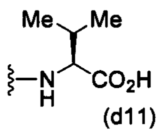
(d11)
,
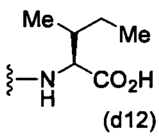
(d12)
,
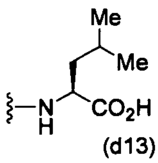
(d13)
,
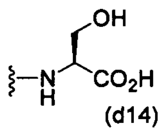
(d14)
,
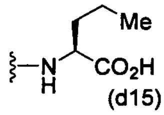
(d15)
,

or
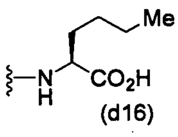
(d16)

(R1, Z, A, W) = (a1, b1, c1, d1), (a1, b1, c1, d2), (a1, b1, c1, d3), (a1, b1, c1, d4), (a1, b1, c1, d5), (a1, b1, c1, d6), (a1, b1, c1, d7), (a1, b1, c1, d8), (a1, b1, c1, d9), (a1, b1, c1, d10), (a1, b1, c1, d11), (a1, b1, c1, d12), (a1, b1, c1, d13), (a1, b1, c1, d14), (a1, b1, c1, d15), (a1, b1, c1, d16), (a1, b1, c2, d1), (a1, b1, c2, d2), (a1, b1, c2, d3), (a1, b1, c2, d4), (a1, b1, c2, d5), (a1, b1, c2, d6), (a1, b1, c2, d7), (a1, b1, c2, d8), (a1, b1, c2, d9), (a1, b1, c2, d10), (a1, b1, c2, d11), (a1, b1, c2, d12), (a1, b1, c2, d13), (a1, b1, c2, d14), (a1, b1, c2, d15), (a1, b1, c2, d16), (a1, b1, c3, d1), (a1, b1, c3, d2), (a1, b1, c3, d3), (a1, b1, c3, d4), (a1, b1, c3, d5), (a1, b1, c3, d6), (a1, b1, c3, d7), (a1, b1, c3, d8), (a1, b1, c3, d9), (a1, b1, c3, d10), (a1, b1, c3, d11), (a1, b1, c3, d12), (a1, b1, c3, d13), (a1, b1, c3, d14), (a1, b1, c3, d15), (a1, b1, c3, d16), (a1, b2, c1, d1), (a1, b2, c1, d2), (a1, b2, c1, d3), (a1, b2, c1, d4), (a1, b2, c1, d5), (a1, b2, c1, d6), (a1, b2, c1, d7), (a1, b2, c1 d8), (a1, b2, c1, d9), (a1, b2, c1, d10), (a1, b2, c1, d11), (a1, b2, c1, d12), (a1, b2, c1, d13), (a1, b2, c1, d14), (a1, b2, c1, d15), (a1, b2, c1, d16), (a1, b2, c2, d1), (a1, b2, c2, d2), (a1, b2, c2, d3), (a1, b2, c2, d4), (a1, b2, c2, d5), (a1, b2, c2, d6), (a1, b2, c2, d7), (a1, b2, c2, d8), (a1, b2, c2, d9), (a1, b2, c2, d10), (a1, b2, c2, d11), (a1, b2, c2, d12), (a1, b2, c2, d13), (a1, b2, c2, d14), (a1, b2, c2, d15), (a1, b2, c2, d16), (a1, b2, c3, d1), (a1, b2, c3, d2), (a1, b2, c3, d3), (a1, b2, c3, d4), (a1, b2, c3, d5), (a1, b2, c3, d6), (a1, b2, c3, d7), (a1, b2, c3, d8), (a1, b2, c3, d9), (a1, b2, c3, d10), (a1, b2, c3, d11), (a1, b2, c3, d12), (a1, b2, c3, d13), (a1, b2, c3, d14), (a1, b2, c3, d15), (a1, b2, c3, d16), (a1, b3, c1, d1), (a1, b3, c1, d2), (a1, b3, c1, d3), (a1, b3, c1, d4), (a1, b3, c1, d5), (a1, b3, c1, d6), (a1, b3, c1, d7), (a1, b3, c1, d8), (a1, b3, c1, d9), (a1, b3, c1, d10), (a1, b3, c1, d11), (a1, b3, c1, d12), (a1, b3, c1, d13), (a1, b3, c1, d14), (a1, b3, c1, d15), (a1, b3, c1, d16), (a1, b3, c2, d1), (a1, b3, c2, d2), (a1, b3, c2, d3), (a1, b3, c2, d4), (a1, b3, c2, d5), (a1, b3, c2, d6), (a1, b3, c2, d7), (a1, b3, c2, d8), (a1, b3, c2, d9), (a1, b3, c2, d10), (a1, b3, c2, d11),

(a1, b3, c2, d12), (a1, b3, c2, d13), (a1, b3, c2, d14), (a1, b3, c2, d15), (a1, b3, c2, d16), (a1, b3, c3, d1), (a1, b3, c3, d2), (a1, b3, c3, d3), (a1, b3, c3, d4), (a1, b3, c3, d5), (a1, b3, c3, d6), (a1, b3, c3, d7), (a1, b3, c3, d8), (a1, b3, c3, d9), (a1, b3, c3, d10), (a1, b3, c3, d11), (a1, b3, c3, d12), (a1, b3, c3, d13), (a1, b3, c3, d14), (a1, b3, c3, d15), (a1, b3, c3, d16), (a1, b4, c1, d1), (a1, b4, c1, d2), (a1, b4, c1, d3), (a1, b4, c1, d4), (a1, b4, c1, d5), (a1, b4, c1, d6), (a1, b4, c1, d7), (a1, b4, c1, d8), (a1, b4, c1, d9), (a1, b4, c1, d10), (a1, b4, c1, d11), (a1, b4, c1, d12), (a1, b4, c1, d13), (a1, b4, c1, d14), (a1, b4, c1, d15), (a1, b4, c1, d16), (a1, b4, c2, d1), (a1, b4, c2, d2), (a1, b4, c2, d3), (a1, b4, c2, d4), (a1, b4, c2, d5), (a1, b4, c2, d6), (a1, b4, c2, d7), (a1, b4, c2, d8), (a1, b4, c2, d9), (a1, b4, c2, d10), (a1, b4, c2, d11), (a1, b4, c2, d12), (a1, b4, c2, d13), (a1, b4, c2, d14), (a1, b4, c2, d15), (a1, b4, c2, d16), (a1, b4, c3, d1), (a1, b4, c3, d2), (a1, b4, c3, d3), (a1, b4, c3, d4), (a1, b4, c3, d5), (a1, b4, c3, d6), (a1, b4, c3, d7), (a1, b4, c3, d8), (a1, b4, c3, d9), (a1, b4, c3, d10), (a1, b4, c3, d11), (a1, b4, c3, d12), (a1, b4, c3, d13), (a1, b4, c3, d14), (a1, b4, c3, d15), (a1, b4, c3, d16),

(a2, b1, c1, d1), (a2, b1, c1, d2), (a2, b1, c1, d3), (a2, b1, c1, d4), (a2, b1, c1, d5), (a2, b1, c1, d6), (a2, b1, c1, d7), (a2, b1, c1, d8), (a2, b1, c1, d9), (a2, b1, c1, d10), (a2, b1, c1, d11), (a2, b1, c1, d12), (a2, b1, c1, d13), (a2, b1, c1, d14), (a2, b1, c1, d15), (a2, b1, c1, d16), (a2, b1, c2, d1), (a2, b1, c2, d2), (a2, b1, c2, d3), (a2, b1, c2, d4), (a2, b1, c2, d5), (a2, b1, c2, d6), (a2, b1, c2, d7), (a2, b1, c2, d8), (a2, b1, c2, d9), (a2, b1, c2, d10), (a2, b1, c2, d11), (a2, b1, c2, d12), (a2, b1, c2, d13), (a2, b1, c2, d14), (a2, b1, c2, d15), (a2, b1, c2, d16), (a2, b1, c3, d1), (a2, b1, c3, d2), (a2, b1, c3, d3), (a2, b1, c3, d4), (a2, b1, c3, d5), (a2, b1, c3, d6), (a2, b1, c3, d7), (a2, b1, c3, d8), (a2, b1, c3, d9), (a2, b1, c3, d10), (a2, b1, c3, d11), (a2, b1, c3, d12), (a2, b1, c3, d13), (a2, b1, c3, d14), (a2, b1, c3, d15), (a2, b1, c3, d16), (a2, b2, c1, d1), (a2, b2, c1, d2), (a2, b2, c1, d3), (a2, b2, c1, d4), (a2, b2, c1, d5), (a2, b2, c1, d6), (a2, b2, c1, d7), (a2, b2, c1, d8), (a2, b2, c1, d9), (a2, b2, c1, d10), (a2, b2, c1, d11), (a2, b2, c1, d12), (a2, b2, c1, d13), (a2, b2, c1, d14), (a2, b2, c1, d15), (a2, b2, c1, d16), (a2, b2, c2, d1), (a2, b2, c2, d2), (a2, b2, c2, d3), (a2, b2, c2, d4), (a2, b2, c2, d5), (a2, b2, c2, d6), (a2, b2, c2, d7), (a2, b2, c2, d8), (a2, b2, c2, d9), (a2, b2, c2, d10), (a2, b2, c2, d11), (a2, b2, c2, d12), (a2, b2, c2, d13), (a2, b2, c2, d14), (a2, b2, c2, d15), (a2, b2, c2, d16), (a2, b2, c3, d1), (a2, b2, c3, d2), (a2, b2, c3, d3), (a2, b2, c3, d4), (a2, b2, c3, d5), (a2, b2, c3, d6), (a2, b2, c3, d7), (a2, b2, c3, d8), (a2, b2, c3, d9), (a2, b2, c3, d10), (a2, b2, c3, d11), (a2, b2, c3, d12), (a2, b2, c3, d13), (a2, b2, c3, d14), (a2, b2, c3, d15), (a2, b2, c3, d16), (a2, b3, c1, d1), (a2, b3, c1, d2), (a2, b3, c1, d3), (a2, b3, c1, d4), (a2, b3, c1, d5), (a2, b3, c1, d6), (a2, b3, c1, d7), (a2, b3, c1, d8), (a2, b3, c1, d9), (a2, b3, c1, d10), (a2, b3, c1, d11), (a2, b3, c1, d12), (a2, b3, c1, d13), (a2, b3, c1, d14), (a2, b3, c1, d15), (a2, b3, c1, d16), (a2, b3, c2, d1), (a2, b3, c2, d2), (a2, b3, c2, d3), (a2, b3, c2, d4), (a2, b3, c2, d5), (a2, b3, c2, d6), (a2, b3, c2, d7), (a2, b3, c2, d8), (a2, b3, c2, d9), (a2, b3, c2, d10), (a2, b3, c2, d11), (a2, b3, c2, d12), (a2, b3, c2, d13), (a2, b3, c2, d14), (a2, b3, c2, d15), (a2, b3, c2, d16), (a2, b3, c3, d1), (a2, b3, c3, d2), (a2, b3, c3, d3), (a2, b3, c3, d4), (a2, b3, c3, d5), (a2, b3, c3, d6), (a2, b3, c3, d7), (a2, b3, c3, d8), (a2, b3, c3, d9), (a2, b3, c3, d10), (a2, b3, c3, d11), (a2, b3, c3, d12), (a2, b3, c3, d13), (a2, b3, c3, d14), (a2, b3, c3, d15), (a2, b3, c3, d16), (a2, b4, c1, d1), (a2, b4, c1, d2), (a2, b4, c1, d3), (a2, b4, c1, d4), (a2, b4, c1, d5), (a2, b4, c1, d6), (a2, b4, c1, d7), (a2, b4, c1, d8), (a2, b4, c1, d9), (a2, b4, c1, d10), (a2, b4, c1, d11), (a2, b4, c1, d12), (a2, b4, c1, d13), (a2, b4, c1, d14), (a2, b4, c1, d15), (a2, b4, c1, d16), (a2, b4, c2, d1), (a2, b4, c2, d2), (a2, b4, c2, d3), (a2, b4, c2, d4), (a2, b4, c2, d5), (a2, b4, c2, d6), (a2, b4, c2, d7), (a2, b4, c2, d8), (a2, b4, c2, d9), (a2, b4, c2, d10), (a2, b4, c2, d11), (a2, b4, c2, d12), (a2, b4, c2, d13), (a2, b4, c2, d14), (a2, b4, c2, d15), (a2, b4, c2, d16), (a2, b4, c3, d1), (a2, b4, c3, d2), (a2, b4, c3, d3), (a2, b4, c3, d4), (a2, b4, c3, d5), (a2, b4, c3, d6), (a2, b4, c3, d7), (a2, b4, c3, d8), (a2, b4, c3, d9), (a2, b4, c3, d10), (a2, b4, c3, d11), (a2, b4, c3, d12), (a2, b4, c3, d13), (a2, b4, c3, d14), (a2, b4, c3, d15), (a2, b4, c3, d16),

(a3, b1, c1, d1), (a3, b1, c1, d2), (a3, b1, c1, d3), (a3, b1, c1, d4), (a3, b1, c1, d5), (a3, b1, c1, d6), (a3, b1, c1, d7), (a3, b1, c1, d8), (a3, b1, c1, d9), (a3, b1, c1, d10), (a3, b1, c1, d11), (a3, b1, c1, d12), (a3, b1, c1, d13), (a3, b1, c1, d14), (a3, b1, c1, d15), (a3, b1, c1, d16), (a3, b1, c2, d1), (a3, b1, c2, d2), (a3, b1, c2, d3), (a3, b1, c2, d4), (a3, b1, c2, d5), (a3, b1, c2, d6), (a3, b1, c2, d7), (a3, b1, c2, d8), (a3, b1, c2, d9), (a3, b1, c2, d10), (a3, b1, c2, d11), (a3, b1, c2, d12), (a3, b1, c2, d13), (a3, b1, c2, d14), (a3, b1, c2, d15), (a3, b1, c2, d16), (a3, b1, c3, d1), (a3, b1, c3, d2), (a3, b1, c3, d3), (a3, b1, c3, d4), (a3, b1, c3, d5), (a3, b1, c3, d6), (a3, b1, c3, d7), (a3, b1, c3, d8), (a3, b1, c3, d9), (a3, b1, c3, d10), (a3, b1, c3, d11), (a3, b1, c3, d12), (a3, b1, c3, d13), (a3, b1, c3, d14), (a3, b1, c3, d15), (a3, b1, c3, d16), (a3, b2, c1, d1), (a3, b2, c1, d2), (a3, b2, c1, d3), (a3, b2, c1, d4), (a3, b2, c1, d5), (a3, b2, c1, d6), (a3, b2, c1, d7), (a3, b2, c1, d8), (a3, b2, c1, d9), (a3, b2, c1, d10), (a3, b2, c1, d11), (a3, b2, c1, d12), (a3, b2, c1, d13), (a3, b2, c1, d14), (a3, b2, c1, d15), (a3, b2, c1, d16), (a3, b2, c2, d1), (a3, b2, c2, d2), (a3, b2, c2, d3), (a3, b2, c2, d4), (a3, b2, c2, d5), (a3, b2, c2, d6), (a3, b2, c2, d7), (a3, b2, c2, d8), (a3, b2, c2, d9), (a3, b2, c2, d10), (a3, b2, c2, d11), (a3, b2, c2, d12), (a3, b2, c2, d13), (a3, b2, c2, d14), (a3, b2, c2, d15), (a3, b2, c2, d16), (a3, b2, c3, d1), (a3, b2, c3, d2), (a3, b2, c3, d3), (a3, b2, c3, d4), (a3, b2, c3, d5), (a3, b2, c3, d6), (a3, b2, c3, d7), (a3, b2, c3, d8), (a3, b2, c3, d9), (a3, b2, c3, d10), (a3, b2, c3, d11), (a3, b2, c3, d12), (a3, b2, c3, d13), (a3, b2, c3, d14), (a3, b2, c3, d15), (a3, b2, c3, d16), (a3, b3, c1, d1), (a3, b3, c1, d2), (a3, b3, c1, d3), (a3, b3, c1, d4), (a3, b3, c1, d5), (a3, b3, c1, d6), (a3, b3, c1, d7), (a3, b3, c1, d8), (a3, b3, c1, d9), (a3, b3, c1, d10), (a3, b3, c1, d11), (a3, b3, c1, d12), (a3, b3, c1, d13), (a3, b3, c1, d14), (a3, b3, c1, d15), (a3, b3, c1, d16), (a3, b3, c2, d1), (a3, b3, c2, d2), (a3, b3, c2, d3), (a3, b3, c2, d4), (a3, b3, c2, d5), (a3, b3, c2, d6), (a3, b3, c2, d7), (a3, b3, c2, d8), (a3, b3, c2, d9), (a3, b3, c2, d10), (a3, b3, c2, d11), (a3, b3, c2, d12), (a3, b3, c2, d13), (a3, b3, c2, d14), (a3, b3, c2, d15), (a3, b3, c2, d16), (a3, b3, c3, d1), (a3, b3, c3, d2), (a3, b3, c3, d3), (a3, b3, c3, d4),

(a3, b3, c3, d5), (a3, b3, c3, d6), (a3, b3, c3, d7), (a3, b3, c3, d8), (a3, b3, c3, d9), (a3, b3, c3, d10), (a3, b3, c3, d11), (a3, b3, c3, d12), (a3, b3, c3, d13), (a3, b3, c3, d14), (a3, b3, c3, d15), (a3, b3, c3, d16), (a3, b4, c1, d1), (a3, b4, c1, d2), (a3, b4, c1, d3), (a3, b4, c1, d4), (a3, b4, c1, d5), (a3, b4, c1, d6), (a3, b4, c1, d7), (a3, b4, c1, d8), (a3, b4, c1, d9), (a3, b4, c1, d10), (a3, b4, c1, d11), (a3, b4, c1, d12), (a3, b4, c1, d13), (a3, b4, c1, d14), (a3, b4, c1, d15), (a3, b4, c1, d16), (a3, b4, c2, d1), (a3, b4, c2, d2), (a3, b4, c2, d3), (a3, b4, c2, d4), (a3, b4, c2, d5), (a3, b4, c2, d6), (a3, b4, c2, d7), (a3, b4, c2, d8), (a3, b4, c2, d9), (a3, b4, c2, d10), (a3, b4, c2, d11), (a3, b4, c2, d12), (a3, b4, c2, d13), (a3, b4, c2, d14), (a3, b4, c2, d15), (a3, b4, c2, d16), (a3, b4, c3, d1), (a3, b4, c3, d2), (a3, b4, c3, d3), (a3, b4, c3, d4), (a3, b4, c3, d5), (a3, b4, c3, d6), (a3, b4, c3, d7), (a3, b4, c3, d8), (a3, b4, c3, d9), (a3, b4, c3, d10), (a3, b4, c3, d11), (a3, b4, c3, d12), (a3, b4, c3, d13), (a3, b4, c3, d14), (a3, b4, c3, d15), (a3, b4, c3, d16),

(a4, b1, c1, d1), (a4, b1, c1, d2), (a4, b1, c1, d3), (a4, b1, c1, d4), (a4, b1, c1, d5), (a4, b1, c1, d6), (a4, b1, c1, d7), (a4, b1, c1, d8), (a4, b1, c1, d9), (a4, b1, c1, d10), (a4, b1, c1, d11), (a4, b1, c1, d12), (a4, b1, c1, d13), (a4, b1, c1, d14), (a4, b1, c1, d15), (a4, b1, c1, d16), (a4, b1, c2, d1), (a4, b1, c2, d2), (a4, b1, c2, d3), (a4, b1, c2, d4), (a4, b1, c2, d5), (a4, b1, c2, d6), (a4, b1, c2, d7), (a4, b1, c2, d8), (a4, b1, c2, d9), (a4, b1, c2, d10), (a4, b1, c2, d11), (a4, b1, c2, d12), (a4, b1, c2, d13), (a4, b1, c2, d14), (a4, b1, c2, d15), (a4, b1, c2, d16), (a4, b1, c3, d1), (a4, b1, c3, d2), (a4, b1, c3, d3), (a4, b1, c3, d4), (a4, b1, c3, d5), (a4, b1, c3, d6), (a4, b1, c3, d7), (a4, b1, c3, d8), (a4, b1, c3, d9), (a4, b1, c3, d10), (a4, b1, c3, d11), (a4, b1, c3, d12), (a4, b1, c3, d13), (a4, b1, c3, d14), (a4, b1, c3, d15), (a4, b1, c3, d16), (a4, b2, c1, d1), (a4, b2, c1, d2), (a4, b2, c1, d3), (a4, b2, c1, d4), (a4, b2, c1, d5), (a4, b2, c1, d6), (a4, b2, c1, d7), (a4, b2, c1, d8), (a4, b2, c1, d9), (a4, b2, c1, d10), (a4, b2, c1, d11), (a4, b2, c1, d12), (a4, b2, c1, d13), (a4, b2, c1, d14), (a4, b2, c1, d15), (a4, b2, c1, d16), (a4, b2, c2, d1), (a4, b2, c2, d2), (a4, b2, c2, d3), (a4, b2, c2, d4), (a4, b2, c2, d5), (a4, b2, c2, d6), (a4, b2, c2, d7), (a4, b2, c2, d8), (a4, b2, c2, d9), (a4, b2, c2, d10), (a4, b2, c2, d11), (a4, b2, c2, d12), (a4, b2, c2, d13), (a4, b2, c2, d14), (a4, b2, c2, d15), (a4, b2, c2, d16), (a4, b2, c3, d1), (a4, b2, c3, d2), (a4, b2, c3, d3), (a4, b2, c3, d4), (a4, b2, c3, d5), (a4, b2, c3, d6), (a4, b2, c3, d7), (a4, b2, c3, d8), (a4, b2, c3, d9), (a4, b2, c3, d10), (a4, b2, c3, d11), (a4, b2, c3, d12), (a4, b2, c3, d13), (a4, b2, c3, d14), (a4, b2, c3, d15), (a4, b2, c3, d16), (a4, b3, c1, d1), (a4, b3, c1, d2), (a4, b3, c1, d3), (a4, b3, c1, d4), (a4, b3, c1, d5), (a4, b3, c1, d6), (a4, b3, c1, d7), (a4, b3, c1, d8), (a4, b3, c1, d9), (a4, b3, c1, d10), (a4, b3, c1, d11), (a4, b3, c1, d12), (a4, b3, c1, d13), (a4, b3, c1, d14), (a4, b3, c1, d15), (a4, b3, c1, d16), (a4, b3, c2, d1), (a4, b3, c2, d2), (a4, b3, c2, d3), (a4, b3, c2, d4), (a4, b3, c2, d5), (a4, b3, c2, d6), (a4, b3, c2, d7), (a4, b3, c2, d8), (a4, b3, c2, d9), (a4, b3, c2, d10), (a4, b3, c2, d11), (a4, b3, c2, d12), (a4, b3, c2, d13), (a4, b3, c2, d14), (a4, b3, c2, d15), (a4, b3, c2, d16), (a4, b3, c3, d1), (a4, b3, c3, d2), (a4, b3, c3, d3), (a4, b3, c3, d4), (a4, b3, c3, d5), (a4, b3, c3, d6), (a4, b3, c3, d7), (a4, b3, c3, d8), (a4, b3, c3, d9), (a4, b3, c3, d10), (a4, b3, c3, d11), (a4, b3, c3, d12), (a4, b3, c3, d13), (a4, b3, c3, d14), (a4, b3, c3, d15), (a4, b3, c3, d16), (a4, b4, c1, d1), (a4, b4, c1, d2), (a4, b4, c1, d3), (a4, b4, c1, d4), (a4, b4, c1, d5), (a4, b4, c1, d6), (a4, b4, c1, d7), (a4, b4, c1, d8), (a4, b4, c1, d9), (a4, b4, c1, d10), (a4, b4, c1, d11), (a4, b4, c1, d12), (a4, b4, c1, d13), (a4, b4, c1, d14), (a4, b4, c1, d15), (a4, b4, c1, d16), (a4, b4, c2, d1), (a4, b4, c2, d2), (a4, b4, c2, d3), (a4, b4, c2, d4), (a4, b4, c2, d5), (a4, b4, c2, d6), (a4, b4, c2, d7), (a4, b4, c2, d8), (a4, b4, c2, d9), (a4, b4, c2, d10), (a4, b4, c2, d11), (a4, b4, c2, d12), (a4, b4, c2, d13), (a4, b4, c2, d14), (a4, b4, c2, d15), (a4, b4, c2, d16), (a4, b4, c3, d1), (a4, b4, c3, d2), (a4, b4, c3, d3), (a4, b4, c3, d4), (a4, b4, c3, d5), (a4, b4, c3, d6), (a4, b4, c3, d7), (a4, b4, c3, d8), (a4, b4, c3, d9), (a4, b4, c3, d10), (a4, b4, c3, d11), (a4, b4, c3, d12), (a4, b4, c3, d13), (a4, b4, c3, d14), (a4, b4, c3, d15), (a4, b4, c3, d16),

(a5, b1, c1, d1), (a5, b1, c1, d2), (a5, b1, c1, d3), (a5, b1, c1, d4), (a5, b1, c1, d5), (a5, b1, c1, d6), (a5, b1, c1, d7), (a5, b1, c1, d8), (a5, b1, c1, d9), (a5, b1, c1, d10), (a5, b1, c1, d11), (a5, b1, c1, d12), (a5, b1, c1, d13), (a5, b1, c1, d14), (a5, b1, c1, d15), (a5, b1, c1, d16), (a5, b1, c2, d1), (a5, b1, c2, d2), (a5, b1, c2, d3), (a5, b1, c2, d4), (a5, b1, c2, d5), (a5, b1, c2, d6), (a5, b1, c2, d7), (a5, b1, c2, d8), (a5, b1, c2, d9), (a5, b1, c2, d10), (a5, b1, c2, d11), (a5, b1, c2, d12), (a5, b1, c2, d13), (a5, b1, c2, d14), (a5, b1, c2, d15), (a5, b1, c2, d16), (a5, b1, c3, d1), (a5, b1, c3, d2), (a5, b1, c3, d3), (a5, b1, c3, d4), (a5, b1, c3, d5), (a5, b1, c3, d6), (a5, b1, c3, d7), (a5, b1, c3, d8), (a5, b1, c3, d9), (a5, b1, c3, d10), (a5, b1, c3, d11), (a5, b1, c3, d12), (a5, b1, c3, d13), (a5, b1, c3, d14), (a5, b1, c3, d15), (a5, b1, c3, d16), (a5, b2, c1, d1), (a5, b2, c1, d2), (a5, b2, c1, d3), (a5, b2, c1, d4), (a5, b2, c1, d5), (a5, b2, c1, d6), (a5, b2, c1, d7), (a5, b2, c1, d8), (a5, b2, c1, d9), (a5, b2, c1, d10), (a5, b2, c1, d11), (a5, b2, c1, d12), (a5, b2, c1, d13), (a5, b2, c1, d14), (a5, b2, c1, d15), (a5, b2, c1, d16), (a5, b2, c2, d1), (a5, b2, c2, d2), (a5, b2, c2, d3), (a5, b2, c2, d4), (a5, b2, c2, d5), (a5, b2, c2, d6), (a5, b2, c2, d7), (a5, b2, c2, d8), (a5, b2, c2, d9), (a5, b2, c2, d10), (a5, b2, c2, d11), (a5, b2, c2, d12), (a5, b2, c2, d13), (a5, b2, c2, d14), (a5, b2, c2, d15), (a5, b2, c2, d16), (a5, b2, c3, d1), (a5, b2, c3, d2), (a5, b2, c3, d3), (a5, b2, c3, d4), (a5, b2, c3, d5), (a5, b2, c3, d6), (a5, b2, c3, d7), (a5, b2, c3, d8), (a5, b2, c3, d9), (a5, b2, c3, d10), (a5, b2, c3, d11), (a5, b2, c3, d12), (a5, b2, c3, d13), (a5, b2, c3, d14), (a5, b2, c3, d15), (a5, b2, c3, d16), (a5, b3, c1, d1), (a5, b3, c1, d2), (a5, b3, c1, d3), (a5, b3, c1, d4), (a5, b3, c1, d5), (a5, b3, c1, d6), (a5, b3, c1, d7), (a5, b3, c1, d8), (a5, b3, c1, d9), (a5, b3, c1, d10), (a5, b3, c1, d11), (a5, b3, c1, d12), (a5, b3, c1, d13), (a5, b3, c1, d14), (a5, b3, c1, d15), (a5, b3, c1, d16), (a5, b3, c2, d1), (a5, b3, c2, d2), (a5, b3, c2, d3), (a5, b3, c2, d4), (a5, b3, c2, d5), (a5, b3, c2, d6), (a5, b3, c2, d7), (a5, b3, c2, d8), (a5, b3, c2, d9), (a5, b3, c2, d10), (a5, b3, c2, d11), (a5, b3, c2, d12), (a5, b3, c2, d13), (a5, b3, c2, d14), (a5, b3, c2, d15), (a5, b3, c2, d16), (a5, b3, c3, d1), (a5, b3, c3, d2), (a5, b3, c3, d3), (a5, b3, c3, d4), (a5, b3, c3, d5), (a5, b3, c3, d6), (a5, b3, c3, d7), (a5, b3, c3, d8), (a5, b3, c3, d9), (a5, b3, c3, d10), (a5, b3, c3, d11), (a5, b3, c3, d12), (a5, b3, c3, d13), (a5, b3, c3, d14), (a5, b3, c3, d15), (a5, b3, c3, d16), (a5, b4, c1, d1), (a5, b4, c1,

d2), (a5, b4, c1, d3), (a5, b4, c1, d4), (a5, b4, c1, d5), (a5, b4, c1, d6), (a5, b4, c1, d7), (a5, b4, c1, d8), (a5, b4, c1, d9), (a5, b4, c1, d10), (a5, b4, c1, d11), (a5, b4, c1, d12), (a5, b4, c1, d13), (a5, b4, c1, d14), (a5, b4, c1, d15), (a5, b4, c1, d16), (a5, b4, c2, d1), (a5, b4, c2, d2), (a5, b4, c2, d3), (a5, b4, c2, d4), (a5, b4, c2, d5), (a5, b4, c2, d6), (a5, b4, c2, d7), (a5, b4, c2, d8), (a5, b4, c2, d9), (a5, b4, c2, d10), (a5, b4, c2, d11), (a5, b4, c2, d12), (a5, b4, c2, d13), (a5, b4, c2, d14), (a5, b4, c2, d15), (a5, b4, c2, d16), (a5, b4, c3, d1), (a5, b4, c3, d2), (a5, b4, c3, d3), (a5, b4, c3, d4), (a5, b4, c3, d5), (a5, b4, c3, d6), (a5, b4, c3, d7), (a5, b4, c3, d8), (a5, b4, c3, d9), (a5, b4, c3, d10), (a5, b4, c3, d11), (a5, b4, c3, d12), (a5, b4, c3, d13), (a5, b4, c3, d14), (a5, b4, c3, d15), (a5, b4, c3, d16),

(a6, b1, c1, d1), (a6, b1, c1, d2), (a6, b1, c1, d3), (a6, b1, c1, d4), (a6, b1, c1, d5), (a6, b1, c1, d6), (a6, b1, c1, d7), (a6, b1, c1, d8), (a6, b1, c1, d9), (a6, b1, c1, d10), (a6, b1, c1, d11), (a6, b1, c1, d12), (a6, b1, c1, d13), (a6, b1, c1, d14), (a6, b1, c1, d15), (a6, b1, c1, d16), (a6, b1, c2, d1), (a6, b1, c2, d2), (a6, b1, c2, d3), (a6, b1, c2, d4), (a6, b1, c2, d5), (a6, b1, c2, d6), (a6, b1, c2, d7), (a6, b1, c2, d8), (a6, b1, c2, d9), (a6, b1, c2, d10), (a6, b1, c2, d11), (a6, b1, c2, d12), (a6, b1, c2, d13), (a6, b1, c2, d14), (a6, b1, c2, d15), (a6, b1, c2, d16), (a6, b1, c3, d1), (a6, b1, c3, d2), (a6, b1, c3, d3), (a6, b1, c3, d4), (a6, b1, c3, d5), (a6, b1, c3, d6), (a6, b1, c3, d7), (a6, b1, c3, d8), (a6, b1, c3, d9), (a6, b1, c3, d10), (a6, b1, c3, d11), (a6, b1, c3, d12), (a6, b1, c3, d13), (a6, b1, c3, d14), (a6, b1, c3, d15), (a6, b1, c3, d16), (a6, b2, c1, d1), (a6, b2, c1, d2), (a6, b2, c1, d3), (a6, b2, c1, d4), (a6, b2, c1, d5), (a6, b2, c1, d6), (a6, b2, c1, d7), (a6, b2, c1, d8), (a6, b2, c1, d9), (a6, b2, c1, d10), (a6, b2, c1, d11), (a6, b2, c1, d12), (a6, b2, c1, d13), (a6, b2, c1, d14), (a6, b2, c1, d15), (a6, b2, c1, d16), (a6, b2, c2, d1), (a6, b2, c2, d2), (a6, b2, c2, d3), (a6, b2, c2, d4), (a6, b2, c2, d5), (a6, b2, c2, d6), (a6, b2, c2, d7), (a6, b2, c2, d8), (a6, b2, c2, d9), (a6, b2, c2, d10), (a6, b2, c2, d11), (a6, b2, c2, d12), (a6, b2, c2, d13), (a6, b2, c2, d14), (a6, b2, c2, d15), (a6, b2, c2, d16), (a6, b2, c3, d1), (a6, b2, c3, d2), (a6, b2, c3, d3), (a6, b2, c3, d4), (a6, b2, c3, d5), (a6, b2, c3, d6), (a6, b2, c3, d7), (a6, b2, c3, d8), (a6, b2, c3, d9), (a6, b2, c3, d10), (a6, b2, c3, d11), (a6, b2, c3, d12), (a6, b2, c3, d13), (a6, b2, c3, d14), (a6, b2, c3, d15), (a6, b2, c3, d16), (a6, b3, c1, d1), (a6, b3, c1, d2), (a6, b3, c1, d3), (a6, b3, c1, d4), (a6, b3, c1, d5), (a6, b3, c1, d6), (a6, b3, c1, d7), (a6, b3, c1, d8), (a6, b3, c1, d9), (a6, b3, c1, d10), (a6, b3, c1, d11), (a6, b3, c1, d12), (a6, b3, c1, d13), (a6, b3, c1, d14), (a6, b3, c1, d15), (a6, b3, c1, d16), (a6, b3, c2, d1), (a6, b3, c2, d2), (a6, b3, c2, d3), (a6, b3, c2, d4), (a6, b3, c2, d5), (a6, b3, c2, d6), (a6, b3, c2, d7), (a6, b3, c2, d8), (a6, b3, c2, d9), (a6, b3, c2, d10), (a6, b3, c2, d11), (a6, b3, c2, d12), (a6, b3, c2, d13), (a6, b3, c2, d14), (a6, b3, c2, d15), (a6, b3, c2, d16), (a6, b3, c3, d1), (a6, b3, c3, d2), (a6, b3, c3, d3), (a6, b3, c3, d4), (a6, b3, c3, d5), (a6, b3, c3, d6), (a6, b3, c3, d7), (a6, b3, c3, d8), (a6, b3, c3, d9), (a6, b3, c3, d10), (a6, b3, c3, d11), (a6, b3, c3, d12), (a6, b3, c3, d13), (a6, b3, c3, d14), (a6, b3, c3, d15), (a6, b3, c3, d16), (a6, b4, c1, d1), (a6, b4, c1, d2), (a6, b4, c1, d3), (a6, b4, c1, d4), (a6, b4, c1, d5), (a6, b4, c1, d6), (a6, b4, c1, d7), (a6, b4, c1, d8), (a6, b4, c1, d9), (a6, b4, c1, d10), (a6, b4, c1, d11), (a6, b4, c1, d12), (a6, b4, c1, d13), (a6, b4, c1, d14), (a6, b4, c1, d15), (a6, b4, c1, d16), (a6, b4, c2, d1), (a6, b4, c2, d2), (a6, b4, c2, d3), (a6, b4, c2, d4), (a6, b4, c2, d5), (a6, b4, c2, d6), (a6, b4, c2, d7), (a6; b4, c2, d8), (a6, b4, c2, d9), (a6, b4, c2, d10), (a6, b4, c2, d11), (a6, b4, c2, d12), (a6, b4, c2, d13), (a6, b4, c2, d14), (a6, b4, c2, d15), (a6, b4, c2, d16), (a6, b4, c3, d1), (a6, b4, c3, d2), (a6, b4, c3, d3), (a6, b4, c3, d4), (a6, b4, c3, d5), (a6, b4, c3, d6), (a6, b4, c3, d7), (a6, b4, c3, d8), (a6, b4, c3, d9), (a6, b4, c3, d10), (a6, b4, c3, d11), (a6, b4, c3, d12), (a6, b4, c3, d13), (a6, b4, c3, d14), (a6, b4, c3, d15), (a6, b4, c3, d16),

(a7, b1, c1, d1), (a7, b1, c1, d2), (a7, b1, c1, d3), (a7, b1, c1, d4), (a7, b1, c1, d5), (a7, b1, c1, d6), (a7, b1, c1, d7), (a7, b1, c1, d8), (a7, b1, c1, d9), (a7, b1, c1, d10), (a7, b1, c1, d11), (a7, b1, c1, d12), (a7, b1, c1, d13), (a7, b1, c1, d14), (a7, b1, c1, d15), (a7, b1, c1, d16), (a7, b1, c2, d1), (a7, b1, c2, d2), (a7, b1, c2, d3), (a7, b1, c2, d4), (a7, b1, c2, d5), (a7, b1, c2, d6), (a7, b1, c2, d7), (a7, b1, c2, d8), (a7, b1, c2, d9), (a7, b1, c2, d10), (a7, b1, c2, d11), (a7, b1, c2, d12), (a7, b1, c2, d13), (a7, b1, c2, d14), (a7, b1, c2, d15), (a7, b1, c2, d16), (a7, b1, c3, d1), (a7, b1, c3, d2), (a7, b1, c3, d3), (a7, b1, c3, d4), (a7, b1, c3, d5), (a7, b1, c3, d6), (a7, b1, c3, d7), (a7, b1, c3, d8), (a7, b1, c3, d9), (a7, b1, c3, d10), (a7, b1, c3, d11), (a7, b1, c3, d12), (a7, b1, c3, d13), (a7, b1, c3, d14), (a7, b1, c3, d15), (a7, b1, c3, d16), (a7, b2, c1, d1), (a7, b2, c1, d2), (a7, b2, c1, d3), (a7, b2, c1, d4), (a7, b2, c1, d5), (a7, b2, c1, d6), (a7, b2, c1, d7), (a7, b2, c1, d8), (a7, b2, c1, d9), (a7, b2, c1, d10), (a7, b2, c1, d11), (a7, b2, c1, d12), (a7, b2, c1, d13), (a7, b2, c1, d14), (a7, b2, c1, d15), (a7, b2, c1, d16), (a7, b2, c2, d1), (a7, b2, c2, d2), (a7, b2, c2, d3), (a7, b2, c2, d4), (a7, b2, c2, d5), (a7, b2, c2, d6), (a7, b2, c2, d7), (a7, b2, c2, d8), (a7, b2, c2, d9), (a7, b2, c2, d10), (a7, b2, c2, d11), (a7, b2, c2, d12), (a7, b2, c2, d13), (a7, b2, c2, d14), (a7, b2, c2, d15), (a7, b2, c2, d16), (a7, b2, c3, d1), (a7, b2, c3, d2), (a7, b2, c3, d3), (a7, b2, c3, d4), (a7, b2, c3, d5), (a7, b2, c3, d6), (a7, b2, c3, d7), (a7, b2, c3, d8), (a7, b2, c3, d9), (a7, b2, c3, d10), (a7, b2, c3, d11), (a7, b2, c3, d12), (a7, b2, c3, d13), (a7, b2, c3, d14), (a7, b2, c3, d15), (a7, b2, c3, d16), (a7, b3, c1, d1), (a7, b3, c1, d2), (a7, b3, c1, d3), (a7, b3, c1, d4), (a7, b3, c1, d5), (a7, b3, c1, d6), (a7, b3, c1, d7), (a7, b3, c1, d8), (a7, b3, c1, d9), (a7, b3, c1, d10), (a7, b3, c1, d11), (a7, b3, c1, d12), (a7, b3, c1, d13), (a7, b3, c1, d14), (a7, b3, c1, d15), (a7, b3, c1, d16), (a7, b3, c2, d1), (a7, b3, c2, d2), (a7, b3, c2, d3), (a7, b3, c2, d4), (a7, b3, c2, d5), (a7, b3, c2, d6), (a7, b3, c2, d7), (a7, b3, c2, d8), (a7, b3, c2, d9), (a7, b3, c2, d10), (a7, b3, c2, d11), (a7, b3, c2, d12), (a7, b3, c2, d13), (a7, b3, c2, d14), (a7, b3, c2, d15), (a7, b3, c2, d16), (a7, b3, c3, d1), (a7, b3, c3, d2), (a7, b3, c3, d3), (a7, b3, c3, d4), (a7, b3, c3, d5), (a7, b3, c3, d6), (a7, b3, c3, d7), (a7, b3, c3, d8), (a7, b3, c3, d9), (a7, b3, c3, d10), (a7, b3, c3, d11), (a7, b3, c3, d12), (a7, b3, c3, d13), (a7, b3, c3, d14), (a7, b3, c3, d15), (a7, b3, c3, d16), (a7, b4, c1, d1), (a7, b4, c1, d2), (a7, b4, c1, d3), (a7, b4, c1, d4), (a7, b4, c1, d5), (a7, b4, c1, d6), (a7, b4, c1, d7), (a7, b4, c1, d8), (a7, b4, c1, d9), (a7, b4, c1, d10), (a7, b4, c1, d11), (a7, b4, c1, d12), (a7, b4, c1, d13), (a7, b4, c1, d14), (a7, b4, c1, d15), (a7, b4, c1,

d16), (a7, b4, c2, d1), (a7, b4, c2, d2), (a7, b4, c2, d3), (a7, b4, c2, d4), (a7, b4, c2, d5), (a7, b4, c2, d6), (a7, b4, c2, d7), (a7, b4, c2, d8), (a7, b4, c2, d9), (a7, b4, c2, d10), (a7, b4, c2, d11), (a7, b4, c2, d12), (a7, b4, c2, d13), (a7, b4, c2, d14), (a7, b4, c2, d15), (a7, b4, c2, d16), (a7, b4, c3, d1), (a7, b4, c3, d2), (a7, b4, c3, d3), (a7, b4, c3, d4), (a7, b4, c3, d5), (a7, b4, c3, d6), (a7, b4, c3, d7), (a7, b4, c3, d8), (a7, b4, c3, d9), (a7, b4, c3, d10), (a7, b4, c3, d11), (a7, b4, c3, d12), (a7, b4, c3, d13), (a7, b4, c3, d14), (a7, b4, c3, d15), (a7, b4, c3, d16),

(a8, b1, c1, d1), (a8, b1, c1, d2), (a8, b1, c1, d3), (a8, b1, c1, d4), (a8, b1, c1, d5), (a8, b1, c1, d6), (a8, b1, c1, d7), (a8, b1, c1, d8), (a8, b1, c1, d9), (a8, b1, c1, d10), (a8, b1, c1, d11), (a8, b1, c1, d12), (a8, b1, c1, d13), (a8, b1, c1, d14), (a8, b1, c1, d15), (a8, b1, c1, d16), (a8, b1, c2, d1), (a8, b1, c2, d2), (a8, b1, c2, d3), (a8, b1, c2, d4), (a8, b1, c2, d5), (a8, b1, c2, d6), (a8, b1, c2, d7), (a8, b1, c2, d8), (a8, b1, c2, d9), (a8, b1, c2, d10), (a8, b1, c2, d11), (a8, b1, c2, d12), (a8, b1, c2, d13), (a8, b1, c2, d14), (a8, b1, c2, d15), (a8, b1, c2, d16), (a8, b1, c3, d1), (a8, b1, c3, d2), (a8, b1, c3, d3), (a8, b1, c3, d4), (a8, b1, c3, d5), (a8, b1, c3, d6), (a8, b1, c3, d7), (a8, b1, c3, d8), (a8, b1, c3, d9), (a8, b1, c3, d10), (a8, b1, c3, d11), (a8, b1, c3, d12), (a8, b1, c3, d13), (a8, b1, c3, d14), (a8, b1, c3, d15), (a8, b1, c3, d16), (a8, b2, c1, d1), (a8, b2, c1, d2), (a8, b2, c1, d3), (a8, b2, c1, d4), (a8, b2, c1, d5), (a8, b2, c1, d6), (a8, b2, c1, d7), (a8, b2, c1, d8), (a8, b2, c1, d9), (a8, b2, c1, d10), (a8, b2, c1, d11), (a8, b2, c1, d12), (a8, b2, c1, d13), (a8, b2, c1, d14), (a8, b2, c1, d15), (a8, b2, c1, d16), (a8, b2, c2, d1), (a8, b2, c2, d2), (a8, b2, c2, d3), (a8, b2, c2, d4), (a8, b2, c2, d5), (a8, b2, c2, d6), (a8, b2, c2, d7), (a8, b2, c2, d8), (a8, b2, c2, d9), (a8, b2, c2, d10), (a8, b2, c2, d11), (a8, b2, c2, d12), (a8, b2, c2, d13), (a8, b2, c2, d14), (a8, b2, c2, d15), (a8, b2, c2, d16), (a8, b2, c3, d1), (a8, b2, c3, d2), (a8, b2, c3, d3), (a8, b2, c3, d4), (a8, b2, c3, d5), (a8, b2, c3, d6), (a8, b2, c3, d7), (a8, b2, c3, d8), (a8, b2, c3, d9), (a8, b2, c3, d10), (a8, b2, c3, d11), (a8, b2, c3, d12), (a8, b2, c3, d13), (a8, b2, c3, d14), (a8, b2, c3, d15), (a8, b2, c3, d16), (a8, b3, c1, d1), (a8, b3, c1, d2), (a8, b3, c1, d3), (a8, b3, c1, d4), (a8, b3, c1, d5), (a8, b3, c1, d6), (a8, b3, c1, d7), (a8, b3, c1, d8), (a8, b3, c1, d9), (a8, b3, c1, d10), (a8, b3, c1, d11), (a8, b3, c1, d12), (a8, b3, c1, d13), (a8, b3, c1, d14), (a8, b3, c1, d15), (a8, b3, c1, d16), (a8, b3, c2, d1), (a8, b3, c2, d2), (a8, b3, c2, d3), (a8, b3, c2, d4), (a8, b3, c2, d5), (a8, b3, c2, d6), (a8, b3, c2, d7), (a8, b3, c2, d8), (a8, b3, c2, d9), (a8, b3, c2, d10), (a8, b3, c2, d11), (a8, b3, c2, d12), (a8, b3, c2, d13), (a8, b3, c2, d14), (a8, b3, c2, d15), (a8, b3, c2, d16), (a8, b3, c3, d1), (a8, b3, c3, d2), (a8, b3, c3, d3), (a8, b3, c3, d4), (a8, b3, c3, d5), (a8, b3, c3, d6), (a8, b3, c3, d7), (a8, b3, c3, d8), (a8, b3, c3, d9), (a8, b3, c3, d10), (a8, b3, c3, d11), (a8, b3, c3, d12), (a8, b3, c3, d13), (a8, b3, c3, d14), (a8, b3, c3, d15), (a8, b3, c3, d16), (a8, b4, c1, d1), (a8, b4, c1, d2), (a8, b4, c1, d3), (a8, b4, c1, d4), (a8, b4, c1, d5), (a8, b4, c1, d6), (a8, b4, c1, d7), (a8, b4, c1, d8), (a8, b4, c1, d9), (a8, b4, c1, d10), (a8, b4, c1, d11), (a8, b4, c1, d12), (a8, b4, c1, d13), (a8, b4, c1, d14), (a8, b4, c1, d15), (a8, b4, c1, d16), (a8, b4, c2, d1), (a8, b4, c2, d2), (a8, b4, c2, d3), (a8, b4, c2, d4), (a8, b4, c2, d5), (a8, b4, c2, d6), (a8, b4, c2, d7), (a8, b4, c2, d8), (a8, b4, c2, d9), (a8, b4, c2, d10), (a8, b4, c2, d11), (a8, b4, c2, d12), (a8, b4, c2, d13), (a8, b4, c2, d14), (a8, b4, c2, d15), (a8, b4, c2, d16), (a8, b4, c3, d1), (a8, b4, c3, d2), (a8, b4, c3, d3), (a8, b4, c3, d4), (a8, b4, c3, d5), (a8, b4, c3, d6), (a8, b4, c3, d7), (a8, b4, c3, d8), (a8, b4, c3, d9), (a8, b4, c3, d10), (a8, b4, c3, d11), (a8, b4, c3, d12), (a8, b4, c3, d13), (a8, b4, c3, d14), (a8, b4, c3, d15), (a8, b4, c3, d16),

(a9, b1, c1, d1), (a9, b1, c1, d2), (a9, b1, c1, d3), (a9, b1, c1, d4), (a9, b1, c1, d5), (a9, b1, c1, d6), (a9, b1, c1, d7), (a9, b1, c1, d8), (a9, b1, c1, d9), (a9, b1, c1, d10), (a9, b1, c1, d11), (a9, b1, c1, d12), (a9, b1, c1, d13), (a9, b1, c1, d14), (a9, b1, c1, d15), (a9, b1, c1, d16), (a9, b1, c2, d1), (a9, b1, c2, d2), (a9, b1, c2, d3), (a9, b1, c2, d4), (a9, b1, c2, d5), (a9, b1, c2, d6), (a9, b1, c2, d7), (a9, b1, c2, d8), (a9, b1, c2, d9), (a9, b1, c2, d10), (a9, b1, c2, d11), (a9, b1, c2, d12), (a9, b1, c2, d13), (a9, b1, c2, d14), (a9, b1, c2, d15), (a9, b1, c2, d16), (a9, b1, c3, d1), (a9, b1, c3, d2), (a9, b1, c3, d3), (a9, b1, c3, d4), (a9, b1, c3, d5), (a9, b1, c3, d6), (a9, b1, c3, d7), (a9, b1, c3, d8), (a9, b1, c3, d9), (a9, b1, c3, d10), (a9, b1, c3, d11), (a9, b1, c3, d12), (a9, b1, c3, d13), (a9, b1, c3, d14), (a9, b1, c3, d15), (a9, b1, c3, d16), (a9, b2, c1, d1), (a9, b2, c1, d2), (a9, b2, c1, d3), (a9, b2, c1, d4), (a9, b2, c1, d5), (a9, b2, c1, d6), (a9, b2, c1, d7), (a9, b2, c1, d8), (a9, b2, c1, d9), (a9, b2, c1, d10), (a9, b2, c1, d11), (a9, b2, c1, d12), (a9, b2, c1, d13), (a9, b2, c1, d14), (a9, b2, c1, d15), (a9, b2, c1, d16), (a9, b2, c2, d1), (a9, b2, c2, d2), (a9, b2, c2, d3), (a9, b2, c2, d4), (a9, b2, c2, d5), (a9, b2, c2, d6), (a9, b2, c2, d7), (a9, b2, c2, d8), (a9, b2, c2, d9), (a9, b2, c2, d10), (a9, b2, c2, d11), (a9, b2, c2, d12), (a9, b2, c2, d13), (a9, b2, c2, d14), (a9, b2, c2, d15), (a9, b2, c2, d16), (a9, b2, c3, d1), (a9, b2, c3, d2), (a9, b2, c3, d3), (a9, b2, c3, d4), (a9, b2, c3, d5), (a9, b2, c3, d6), (a9, b2, c3, d7), (a9, b2, c3, d8), (a9, b2, c3, d9), (a9, b2, c3, d10), (a9, b2, c3, d11), (a9, b2, c3, d12), (a9, b2, c3, d13), (a9, b2, c3, d14), (a9, b2, c3, d15), (a9, b2, c3, d16), (a9, b3, c1, d1), (a9, b3, c1, d2), (a9, b3, c1, d3), (a9, b3, c1, d4), (a9, b3, c1, d5), (a9, b3, c1, d6), (a9, b3, c1, d7), (a9, b3, c1, d8), (a9, b3, c1, d9), (a9, b3, c1, d10), (a9, b3, c1, d11), (a9, b3, c1, d12), (a9, b3, c1, d13), (a9, b3, c1, d14), (a9, b3, c1, d15), (a9, b3, c1, d16), (a9, b3, c2, d1), (a9, b3, c2, d2), (a9, b3, c2, d3), (a9, b3, c2, d4), (a9, b3, c2, d5), (a9, b3, c2, d6), (a9, b3, c2, d7), (a9, b3, c2, d8), (a9, b3, c2, d9), (a9, b3, c2, d10), (a9, b3, c2, d11), (a9, b3, c2, d12), (a9, b3, c2, d13), (a9, b3, c2, d14), (a9, b3, c2, d15), (a9, b3, c2, d16), (a9, b3, c3, d1), (a9, b3, c3, d2), (a9, b3, c3, d3), (a9, b3, c3, d4), (a9, b3, c3, d5), (a9, b3, c3, d6), (a9, b3, c3, d7), (a9, b3, c3, d8), (a9, b3, c3, d9), (a9, b3, c3, d10), (a9, b3, c3, d11), (a9, b3, c3, d12), (a9, b3, c3, d13), (a9, b3, c3, d14), (a9, b3, c3, d15), (a9, b3, c3, d16), (a9, b4, c1, d1), (a9, b4, c1, d2), (a9, b4, c1, d3), (a9, b4, c1, d4), (a9, b4, c1, d5), (a9, b4, c1, d6), (a9, b4, c1, d7), (a9, b4, c1, d8), (a9, b4, c1, d9), (a9, b4, c1, d10), (a9, b4, c1, d11), (a9, b4, c1, d12), (a9, b4, c1, d13), (a9, b4, c1, d14), (a9, b4, c1, d15), (a9, b4, c1, d16), (a9, b4, c2, d1), (a9, b4, c2, d2), (a9, b4, c2, d3), (a9, b4, c2, d4), (a9, b4, c2, d5), (a9, b4, c2, d6), (a9, b4, c2, d7), (a9, b4, c2, d8), (a9, b4, c2, d9), (a9, b4, c2, d10), (a9, b4, c2, d11), (a9, b4, c2, d12), (a9, b4, c2, d13), (a9, b4,

c2, d14), (a9, b4, c2, d15), (a9, b4, c2, d16), (a9, b4, c3, d1), (a9, b4, c3, d2), (a9, b4, c3, d3), (a9, b4, c3, d4), (a9, b4, c3, d5), (a9, b4, c3, d6), (a9, b4, c3, d7), (a9, b4, c3, d8), (a9, b4, c3, d9), (a9, b4, c3, d10), (a9, b4, c3, d11), (a9, b4, c3, d12), (a9, b4, c3, d13), (a9, b4, c3, d14), (a9, b4, c3, d15), (a9, b4, c3, d16),

(a10, b1, c1, d1), (a10, b1, c1, d2), (a10, b1, c1, d3), (a10, b1, c1, d4), (a10, b1, c1, d5), (a10, b1, c1, d6), (a10, b1, c1, d7), (a10, b1, c1, d8), (a10, b1, c1, d9), (a10, b1, c1, d10), (a10, b1, c1, d11), (a10, b1, c1, d12), (a10, b1, c1, d13), (a10, b1, c1, d14), (a10, b1, c1, d15), (a10, b1, c1, d16), (a10, b1, c2, d1), (a10, b1, c2, d2), (a10, b1, c2, d3), (a10, b1, c2, d4), (a10, b1, c2, d5), (a10, b1, c2, d6), (a10, b1, c2, d7), (a10, b1, c2, d8), (a10, b1, c2, d9), (a10, b1, c2, d10), (a10, b1, c2, d11), (a10, b1, c2, d12), (a10, b1, c2, d13), (a10, b1, c2, d14), (a10, b1, c2, d15), (a10, b1, c2, d16), (a10, b1, c3, d1), (a10, b1, c3, d2), (a10, b1, c3, d3), (a10, b1, c3, d4), (a10, b1, c3, d5), (a10, b1, c3, d6), (a10, b1, c3, d7), (a10, b1, c3, d8), (a10, b1, c3, d9), (a10, b1, c3, d10), (a10, b1, c3, d11), (a10, b1, c3, d12), (a10, b1, c3, d13), (a10, b1, c3, d14), (a10, b1, c3, d15), (a10, b1, c3, d16), (a10, b2, c1, d1), (a10, b2, c1, d2), (a10, b2, c1, d3), (a10, b2, c1, d4), (a10, b2, c1, d5), (a10, b2, c1, d6), (a10, b2, c1, d7), (a10, b2, c1, d8), (a10, b2, c1, d9), (a10, b2, c1, d10), (a10, b2, c1, d11), (a10, b2, c1, d12), (a10, b2, c1, d13), (a10, b2, c1, d14), (a10, b2, c1, d15), (a10, b2, c1, d16), (a10, b2, c2, d1), (a10, b2, c2, d2), (a10, b2, c2, d3), (a10, b2, c2, d4), (a10, b2, c2, d5), (a10, b2, c2, d6), (a10, b2, c2, d7), (a10, b2, c2, d8), (a10, b2, c2, d9), (a10, b2, c2, d10), (a10, b2, c2, d11), (a10, b2, c2, d12), (a10, b2, c2, d13), (a10, b2, c2, d14), (a10, b2, c2, d15), (a10, b2, c2, d16), (a10, b2, c3, d1), (a10, b2, c3, d2), (a10, b2, c3, d3), (a10, b2, c3, d4), (a10, b2, c3, d5), (a10, b2, c3, d6), (a10, b2, c3, d7), (a10, b2, c3, d8), (a10, b2, c3, d9), (a10, b2, c3, d10), (a10, b2, c3, d11), (a10, b2, c3, d12), (a10, b2, c3, d13), (a10, b2, c3, d14), (a10, b2, c3, d15), (a10, b2, c3, d16), (a10, b3, c1, d1), (a10, b3, c1, d2), (a10, b3, c1, d3), (a10, b3, c1, d4), (a10, b3, c1, d5), (a10, b3, c1, d6), (a10, b3, c1, d7), (a10, b3, c1, d8), (a10, b3, c1, d9), (a10, b3, c1, d10), (a10, b3, c1, d11), (a10, b3, c1, d12), (a10, b3, c1, d13), (a10, b3, c1, d14), (a10, b3, c1, d15), (a10, b3, c1, d16), (a10, b3, c2, d1), (a10, b3, c2, d2), (a10, b3, c2, d3), (a10, b3, c2, d4), (a10, b3, c2, d5), (a10, b3, c2, d6), (a10, b3, c2, d7), (a10, b3, c2, d8), (a10, b3, c2, d9), (a10, b3, c2, d10), (a10, b3, c2, d11), (a10, b3, c2, d12), (a10, b3, c2, d13), (a10, b3, c2, d14), (a10, b3, c2, d15), (a10, b3, c2, d16), (a10, b3, c3, d1), (a10, b3, c3, d2), (a10, b3, c3, d3), (a10, b3, c3, d4), (a10, b3, c3, d5), (a10, b3, c3, d6), (a10, b3, c3, d7), (a10, b3, c3, d8), (a10, b3, c3, d9), (a10, b3, c3, d10), (a10, b3, c3, d11), (a10, b3, c3, d12), (a10, b3, c3, d13), (a10, b3, c3, d14), (a10, b3, c3, d15), (a10, b3, c3, d16), (a10, b4, c1, d1), (a10, b4, c1, d2), (a10, b4, c1, d3), (a10, b4, c1, d4), (a10, b4, c1, d5), (a10, b4, c1, d6), (a10, b4, c1, d7), (a10, b4, c1, d8), (a10, b4, c1, d9), (a10, b4, c1, d10), (a10, b4, c1, d11), (a10, b4, c1, d12), (a10, b4, c1, d13), (a10, b4, c1, d14), (a10, b4, c1, d15), (a10, b4, c1, d16), (a10, b4, c2, d1), (a10, b4, c2, d2), (a10, b4, c2, d3), (a10, b4, c2, d4), (a10, b4, c2, d5), (a10, b4, c2, d6), (a10, b4, c2, d7), (a10, b4, c2, d8), (a10, b4, c2, d9), (a10, b4, c2, d10), (a10, b4, c2, d11), (a10, b4, c2, d12), (a10, b4, c2, d13), (a10, b4, c2, d14), (a10, b4, c2, d15), (a10, b4, c2, d16), (a10, b4, c3, d1), (a10, b4, c3, d2), (a10, b4, c3, d3), (a10, b4, c3, d4), (a10, b4, c3, d5), (a10, b4, c3, d6), (a10, b4, c3, d7), (a10, b4, c3, d8), (a10, b4, c3, d9), (a10, b4, c3, d10), (a10, b4, c3, d11), (a10, b4, c3, d12), (a10, b4, c3, d13), (a10, b4, c3, d14), (a10, b4, c3, d15), (a10, b4, c3, d16),

(a11, b1, c1, d1), (a11, b1, c1, d2), (a11, b1, c1, d3), (a11, b1, c1, d4), (a11, b1, c1, d5), (a11, b1, c1, d6), (a11, b1, c1, d7), (a11, b1, c1, d8), (a11, b1, c1, d9), (a11, b1, c1, d10), (a11, b1, c1, d11), (a11, b1, c1, d12), (a11, b1, c1, d13), (a11, b1, c1, d14), (a11, b1, c1, d15), (a11, b1, c1, d16), (a11, b1, c2, d1), (a11, b1, c2, d2), (a11, b1, c2, d3), (a11, b1, c2, d4), (a11, b1, c2, d5), (a11, b1, c2, d6), (a11, b1, c2, d7), (a11, b1, c2, d8), (a11, b1, c2, d9), (a11, b1, c2, d10), (a11, b1, c2, d11), (a11, b1, c2, d12), (a11, b1, c2, d13), (a11, b1, c2, d14), (a11, b1, c2, d15), (a11, b1, c2, d16), (a11, b1, c3, d1), (a11, b1, c3, d2), (a11, b1, c3, d3), (a11, b1, c3, d4), (a11, b1, c3, d5), (a11, b1, c3, d6), (a11, b1, c3, d7), (a11, b1, c3, d8), (a11, b1, c3, d9), (a11, b1, c3, d10), (a11, b1, c3, d11), (a11, b1, c3, d12), (a11, b1, c3, d13), (a11, b1, c3, d14), (a11, b1, c3, d15), (a11, b1, c3, d16), (a11, b2, c1, d1), (a11, b2, c1, d2), (a11, b2, c1, d3), (a11, b2, c1, d4), (a11, b2, c1, d5), (a11, b2, c1, d6), (a11, b2, c1, d7), (a11, b2, c1, d8), (a11, b2, c1, d9), (a11, b2, c1, d10), (a11, b2, c1, d11), (a11, b2, c1, d12), (a11, b2, c1, d13), (a11, b2, c1, d14), (a11, b2, c1, d15), (a11, b2, c1, d16), (a11, b2, c2, d1), (a11, b2, c2, d2), (a11, b2, c2, d3), (a11, b2, c2, d4), (a11, b2, c2, d5), (a11, b2, c2, d6), (a11, b2, c2, d7), (a11, b2, c2, d8), (a11, b2, c2, d9), (a11, b2, c2, d10), (a11, b2, c2, d11), (a11, b2, c2, d12), (a11, b2, c2, d13), (a11, b2, c2, d14), (a11, b2, c2, d15), (a11, b2, c2, d16), (a11, b2, c3, d1), (a11, b2, c3, d2), (a11, b2, c3, d3), (a11, b2, c3, d4), (a11, b2, c3, d5), (a11, b2, c3, d6), (a11, b2, c3, d7), (a11, b2, c3, d8), (a11, b2, c3, d9), (a11, b2, c3, d10), (a11, b2, c3, d11), (a11, b2, c3, d12), (a11, b2, c3, d13), (a11, b2, c3, d14), (a11, b2, c3, d15), (a11, b2, c3, d16), (a11, b3, c1, d1), (a11, b3, c1, d2), (a11, b3, c1, d3), (a11, b3, c1, d4), (a11, b3, c1, d5), (a11, b3, c1, d6), (a11, b3, c1, d7), (a11, b3, c1, d8), (a11, b3, c1, d9), (a11, b3, c1, d10), (a11, b3, c1, d11), (a11, b3, c1, d12), (a11, b3, c1, d13), (a11, b3, c1, d14), (a11, b3, c1, d15), (a11, b3, c1, d16), (a11, b3, c2, d1), (a11, b3, c2, d2), (a11, b3, c2, d3), (a11, b3, c2, d4), (a11, b3, c2, d5), (a11, b3, c2, d6), (a11, b3, c2, d7), (a11, b3, c2, d8), (a11, b3, c2, d9), (a11, b3, c2, d10), (a11, b3, c2, d11), (a11, b3, c2, d12), (a11, b3, c2, d13), (a11, b3, c2, d14), (a11, b3, c2, d15), (a11, b3, c2, d16), (a11, b3, c3, d1), (a11, b3, c3, d2), (a11, b3, c3, d3), (a11, b3, c3, d4), (a11, b3, c3, d5), (a11, b3, c3, d6), (a11, b3, c3, d7), (a11, b3, c3, d8), (a11, b3, c3, d9), (a11, b3, c3, d10), (a11, b3, c3, d11), (a11, b3, c3, d12), (a11, b3, c3, d13), (a11, b3, c3, d14), (a11, b3, c3, d15), (a11, b3, c3, d16), (a11, b4, c1, d1), (a11, b4, c1, d2), (a11, b4, c1, d3), (a11, b4, c1, d4), (a11, b4, c1, d5), (a11, b4, c1, d6), (a11, b4, c1, d7), (a11, b4, c1, d8), (a11, b4, c1, d9), (a11, b4, c1, d10), (a11, b4, c1, d11), (a11, b4, c1, d12), (a11, b4, c1, d13), (a11, b4, c1, d14), (a11, b4, c1, d15), (a11, b4, c1, d16),

(a12, b4, c2, d1), (a12, b4, c2, d2), (a12, b4, c2, d3), (a12, b4, c2, d4), (a12, b4, c2, d5), (a12, b4, c2, d6), (a12, b4, c2, d7), (a12, b4, c2, d8), (a12, b4, c2, d9), (a12, b4, c2, d10), (a12, b4, c2, d11), (a12, b4, c2, d12), (a12, b4, c2, d13), (a12, b4, c2, d14), (a12, b4, c2, d15), (a12, b4, c2, d16), (a12, b4, c3, d1), (a12, b4, c3, d2), (a12, b4, c3, d3), (a12, b4, c3, d4), (a12, b4, c3, d5), (a12, b4, c3, d6), (a12, b4, c3, d7), (a12, b4, c3, d8), (a12, b4, c3, d9), (a12, b4, c3, d10), (a12, b4, c3, d11), (a12, b4, c3, d12), (a12, b4, c3, d13), (a12, b4, c3, d14), (a12, b4, c3, d15), (a12, b4, c3, d16), (a12, b1, c1, d1), (a12, b1, c1, d2), (a12, b1, c1, d3), (a12, b1, c1, d4), (a12, b1, c1, d5), (a12, b1, c1, d6), (a12, b1, c1, d7), (a12, b1, c1, d8), (a12, b1, c1, d9), (a12, b1, c1, d10), (a12, b1, c1, d11), (a12, b1, c1, d12), (a12, b1, c1, d13), (a12, b1, c1, d14), (a12, b1, c1, d15), (a12, b1, c1, d16), (a12, b1, c2, d1), (a12, b1, c2, d2), (a12, b1, c2, d3), (a12, b1, c2, d4), (a12, b1, c2, d5), (a12, b1, c2, d6), (a12, b1, c2, d7), (a12, b1, c2, d8), (a12, b1, c2, d9), (a12, b1, c2, d10), (a12, b1, c2, d11), (a12, b1, c2, d12), (a12, b1, c2, d13), (a12, b1, c2, d14), (a12, b1, c2, d15), (a12, b1, c2, d16), (a12, b1, c3, d1), (a12, b1, c3, d2), (a12, b1, c3, d3), (a12, b1, c3, d4), (a12, b1, c3, d5), (a12, b1, c3, d6), (a12, b1, c3, d7), (a12, b1, c3, d8), (a12, b1, c3, d9), (a12, b1, c3, d10), (a12, b1, c3, d11), (a12, b1, c3, d12), (a12, b1, c3, d13), (a12, b1, c3, d14), (a12, b1, c3, d15), (a12, b1, c3, d16), (a12, b2, c1, d1), (a12, b2, c1, d2), (a12, b2, c1, d3), (a12, b2, c1, d4), (a12, b2, c1, d5), (a12, b2, c1, d6), (a12, b2, c1, d7), (a12, b2, c1, d8), (a12, b2, c1, d9), (a12, b2, c1, d10), (a12, b2, c1, d11), (a12, b2, c1, d12), (a12, b2, c1, d13), (a12, b2, c1, d14), (a12, b2, c1, d15), (a12, b2, c1, d16), (a12, b2, c2, d1), (a12, b2, c2, d2), (a12, b2, c2, d3), (a12, b2, c2, d4), (a12, b2, c2, d5), (a12, b2, c2, d6), (a12, b2, c2, d7), (a12, b2, c2, d8), (a12, b2, c2, d9), (a12, b2, c2, d10), (a12, b2, c2, d11), (a12, b2, c2, d12), (a12, b2, c2, d13), (a12, b2, c2, d14), (a12, b2, c2, d15), (a12, b2, c2, d16), (a12, b2, c3, d1), (a12, b2, c3, d2), (a12, b2, c3, d3), (a12, b2, c3, d4), (a12, b2, c3, d5), (a12, b2, c3, d6), (a12, b2, c3, d7), (a12, b2, c3, d8), (a12, b2, c3, d9), (a12, b2, c3, d10), (a12, b2, c3, d11), (a12, b2, c3, d12), (a12, b2, c3, d13), (a12, b2, c3, d14), (a12, b2, c3, d15), (a12, b2, c3, d16), (a12, b3, c1, d1), (a12, b3, c1, d2), (a12, b3, c1, d3), (a12, b3, c1, d4), (a12, b3, c1, d5), (a12, b3, c1, d6), (a12, b3, c1, d7), (a12, b3, c1, d8), (a12, b3, c1, d9), (a12, b3, c1, d10), (a12, b3, c1, d11), (a12, b3, c1, d12), (a12, b3, c1, d13), (a12, b3, c1, d14), (a12, b3, c1, d15), (a12, b3, c1, d16), (a12, b3, c2, d1), (a12, b3, c2, d2), (a12, b3, c2, d3), (a12, b3, c2, d4), (a12, b3, c2, d5), (a12, b3, c2, d6), (a12, b3, c2, d7), (a12, b3, c2, d8), (a12, b3, c2, d9), (a12, b3, c2, d10), (a12, b3, c2, d11), (a12, b3, c2, d12), (a12, b3, c2, d13), (a12, b3, c2, d14), (a12, b3, c2, d15), (a12, b3, c2, d16), (a12, b3, c3, d1), (a12, b3, c3, d2), (a12, b3, c3, d3), (a12, b3, c3, d4), (a12, b3, c3, d5), (a12, b3, c3, d6), (a12, b3, c3, d7), (a12, b3, c3, d8), (a12, b3, c3, d9), (a12, b3, c3, d10), (a12, b3, c3, d11), (a12, b3, c3, d12), (a12, b3, c3, d13), (a12, b3, c3, d14), (a12, b3, c3, d15), (a12, b3, c3, d16), (a12, b4, c1, d1), (a12, b4, c1, d2), (a12, b4, c1, d3), (a12, b4, c1, d4), (a12, b4, c1, d5), (a12, b4, c1, d6), (a12, b4, c1, d7), (a12, b4, c1, d8), (a12, b4, c1, d9), (a12, b4, c1, d10), (a12, b4, c1, d11), (a12, b4, c1, d12), (a12, b4, c1, d13), (a12, b4, c1, d14), (a12, b4, c1, d15), (a12, b4, c1, d16), (a12, b4, c2, d1), (a12, b4, c2, d2), (a12, b4, c2, d3), (a12, b4, c2, d4), (a12, b4, c2, d5), (a12, b4, c2, d6), (a12, b4, c2, d7), (a12, b4, c2, d8), (a12, b4, c2, d9), (a12, b4, c2, d10), (a12, b4, c2, d11), (a12, b4, c2, d12), (a12, b4, c2, d13), (a12, b4, c2, d14), (a12, b4, c2, d15), (a12, b4, c2, d16), (a12, b4, c3, d1), (a12, b4, c3, d2), (a12, b4, c3, d3), (a12, b4, c3, d4), (a12, b4, c3, d5), (a12, b4, c3, d6), (a12, b4, c3, d7), (a12, b4, c3, d8), (a12, b4, c3, d9), (a12, b4, c3, d10), (a12, b4, c3, d11), (a12, b4, c3, d12), (a12, b4, c3, d13), (a12, b4, c3, d14), (a12, b4, c3, d15), (a12, b4, c3, d16),

(a13, b1, c1, d1), (a13, b1, c1, d2), (a13, b1, c1, d3), (a13, b1, c1, d4), (a13, b1, c1, d5), (a13, b1, c1, d6), (a13, b1, c1, d7), (a13, b1, c1, d8), (a13, b1, c1, d9), (a13, b1, c1, d10), (a13, b1, c1, d11), (a13, b1, c1, d12), (a13, b1, c1, d13), (a13, b1, c1, d14), (a13, b1, c1, d15), (a13, b1, c1, d16), (a13, b1, c2, d1), (a13, b1, c2, d2), (a13, b1, c2, d3), (a13, b1, c2, d4), (a13, b1, c2, d5), (a13, b1, c2, d6), (a13, b1, c2, d7), (a13, b1, c2, d8), (a13, b1, c2, d9), (a13, b1, c2, d10), (a13, b1, c2, d11), (a13, b1, c2, d12), (a13, b1, c2, d13), (a13, b1, c2, d14), (a13, b1, c2, d15), (a13, b1, c2, d16), (a13, b1, c3, d1), (a13, b1, c3, d2), (a13, b1, c3, d3), (a13, b1, c3, d4), (a13, b1, c3, d5), (a13, b1, c3, d6), (a13, b1, c3, d7), (a13, b1, c3, d8), (a13, b1, c3, d9), (a13, b1, c3, d10), (a13, b1, c3, d11), (a13, b1, c3, d12), (a13, b1, c3, d13), (a13, b1, c3, d14), (a13, b1, c3, d15), (a13, b1, c3, d16), (a13, b2, c1, d1), (a13, b2, c1, d2), (a13, b2, c1, d3), (a13, b2, c1, d4), (a13, b2, c1, d5), (a13, b2, c1, d6), (a13, b2, c1, d7), (a13, b2, c1, d8), (a13, b2, c1, d9), (a13, b2, c1, d10), (a13, b2, c1, d11), (a13, b2, c1, d12), (a13, b2, c1, d13), (a13, b2, c1, d14), (a13, b2, c1, d15), (a13, b2, c1, d16), (a13, b2, c2, d1), (a13, b2, c2, d2), (a13, b2, c2, d3), (a13, b2, c2, d4), (a13, b2, c2, d5), (a13, b2, c2, d6), (a13, b2, c2, d7), (a13, b2, c2, d8), (a13, b2, c2, d9), (a13, b2, c2, d10), (a13, b2, c2, d11), (a13, b2, c2, d12), (a13, b2, c2, d13), (a13, b2, c2, d14), (a13, b2, c2, d15), (a13, b2, c2, d16), (a13, b2, c3, d1), (a13, b2, c3, d2), (a13, b2, c3, d3), (a13, b2, c3, d4), (a13, b2, c3, d5), (a13, b2, c3, d6), (a13, b2, c3, d7), (a13, b2, c3, d8), (a13, b2, c3, d9), (a13, b2, c3, d10), (a13, b2, c3, d11), (a13, b2, c3, d12), (a13, b2, c3, d13), (a13, b2, c3, d14), (a13, b2, c3, d15), (a13, b2, c3, d16), (a13, b3, c1, d1), (a13, b3, c1, d2), (a13, b3, c1, d3), (a13, b3, c1, d4), (a13, b3, c1, d5), (a13, b3, c1, d6), (a13, b3, c1, d7), (a13, b3, c1, d8), (a13, b3, c1, d9), (a13, b3, c1, d10), (a13, b3, c1, d11), (a13, b3, c1, d12), (a13, b3, c1, d13), (a13, b3, c1, d14), (a13, b3, c1, d15), (a13, b3, c1, d16), (a13, b3, c2, d1), (a13, b3, c2, d2), (a13, b3, c2, d3), (a13, b3, c2, d4), (a13, b3, c2, d5), (a13, b3, c2, d6), (a13, b3, c2, d7), (a13, b3, c2, d8), (a13, b3, c2, d9), (a13, b3, c2, d10), (a13, b3, c2, d11), (a13, b3, c2, d12), (a13, b3, c2, d13), (a13, b3, c2, d14), (a13, b3, c2, d15), (a13, b3, c2, d16), (a13, b3, c3, d1), (a13, b3, c3, d2), (a13, b3, c3, d3), (a13, b3, c3, d4), (a13, b3, c3, d5), (a13, b3, c3, d6), (a13, b3, c3, d7), (a13, b3, c3, d8), (a13, b3, c3, d9), (a13, b3, c3, d10), (a13, b3, c3, d11), (a13, b3, c3, d12), (a13, b3, c3, d13), (a13, b3, c3, d14), (a13, b3, c3, d15), (a13, b3, c3, d16), (a13, b4, c1, d1), (a13, b4, c1, d2), (a13, b4, c1, d3), (a13, b4, c1, d4), (a13, b4, c1,

d5), (a13, b4, c1, d6), (a13, b4, c1, d7), (a13, b4, c1, d8), (a13, b4, c1, d9), (a13, b4, c1, d10), (a13, b4, c1, d11), (a13, b4, c1, d12), (a13, b4, c1, d13), (a13, b4, c1, d14), (a13, b4, c1, d15), (a13, b4, c1, d16), (a13, b4, c2, d1), (a13, b4, c2, d2), (a13, b4, c2, d3), (a13, b4, c2, d4), (a13, b4, c2, d5), (a13, b4, c2, d6), (a13, b4, c2, d7), (a13, b4, c2, d8), (a13, b4, c2, d9), (a13, b4, c2, d10), (a13, b4, c2, d11), (a13, b4, c2, d12), (a13, b4, c2, d13), (a13, b4, c2, d14), (a13, b4, c2, d15), (a13, b4, c2, d16), (a13, b4, c3, d1), (a13, b4, c3, d2), (a13, b4, c3, d3), (a13, b4, c3, d4), (a13, b4, c3, d5), (a13, b4, c3, d6), (a13, b4, c3, d7), (a13, b4, c3, d8), (a13, b4, c3, d9), (a13, b4, c3, d10), (a13, b4, c3, d11), (a13, b4, c3, d12), (a13, b4, c3, d13), (a13, b4, c3, d14), (a13, b4, c3, d15), (a13, b4, c3, d16),

(a14, b1, c1, d1), (a14, b1, c1, d2), (a14, b1, c1, d3), (a14, b1, c1, d4), (a14, b1, c1, d5), (a14, b1, c1, d6), (a14, b1, c1, d7), (a14, b1, c1, d8), (a14, b1, c1, d9), (a14, b1, c1, d10), (a14, b1, c1, d11), (a14, b1, c1, d12), (a14, b1, c1, d13), (a14, b1, c1, d14), (a14, b1, c1, d15), (a14, b1, c1, d16), (a14, b1, c2, d1), (a14, b1, c2, d2), (a14, b1, c2, d3), (a14, b1, c2, d4), (a14, b1, c2, d5), (a14, b1, c2, d6), (a14, b1, c2, d7), (a14, b1, c2, d8), (a14, b1, c2, d9), (a14, b1, c2, d10), (a14, b1, c2, d11), (a14, b1, c2, d12), (a14, b1, c2, d13), (a14, b1, c2, d14), (a14, b1, c2, d15), (a14, b1, c2, d16), (a14, b1, c3, d1), (a14, b1, c3, d2), (a14, b1, c3, d3), (a14, b1, c3, d4), (a14, b1, c3, d5), (a14, b1, c3, d6), (a14, b1, c3, d7), (a14, b1, c3, d8), (a14, b1, c3, d9), (a14, b1, c3, d10), (a14, b1, c3, d11), (a14, b1, c3, d12), (a14, b1, c3, d13), (a14, b1, c3, d14), (a14, b1, c3, d15), (a14, b1, c3, d16), (a14, b2, c1, d1), (a14, b2, c1, d2), (a14, b2, c1, d3), (a14, b2, c1, d4), (a14, b2, c1, d5), (a14, b2, c1, d6), (a14, b2, c1, d7), (a14, b2, c1, d8), (a14, b2, c1, d9), (a14, b2, c1, d10), (a14, b2, c1, d11), (a14, b2, c1, d12), (a14, b2, c1, d13), (a14, b2, c1, d14), (a14, b2, c1, d15), (a14, b2, c1, d16), (a14, b2, c2, d1), (a14, b2, c2, d2), (a14, b2, c2, d3), (a14, b2, c2, d4), (a14, b2, c2, d5), (a14, b2, c2, d6), (a14, b2, c2, d7), (a14, b2, c2, d8), (a14, b2, c2, d9), (a14, b2, c2, d10), (a14, b2, c2, d11), (a14, b2, c2, d12), (a14, b2, c2, d13), (a14, b2, c2, d14), (a14, b2, c2, d15), (a14, b2, c2, d16), (a14, b2, c3, d1), (a14, b2, c3, d2), (a14, b2, c3, d3), (a14, b2, c3, d4), (a14, b2, c3, d5), (a14, b2, c3, d6), (a14, b2, c3, d7), (a14, b2, c3, d8), (a14, b2, c3, d9), (a14, b2, c3, d10), (a14, b2, c3, d11), (a14, b2, c3, d12), (a14, b2, c3, d13), (a14, b2, c3, d14), (a14, b2, c3, d15), (a14, b2, c3, d16), (a14, b3, c1, d1), (a14, b3, c1, d2), (a14, b3, c1, d3), (a14, b3, c1, d4), (a14, b3, c1, d5), (a14, b3, c1, d6), (a14, b3, c1, d7), (a14, b3, c1, d8), (a14, b3, c1, d9), (a14, b3, c1, d10), (a14, b3, c1, d11), (a14, b3, c1, d12), (a14, b3, c1, d13), (a14, b3, c1, d14), (a14, b3, c1, d15), (a14, b3, c1, d16), (a14, b3, c2, d1), (a14, b3, c2, d2), (a14, b3, c2, d3), (a14, b3, c2, d4), (a14, b3, c2, d5), (a14, b3, c2, d6), (a14, b3, c2, d7), (a14, b3, c2, d8), (a14, b3, c2, d9), (a14, b3, c2, d10), (a14, b3, c2, d11), (a14, b3, c2, d12), (a14, b3, c2, d13), (a14, b3, c2, d14), (a14, b3, c2, d15), (a14, b3, c2, d16), (a14, b3, c3, d1), (a14, b3, c3, d2), (a14, b3, c3, d3), (a14, b3, c3, d4), (a14, b3, c3, d5), (a14, b3, c3, d6), (a14, b3, c3, d7), (a14, b3, c3, d8), (a14, b3, c3, d9), (a14, b3, c3, d10), (a14, b3, c3, d11), (a14, b3, c3, d12), (a14, b3, c3, d13), (a14, b3, c3, d14), (a14, b3, c3, d15), (a14, b3, c3, d16), (a14, b4, c1, d1), (a14, b4, c1, d2), (a14, b4, c1, d3), (a14, b4, c1, d4), (a14, b4, c1, d5), (a14, b4, c1, d6), (a14, b4, c1, d7), (a14, b4, c1, d8), (a14, b4, c1, d9), (a14, b4, c1, d10), (a14, b4, c1, d11), (a14, b4, c1, d12), (a14, b4, c1, d13), (a14, b4, c1, d14), (a14, b4, c1, d15), (a14, b4, c1, d16), (a14, b4, c2, d1), (a14, b4, c2, d2), (a14, b4, c2, d3), (a14, b4, c2, d4), (a14, b4, c2, d5), (a14, b4, c2, d6), (a14, b4, c2, d7), (a14, b4, c2, d8), (a14, b4, c2, d9), (a14, b4, c2, d10), (a14, b4, c2, d11), (a14, b4, c2, d12), (a14, b4, c2, d13), (a14, b4, c2, d14), (a14, b4, c2, d15), (a14, b4, c2, d16), (a14, b4, c3, d1), (a14, b4, c3, d2), (a14, b4, c3, d3), (a14, b4, c3, d4), (a14, b4, c3, d5), (a14, b4, c3, d6), (a14, b4, c3, d7), (a14, b4, c3, d8), (a14, b4, c3, d9), (a14, b4, c3, d10), (a14, b4, c3, d11), (a14, b4, c3, d12), (a14, b4, c3, d13), (a14, b4, c3, d14), (a14, b4, c3, d15), (a14, b4, c3, d16),

(a15, b1, c1, d1), (a15, b1, c1, d2), (a15, b1, c1, d3), (a15, b1, c1, d4), (a15, b1, c1, d5), (a15, b1, c1, d6), (a15, b1, c1, d7), (a15, b1, c1, d8), (a15, b1, c1, d9), (a15, b1, c1, d10), (a15, b1, c1, d11), (a15, b1, c1, d12), (a15, b1, c1, d13), (a15, b1, c1, d14), (a15, b1, c1, d15), (a15, b1, c1, d16), (a15, b1, c2, d1), (a15, b1, c2, d2), (a15, b1, c2, d3), (a15, b1, c2, d4), (a15, b1, c2, d5), (a15, b1, c2, d6), (a15, b1, c2, d7), (a15, b1, c2, d8), (a15, b1, c2, d9), (a15, b1, c2, d10), (a15, b1, c2, d11), (a15, b1, c2, d12), (a15, b1, c2, d13), (a15, b1, c2, d14), (a15, b1, c2, d15), (a15, b1, c2, d16), (a15, b1, c3, d1), (a15, b1, c3, d2), (a15, b1, c3, d3), (a15, b1, c3, d4), (a15, b1, c3, d5), (a15, b1, c3, d6), (a15, b1, c3, d7), (a15, b1, c3, d8), (a15, b1, c3, d9), (a15, b1, c3, d10), (a15, b1, c3, d11), (a15, b1, c3, d12), (a15, b1, c3, d13), (a15, b1, c3, d14), (a15, b1, c3, d15), (a15, b1, c3, d16), (a15, b2, c1, d1), (a15, b2, c1, d2), (a15, b2, c1, d3), (a15, b2, c1, d4), (a15, b2, c1, d5), (a15, b2, c1, d6), (a15, b2, c1, d7), (a15, b2, c1, d8), (a15, b2, c1, d9), (a15, b2, c1, d10), (a15, b2, c1, d11), (a15, b2, c1, d12), (a15, b2, c1, d13), (a15, b2, c1, d14), (a15, b2, c1, d15), (a15, b2, c1, d16), (a15, b2, c2, d1), (a15, b2, c2, d2), (a15, b2, c2, d3), (a15, b2, c2, d4), (a15, b2, c2, d5), (a15, b2, c2, d6), (a15, b2, c2, d7), (a15, b2, c2, d8), (a15, b2, c2, d9), (a15, b2, c2, d10), (a15, b2, c2, d11), (a15, b2, c2, d12), (a15, b2, c2, d13), (a15, b2, c2, d14), (a15, b2, c2, d15), (a15, b2, c2, d16), (a15, b2, c3, d1), (a15, b2, c3, d2), (a15, b2, c3, d3), (a15, b2, c3, d4), (a15, b2, c3, d5), (a15, b2, c3, d6), (a15, b2, c3, d7), (a15, b2, c3, d8), (a15, b2, c3, d9), (a15, b2, c3, d10), (a15, b2, c3, d11), (a15, b2, c3, d12), (a15, b2, c3, d13), (a15, b2, c3, d14), (a15, b2, c3, d15), (a15, b2, c3, d16), (a15, b3, c1, d1), (a15, b3, c1, d2), (a15, b3, c1, d3), (a15, b3, c1, d4), (a15, b3, c1, d5), (a15, b3, c1, d6), (a15, b3, c1, d7), (a15, b3, c1, d8), (a15, b3, c1, d9), (a15, b3, c1, d10), (a15, b3, c1, d11), (a15, b3, c1, d12), (a15, b3, c1, d13), (a15, b3, c1, d14), (a15, b3, c1, d15), (a15, b3, c1, d16), (a15, b3, c2, d1), (a15, b3, c2, d2), (a15, b3, c2, d3), (a15, b3, c2, d4), (a15, b3, c2, d5), (a15, b3, c2, d6), (a15, b3, c2, d7), (a15, b3, c2, d8), (a15, b3, c2, d9), (a15, b3, c2, d10), (a15, b3, c2, d11), (a15, b3, c2, d12), (a15, b3, c2, d13), (a15, b3, c2, d14), (a15, b3, c2, d15), (a15, b3, c2, d16), (a15, b3, c3, d1), (a15, b3, c3, d2), (a15, b3, c3, d3), (a15, b3, c3, d4), (a15, b3, c3, d5), (a15, b3, c3, d6), (a15, b3, c3, d7), (a15, b3, c3, d8),

(a15, b3, c3, d9), (a15, b3, c3, d10), (a15, b3, c3, d11), (a15, b3, c3, d12), (a15, b3, c3, d13), (a15, b3, c3, d14), (a15, b3, c3, d15), (a15, b3, c3, d16), (a15, b4, c1, d1), (a15, b4, c1, d2), (a15, b4, c1, d3), (a15, b4, c1, d4), (a15, b4, c1, d5), (a15, b4, c1, d6), (a15, b4, c1, d7), (a15, b4, c1, d8), (a15, b4, c1, d9), (a15, b4, c1, d10), (a15, b4, c1, d11), (a15, b4, c1, d12), (a15, b4, c1, d13), (a15, b4, c1, d14), (a15, b4, c1, d15), (a15, b4, c1, d16), (a15, b4, c2, d1), (a15, b4, c2, d2), (a15, b4, c2, d3), (a15, b4, c2, d4), (a15, b4, c2, d5), (a15, b4, c2, d6), (a15, b4, c2, d7), (a15, b4, c2, d8), (a15, b4, c2, d9), (a15, b4, c2, d10), (a15, b4, c2, d11), (a15, b4, c2, d12), (a15, b4, c2, d13), (a15, b4, c2, d14), (a15, b4, c2, d15), (a15, b4, c2, d16), (a15, b4, c3, d1), (a15, b4, c3, d2), (a15, b4, c3, d3), (a15, b4, c3, d4), (a15, b4, c3, d5), (a15, b4, c3, d6), (a15, b4, c3, d7), (a15, b4, c3, d8), (a15, b4, c3, d9), (a15, b4, c3, d10), (a15, b4, c3, d11), (a15, b4, c3, d12), (a15, b4, c3, d13), (a15, b4, c3, d14), (a15, b4, c3, d15), (a15, b4, c3, d16),

(a16, b1, c1, d1), (a16, b1, c1, d2), (a16, b1, c1, d3), (a16, b1, c1, d4), (a16, b1, c1, d5), (a16, b1, c1, d6), (a16, b1, c1, d7), (a16, b1, c1, d8), (a16, b1, c1, d9), (a16, b1, c1, d10), (a16, b1, c1, d11), (a16, b1, c1, d12), (a16, b1, c1, d13), (a16, b1, c1, d14), (a16, b1, c1, d15), (a16, b1, c1, d16), (a16, b1, c2, d1), (a16, b1, c2, d2), (a16, b1, c2, d3), (a16, b1, c2, d4), (a16, b1, c2, d5), (a16, b1, c2, d6), (a16, b1, c2, d7), (a16, b1, c2, d8), (a16, b1, c2, d9), (a16, b1, c2, d10), (a16, b1, c2, d11), (a16, b1, c2, d12), (a16, b1, c2, d13), (a16, b1, c2, d14), (a16, b1, c2, d15), (a16, b1, c2, d16), (a16, b1, c3, d1), (a16, b1, c3, d2), (a16, b1, c3, d3), (a16, b1, c3, d4), (a16, b1, c3, d5), (a16, b1, c3, d6), (a16, b1, c3, d7), (a16, b1, c3, d8), (a16, b1, c3, d9), (a16, b1, c3, d10), (a16, b1, c3, d11), (a16, b1, c3, d12), (a16, b1, c3, d13), (a16, b1, c3, d14), (a16, b1, c3, d15), (a16, b1, c3, d16), (a16, b2, c1, d1), (a16, b2, c1, d2), (a16, b2, c1, d3), (a16, b2, c1, d4), (a16, b2, c1, d5), (a16, b2, c1, d6), (a16, b2, c1, d7), (a16, b2, c1, d8), (a16, b2, c1, d9), (a16, b2, c1, d10), (a16, b2, c1, d11), (a16, b2, c1, d12), (a16, b2, c1, d13), (a16, b2, c1, d14), (a16, b2, c1, d15), (a16, b2, c1, d16), (a16, b2, c2, d1), (a16, b2, c2, d2), (a16, b2, c2, d3), (a16, b2, c2, d4), (a16, b2, c2, d5), (a16, b2, c2, d6), (a16, b2, c2, d7), (a16, b2, c2, d8), (a16, b2, c2, d9), (a16, b2, c2, d10), (a16, b2, c2, d11), (a16, b2, c2, d12), (a16, b2, c2, d13), (a16, b2, c2, d14), (a16, b2, c2, d15), (a16, b2, c2, d16), (a16, b2, c3, d1), (a16, b2, c3, d2), (a16, b2, c3, d3), (a16, b2, c3, d4), (a16, b2, c3, d5), (a16, b2, c3, d6), (a16, b2, c3, d7), (a16, b2, c3, d8), (a16, b2, c3, d9), (a16, b2, c3, d10), (a16, b2, c3, d11), (a16, b2, c3, d12), (a16, b2, c3, d13), (a16, b2, c3, d14), (a16, b2, c3, d15), (a16, b2, c3, d16), (a16, b3, c1, d1), (a16, b3, c1, d2), (a16, b3, c1, d3), (a16, b3, c1, d4), (a16, b3, c1, d5), (a16, b3, c1, d6), (a16, b3, c1, d7), (a16, b3, c1, d8), (a16, b3, c1, d9), (a16, b3, c1, d10), (a16, b3, c1, d11), (a16, b3, c1, d12), (a16, b3, c1, d13), (a16, b3, c1, d14), (a16, b3, c1, d15), (a16, b3, c1, d16), (a16, b3, c2, d1), (a16, b3, c2, d2), (a16, b3, c2, d3), (a16, b3, c2, d4), (a16, b3, c2, d5), (a16, b3, c2, d6), (a16, b3, c2, d7), (a16, b3, c2, d8), (a16, b3, c2, d9), (a16, b3, c2, d10), (a16, b3, c2, d11), (a16, b3, c2, d12), (a16, b3, c2, d13), (a16, b3, c2, d14), (a16, b3, c2, d15), (a16, b3, c2, d16), (a16, b3, c3, d1), (a16, b3, c3, d2), (a16, b3, c3, d3), (a16, b3, c3, d4), (a16, b3, c3, d5), (a16, b3, c3, d6), (a16, b3, c3, d7), (a16, b3, c3, d8), (a16, b3, c3, d9), (a16, b3, c3, d10), (a16, b3, c3, d11), (a16, b3, c3, d12), (a16, b3, c3, d13), (a16, b3, c3, d14), (a16, b3, c3, d15), (a16, b3, c3, d16), (a16, b4, c1, d1), (a16, b4, c1, d2), (a16, b4, c1, d3), (a16, b4, c1, d4), (a16, b4, c1, d5), (a16, b4, c1, d6), (a16, b4, c1, d7), (a16, b4, c1, d8), (a16, b4, c1, d9), (a16, b4, c1, d10), (a16, b4, c1, d11), (a16, b4, c1, d12), (a16, b4, c1, d13), (a16, b4, c1, d14), (a16, b4, c1, d15), (a16, b4, c1, d16), (a16, b4, c2, d1), (a16, b4, c2, d2), (a16, b4, c2, d3), (a16, b4, c2, d4), (a16, b4, c2, d5), (a16, b4, c2, d6), (a16, b4, c2, d7), (a16, b4, c2, d8), (a16, b4, c2, d9), (a16, b4, c2, d10), (a16, b4, c2, d11), (a16, b4, c2, d12), (a16, b4, c2, d13), (a16, b4, c2, d14), (a16, b4, c2, d15), (a16, b4, c2, d16), (a16, b4, c3, d1), (a16, b4, c3, d2), (a16, b4, c3, d3), (a16, b4, c3, d4), (a16, b4, c3, d5), (a16, b4, c3, d6), (a16, b4, c3, d7), (a16, b4, c3, d8), (a16, b4, c3, d9), (a16, b4, c3, d10), (a16, b4, c3, d11), (a16, b4, c3, d12), (a16, b4, c3, d13), (a16, b4, c3, d14), (a16, b4, c3, d15), (a16, b4, c3, d16),

(a17, b1, c1, d1), (a17, b1, c1, d2), (a17, b1, c1, d3), (a17, b1, c1, d4), (a17, b1, c1, d5), (a17, b1, c1, d6), (a17, b1, c1, d7), (a17, b1, c1, d8), (a17, b1, c1, d9), (a17, b1, c1, d10), (a17, b1 c1, d11), (a17, b1, c1, d12), (a17, b1, c1, d13), (a17, b1, c1, d14), (a17, b1, c1, d15), (a17, b1, c1, d16), (a17, b1, c2, d1), (a17, b1, c2, d2), (a17, b1, c2, d3), (a17, b1, c2, d4), (a17, b1, c2, d5), (a17, b1, c2, d6), (a17, b1, c2, d7), (a17, b1, c2, d8), (a17, b1, c2, d9), (a17, b1, c2, d10), (a17, b1, c2, d11), (a17, b1, c2, d12), (a17, b1, c2, d13), (a17, b1, c2, d14), (a17, b1, c2, d15), (a17, b1, c2, d16), (a17, b1, c3, d1), (a17, b1, c3, d2), (a17, b1, c3, d3), (a17, b1, c3, d4), (a17, b1, c3, d5), (a17, b1, c3, d6), (a17, b1, c3, d7), (a17, b1, c3, d8), (a17, b1, c3, d9), (a17, b1, c3, d10), (a17, b1, c3, d11), (a17, b1, c3, d12), (a17, b1, c3, d13), (a17, b1, c3, d14), (a17, b1, c3, d15), (a17, b1, c3, d16), (a17, b2, c1, d1), (a17, b2, c1, d2), (a17, b2, c1, d3), (a17, b2, c1, d4), (a17, b2, c1, d5), (a17, b2, c1, d6), (a17, b2, c1, d7), (a17, b2, c1, d8), (a17, b2, c1, d9), (a17, b2, c1, d10), (a17, b2, c1, d11), (a17, b2, c1, d12), (a17, b2, c1, d13), (a17, b2, c1, d14), (a17, b2, c1, d15), (a17, b2, c1, d16), (a17, b2, c2, d1), (a17, b2, c2, d2), (a17, b2, c2, d3), (a17, b2, c2, d4), (a17, b2, c2, d5), (a17, b2, c2, d6), (a17, b2, c2, d7), (a17, b2, c2, d8), (a17, b2, c2, d9), (a17, b2, c2, d10), (a17, b2, c2, d11), (a17, b2, c2, d12), (a17, b2, c2, d13), (a17, b2, c2, d14), (a17, b2, c2, d15), (a17, b2, c2, d16), (a17, b2, c3, d1), (a17, b2, c3, d2), (a17, b2, c3, d3), (a17, b2, c3, d4), (a17, b2, c3, d5), (a17, b2, c3, d6), (a17, b2, c3, d7), (a17, b2, c3, d8), (a17, b2, c3, d9), (a17, b2, c3, d10), (a17, b2, c3, d11), (a17, b2, c3, d12), (a17, b2, c3, d13), (a17, b2, c3, d14), (a17, b2, c3, d15), (a17, b2, c3, d16), (a17, b3, c1, d1), (a17, b3, c1, d2), (a17, b3, c1, d3), (a17, b3, c1, d4), (a17, b3, c1, d5), (a17, b3, c1, d6), (a17, b3, c1, d7), (a17, b3, c1, d8), (a17, b3, c1, d9), (a17, b3, c1, d10), (a17, b3, c1, d11), (a17, b3, c1, d12), (a17, b3, c1, d13), (a17, b3, c1, d14), (a17, b3, c1, d15), (a17, b3, c1, d16), (a17, b3, c2, d1), (a17, b3, c2, d2), (a17, b3, c2, d3), (a17, b3, c2, d4), (a17, b3, c2, d5), (a17, b3, c2, d6), (a17, b3, c2, d7), (a17, b3, c2, d8), (a17, b3, c2, d9), (a17, b3, c2, d10), (a17, b3, c2, d11),

(a17, b3, c2, d12), (a17, b3, c2, d13), (a17, b3, c2, d14), (a17, b3, c2, d15), (a17, b3, c2, d16), (a17, b3, c3, d1), (a17, b3, c3, d2), (a17, b3, c3, d3), (a17, b3, c3, d4), (a17, b3, c3, d5), (a17, b3, c3, d6), (a17, b3, c3, d7), (a17, b3, c3, d8), (a17, b3, c3, d9), (a17, b3, c3, d10), (a17, b3, c3, d11), (a17, b3, c3, d12), (a17, b3, c3, d13), (a17, b3, c3, d14), (a17, b3, c3, d15), (a17, b3, c3, d16), (a17, b4, c1, d1), (a17, b4, c1, d2), (a17, b4, c1, d3), (a17, b4, c1, d4), (a17, b4, c1, d5), (a17, b4, c1, d6), (a17, b4, c1, d7), (a17, b4, c1, d8), (a17, b4, c1, d9), (a17, b4, c1, d10), (a17, b4, c1, d11), (a17, b4, c1, d12), (a17, b4, c1, d13), (a17, b4, c1, d14), (a17, b4, c1, d15), (a17, b4, c1, d16), (a17, b4, c2, d1), (a17, b4, c2, d2), (a17, b4, c2, d3), (a17, b4, c2, d4), (a17, b4, c2, d5), (a17, b4, c2, d6), (a17, b4, c2, d7), (a17, b4, c2, d8), (a17, b4, c2, d9), (a17, b4, c2, d10), (a17, b4, c2, d11), (a17, b4, c2, d12), (a17, b4, c2, d13), (a17, b4, c2, d14), (a17, b4, c2, d15), (a17, b4, c2, d16), (a17, b4, c3, d1), (a17, b4, c3, d2), (a17, b4, c3, d3), (a17, b4, c3, d4), (a17, b4, c3, d5), (a17, b4, c3, d6), (a17, b4, c3, d7), (a17, b4, c3, d8), (a17, b4, c3, d9), (a17, b4, c3, d10), (a17, b4, c3, d11), (a17, b4, c3, d12), (a17, b4, c3, d13), (a17, b4, c3, d14), (a17, b4, c3, d15), (a17, b4, c3, d16),

(a18, b1, c1, d1), (a18, b1, c1, d2), (a18, b1, c1, d3), (a18, b1, c1, d4), (a18, b1, c1, d5), (a18, b1, c1, d6), (a18, b1, c1, d7), (a18, b1, c1, d8), (a18, b1, c1, d9), (a18, b1, c1, d10), (a18, b1, c1, d11), (a18, b1, c1, d12), (a18, b1, c1, d13), (a18, b1, c1, d14), (a18, b1, c1, d15), (a18, b1, c1, d16), (a18, b1, c2, d1), (a18, b1, c2, d2), (a18, b1, c2, d3), (a18, b1, c2, d4), (a18, b1, c2, d5), (a18, b1, c2, d6), (a18, b1, c2, d7), (a18, b1, c2, d8), (a18, b1, c2, d9), (a18, b1, c2, d10), (a18, b1, c2, d11), (a18, b1, c2, d12), (a18, b1, c2, d13), (a18, b1, c2, d14), (a18, b1, c2, d15), (a18, b1, c2, d16), (a18, b1, c3, d1), (a18, b1, c3, d2), (a18, b1, c3, d3), (a18, b1, c3, d4), (a18, b1, c3, d5), (a18, b1, c3, d6), (a18, b1, c3, d7), (a18, b1, c3, d8), (a18, b1, c3, d9), (a18, b1, c3, d10), (a18, b1, c3, d11), (a18, b1, c3, d12), (a18, b1, c3, d13), (a18, b1, c3, d14), (a18, b1, c3, d15), (a18, b1, c3, d16), (a18, b2, c1, d1), (a18, b2, c1, d2), (a18, b2, c1, d3), (a18, b2, c1, d4), (a18, b2, c1, d5), (a18, b2, c1, d6), (a18, b2, c1, d7), (a18, b2, c1, d8), (a18, b2, c1, d9), (a18, b2, c1, d10), (a18, b2, c1, d11), (a18, b2, c1, d12), (a18, b2, c1, d13), (a18, b2, c1, d14), (a18, b2, c1, d15), (a18, b2, c1, d16), (a18, b2, c2, d1), (a18, b2, c2, d2), (a18, b2, c2, d3), (a18, b2, c2, d4), (a18, b2, c2, d5), (a18, b2, c2, d6), (a18, b2, c2, d7), (a18, b2, c2, d8), (a18, b2, c2, d9), (a18, b2, c2, d10), (a18, b2, c2, d11), (a18, b2, c2, d12), (a18, b2, c2, d13), (a18, b2, c2, d14), (a18, b2, c2, d15), (a18, b2, c2, d16), (a18, b2, c3, d1), (a18, b2, c3, d2), (a18, b2, c3, d3), (a18, b2, c3, d4), (a18, b2, c3, d5), (a18, b2, c3, d6), (a18, b2, c3, d7), (a18, b2, c3, d8), (a18, b2, c3, d9), (a18, b2, c3, d10), (a18, b2, c3, d11), (a18, b2, c3, d12), (a18, b2, c3, d13), (a18, b2, c3, d14), (a18, b2, c3, d15), (a18, b2, c3, d16), (a18, b3, c1, d1), (a18, b3, c1, d2), (a18, b3, c1, d3), (a18, b3, c1, d4), (a18, b3, c1, d5), (a18, b3, c1, d6), (a18, b3, c1, d7), (a18, b3, c1, d8), (a18, b3, c1, d9), (a18, b3, c1, d10), (a18, b3, c1, d11), (a18, b3, c1, d12), (a18, b3, c1, d13), (a18, b3, c1, d14), (a18, b3, c1, d15), (a18, b3, c1, d16), (a18, b3, c2, d1), (a18, b3, c2, d2), (a18, b3, c2, d3), (a18, b3, c2, d4), (a18, b3, c2, d5), (a18, b3, c2, d6), (a18, b3, c2, d7), (a18, b3, c2, d8), (a18, b3, c2, d9), (a18, b3, c2, d10), (a18, b3, c2, d11), (a18, b3, c2, d12), (a18, b3, c2, d13), (a18, b3, c2, d14), (a18, b3, c2, d15), (a18, b3, c2, d16), (a18, b3, c3, d1), (a18, b3, c3, d2), (a18, b3, c3, d3), (a18, b3, c3, d4), (a18, b3, c3, d5), (a18, b3, c3, d6), (a18, b3, c3, d7), (a18, b3, c3, d8), (a18, b3, c3, d9), (a18, b3, c3, d10), (a18, b3, c3, d11), (a18, b3, c3, d12), (a18, b3, c3, d13), (a18, b3, c3, d14), (a18, b3, c3, d15), (a18, b3, c3, d16), (a18, b4, c1, d1), (a18, b4, c1, d2), (a18, b4, c1, d3), (a18, b4, c1, d4), (a18, b4, c1, d5), (a18, b4, c1, d6), (a18, b4, c1, d7), (a18, b4, c1, d8), (a18, b4, c1, d9), (a18, b4, c1, d10), (a18, b4, c1, d11), (a18, b4, c1, d12), (a18, b4, c1, d13), (a18, b4, c1, d14), (a18, b4, c1, d15), (a18, b4, c1, d16), (a18, b4, c2, d1), (a18, b4, c2, d2), (a18, b4, c2, d3), (a18, b4, c2, d4), (a18, b4, c2, d5), (a18, b4, c2, d6), (a18, b4, c2, d7), (a18, b4, c2, d8), (a18, b4, c2, d9), (a18, b4, c2, d10), (a18, b4, c2, d11), (a18, b4, c2, d12), (a18, b4, c2, d13), (a18, b4, c2, d14), (a18, b4, c2, d15), (a18, b4, c2, d16), (a18, b4, c3, d1), (a18, b4, c3, d2), (a18, b4, c3, d3), (a18, b4, c3, d4), (a18, b4, c3, d5), (a18, b4, c3, d6), (a18, b4, c3, d7), (a18, b4, c3, d8), (a18, b4, c3, d9), (a18, b4, c3, d10), (a18, b4, c3, d11), (a18, b4, c3, d12), (a18, b4, c3, d13), (a18, b4, c3, d14), (a18, b4, c3, d15), (a18, b4, c3, d16),

(a19, b1, c1, d1), (a19, b1, c1, d2), (a19, b1, c1, d3), (a19, b1, c1, d4), (a19, b1, c1, d5), (a19, b1, c1, d6), (a19, b1, c1, d7), (a19, b1, c1, d8), (a19, b1, c1, d9), (a19, b1, c1, d10), (a19, b1, c1, d11), (a19, b1, c1, d12), (a19, b1, c1, d13), (a19, b1, c1, d14), (a19, b1, c1, d15), (a19, b1, c1, d16), (a19, b1, c2, d1), (a19, b1, c2, d2), (a19, b1, c2, d3), (a19, b1, c2, d4), (a19, b1, c2, d5), (a19, b1, c2, d6), (a19, b1, c2, d7), (a19, b1, c2, d8), (a19, b1, c2, d9), (a19, b1, c2, d10), (a19, b1, c2, d11), (a19, b1, c2, d12), (a19, b1, c2, d13), (a19, b1, c2, d14), (a19, b1, c2, d15), (a19, b1, c2, d16), (a19, b1, c3, d1), (a19, b1, c3, d2), (a19, b1, c3, d3), (a19, b1, c3, d4), (a19, b1, c3, d5), (a19, b1, c3, d6), (a19, b1, c3, d7), (a19, b1, c3, d8), (a19, b1, c3, d9), (a19, b1, c3, d10), (a19, b1, c3, d11), (a19, b1, c3, d12), (a19, b1, c3, d13), (a19, b1, c3, d14), (a19, b1, c3, d15), (a19, b1, c3, d16), (a19, b2, c1, d1), (a19, b2, c1, d2), (a19, b2, c1, d3), (a19, b2, c1, d4), (a19, b2, c1, d5), (a19, b2, c1, d6), (a19, b2, c1, d7), (a19, b2, c1, d8), (a19, b2, c1, d9), (a19, b2, c1, d10), (a19, b2, c1, d11), (a19, b2, c1, d12), (a19, b2, c1, d13), (a19, b2, c1, d14), (a19, b2, c1, d15), (a19, b2, c1, d16), (a19, b2, c2, d1), (a19, b2, c2, d2), (a19, b2, c2, d3), (a19, b2, c2, d4), (a19, b2, c2, d5), (a19, b2, c2, d6), (a19, b2, c2, d7), (a19, b2, c2, d8), (a19, b2, c2, d9), (a19, b2, c2, d10), (a19, b2, c2, d11), (a19, b2, c2, d12), (a19, b2, c2, d13), (a19, b2, c2, d14), (a19, b2, c2, d15), (a19, b2, c2, d16), (a19, b2, c3, d1), (a19, b2, c3, d2), (a19, b2, c3, d3), (a19, b2, c3, d4), (a19, b2, c3, d5), (a19, b2, c3, d6), (a19, b2, c3, d7), (a19, b2, c3, d8), (a19, b2, c3, d9), (a19, b2, c3, d10), (a19, b2, c3, d11), (a19, b2, c3, d12), (a19, b2, c3, d13), (a19, b2, c3, d14), (a19, b2, c3, d15), (a19, b2, c3, d16), (a19, b3, c1, d1), (a19, b3, c1, d2), (a19, b3, c1, d3), (a19, b3, c1, d4), (a19, b3, c1, d5), (a19, b3, c1, d6), (a19, b3, c1, d7), (a19, b3, c1, d8), (a19, b3, c1, d9), (a19, b3, c1, d10), (a19, b3, c1, d11), (a19, b3, c1, d12), (a19, b3, c1, d13), (a19, b3, c1,

d14), (a19, b3, c1, d15), (a19, b3, c1, d16), (a19, b3, c2, d1), (a19, b3, c2, d2), (a19, b3, c2, d3), (a19, b3, c2, d4), (a19, b3, c2, d5), (a19, b3, c2, d6), (a19, b3, c2, d7), (a19, b3, c2, d8), (a19, b3, c2, d9), (a19, b3, c2, d10), (a19, b3, c2, d11), (a19, b3, c2, d12), (a19, b3, c2, d13), (a19, b3, c2, d14), (a19, b3, c2, d15), (a19, b3, c2, d16), (a19, b3, c3, d1), (a19, b3, c3, d2), (a19, b3, c3, d3), (a19, b3, c3, d4), (a19, b3, c3, d5), (a19, b3, c3, d6), (a19, b3, c3, d7), (a19, b3, c3, d8), (a19, b3, c3, d9), (a19, b3, c3, d10), (a19, b3, c3, d11), (a19, b3, c3, d12), (a19, b3, c3, d13), (a19, b3, c3, d14), (a19, b3, c3, d15), (a19, b3, c3, d16), (a19, b4, c1, d1), (a19, b4, c1, d2), (a19, b4, c1, d3), (a19, b4, c1, d4), (a19, b4, c1, d5), (a19, b4, c1, d6), (a19, b4, c1, d7), (a19, b4, c1, d8), (a19, b4, c1, d9), (a19, b4, c1, d10), (a19, b4, c1, d11), (a19, b4, c1, d12), (a19, b4, c1, d13), (a19, b4, c1, d14), (a19, b4, c1, d15), (a19, b4, c1, d16), (a19, b4, c2, d1), (a19, b4, c2, d2), (a19, b4, c2, d3), (a19, b4, c2, d4), (a19, b4, c2, d5), (a19, b4, c2, d6), (a19, b4, c2, d7), (a19, b4, c2, d8), (a19, b4, c2, d9), (a19, b4, c2, d10), (a19, b4, c2, d11), (a19, b4, c2, d12), (a19, b4, c2, d13), (a19, b4, c2, d14), (a19, b4, c2, d15), (a19, b4, c2, d16), (a19, b4, c3, d1), (a19, b4, c3, d2), (a19, b4, c3, d3), (a19, b4, c3, d4), (a19, b4, c3, d5), (a19, b4, c3, d6), (a19, b4, c3, d7), (a19, b4, c3, d8), (a19, b4, c3, d9), (a19, b4, c3, d10), (a19, b4, c3, d11), (a19, b4, c3, d12), (a19, b4, c3, d13), (a19, b4, c3, d14), (a19, b4, c3, d15), (a19, b4, c3, d16),

(a20, b1, c1, d1), (a20, b1, c1, d2), (a20, b1, c1, d3), (a20, b1, c1, d4), (a20, b1, c1, d5), (a20, b1, c1, d6), (a20, b1, c1, d7), (a20, b1, c1, d8), (a20, b1, c1, d9), (a20, b1, c1, d10), (a20, b1, c1, d11), (a20, b1, c1, d12), (a20, b1, c1, d13), (a20, b1, c1, d14), (a20, b1, c1, d15), (a20, b1, c1, d16), (a20, b1, c2, d1), (a20, b1, c2, d2), (a20, b1, c2, d3), (a20, b1, c2, d4), (a20, b1, c2, d5), (a20, b1, c2, d6), (a20, b1, c2, d7), (a20, b1, c2, d8), (a20, b1, c2, d9), (a20, b1, c2, d10), (a20, b1, c2, d11), (a20, b1, c2, d12), (a20, b1, c2, d13), (a20, b1, c2, d14), (a20, b1, c2, d15), (a20, b1, c2, d16), (a20, b1, c3, d1), (a20, b1, c3, d2), (a20, b1, c3, d3), (a20, b1, c3, d4), (a20, b1, c3, d5), (a20, b1, c3, d6), (a20, b1, c3, d7), (a20, b1, c3, d8), (a20, b1, c3, d9), (a20, b1, c3, d10), (a20, b1, c3, d11), (a20, b1, c3, d12), (a20, b1, c3, d13), (a20, b1, c3, d14), (a20, b1, c3, d15), (a20, b1, c3, d16), (a20, b2, c1, d1), (a20, b2, c1, d2), (a20, b2, c1, d3), (a20, b2, c1, d4), (a20, b2, c1, d5), (a20, b2, c1, d6), (a20, b2, c1, d7), (a20, b2, c1, d8), (a20, b2, c1, d9), (a20, b2, c1, d10), (a20, b2, c1, d11), (a20, b2, c1, d12), (a20, b2, c1, d13), (a20, b2, c1, d14), (a20, b2, c1, d15), (a20, b2, c1, d16), (a20, b2, c2, d1), (a20, b2, c2, d2), (a20, b2, c2, d3), (a20, b2, c2, d4), (a20, b2, c2, d5), (a20, b2, c2, d6), (a20, b2, c2, d7), (a20, b2, c2, d8), (a20, b2, c2, d9), (a20, b2, c2, d10), (a20, b2, c2, d11), (a20, b2, c2, d12), (a20, b2, c2, d13), (a20, b2, c2, d14), (a20, b2, c2, d15), (a20, b2, c2, d16), (a20, b2, c3, d1), (a20, b2, c3, d2), (a20, b2, c3, d3), (a20, b2, c3, d4), (a20, b2, c3, d5), (a20, b2, c3, d6), (a20, b2, c3, d7), (a20, b2, c3, d8), (a20, b2, c3, d9), (a20, b2, c3, d10), (a20, b2, c3, d11), (a20, b2, c3, d12), (a20, b2, c3, d13), (a20, b2, c3, d14), (a20, b2, c3, d15), (a20, b2, c3, d16), (a20, b3, c1, d1), (a20, b3, c1, d2), (a20, b3, c1, d3), (a20, b3, c1, d4), (a20, b3, c1, d5), (a20, b3, c1, d6), (a20, b3, c1, d7), (a20, b3, c1, d8), (a20, b3, c1, d9), (a20, b3, c1, d10), (a20, b3, c1, d11), (a20, b3, c1, d12), (a20, b3, c1, d13), (a20, b3, c1, d14), (a20, b3, c1, d15), (a20, b3, c1, d16), (a20, b3, c2, d1), (a20, b3, c2, d2), (a20, b3, c2, d3), (a20, b3, c2, d4), (a20, b3, c2, d5), (a20, b3, c2, d6), (a20, b3, c2, d7), (a20, b3, c2, d8), (a20, b3, c2, d9), (a20, b3, c2, d10), (a20, b3, c2, d11), (a20, b3, c2, d12), (a20, b3, c2, d13), (a20, b3, c2, d14), (a20, b3, c2, d15), (a20, b3, c2, d16), (a20, b3, c3, d1), (a20, b3, c3, d2), (a20, b3, c3, d3), (a20, b3, c3, d4), (a20, b3, c3, d5), (a20, b3, c3, d6), (a20, b3, c3, d7), (a20, b3, c3, d8), (a20, b3, c3, d9), (a20, b3, c3, d10), (a20, b3, c3, d11), (a20, b3, c3, d12), (a20, b3, c3, d13), (a20, b3, c3, d14), (a20, b3, c3, d15), (a20, b3, c3, d16), (a20, b4, c1, d1), (a20, b4, c1, d2), (a20, b4, c1, d3), (a20, b4, c1, d4), (a20, b4, c1, d5), (a20, b4, c1, d6), (a20, b4, c1, d7), (a20, b4, c1, d8), (a20, b4, c1, d9), (a20, b4, c1, d10), (a20, b4, c1, d11), (a20, b4, c1, d12), (a20, b4, c1, d13), (a20, b4, c1, d14), (a20, b4, c1, d15), (a20, b4, c1, d16), (a20, b4, c2, d1), (a20, b4, c2, d2), (a20, b4, c2, d3), (a20, b4, c2, d4), (a20, b4, c2, d5), (a20, b4, c2, d6), (a20, b4, c2, d7), (a20, b4, c2, d8), (a20, b4, c2, d9), (a20, b4, c2, d10), (a20, b4, c2, d11), (a20, b4, c2, d12), (a20, b4, c2, d13), (a20, b4, c2, d14), (a20, b4, c2, d15), (a20, b4, c2, d16), (a20, b4, c3, d1), (a20, b4, c3, d2), (a20, b4, c3, d3), (a20, b4, c3, d4), (a20, b4, c3, d5), (a20, b4, c3, d6), (a20, b4, c3, d7), (a20, b4, c3, d8), (a20, b4, c3, d9), (a20, b4, c3, d10), (a20, b4, c3, d11), (a20, b4, c3, d12), (a20, b4, c3, d13), (a20, b4, c3, d14), (a20, b4, c3, d15), (a20, b4, c3, d16) are synthesized.

Test Example 1 Preparation of MMP

Various human MMPs (MMP-2, MMP-9 and MMP-13) were purchased from Calbiochem (EMD/MERCK Biosciences).

Since the purchased MMP-13 was Proenzyme, the activated one was used, which was incubated at 37°C for 2 hours after the successive addition of an assay buffer (containing 50 mM Tris-HCl (pH7.6), 0.3 M NaCl, 10 mM $CaCl_2$, 0.005% Brij35) and APMA (final concentration 1mM) to the enzyme solution.

Test Example 2 Method for measuring enzyme inhibiting activity of various MMPs

The enzyme activity of MMPs was measured according to the method described in C. Graham Knight, Frances Willenbrock and Gillian Murphy: A novel coumarin-labelled peptide for sensitive continuous assays of the matrix metalloproteinases: FEBS LETT., 296, (1992), 263-266. As a substrate, MOCAc-Pro-Leu-Gly-Leu-$A_2$Pr(DNP)-Ala-Arg-$NH_2$ (Peptide Institute, Inc. Osaka, Japan) was used, and the following four assays are performed per compound (inhibitor)

for measuring the inhibiting activity.

(A) Substrate (synthetic substrate), enzyme (MMPs), inhibitor
(B) Substrate (synthetic substrate), inhibitor
(C) Substrate (synthetic substrate), enzyme (MMPs)
(D) Substrate (synthetic substrate)

In each assay, the fluorescent intensity was measured, and inhibition (%) was calculated by the following equation.

$$\text{Inhibition } (\%) = [I-(A-B)/(C-D)] \times 100$$

$IC_{50}$ indicates a concentration at which inhibition (%) is 50%.
Inhibiting activity values for MMP-2, MMP-9 and MMP-13 measured by the aforementioned method are shown in Tables 1 to 8.
Compounds not described in the Tables, whose inhibiting activity for MMP-13 is less than 10nM, include I-18, I-19, I-28, I-32, I-56, I-67, I-68, I-71, I-104, I-106, I-107 and the like.
Moreover, compounds whose inhibitinng activity for MMP-13 is 10-100nM include I-26, I-27, I-29, I-55, I-58, I-59, I-60, I-62, I-63, I-69, I-72, I-80, I-81, I-82, I-83, I-92, I-93, I-95, I-96, I-99, I-101, I-105, I-108, I-109, I-110, I-111, I-161 and the like.
Inhibiting activity for the MMP-13 of these compounds is 100 times or more strong compared to the inhibition activity for MMP-2 or MMP-9.
[Table 1]

| No | IC50 (uM) | | |
|---|---|---|---|
| | MMP-2 | MMP-9 | MMP-13 |
| I-1 | 0.1 - 1 | > 10 | 0.0021 |
| I-2 | 0.1 - 1 | > 10 | 0.0038 |
| I-33 | 1 - 10 | > 10 | 0.011 |
| I-112 | > 10 | > 10 | 0.062 |
| I-113 | > 10 | > 10 | 0.071 |
| I-114 | 1-10 | > 10 | 0.011 |
| I-115 | 1-10 | >10 | 0.012 |

[Table 2]

| No | IC50 (uM) | | |
|---|---|---|---|
| | MMP-2 | MMP-9 | MMP-13 |
| III-1 | 1 - 10 | > 10 | 0.003 |
| III-2 | 1 - 10 | > 10 | 0.009 |
| III-4 | 1 - 10 | > 10 | 0.006 |
| III-5 | 1 - 10 | > 10 | 0.005 |
| III-6 | > 10 | > 10 | 0.028 |
| III-10 | > 10 | > 10 | 0.013 |
| III-12 | > 10 | > 10 | 0.004 |

(continued)

| No | IC50 (uM) | | |
|---|---|---|---|
| | MMP-2 | MMP-9 | MMP-13 |
| III-17 | > 10 | > 10 | 0.025 |
| III-24 | > 10 | > 10 | 0.026 |
| III-25 | > 10 | > 10 | 0.018 |
| III-27 | > 10 | > 10 | 0.019 |
| III-30 | 1 -10 | > 10 | 0.022 |
| III-37 | 0.1 - 1 | > 10 | 0.001 |
| III-38 | > 10 | > 10 | 0.029 |
| III-45 | > 10 | > 10 | 0.013 |
| III-46 | > 10 | > 10 | 0.018 |
| III-59 | > 10 | > 10 | 0.006 |
| III-60 | > 10 | > 10 | 0.006 |

[Table 3]

| | | | |
|---|---|---|---|
| III-68 | > 10 | > 10 | 0.005 |
| III-69 | > 10 | > 10 | 0.007 |
| III-72 | > 10 | > 10 | 0.021 |
| III-73 | > 10 | > 10 | 0.023 |
| III-80 | >10 | >10 | 0.013 |
| III-81 | 1 - 10 | > 10 | 0.006 |
| III-86 | 1 - 10 | > 10 | 0.009 |
| III-87 | > 10 | > 10 | 0.025 |
| III-94 | 1 - 10 | 1 - 10 | 0.009 |
| III-99 | 1 - 10 | > 10 | 0.025 |
| III-116 | > 10 | > 10 | 0.023 |
| III-118 | > 10 | > 10 | 0.028 |
| III-121 | > 10 | > 10 | 0.025 |
| III-135 | 1 - 10 | > 10 | 0.009 |
| III-137 | 1 - 10 | > 10 | 0.017 |
| III-138 | 1 - 10 | > 10 | 0.018 |
| III-144 | 1 - 10 | > 10 | 0.010 |
| III-150 | 1 - 10 | > 10 | 0.007 |
| III-152 | 1 - 10 | > 10 | 0.008 |

[Table 4]

| | | | |
|---|---|---|---|
| III-153 | 1 - 10 | > 10 | 0.004 |

(continued)

| | | | |
|---|---|---|---|
| III-154 | > 10 | > 10 | 0.021 |
| III-156 | 1 - 10 | > 10 | 0.010 |
| III-157 | > 10 | > 10 | 0.019 |
| III-161 | 1 - 10 | > 10 | 0.005 |
| III-167 | > 10 | > 10 | 0.014 |
| III-169 | 1 - 10 | > 10 | 0.003 |
| III-170 | 1 - 10 | > 10 | 0.004 |
| III-171 | 1 - 10 | > 10 | 0.007 |
| III-172 | > 10 | > 10 | 0.010 |
| III-177 | 1 - 10 | > 10 | 0.009 |
| III-179 | > 10 | > 10 | 0.012 |
| III-181 | 1 - 10 | > 10 | 0.006 |
| III-184 | 1 - 10 | > 10 | 0.014 |
| III-186 | 1 -10 | > 10 | 0.012 |
| III-187 | 1 - 10 | > 10 | 0.007 |
| III-189 | 1 - 10 | > 10 | 0.005 |
| III-192 | 1 - 10 | > 10 | 0.008 |
| III-193 | 1 - 10 | > 10 | 0.005 |

[Table 5]

| | | | |
|---|---|---|---|
| III-194 | > 10 | > 10 | 0.026 |
| III-195 | > 10 | > 10 | 0.007 |
| III-196 | > 10 | > 10 | 0.020 |
| III-199 | 1 - 10 | > 10 | 0.004 |
| III-200 | 1 - 10 | > 10 | 0.005 |
| III-201 | 0.1 - 1 | > 10 | 0.001 |
| III-205 | 1 - 10 | > 10 | 0.004 |
| III-206 | 0.1 - 1 | > 10 | 0.001 |
| III-207 | 1 - 10 | > 10 | 0.004 |
| III-210 | > 10 | > 10 | 0.017 |
| III-212 | 1 - 10 | > 10 | 0.004 |
| III-214 | 1 - 10 | > 10 | 0.006 |
| III-216 | > 10 | >10 | 0.017 |
| III-218 | > 10 | >10 | 0.019 |
| III-222 | > 10 | > 10 | 0.023 |
| III-223 | 1 - 10 | > 10 | 0.017 |
| III-224 | 1 - 10 | > 10 | 0.016 |
| III-226 | > 10 | > 10 | 0.023 |

(continued)

| | | | |
|---|---|---|---|
| III-230 | 1 - 10 | > 10 | 0.007 |

[Table 6]

| No | IC50 (uM) | | |
|---|---|---|---|
| | MMP-2 | MMP-9 | MMP-13 |
| V-1 | 1 - 10 | > 10 | 0.046 |
| V-2 | 1 | > 10 | 0.001 |
| V-3 | > 10 | > 10 | 0.002 |
| V-5 | 1-10 | > 10 | 0.018 |
| V-6 | > 10 | > 10 | 0.019 |
| V-7 | 1-10 | > 10 | 0.012 |
| V-8 | 1 - 10 | > 10 | 0.007 |
| V-11 | 1-10 | > 10 | 0.014 |
| V-12 | 1 - 10 | > 10 | 0.004 |
| V-14 | 1 - 10 | > 10 | 0.006 |
| V-15 | 1 - 10 | > 10 | 0.013 |
| V-19 | 1 - 10 | > 10 | 0.008 |
| V-20 | 1 - 10 | > 10 | 0.001 |
| V-22 | > 10 | > 10 | 0.008 |
| V-23 | 1 -10 | > 10 | 0.004 |
| V-24 | > 10 | > 10 | 0.023 |

[Table 7]

| | | | |
|---|---|---|---|
| V-25 | 1 - 10 | > 10 | 0.009 |
| V-26 | 1 - 10 | > 10 | 0.024 |
| V-28 | 1 - 10 | > 10 | 0.016 |
| V-30 | 1 - 10 | > 10 | 0.001 |
| V-31 | 1 - 10 | > 10 | 0.003 |
| V-32 | 1 - 10 | > 10 | 0.016 |
| V-33 | 1 - 10 | > 10 | 0.003 |
| V-34 | 1 - 10 | > 10 | 0.005 |
| V-36 | 1 - 10 | 1 - 10 | 0.005 |
| V-37 | > 10 | > 10 | 0.020 |
| V-40 | 1 - 10 | 1 - 10 | 0.008 |
| V-41 | 1 - 10 | > 10 | 0.002 |
| V-43 | > 10 | > 10 | 0.019 |
| V-44 | 1 - 10 | > 10 | 0.016 |

(continued)

| | | | |
|---|---|---|---|
| V-47 | 1 - 10 | > 10 | 0.004 |
| V-51 | 1 - 10 | > 10 | 0.008 |
| V-52 | 1 - 10 | 1 - 10 | 0.012 |

[Table 8]

| | | | |
|---|---|---|---|
| V-56 | 1 - 10 | > 10 | 0.016 |
| V-57 | 1 - 10 | > 10 | 0.013 |
| V-59 | 1 - 10 | > 10 | 0.010 |
| V-60 | > 10 | > 10 | 0.021 |
| V-63 | 1 - 10 | > 10 | 0.008 |
| V-65 | > 10 | > 10 | 0.017 |
| V-68 | 1 - 10 | 1 - 10 | 0.005 |
| V-71 | > 10 | > 10 | 0.008 |
| V-74 | 1 - 10 | > 10 | 0.016 |
| V-76 | > 10 | > 10 | 0.018 |
| V-77 | > 10 | > 10 | 0.004 |
| V-78 | > 10 | > 10 | 0.004 |
| V-79 | > 10 | > 10 | 0.004 |
| V-85 | 1 - 10 | > 10 | 0.002 |
| V-86 | 1 - 10 | > 10 | 0.012 |
| V-88 | 1 - 10 | > 10 | 0.024 |

Preparation Examples

Preparation Example 1

A granule containing the following ingredients is produced.

| Ingredient | Active ingredient 10 mg |
|---|---|
| Lactose | 700 mg |
| Corn starch | 274 mg |
| HPC-L | 16 mg |
| | 1000 mg |

The active ingredient and lactose are passed through a 60 mesh sieve. Corn starch is passed through a 120 mesh sieve. These are mixed by a V-type mixing machine. To the mixed powder is added a HPC-L (low viscosity hydroxypropylcellulose) aqueous solution, the materials are kneaded, granulated (extrusion granulation, pore diameter 0.5-1 mm), and dried. The resulting dried granule is passed through a vibration sieve (12/60 mesh) to give a granule.

Preparation Examples 2

A filling powder into capsules, having the following ingredients, is produced.

| Ingredient | Active ingredient 10 mg |
|---|---|
| Lactose | 79 mg |
| Corn starch | 10 mg |
| Magnesium stearate | 1 mg |
| | 100 mg |

The active ingredient and lactose are passed through a 60 mesh sieve. Corn starch is passed through a 120 mesh sieve. These are mixed with magnesium stearate are by a V-type mixing machine. The 10% powder, 100 mg, is filled into a No.5 hard-gelatin capsule.

Preparation Examples 3

A tablet having the following ingredients is produced.

| Ingredient | Active ingredient 10 mg |
|---|---|
| Lactose | 90 mg |
| Microcrystalline cellulose | 30 mg |
| CMC-Na | 15 mg |
| Magnesium stearate | 5 mg |
| | 150 mg |

The active ingredient, lactose, microcrystalline cellulose, and CMC-Na (carboxymethylcellulose sodium salt) are passed through a 60 mesh sieve, and mixed. Magnesium stearate is mixed with the above powder to give the mixed powder for preparing a tablet. The obtained mixed powder is compressed to give a 150-mg tablet.

Preparation Example 4

An aerosol solution containing the following ingredients is produced.

| | Weight |
|---|---|
| Active ingredient | 0.25 |
| Ethanol | 25.75 |
| Propellant 22 (chlorodifluoromethane) | 74.00 |
| Total | 100.00 |

The active ingredient and ethanol are mixed and added to a portionof the propellant 22. After being cooled to -30°C, the mixture is transferred to a filling device. Then, a required amount is supplied to a stainless steel container, and is diluted with a rest of propellant. A valve unit is attached to the container.

Preparation Example 5

An intravenous preparation for vein is produced as follows:

| | |
|---|---|
| Active ingredient | 100 mg |
| Physiological saline | 1000 ml |

A solution of the aforementioned ingredients is intravenously administered to a patient usually at a rate of 1 ml per minutes.

Preparation Example 6

A transdermal preparation containing the following ingredients is produced.

| | |
|---|---|
| Active ingredient | 10 mg |
| Isopropyl myristate | 990 mg |
| | 1000 mg |

After the compound represented by the formula (I) is dispersed into isopropyl myristate, the dispersion is mixed with an acryl-based adhesive (e.g. Nicazol), and then a transdermal preparation was obtained by applying to an application support.

Preparation Example 7

An ointment containing the following ingredients is produced.

| | |
|---|---|
| Active ingredient | 10 mg |
| Liquid paraffin | 75 mg |
| White vaseline | 925 mg |
| | 1000 mg |

The compound represented by the formula (I) and liquid paraffin are dispersed, and kneaded with white vaseline to give an ointment.

[Industrial applicability]

The sulfonylurea derivatives having the MMP-13 selective inhibiting activity of the present invention are useful as a medicine for treating or preventing a disease associated with MMP-13, particularly osteoarthritis.

## Claims

**1.** A compound represented by the general formula (I):

[Chemical formula 1]

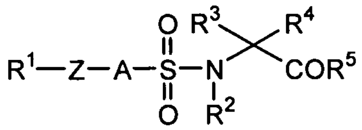

(I)

wherein $R^1$ is

a) a group represented by the formula:

[Chemical formula 2]

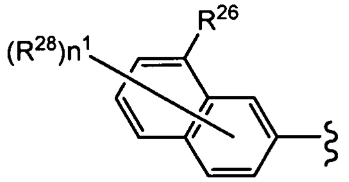

,

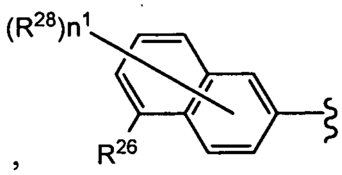

,

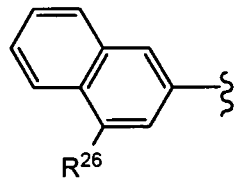

or

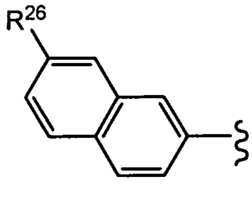

wherein $R^{26}$ and $R^{28}$ are each independently halogen, optionally substituted lower alkyl, optionally substituted lower alkenyl, optionally substituted lower alkyloxy, lower alkylthio, aralkylthio, optionally substituted amino, carboxy, hydroxyl, cyano, lower alkyloxycarbonyl, optionally substituted aminocarbonyl, optionally substituted aminoxalyl, acyl, lower alkylsulfonyl, lower alkylsulfinyl, aralkylsulfonyl, aralkylsulfinyl, optionally substituted aminosulfonyl, optionally substituted heteroaryl or an optionally substituted non-aromatic heterocyclic group ;
$n^1$ is an integer of 0 to 3, and $R^{28}$ may be substituted on all substitutable atoms on the ring;
b) a group represented by the formula:

[Chemical formula 3]

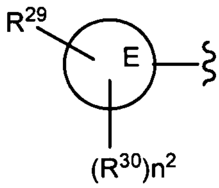

wherein E ring is benzene, pyridine, 2-pyridone, thiazole,1H-pyrimidine- 2 -one, tetrahydrothienopyrimidine or oxazole;
$R^{29}$ is a halogen, acyl, optionally substituted lower alkyl, optionally substituted lower alkyloxy, optionally substituted lower alkenyl, optionally substituted aryl, optionally substituted heteroaryl, an optionally substituted non-aromatic carbocyclic group, an optionally substituted non-aromatic heterocyclic group, optionally substituted aminocarbonyl, optionally substituted amino or optionally substituted hydrazino;
$R^{30}$ is each independently halogen, hydroxy, nitro, optionally substituted lower alkyl, optionally substituted lower alkenyl, optionally substituted lower alkyloxy, optionally substituted aryloxy, optionally substituted aralkyl, optionally substituted heteroarylalkyl, lower alkylthio, aralkylthio, optionally substituted amino, carboxy, cyano, lower alkyloxycarbonyl, optionally substituted aminocarbonyl, optionally substituted aminoxalyl, acyl, lower alkylsulfonyl, lower alkylsulfinyl, aralkylsulfonyl, aralkylsulfinyl, optionally substituted aminosulfonyl, optionally substituted aryl, optionally substituted heteroaryl or an optionally substituted non-aromatic heterocyclic group;
$n^2$ is an integer of 0 to 2, and $R^{29}$ and $R^{30}$ may be substituted on all substitutable atoms on the ring or
c) a group represented by the formula:

[Chemical formula 4]

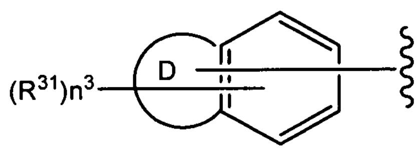

wherein D ring is a non-aromatic carbocyclic ring, a non-aromatic heterocyclic ring or an aromatic heterocyclic ring;
$R^{31}$ is each independently halogen, optionally substituted lower alkyl, optionally substituted lower alkenyl, optionally substituted lower alkyloxy, alkylthio, aralkylthio, optionally substituted amino, carboxy, cyano, lower alkyloxycarbonyl, lower alkyloxy lower alkylcarbonyl, optionally substituted aminocarbonyl, optionally substituted aminoxalyl, acyl, lower alkylsulfonyl, lower alkylsulfinyl, aralkylsulfonyl, aralkylsulfinyl, optionally substituted aminosulfonyl, oxo, thioxo, imino, hydroxyl, hydroxyimino, optionally substituted lower alkyloxyimino, optionally substituted heteroaryl, optionally substituted aralkyl, optionally substituted heteroarylalkyl, optionally substituted aralkyloxy, optionally substituted aryl or an optionally substituted non-aromatic heterocyclic group;
all substitutable atoms of the benzene ring and all substitutable atoms of D ring may be bonded to "Z", $n^3$ is an integer of 0 to 4;
$R^{31}$ may be substituted on all substitutable atoms of the benzene ring and all substitutable atoms of D ring;
provided that when $n^3$ is an integer of 0, D ring is not unsubstitu ted indole;
Z is -O-, -S-, -SO-, $-SO_2-$, $-N(R^{10})-$, $-N(R^{10})-C(=O)-$, $-C(=O)-N(R^{10})-$, - $N(R^{10})-SO_2-$, $-SO_2-N(R^{10})-$, -C(=O)-, -O-C(=O)-, -C(=O)-O-, $-N(R^{10})-C(=O)-N(R^{10})-$, $-N(R^{10})-C(=S)-N(R^{10})-$, $-O-N=C(R^8)-$, $-C(R^8)=N-O-$, $-O-C(=O)-N(R^{10})-$, $-N(R^{10})-C(=O)-O-$, -C(=O)C(=O)-, an optionally substituted non-aromatic carbocyclic group, an optionally substituted non-aromatic heterocyclic group, optionally substituted C1-C5 alkylene which may be intervened with a substituent selected from Substituent group a, or optionally substituted C2-C5 alkenylene which may be intervened with a substituent selected from Substituent group a, or optionally substituted C2-C5 alkynylene which may be intervened with a substituent selected from Substituent group a, wherein the Substituent group a consists of -O-, -S-, -SO-, $-SO_2-$, $-N(R^{10})-$, $-N(R^{10})-C(=O)-$, $-C(=O)-N(R^{10})-$, $-N(R^{10})-SO_2-$, $-SO_2-N(R^{10})-$, -C(=O)-, -O-C(=O)-, - C(=O)-O-, $-N(R^{10})-C(=O)-N(R^{10})-$, $-N(R^{10})-C(=S)-N(R^{10})-$, $-O-N=C(R^8)-$, - $C(R^8)=N-O-$, $-O-C(=O)-N(R^{10})-$, $-N(R^{10})-C(=O)-O-$, and -C(=O)-C(=O)-, $R^8$ and $R^{10}$ are each independently a hydrogen atom or lower alkyl;
A is a group represented by the formula;

[Chemical formula 5]

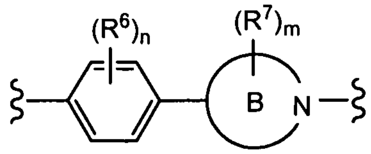

or

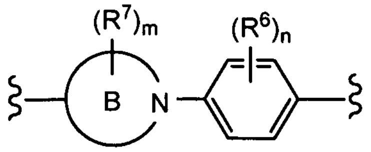

wherein $R^6$ and $R^7$ are each independently halogen, lower alkyl, cycloalkyl, lower alkenyl, lower alkynyl, lower alkyloxy, lower alkenyloxy, lower alkylthio, halo lower alkyl, halo lower alkyloxy, halo lower alkylthio, hydroxy, hydroxy lower alkyl, mercapto, carboxy, lower alkyloxycarbonyl, lower alkylsulfonyl, acyl, acyloxy, nitro, cyano, optionally substituted amino, or optionally substituted aminocarbonyl; B ring is a nitrogen-containing heterocyclic ring which is optionally contained a further nitrogen atom, an oxygen atom, and/or a sulfur atom in the ring;
m and n are each independently an integer of 0 to 3;
$R^2$ is a hydrogen atom, optionally substituted lower alkyl, optionally substituted aralkyl, optionally substituted heteroarylalkyl, optionally substituted aryl, or optionally substituted heteroaryl;
$R^3$ is a hydrogen atom, optionally substituted lower alkyl, optionally substituted aralkyl, optionally substituted heteroarylalkyl, optionally substituted aryl, or optionally substituted heteroaryl;
$R^4$ is a hydrogen atom, or
$R^3$ and $R^4$ may be taken together with an adjacent carbon atom to form a 5- to 6-membered non-aromatic carbocyclic ring or a 5- to 6-membered non-aromatic heterocyclic ring;
$R^5$ is hydroxy, lower alkyloxy, or -NHOH;
an optically active isomer , a pharmaceutically acceptable salt or a solvate thereof.

2. The compound according to claim 1, wherein $R^1$ is a group represented by the formula:

[Chemical formula 6]

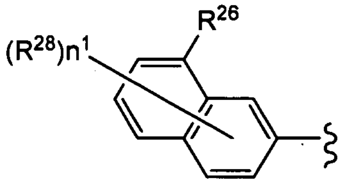
,
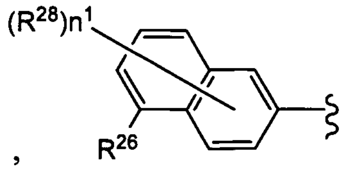
,
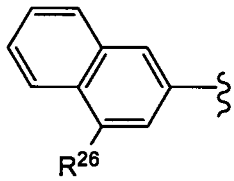

or

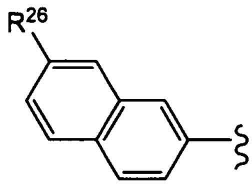

wherein $R^{26}$, $R^{28}$ and $n^1$ are as defined in claim 1, an optically active isomer, a pharmaceutically acceptable salt, or a solvate thereof.

**3.** The compound according to claim 1, wherein $R^1$ is a group represented by the formula:

[Chemical formula 8]

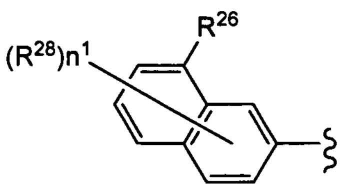

or

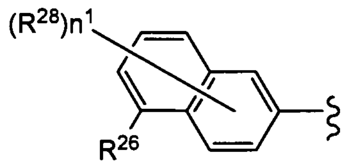

wherein $R^{26}$, $R^{28}$ and $n^1$ are as defined in claim 1, an optically active isomer, a pharmaceutically acceptable salt, or a solvate thereof.

**4.** The compound according to claim 1, wherein $R^1$ is a group represented by the formula:

[Chemical formula 8]

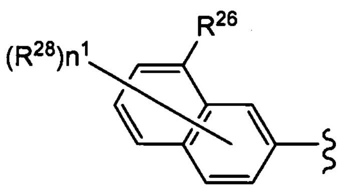

wherein $R^{26}$, $R^{28}$ and $n^1$ are as defined in claim 1, an optically active isomer, a pharmaceutically acceptable salt, or a solvate thereof.

**5.** The compound according to claim 1, wherein $R^1$ is a group represented by the formula:

[Chemical formula 9]

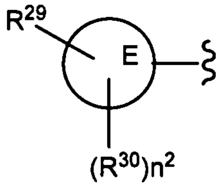

wherein E ring, $R^{29}$, $R^{30}$ and $n^2$ are as defined in claim 1, an optically active isomer, a pharmaceutically acceptable salt, or a solvate thereof.

**6.** The compound according to claim 1, wherein $R^1$ is a group represented by the formula:

[Chemical formula 10]

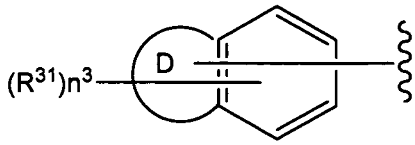

wherein D ring, $R^{31}$ and $n^3$ are as defined in claim 1, an optically active isomer, a pharmaceutically acceptable salt, or a solvate thereof.

**7.** The compound according to claim 1, wherein A is a group represented by the formula:

[Chemical formula 11]

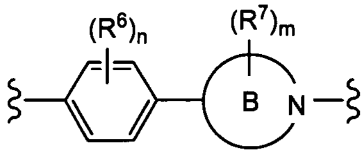

wherein $R^6$, $R^7$, B ring, m and n are as defined in claim 1, an optically active isomer, a pharmaceutically acceptable salt, or a solvate thereof.

**8.** The compound according to claim 1, wherein A is a group represented by the formula:

[Chemical formula 12]

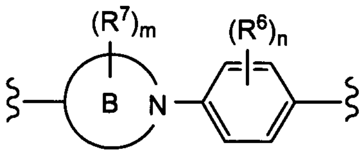

wherein $R^6$, $R^7$, B ring, m and n are as defined in claim 1, an optically active isomer, a pharmaceutically acceptable salt, or a solvate thereof.

**9.** The compound according to claim 1, wherein Z is $-C(R^8)(R^9)-$, -O-, -S-, - SO-, $-SO_2-$, $N(R^{10})-$, $-C(R^8)(R^9)-C(R^8)(R^9)-$, $-C(R^8)=C(R^9)-$, -C≡C-, $-O-C(R^8)(R^9)-$, $-C(R^8)(R^9)-O-$, $-S-C(R^8)(R^9)-$, $-C(R^8)(R^9)-S-$, $-SO-C(R^8)(R^9)-$, $-C(R^8)(R^9)-SO-$, $-SO_2-C(R^8)(R^9)-$, $-C(R^8)(R^9)-SO_2-$, $- N(R^{10})-C(R^8)(R^9)-$, $-C(R^8)(R^9)-N(R^{10})-$, $-N(R^{10})-C(=O)-$, -C(=O)-N

$(R^{10})$-, -$N(R^{10})$-$SO_2$-, -$SO_2$-$N(R^{10})$-, -$C(R^8)(R^9)$-C(=O)-, -C(=O)-$C(R^8)(R^9)$-, -C(=O)-C(=O)-, -$C(R^8)(R^9)$-$C(R^8)(R^9)$-$C(R^8)(R^9)$-, - $C(R^8)(R^9)$-$C(R^8)$=$C(R^9)$-, -$C(R^8)$=$C(R^9)$-$C(R^8)(R^9)$-, -$C(R^8)(R^9)$-C≡C-, - C≡C-$C(R^8)(R^9)$-, -O-$C(R^8)(R^9)$-$C(R^8)(R^9)$-, -$C(R^8)(R^9)$-O-$C(R^8)(R^9)$-, - $C(R^8)(R^9)$-$C(R^8)(R^9)$-O-, -S-$C(R^8)(R^9)$-$C(R^8)(R^9)$-, -$C(R^8)(R^9)$-S-$C(R^8)(R^9)$-, -$C(R^8)(R^9)$-$C(R^8)(R^9)$-S-, -SO-$C(R^8)(R^9)$-$C(R^8)(R^9)$-, - $C(R^8)(R^9)$-SO-$C(R^8)(R^9)$-, -$C(R^8)(R^9)$-$C(R^8)(R^9)$-SO-, -$SO_2$-$C(R^8)(R^9)$-$C(R^8)(R^9)$-, -$C(R^8)(R^9)$-$SO_2$-$C(R^8)(R^9)$-, -$C(R^8)(R^9)$-$C(R^8)(R^9)$-$SO_2$-, - $N(R^{10})$-$C(R^8)(R^9)$-$C(R^8)(R^9)$-, -$C(R^8)(R^9)$-$N(R^{10})$-$C(R^8)(R^9)$-, - $C(R^8)(R^9)$-$C(R^8)(R^9)$-$N(R^{10})$-, -C(=O)-$C(R^8)(R^9)$-$C(R^8)(R^9)$-, - $C(R^8)(R^9)$-C(=O)-$C(R^8)(R^9)$-, -$C(R^8)(R^9)$-$C(R^8)(R^9)$-C(=O)-, -$C(R^8)(R^9)$-$N(R^{10})$-C(=O)-, -$N(R^{10})$-C(=O)-$C(R^8)(R^9)$-, -$C(R^8)(R^9)$-C(=O)-$N(R^{10})$-, - C(=O)-$N(R^{10})$-$C(R^8)(R^9)$-, -$C(R^8)(R^9)$-$N(R^{10})$-$SO_2$-, -$N(R^{10})$-$SO_2$-$C(R^8)(R^9)$-, -$C(R^8)(R^9)$-$SO_2$-$N(R^{10})$-, -$SO_2$-$N(R^{10})$-$C(R^8)(R^9)$-, -$N(R^{10})$-C(=O)-$N(R^{10})$-, -$N(R^{10})$-C(=S)-$N(R^{10})$-, -O-N=$C(R^8)$-, -$C(R^8)$=N-O-, -O-C(=O)-$C(R^8)(R^9)$-, -$C(R^8)(R^9)$-C(=O)-O-, -O-C(=O)-$N(R^{10})$-, or -$N(R^{10})$-C(=O)-O-, wherein $R^8$, $R^9$ and $R^{10}$ are each independently a hydrogen atom or lower alkyl, an optically active isomer, a pharmaceutically acceptable salt, or a solvate thereof.

**10.** The compound according to claim 1, wherein Z is -C≡C-, or -CH=CH-, an optically active isomer, a pharmaceutically acceptable salt, or a solvate thereof.

**11.** The compound according to claim 1, wherein a group represented by the formula:

[Chemical formula 13]

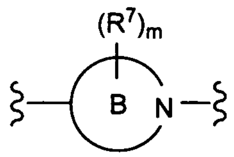

is a group represented by the formula:

[Chemical formula 14]

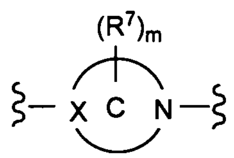

wherein X is a carbon atom or a nitrogen atom, C ring is a 5- to 8-membered nitrogen-containing heterocyclic ring, and $R^7$ and m are as defined in claim 1, an optically active isomer, a pharmaceutically acceptable salt, or a solvate thereof.

**12.** The compound according to claim 1, wherein $R^2$ is a hydrogen atom, an optically active isomer, a pharmaceutically acceptable salt, or a solvate thereof.

**13.** The compound according to claim 1, wherein $R^3$ is a hydrogen atom, methyl, ethyl, n-propyl, isopropyl, n-butyl, s-butyl, isobutyl, tert-butyl, hydroxymethyl, 1-hydroxyethyl, 2-hydroxyethyl, carboxymethyl, carboxyethyl, methyloxymethyl, methylthiomethyl, 2-methylthioethyl, phenyl, 4-hydroxyphenyl, 4-fluorophenyl, 4-methyloxyphenyl, benzyl, 4-hydroxybenzyl, 4-fluorobenzyl, 4-chlorobenzyl, 4-methoxybenzyl, indol-3-ylmethyl, 1-carboxymethylindol-3-ylmethyl, thiazol-4-ylmethyl, carboxymethyloxybenzyl, or cyclopropylmethyl;
$R^4$ is a hydrogen atom; or
$R^3$ and $R^4$ may be taken together with an adjacent carbon to form a ring represented by the formula:

[Chemical formula 8]

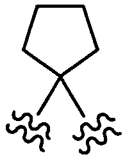 、 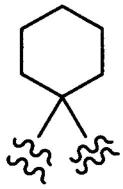 or 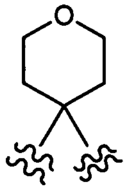

an optically active isomer, a pharmaceutically acceptable salt, or a solvate thereof.

**14.** The compound according to claim 1, wherein $R^5$ is hydroxy, an optically active isomer, a pharmaceutically acceptable salt, or a solvate thereof.

**15.** A pharmaceutical composition containing the compound as defined in any one of claims 1 to 13 as an active ingredient.

**16.** A pharmaceutical composition for inhibiting MMP-13 which contains the compound as defined in any one of claims 1 to 13 as an active ingredient.

**17.** A method of treating a disease resulting from, or associated with MMP-13 of a mammal, comprising administering to a mammal including a human therapeutically effective amount of the compound as defined in any one of claims 1 to 13.

**18.** Use of the compound as defined in any one of claims 1 to 13, for preparing a medicament for treating a disease resulting from or associated with MMP-13.

**INTERNATIONAL SEARCH REPORT**

International application No.
PCT/JP2007/071192

A. CLASSIFICATION OF SUBJECT MATTER
*C07D209/08*(2006.01)i, *A61K31/4418*(2006.01)i, *A61K31/451*(2006.01)i, *A61K31/495*(2006.01)i, *A61K31/496*(2006.01)i, *A61K31/5377*(2006.01)i, *A61K31/551*(2006.01)i, *A61P1/02*(2006.01)i, *A61P19/02*(2006.01)i,
According to International Patent Classification (IPC) or to both national classification and IPC

B. FIELDS SEARCHED
Minimum documentation searched (classification system followed by classification symbols)
C07D209/08, A61K31/4418, A61K31/451, A61K31/495, A61K31/496, A61K31/5377, A61K31/551, A61P1/02, A61P19/02, A61P27/02, A61P29/00, A61P31/12, A61P31/18, A61P35/00, A61P35/04, A61P43/00, C07D209/86, C07D211/96, C07D213/81,

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAPLUS(STN), REGISTRY(STN), MEDLINE(STN), BIOSIS(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| P,X | WO 2007/102392 A1 (Shionogi & Co., Ltd.), 13 September, 2007 (13.09.07), Full text (Family: none) | 1-16,18 |
| X | WO 02/06274 A1 (AMERICAN HOME PRODUCTS CORP.), 24 January, 2002 (24.01.02), Full text & US 2002/028797 A1 & US 2003/027795 A1 | 1,5,8,11-15 |
| X | JP 2001-523222 A (F. Hoffmann-La Roche AG.), 20 November, 2001 (20.11.01), Pages 23 to 24; page 158, & WO 98/32748 A1 & EP 958287 A1 & US 6376506 B1 | 1,5,7,9, 11-13,15,16, 18 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 November, 2007 (19.11.07) | 27 November, 2007 (27.11.07) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2007/071192

C (Continuation). DOCUMENTS CONSIDERED TO BE RELEVANT

| Category* | Citation of document, with indication, where appropriate, of the relevant passages | Relevant to claim No. |
|---|---|---|
| A | US 6153612 A (Warner-Lambert Co.), 28 November, 2000 (28.11.00), Full text & US 6297247 B1 | 1-16,18 |
| A | WO 03/080042 A1 (Shionogi & Co., Ltd.), 02 October, 2003 (02.10.03), Full text & EP 1491190 A1 & AU 2003221160 A1 & US 2005/227994 A1 | 1-16,18 |
| A | WO 2005/061459 A1 (WYETH), 07 July, 2005 (07.07.05), Full text & JP 2007-516245 A & EP 1689716 A1 & US 2005/130973 A1 | 1-16,18 |
| A | TAMURA, Y. et al., Highly Selective and Orally Active Inhibitors of Type IV Collagenase (MMP-9 and MMP-2): N-Sulfonylamino Acid Derivatives, Journal of Medicinal Chemistry, 1998, Vol.41, No.4, pp.640-649 | 1-16,18 |

Form PCT/ISA/210 (continuation of second sheet) (April 2007)

**INTERNATIONAL SEARCH REPORT**

International application No.
PCT/JP2007/071192

Continuation of A. CLASSIFICATION OF SUBJECT MATTER
(International Patent Classification (IPC))

*A61P27/02*(2006.01)i, *A61P29/00*(2006.01)i, *A61P31/12*(2006.01)i, *A61P31/18*(2006.01)i, *A61P35/00*(2006.01)i, *A61P35/04*(2006.01)i, *A61P43/00*(2006.01)i, *C07D209/86*(2006.01)i, *C07D211/96*(2006.01)i, *C07D213/81*(2006.01)i, *C07D215/20*(2006.01)i, *C07D241/04*(2006.01)i, *C07D243/08*(2006.01)i, *C07D263/32*(2006.01)i, *C07D277/28*(2006.01)i, *C07D295/22*(2006.01)i, *C07D307/81*(2006.01)i, *C07D307/91*(2006.01)i

(According to International Patent Classification (IPC) or to both national classification and IPC)

Continuation of B. FIELDS SEARCHED
Minimum documentation searched (International Patent Classification (IPC))

C07D215/20, C07D241/04, C07D243/08, C07D263/32, C07D277/28, C07D295/22, C07D307/81, C07D307/91

Minimum documentation searched (classification system followed by classification symbols)

Form PCT/ISA/210 (extra sheet) (April 2007)

**INTERNATIONAL SEARCH REPORT**

International application No.
PCT/JP2007/071192

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 17
because they relate to subject matter not required to be searched by this Authority, namely:
Claim 17 pertains to methods for treatment of the human body by therapy and thus relates to a subject matter which this International Searching Authority is not required, under the provisions of Article 17(2)(a)(i) of the PCT and Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9727174 A
- WO 9904780 A
- WO 0015213 A
- WO 0183431 A
- WO 0228844 A
- WO 0183461 A
- WO 03080042 A
- JP 2000319250 A
- WO 05061459 A
- WO 9832748 A
- WO 2007102392 A
- WO 0046189 A


### Non-patent literature cited in the description


- **Yoshinori Tamura et al.** *J. Med. Chem.,* 1998, vol. 41 (4), 640-649
- *Tetrahedron Lett.,* 1998, vol. 39, 617-620
- Experimental Chemistry Course. Chemical Society of Japan, vol. 22
- **Gary M. Ksander.** *J. Med. Chem.,* 1995, vol. 38, 1689-1700
- New Experimental Chemistry Course. Chemical Society of Japan, vol. 14
- **Theodora W Green.** Protective Groups in Organic Synthesis. John Wiley & Sons
- **Meacock et al.** *J. Exp. Path.,* 1990, vol. 71, 279-293
- **Fletcher et al.** *J. Pharmacol. Exp. Ther.,* vol. 284 (2), 714-721
- **C. Graham Knight.** Frances Willenbrock and Gillian Murphy: A novel coumarin-labelled peptide for sensitive continuous assays of the matrix metalloproteinases. *FEBS LETT.,* 1992, vol. 296, 263-266